(19) Europäisches Patentamt
European Patent Office
Office européen des brevets

(11) EP 1 162 268 A2

(12) EUROPEAN PATENT APPLICATION

(43) Date of publication:
**12.12.2001 Bulletin 2001/50**

(21) Application number: **01116253.4**

(22) Date of filing: **10.09.1999**

(51) Int Cl.[7]: **C12N 15/53**, C12N 9/02, C12N 15/63, C12N 5/10, C12P 7/44, C12R 1/74

(84) Designated Contracting States:
**AT BE CH CY DE DK ES FI FR GB GR IE IT LI LU MC NL PT SE**

(30) Priority: **05.10.1998 US 103099**
**10.03.1999 US 123555**

(62) Document number(s) of the earlier application(s) in accordance with Art. 76 EPC:
**99946862.2 / 1 135 506**

(71) Applicant: **Cognis Corporation**
**Gulph Mills, PA 19406 (US)**

(72) Inventors:
- **Wilson, Ron C.**
  **Loveland, OH 45140 (US)**
- **Craft, David L.**
  **Fort Thomas, KY 41075 (US)**
- **Eirich, Dudley L.**
  **Cincinnati, OH 45150 (US)**
- **Eshoo, Mark**
  **Berkley, CA 94705 (US)**
- **Madduri, Krishna M.**
  **Indianapolis, IN 46268 (US)**
- **Cornett, Cathy A.**
  **Crescent Springs, KY 41017 (US)**
- **Brenner, Alfred A.**
  **Santa Rosa, CA 95403 (US)**
- **Tang, Maria**
  **Fairfield, CA 94533 (US)**
- **Loper, John C.**
  **Cincinnati, OH 45213 (US)**
- **Gleeson, Martin**
  **San Diego, CA 92130 (US)**

(74) Representative:
**Weber, Thomas, Dr. Dipl.-Chem. et al**
**Patentanwälte**
**von Kreisler-Selting-Werner,**
**Bahnhofsvorplatz 1 (Deichmannhaus)**
**50667 Köln (DE)**

Remarks:
This application was filed on 05 - 07 - 2001 as a divisional application to the application mentioned under INID code 62.

# (54) Cytochrome P450 monooxygenase and NADPH cytochrome P450 oxidoreductase genes and proteins related to the omega hydroxylase complex of Candida tropicalis and methods relating thereto

(57) Novel genes have been isolated which encode cytochrome P450 and NADPH reductase enzymes of the ω-hydroxylase complex of *C. tropicalis* 20336. Vectors including these genes, transfected host cells and transformed host cells are provided. Methods of producing of cytochrome P450 and NADPH reductase enzymes are also provided which involve transforming a host cell with a gene encoding these enzymes and culturing the cells. Methods of increasing the production of a dicarboxylic acid and methods of increasing production of the aforementioned enzymes are also provided which involve increasing in the host cell the number of genes encoding these enzymes. A method for discriminating members of a gene family by quantifying the expression of genes is also provided.

EP 1 162 268 A2

**Description**

## CROSS REFERENCE TO RELATED APPLICATIONS

This application claims priority to U.S. Provisional Application Serial No. 60/103,099 filed October 5, 1998, and U.S. Provisional Application Serial No. 60/083,798 filed May 1, 1998.

## BACKGROUND

### 1. Field of the Invention

The present invention relates to novel genes which encode enzymes of the ω-hydroxylase complex in yeast *Candida tropicalis* strains. In particular, the invention relates to novel genes encoding the cytochrome P450 and NADPH reductase enzymes of the ω-hydroxylase complex in yeast *Candida tropicalis,* and to a method of quantitating the expression of genes.

### 2. Description of the Related Art

Aliphatic dioic acids are versatile chemical intermediates useful as raw materials for the preparation of perfumes, polymers, adhesives and macrolid antibiotics. While several chemical routes to the synthesis of long-chain alpha, ω-dicarboxylic acids are available, the synthesis is not easy and most methods result in mixtures containing shorter chain lengths. As a result, extensive purification steps are necessary. While it is known that long-chain dioic acids can also be produced by microbial transformation of alkanes, fatty acids or esters thereof, chemical synthesis has remained the most commercially viable route, due to limitations with the current biological approaches.

Several strains of yeast are known to excrete alpha, ω-dicarboxylic acids as a byproduct when cultured on alkanes or fatty acids as the carbon source. In particular, yeast belonging to the Genus *Candida,* such as *C. albicans, C. cloacae, C. guillermondii, C. intermedia, C. lipolytica, C. maltosa, C. parapsilosis* and *C. zeylenoides* are known to produce such dicarboxylic acids (*Agr. Biol. Chem.* 35: 2033-2042 (1971)). Also, various strains of C. *tropicalis* are known to produce dicarboxylic acids ranging in chain lengths from $C_{11}$ through $C_{18}$ (Okino et al., BM Lawrence, BD Mookherjee and BJ Willis (eds), in *Flavors and Fragrances: A World Perspective.* Proceedings of the 10th International Conference of Essential Oils, Flavors and Fragrances, Elsevier Science Publishers BV Amsterdam (1988)), and are the basis of several patents as reviewed by Bühler and Schindler, in *Aliphatic Hydrocarbons in Biotechnology,* H. J. Rehm and G. Reed (eds), Vol. 169, Verlag Chemie, Weinheim (1984).

Studies of the biochemical processes by which yeasts metabolize alkanes and fatty acids have revealed three types of oxidation reactions: α-oxidation of alkanes to alcohols, ω-oxidation of fatty acids to alpha, ω-dicarboxylic acids and the degradative β-oxidation of fatty acids to $CO_2$ and water. The first two types of oxidations are catalyzed by microsomal enzymes while the last type takes place in the peroxisomes. In *C. tropicalis,* the first step in the ωoxidation pathway is catalyzed by a membrane-bound enzyme complex (ω-hydroxylase complex) including a cytochrome P450 monooxygenase and a NADPH cytochrome reductase. This hydroxylase complex is responsible for the primary oxidation of the terminal methyl group in alkanes and fatty acids (Gilewicz et al., *Can. J. Microbiol.* 25:201 (1979)). The genes which encode the cytochrome P450 and NADPH reductase components of the complex have previously been identified as P450ALK and P450RED respectively, and have also been cloned and sequenced (Sanglard et al., *Gene* 76:121-136 (1989)). P450ALK has also been designated P450ALK1. More recently, ALK genes have been designated by the symbol *CYP* and RED genes have been designated by the symbol *CPR.* See, e.g., Nelson, *Pharmacogenetics* 6(1):1-42 (1996), which is incorporated herein by reference. See also Ohkuma et al., *DNA and Cell Biology* 14:163-173 (1995), Seghezzi et al., *DNA and Cell Biology,* 11:767-780 (1992) and Kargel et al., *Yeast* 12:333-348 (1996), each incorporated herein by reference. For example, P450ALK is also designated *CYP52* according to the nomenclature of Nelson, *supra.* Fatty acids are ultimately formed from alkanes after two additional oxidation steps, catalyzed by alcohol oxidase (Kemp et al., *Appl. Microbiol. and Biotechnol.* 28: 370-374 (1988)) and aldehyde dchydrogenase. The fatty acids can be further oxidized through the same or similar pathway to the corresponding dicarboxylic acid. The ω-oxidation of fatty acids proceeds via the ω-hydroxy fatty acid and its aldehyde derivative, to the corresponding dicarboxylic acid without the requirement for CoA activation. However, both fatty acids and dicarboxylic acids can be degraded, after activation to the corresponding acyl-CoA ester through the β-oxidation pathway in the peroxisomes, leading to chain shortening. In mammalian systems, both fatty acid and dicarboxylic acid products of ω-oxidation are activated to their CoA-esters at equal rates and are substrates for both mitochondrial and peroxisomal β-oxidation (*J. Biochem.,* 102:225-234 (1987)). In yeast, β-oxidation takes place solely in the peroxisomes (*Agr.Biol.Chem.* 49: 1821-1828 (1985)).

The production of dicarboxylic acids by fermentation of unsaturated $C_{14}$-$C_{16}$ monocarboxylic acids using a strain of the species *C. tropicalis* is disclosed in U.S. Patent 4,474,882. The unsaturated dicarboxylic acids correspond to the starting materials in the number and position of the double bonds. Similar processes in which other special microorganisms are used are described in U.S. Patents 3,975,234 and 4,339,536, in British Patent Specification 1,405,026 and in German Patent Publications 21 64 626, 28 53 847, 29 37 292, 29 51 177, and 21 40 133.

Cytochromes P450 (P450s) are terminal monooxidases of a multicomponent enzyme system as described above. They comprise a superfamily of proteins which exist widely in nature having been isolated from a variety of organisms as described e.g., in Nelson, *supra.* These organisms include various mammals, fish, invertebrates, plants, mollusk, crustaceans, lower eukaryotes and bacteria (Nelson, *supra).* First discovered in rodent liver microsomes as a carbon-monoxide binding pigment as described, e.g., in Garfinkel, *Arch. Biochem. Biophys.* 77:493-509 (1958), which is incorporated herein by reference, P450s were later named based on their absorption at 450 nm in a reduced-CO coupled difference spectrum as described, e.g., in Omura et al., *J. Biol. Chem.* 239:2370-2378 (1964), which is incorporated herein by reference.

P450s catalyze the metabolism of a variety of endogenous and exogenous compounds (Nelson, *supra).* Endogenous compounds include steroids, prostanoids, eicosanoids, fat-soluble vitamins, fatty acids, mammalian alkaloids, leukotrines, biogenic amines and phytolexins (Nelson, *supra).* P450 metabolism involves such reactions as epoxidation, hydroxylation, deakylation, N-hydroxylation, sulfoxidation, desulfuration and reductive dehalogenation. These reactions generally make the compound more water soluble, which is conducive for excretion, and more electrophilic. These electrophilic products can have detrimental effects if they react with DNA or other cellular constituents. However, they can react through conjugation with low molecular weight hydrophilic substances resulting in glucoronidation, sulfation, acetylation, amino acid conjugation or glutathione conjugation typically leading to inactivation and elimination as described, e.g., in Klaassen et al., *Toxicology,* 3rd ed, Macmillan, New York, 1986, incorporated herein by reference.

P450s are heme thiolate proteins consisting of a heme moiety bound to a single polypeptide chain of 45,000 to 55,000 Da. The iron of the heme prosthetic group is located at the center of a protoporphyrin ring. Four ligands of the heme iron can be attributed to the porphyrin ring. The fifth ligand is a thiolate anion from a cysteinyl residue of the polypeptide. The sixth ligand is probably a hydroxyl group from an amino acid residue, or a moiety with a similar field strength such as a water molecule as described, e.g., in Goeptar et al., *Critical Reviews in Toxicology* 25(1):25-65 (1995), incorporated herein by reference.

Monooxygenation reactions catalyzed by cytochromes P450 in a eukaryotic membrane-bound system require the transfer of electrons from NADPH to P450 via NADPH-cytochrome P450 reductase (*CPR*) as described, e.g., in Taniguchi et al., *Arch. Biochem. Biophys.* 232:585 (1984), incorporated herein by reference. *CPR* is a flavoprotein of approximately 78,000 Da containing 1 mol of flavin adenine dinucleotide (FAD) and 1 mol of flavin mononucleotide (FMN) per mole of enzyme as described, e.g., in Potter et al., *J. Biol. Chem.* 258:6906 (1983), incorporated herein by reference. The FAD moiety of *CPR* is the site of electron entry into the enzyme, whereas FMN is the electron-donating site to P450 as described, e.g., in Vermilion et al., *J. Biol. Chem.* 253:8812 (1978), incorporated herein by reference. The overall reaction is as follows:

$$H^+ + RH + NADPH + O_2 \rightarrow ROH + NADP^+ + H_2O$$

Binding of a substrate to the catalytic site of P450 apparently results in a conformational change initiating electron transfer from *CPR* to P450. Subsequent to the transfer of the first electron, $O_2$ binds to the $Fe_2^+$-P450 substrate complex to form $Fe_3^+$ -P450-substrate complex. This complex is then reduced by a second electron from *CPR,* or, in some cases, NADH via cytochrome b5 and NADH-cytochrome b5 reductase as described, e.g., in Guengerich et al., *Arch. Biochem. Biophys.* 205:365 (1980), incorporated herein by reference. One atom of this reactive oxygen is introduced into the substrate, while the other is reduced to water. The oxygenated substrate then dissociates, regenerating the oxidized form of the cytochrome P450 as described, e.g., in Klassen, Amdur and Doull, *Casarett and Doull's Toxicology,* Macmillan, New York (1986), incorporated herein by reference.

The P450 reaction cycle can be short-circuited in such a way that $O_2$ is reduced to $O_2^-$ and/or $H_2O_2$ instead of being utilized for substrate oxygenation. This side reaction is often referred to as the "uncoupling" of cytochrome P450 as described, e.g., in Kuthen et al., *Eur. J. Biochem*. 126:583 (1982) and Poulos et al., *FASEB J.* 6:674 (1992), both of which are incorporated herein by reference. The formation of these oxygen radicals may lead to oxidative cell damage as described, e.g., in Mukhopadhyay, *J. Biol. Chem.* 269(18):13390-13397 (1994) and Ross et al., *Biochem. Pharm*. 49(7):979-989 (1995), both of which are incorporated herein by reference. It has been proposed that cytochrome b5's effect on P450 binding to the *CPR* results in a more stable complex which is less likely to become "uncoupled" as described, e.g., in Yamazaki et al., *Arch. Biochem. Biophys.* 325(2):174-182 (1996), incorporated herein by reference.

P450 families are assigned based upon protein sequence comparisons. Notwithstanding a certain amount of heterogeneity, a practical classification of P450s into families can be obtained based on deduced amino acid sequence similarity. P450s with amino acid sequence similarity of between about 40 - 80% are considered to be in the same family, with sequences of about > 55% belonging to the same subfamily. Those with sequence similarity of about < 40% are generally listed as members of different P450 gene families (Nelson, *supra*). *A* value of about > 97% is taken to indicate allelic variants of the same gene, unless proven otherwise based on catalytic activity, sequence divergence in non-translated regions of the gene sequence, or chromosomal mapping.

The most highly conserved region is the HR2 consensus containing the invariant cysteine residue near the carboxyl terminus which is required for heme binding as described, e.g., in Gotoh et al. *J. Biochem.* 93:807-817 (1983) and Motohashi et al., *J. Biochem.* 101:879-997 (1987), both of which are incorporated herein by reference. Additional consensus regions, including the central region of helix I and the transmembrane region, have also been identified, as described, e.g, in Goeptar et al., *supra* and Kalb et al., *PNAS.* 85:7221-7225 (1988), incorporated herein by reference, although the HR2 cysteine is the only invariant amino acid among P450s.

Short chain (≤C12) aliphatic dicarboxylic acids (diacids) are important industrial intermediates in the manufacture of diesters and polymers, and find application as thermoplastics, plasticizing agents, lubricants, hydraulic fluids, agricultural chemicals, pharmaceuticals, dyes, surfactants, and adhesives. The high price and limited availability of short chain diacids are due to constraints imposed by the existing chemical synthesis.

Long-chain diacids (aliphatic α, ω-dicarboxylic acids with carbon numbers of 12 or greater, hereafter also referred to as diacids) ($HOOC-(CH_2)_n-COOH$) are a versatile family of chemicals with demonstrated and potential utility in a variety of chemical products including plastics, adhesives, and fragrances. Unfortunately, the full market potential of diacids has not been realized because chemical processes produce only a limited range of these materials at a relatively high price. In addition, chemical processes for the production of diacids have a number of limitations and disadvantages. All the chemical processes are restricted to the production of diacids of specific carbon chain lengths. For example, the dodecanedioic acid process starts with butadiene. The resulting product diacids are limited to multiples of four-carbon lengths and, in practice, only dodecanedioic acid is made. The dodecanedioic process is based on nonrenewable petrochemical feedstocks. The multireaction conversion process produces unwanted byproducts, which result in yield losses, $NO_x$ pollution and heavy metal wastes.

Long-chain diacids offer potential advantages over shorter chain diacids, but their high selling price and limited commercial availability prevent widespread growth in many of these applications. Biocatalysis offers an innovative way to overcome these limitations with a process that produces a wide range of diacid products from renewable feedstocks. However, there is no commercially viable bioprocess to produce long chain diacids from renewable resources.

## SUMMARY OF THE INVENTION

An isolated nucleic acid is provided which encodes a *CPRA* protein having the amino acid sequence set forth in SEQ ID NO: 83. An isolated nucleic acid is also provided which includes a coding region defined by nucleotides 1006-3042 as set forth in SEQ ID NO: 81. An isolated protein is provided which includes an amino acid sequence as set forth in SEQ ID NO: 83. A vector is provided which includes a nucleotide sequence encoding *CPRA* protein including an amino acid sequence as set forth in SEQ ID NO: 83. A host cell is provided which is transfected or transformed with the nucleic acid encoding *CPRA* protein having an amino acid sequence as set forth in SEQ ID NO: 83. A method of producing a *CPRA* protein including an amino acid sequence as set forth in SEQ ID NO: 83 is also provided which includes a) transforming a suitable host cell with a DNA sequence that encodes the protein having the amino acid sequence as set forth in SEQ ID NO: 83; and b) culturing the cell under conditions favoring the expression of the protein.

An isolated nucleic acid is provided which encodes a *CPRB* protein having the amino acid sequence set forth in SEQ ID NO: 84. An isolated nucleic acid is provided which includes a coding region defined by nucleotides 1033-3069 as set forth in SEQ ID NO: 82. An isolated protein is provided which includes an amino acid sequence as set forth in SEQ ID NO: 84. A vector is provided which includes a nucleotide sequence encoding *CPRB* protein including an amino acid sequence as set forth in SEQ ID NO: 84. A host cell is provided which is transfected or transformed with the nucleic acid encoding *CPRB* protein having an amino acid sequence as set forth in SEQ ID NO: 84. A method of producing a *CPRB* protein including an amino acid sequence as set forth in SEQ ID NO: 84 is provided which includes a) transforming a suitable host cell with a DNA sequence that encodes the protein having the amino acid sequence as set forth in SEQ ID NO: 84; and b) culturing the cell under conditions favoring the expression of the protein.

An isolated nucleic acid is provided which encodes a *CYP52A1A* protein having the amino acid sequence set forth in SEQ ID NO: 95. An isolated nucleic acid is provided which includes a coding region defined by nucleotides 1177-2748 as set forth in SEQ ID NO: 85. An isolated protein is provided which includes an amino acid sequence as set forth in SEQ ID NO: 95. A vector is provided which includes a nucleotide sequence encoding *CYP52A1A* protein including an amino acid sequence as set forth in SEQ ID NO: 95. A host cell is provided which is transfected or transformed with the nucleic acid encoding *CYP52A1A* protein having an amino acid sequence as set forth in SEQ ID NO: 95. A method of producing a *CYP52A1A* protein including an amino acid sequence as set forth in SEQ ID NO: 95 is provided which includes a) transforming a suitable host cell with a DNA sequence that encodes the protein having the amino acid sequence as set forth in SEQ ID NO: 95; and b) culturing the cell under conditions favoring the expression of the protein.

An isolated nucleic acid encoding a *CYP52A2A* protein is provided which has the amino acid sequence set forth in SEQ ID NO: 96. An isolated nucleic acid is provided which includes a coding region defined by nucleotides 1199-2767 as set forth in SEQ ID NO: 86. An isolated protein is provided which includes an amino acid sequence as set forth in SEQ ID NO: 96. A vector is provided which includes a nucleotide sequence encoding *CYP52A2A* protein including an amino acid sequence as set forth in SEQ ID NO: 96. A host cell is provided which is transfected or transformed with the nucleic acid encoding *CYP52A2A* protein having an amino acid sequence as set forth in SEQ ID NO: 96. A method of producing a *CYP52A2A* protein including an amino acid sequence as set forth in SEQ ID NO: 96 is provided which includes a) transforming a suitable host cell with a DNA sequence that encodes the protein having the amino acid sequence as set forth in SEQ ID NO: 96; and b) culturing the cell under conditions favoring the expression of the protein.

An isolated nucleic acid encoding a *CYP52A2B* protein is provided which has the amino acid sequence set forth in SEQ ID NO: 97. An isolated nucleic acid is provided which includes a coding region defined by nucleotides 1072-2640 as set forth in SEQ ID NO: 87. An isolated protein is provided which includes an amino acid sequence as set forth in SEQ ID NO: 97. A vector is provided which includes a nucleotide sequence encoding *CYP52A2B* protein including an amino acid sequence as set forth in SEQ ID NO: 97. A host cell is provided which is transfected or transformed with the nucleic acid encoding *CYP52A2B* protein having an amino acid sequence as set forth in SEQ ID NO: 97. A method of producing a *CYP52A2B* protein including an amino acid sequence as set forth in SEQ ID NO: 97 is provided which includes a) transforming a suitable host cell with a DNA sequence that encodes the protein having the amino acid sequence as set forth in SEQ ID NO: 97; and b) culturing the cell under conditions favoring the expression of the protein.

An isolated nucleic acid encoding a *CYP52A3A* protein is provided which has the amino acid sequence set forth in SEQ ID NO: 98. An isolated nucleic acid is provided which includes a coding region defined by nucleotides 1126-2748 as set forth in SEQ ID NO: 88. An isolated protein is provided which includes an amino acid sequence as set forth in SEQ ID NO: 98. A vector is provided which includes a nucleotide sequence encoding *CYP52A3A* protein including an amino acid sequence as set forth in SEQ ID NO: 98. A host cell is provided which is transfected or transformed with the nucleic acid encoding *CYP52A3A* protein having an amino acid sequence as set forth in SEQ ID NO: 98. A method of producing a *CYP52A3A* protein including an amino acid sequence as set forth in SEQ ID NO: 98 is provided which includes a) transforming a suitable host cell with a DNA sequence that encodes the protein having the amino acid sequence as set forth in SEQ ID NO: 98; and b) culturing the cell under conditions favoring the expression of the protein.

An isolated nucleic acid encoding a *CYP52A3B* protein is provided having the amino acid sequence as set forth in SEQ ID NO: 99. An isolated nucleic acid is provided which includes a coding region defined by nucleotides 913-2535 as set forth in SEQ ID NO: 89. An isolated protein is provided which includes an amino acid sequence as set forth in SEQ ID NO: 99. A vector is provided which includes a nucleotide sequence encoding *CYP52A3B* protein including an amino acid sequence as set forth in SEQ ID NO: 99. A host cell is provided which is transfected or transformed with the nucleic acid encoding *CYP52A3B* protein having an amino acid sequence as set forth in SEQ ID NO: 99. A method of producing a *CYP52A3B* protein including an amino acid sequence as set forth in SEQ ID NO: 99 is provided which includes a) transforming a suitable host cell with a DNA sequence that encodes the protein having the amino acid sequence as set forth in SEQ ID NO: 99; and b) culturing the cell under conditions favoring the expression of the protein.

An isolated nucleic acid encoding a *CYP52A5A* protein is provided having the amino acid sequence set forth in SEQ ID NO: 100. An isolated nucleic acid is provided which includes a coding region defined by nucleotides 1103-2656 as set forth in SEQ ID NO: 90. An isolated protein is provided which includes an amino acid sequence as set forth in SEQ ID NO: 100. A vector is provided which includes a nucleotide sequence encoding *CYP52A5A* protein including an amino acid sequence as set forth in SEQ ID NO: 100. A host cell is provided which is transfected or transformed with the nucleic acid encoding *CYP52A5A* protein having an amino acid sequence as set forth in SEQ ID NO: 100. A method of producing a *CYP52A5A* protein including an amino acid sequence as set forth in SEQ ID NO: 100 is provided which includes a) transforming a suitable host cell with a DNA sequence that encodes the protein having the amino acid sequence as set forth in SEQ ID NO: 100; and b) culturing the cell under conditions favoring the expression of the protein.

An isolated nucleic acid encoding a *CYP52A5B* protein is provided having the amino acid sequence as set forth in SEQ ID NO: 101. An isolated nucleic acid is provided which includes a coding region defined by nucleotides 1142-2695 as set forth in SEQ ID NO: 91. An isolated protein is provided which includes an amino acid sequence as set forth in SEQ ID NO: 101. A vector is provided which includes a nucleotide sequence encoding *CYP52A5B* protein including the amino acid sequence as set forth in SEQ ID NO: 101. A host cell is provided which is transfected or transformed with the nucleic acid encoding *CYP52A5B* protein having the amino acid sequence as set forth in SEQ ID NO: 101. A method of producing a *CYP52A5B* protein including an amino acid sequence as set forth in SEQ ID NO: 101 is provided which includes a) transforming a suitable host cell with a DNA sequence that encodes the protein having the amino acid sequence as set forth in SEQ ID NO: 101; and b) culturing the cell under conditions favoring the expression of the protein.

An isolated nucleic acid encoding a *CYP52A8A* protein is provided having the amino acid sequence set forth in SEQ ID NO: 102. An isolated nucleic acid is provided which includes a coding region defined by nucleotides 464-2002 as set forth in SEQ ID NO: 92. An isolated protein is provided which includes an amino acid sequence as set forth in SEQ ID NO: 102. A vector is provided which includes a nucleotide sequence encoding *CYP52A8A* protein including an amino acid sequence as set forth in SEQ ID NO: 102. A host cell is provided which is transfected or transformed with the nucleic acid encoding *CYP52A8A* protein having an amino acid sequence as set forth in SEQ ID NO: 102. A method of producing a *CYP52A8A* protein including an amino acid sequence as set forth in SEQ ID NO: 102 is provided which includes a) transforming a suitable host cell with a DNA sequence that encodes the protein having the amino acid sequence as set forth in SEQ ID NO: 102; and b) culturing the cell under conditions favoring the expression of the protein.

An isolated nucleic acid encoding a *CYP52A8B* protein is provided having the amino acid sequence set forth in SEQ ID NO: 103. An isolated nucleic acid is provided which includes a coding region defined by nucleotides 1017-2555 as set forth in SEQ ID NO: 93. An isolated protein is provided which includes an amino acid sequence as set forth in SEQ ID NO: 103. A vector is provided which includes a nucleotide sequence encoding *CYP52A8B* protein including an amino acid sequence as set forth in SEQ ID NO: 103. A host cell is provided which is transfected or transformed with the nucleic acid encoding *CYP52A8B* protein having an amino acid sequence as set forth in SEQ ID NO: 103. A method of producing a *CYP52A8B* protein including an amino acid sequence as set forth in SEQ ID NO: 103 is provided which includes a) transforming a suitable host cell with a DNA sequence that encodes the protein having the amino acid sequence as set forth in SEQ ID NO: 103; and b) culturing the cell under conditions favoring the expression of the protein.

An isolated nucleic acid encoding a *CYP52D4A* protein is provided having the amino acid sequence set forth in SEQ ID NO: 104. An isolated nucleic acid is provided including a coding region defined by nucleotides 767-2266 as set forth in SEQ ID NO: 94. An isolated protein is provided which includes an amino acid sequence as set forth in SEQ ID NO: 104. A vector is provided which includes a nucleotide sequence encoding *CYP52D4A* protein including an amino acid sequence as set forth in SEQ ID NO: 104. A host cell is provided which is transfected or transformed with the nucleic acid encoding *CYP52D4A* protein having an amino acid sequence as set forth in SEQ ID NO: 104. A method of producing a *CYP52D4A* protein including an amino acid sequence as set forth in SEQ ID NO: 104 is provided which includes a) transforming a suitable host cell with a DNA sequence that encodes the protein having the amino acid sequence as set forth in SEQ ID NO: 104; and b) culturing the cell under conditions favoring the expression of the protein.

A method for discriminating members of a gene family by quantifying the amount of target mRNA in a sample is provided which includes a) providing an organism containing a target gene; b) culturing the organism with an organic substrate which causes upregulation in the activity of the target gene; c) obtaining a sample of total RNA from the organism at a first point in time; d) combining at least a portion of the sample of the total RNA with a known amount of competitor RNA to form an RNA mixture, wherein the competitor RNA is substantially similar to the target mRNA but has a lesser number of nucleotides compared to the target mRNA; e) adding reverse transcriptase to the RNA mixture in a quantity sufficient to form corresponding target DNA and competitor DNA; (f) conducting a polymerase chain reaction in the presence of at least one primer specific for at least one substantially non-homologous region of the target DNA within the gene family, the primer also specific for the competitor DNA; g) repeating steps (c-f) using increasing amounts of the competitor RNA while maintaining a substantially constant amount of target RNA; h) determining the point at which the amount of target DNA is substantially equal to the amount of competitor DNA; i) quantifying the results by comparing the ratio of the concentration of unknown target to the known concentration of competitor; and j) obtaining a sample of total RNA from the organism at another point in time and repeating steps (d-i).

A method for increasing production of a dicarboxylic acid is provided which includes a) providing a host cell having a naturally occurring number of *CPRA* genes; b) increasing, in the host cell, the number of *CPRA* genes which encode a *CPRA* protein having the amino acid sequence as set forth in SEQ ID NO: 83; c) culturing the host cell in media containing an organic substrate which upregulates the *CPRA* gene, to effect increased production of dicarboxylic acid.

A method for increasing the production of a *CPRA* protein having an amino acid sequence as set forth in SEQ ID NO: 83 is provided which includes a) transforming a host cell having a naturally occurring amount of *CPRA* protein with an increased copy number of a *CPRA* gene that encodes the *CPRA* protein having the amino acid sequence as set forth in SEQ ID NO: 83; and b) culturing the cell and thereby increasing expression of the protein compared with that of a host cell containing a naturally occurring copy number of the *CPRA* gene.

A method for increasing production of a dicarboxylic acid is provided which includes a) providing a host cell having a naturally occurring number of *CPRB* genes; b) increasing, in the host cell, the number of *CPRB* genes which encode a *CPRB* protein having the amino acid sequence as set forth in SEQ ID NO: 84; c) culturing the host cell in media containing an organic substrate which upregulates the *CPRB* gene, to effect increased production of dicarboxylic acid.

A method for increasing the production of a *CPRB* protein having an amino acid sequence as set forth in SEQ ID NO: 84 is provided which includes a) transforming a host cell having a naturally occurring amount of *CPRB* protein with an increased copy number of a *CPRB* gene that encodes the *CPRB* protein having the amino acid sequence as set forth in SEQ ID NO: 84; and b) culturing the cell and thereby increasing expression of the protein compared with that of a host cell containing a naturally occurring copy number of the *CPRB* gene.

A method for increasing production of a dicarboxylic acid is provided which includes a) providing a host cell having a naturally occurring number of *CYP52A1A* genes; b) increasing, in the host cell, the number of *CYP52A1A* genes which encode a *CYP52A1A* protein having the amino acid sequence as set forth in SEQ ID NO: 95; c) culturing the host cell in media containing an organic substrate which upregulates the *CYP52A1A* gene, to effect increased production of dicarboxylic acid.

A method for increasing the production of a *CYP52A1A* protein having an amino acid sequence as set forth in SEQ ID NO: 95 is provided which includes a) transforming a host cell having a naturally occurring amount of *CYP52A1A* protein with an increased copy number of a *CYP52A1A* gene that encodes the *CYP52A1A* protein having the amino acid sequence as set forth in SEQ ID NO: 95; and b) culturing the cell and thereby increasing expression of the protein compared with that of a host cell containing a naturally occurring copy number of the *CYP52A1A* gene.

A method for increasing production of a dicarboxylic acid is provided which includes a) providing a host cell having a naturally occurring number of *CYP52A2A* genes; b) increasing, in the host cell, the number of *CYP52A2A* genes which encode a *CYP52A2A* protein having the amino acid sequence as set forth in SEQ ID NO: 96; c) culturing the host cell in media containing an organic substrate which upregulates the *CYP52A2A* gene, to effect increased production of dicarboxylic acid.

A method for increasing the production of a *CYP52A2A* protein having an amino acid sequence as set forth in SEQ ID NO: 96 is provided which includes a) transforming a host cell having a naturally occurring amount of *CYP52A2A* protein with an increased copy number of a *CYP52A2A* gene that encodes the *CYP52A2A* protein having the amino acid sequence as set forth in SEQ ID NO: 96; and b) culturing the cell and thereby increasing expression of the protein compared with that of a host cell containing a naturally occurring copy number of the *CYP52A2A* gene.

A method for increasing production of a dicarboxylic acid is provided which includes a) providing a host cell having a naturally occurring number of *CYP52A2B* genes; b) increasing, in the host cell, the number of *CYP52A2B* genes which encode a *CYP52A2B* protein having the amino acid sequence as set forth in SEQ ID NO: 97; c) culturing the host cell in media containing an organic substrate which upregulates the *CYP52A2B* gene, to effect increased production of dicarboxylic acid.

A method for increasing the production of a *CYP52A2B* protein having an amino acid sequence as set forth in SEQ ID NO: 97 is provided which includes a) transforming a host cell having a naturally occurring amount of *CYP52A2B* protein with an increased copy number of a *CYP52A2B* gene that encodes the *CYP52A2B* protein having the amino acid sequence as set forth-in SEQ ID NO: 97; and b) culturing the cell and thereby increasing expression of the protein compared with that of a host cell containing a naturally occurring copy number of the *CYP52A2B* gene.

A method for increasing production of a dicarboxylic acid is provided which includes a) providing a host cell having a naturally occurring number of *CYP52A3A* genes; b) increasing, in the host cell, the number of *CYP52A3A* genes which encode a *CYP52A3A* protein having the amino acid sequence as set forth in SEQ ID NO: 98; c) culturing the host cell in media containing an organic substrate which upregulates *CYP52A3A* gene, to effect increased production of dicarboxylic acid.

A method for increasing the production of a *CYP52A3A* protein having an amino acid sequence as set forth in SEQ ID NO: 98 is provided which includes a) transforming a host cell having a naturally occurring amount of *CYP52A3A* protein with an increased copy number of a *CYP52A3A* gene that encodes the *CYP52A3A* protein having the amino acid sequence as set forth in SEQ ID NO: 98; and b) culturing the cell and thereby increasing expression of the protein compared with that of a host cell containing a naturally occurring copy number of the *CYP52A3A* gene.

A method for increasing production of a dicarboxylic acid is provided which includes a) providing a host cell having a naturally occurring number of *CYP52A3B* genes; b) increasing, in the host cell, the number of *CYP52A3B* genes which encode a *CYP52A3B* protein having the amino acid sequence as set forth in SEQ ID NO: 99; c) culturing the host cell in media containing an organic substrate which upregulates the *CYP52A3B* gene, to effect increased production of dicarboxylic acid.

A method for increasing the production of a *CYP52A3B* protein having an amino acid sequence as set forth in SEQ ID NO: 99 is provided which includes a) transforming a host cell having a naturally occurring amount of *CYP52A3B* protein with an increased copy number of a *CYP52A3B* gene that encodes the *CYP52A3B* protein having the amino acid sequence as set forth in SEQ ID NO: 99; and b) culturing the cell and thereby increasing expression of the protein compared with that of a host cell containing a naturally occurring copy number of the *CYP52A3B* gene.

A method for increasing production of a dicarboxylic acid is provided which includes a) providing a host cell having a naturally occurring number of *CYP52A5A* genes; b) increasing, in the host cell, the number of *CYP52A5A* genes which encode a *CYP52A5A* protein having the amino acid sequence as set forth in SEQ ID NO: 100; c) culturing the host cell in media containing an organic substrate which upregulates the *CYP52A5A* gene, to effect increased production of dicarboxylic acid.

A method for increasing the production of a *CYP52A5A* protein having an amino acid sequence as set forth in SEQ ID NO: 100 is provided which includes a) transforming a host cell having a naturally occurring amount of *CYP52A5A* protein with an increased copy number of a *CYP52A5A* gene that encodes the *CYP52A5A* protein having the amino acid sequence as set forth in SEQ ID NO: 100; and b) culturing the cell and thereby increasing expression of the protein compared with that of a host cell containing a naturally occurring copy number of the *CYP52A5A* gene.

A method for increasing production of a dicarboxylic acid is provided which includes a) providing a host cell having a naturally occurring number of *CYP52A5B* genes; b) increasing, in the host cell, the number of *CYP52A5B* genes which encode a *CYP52A5B* protein having the amino acid sequence as set forth in SEQ ID NO: 101; c) culturing the host cell in media containing an organic substrate which upregulates the *CYP52A5B* gene, to effect increased production of dicarboxylic acid.

A method for increasing the production of a *CYP52A5B* protein having an amino acid sequence as set forth in SEQ ID NO: 101 is provided which includes a) transforming a host cell having a naturally occurring amount of *CYP52A5B* protein with an increased copy number of a *CYP52A5B* gene that encodes the *CYP52A5B* protein having the amino acid sequence as set forth in SEQ ID NO: 101; and b) culturing the cell and thereby increasing expression of the protein compared with that of a host cell containing a naturally occurring copy number of the *CYP52A5B* gene.

A method for increasing production of a dicarboxylic acid is provided which includes a) providing a host cell having a naturally occurring number of *CYP52A8A* genes; b) increasing, in the host cell, the number of *CYP52A8A* genes which encode a *CYP52A8A* protein having the amino acid sequence as set forth in SEQ ID NO: 102; c) culturing the host cell in media containing an organic substrate which upregulates the *CYP52A8A* gene, to effect increased production of dicarboxylic acid.

A method for increasing the production of a *CYP52A8A* protein having an amino acid sequence as set forth in SEQ ID NO: 102 is provided which includes a) transforming a host cell having a naturally occurring amount of *CYP52A8A* protein with an increased copy number of a *CYP52A8A* gene that encodes the *CYP52A8A* protein having the amino acid sequence as set forth in SEQ ID NO: 102; and b) culturing the cell and thereby increasing expression of the protein compared with that of a host cell containing a naturally occurring copy number of the *CYP52A8A* gene.

A method for increasing production of a dicarboxylic acid is provided which includes a) providing a host cell having a naturally occurring number of *CYP52A8B* genes; b) increasing, in the host cell, the number of *CYP52A8B* genes which encode a *CYP52A8B* protein having the amino acid sequence as set forth in SEQ ID NO: 103; c) culturing the host cell in media containing an organic substrate which upregulates the *CYP52A8B* gene, to effect increased production of dicarboxylic acid.

A method for increasing the production of a *CYP52A8B* protein having an amino acid sequence as set forth in SEQ ID NO: 103 is provided which includes a) transforming a host cell having a naturally occurring amount of *CYP52A8B* protein with an increased copy number of a *CYP52A8B* gene that encodes the *CYP52A8B* protein having the amino acid sequence as set forth in SEQ ID NO: 103; and b) culturing the cell and thereby increasing expression of the protein compared with that of a host cell containing a naturally occurring copy number of the *CYP52A8B* gene.

A method for increasing production of a dicarboxylic acid is provided which includes a) providing a host cell having a naturally occurring number of *CYP52D4A* genes; b) increasing, in the host cell, the number of *CYP52D4A* genes which encode a *CYP52D4A* protein having the amino acid sequence as set forth in SEQ ID NO: 104; c) culturing the host cell in media containing an organic substrate which upregulates the *CYP52D4A* gene, to effect increased production of dicarboxylic acid.

A method for increasing the production of a *CYP52D4A* protein having an amino acid sequence as set forth in SEQ ID NO: 104 is provided which includes a) transforming a host cell having a naturally occurring amount of *CYP52D4A* protein with an increased copy number of a *CYP52D4A* gene that encodes the *CYP52D4A* protein having the amino acid sequence as set forth in SEQ ID NO: 104; and b) culturing the cell and thereby increasing expression of the. protein compared with that of a host cell containing a naturally occurring copy number of the *CYP52D4A* gene.

## BRIEF DESCRIPTION OF THE DRAWINGS

Figure 1 is a schematic representation of cloning vector pTriplEx from Clontech™ Laboratories, Inc. Selected restriction sites within the multiple cloning site are shown.

Figure 2A is a map of the ZAP Express™ vector.
Figure 2B is a schematic representation of cloning phagemid vector pBK-CMV.
Figure 3 is a double stranded DNA sequence of a portion of the 5 prime coding region of the *CYP52A5A* gene (SEQ ID NO: 36).
Figure 4 is a diagrammatic representation of highly conserved regions of *CYP* and *CPR* gene protein sequences. Helix I represents the putative substrate binding site and HR2 represents the heme binding region. The FMN, FAD and NADPH binding regions are indicated below the *CPR* gene.
Figure 5 is a diagrammatic representation of the plasmid pHKM1 containing the truncated *CPRA* gene present in the pTriplEx vector. A detailed restriction map of only the sequenced region is shown at the top. The bar indicates the open reading frame. The direction of transcription is indicated by an arrow under the open reading frame.
Figure 6 is a diagrammatic representation of the plasmid pHKM4 containing the truncated *CPRA* gene present in the pTriplEx vector. A detailed restriction map of only the sequenced region is shown at the top. The bar indicates the open reading frame. The direction of transcription is indicated by an arrow under the open reading frame.
Figure 7 is a diagrammatic representation of the plasmid pHKM9 containing the *CPRB* gene (SEQ ID NO: 82) present in the pBK-CMV vector. A detailed restriction map of only the sequenced region is shown at the top. The bar indicates the open reading frame. The direction of transcription is indicated by an arrow under the open reading frame.
Figure 8 is a diagrammatic representation of the plasmid pHKM11 containing the *CYP52A1A* gene (SEQ ID NO: 85) present in the pBK-CMV vector. A detailed restriction map of only the sequenced region is shown at the top. The bar indicates the open reading frame. The direction of transcription is indicated by an arrow under the open reading frame.
Figure 9 is a diagrammatic representation of the plasmid pHKM12 containing the *CYP52A8A* gene (SEQ ID NO: 92) present in the pBK-CMV vector. A detailed restriction map of only the sequenced region is shown at the top. The bar indicates the open reading frame. The direction of transcription is indicated by an arrow under the open reading frame.
Figure 10 is a diagrammatic representation of the plasmid pHKM13 containing the *CYP52D4A* gene (SEQ ID NO: 94) present in the pBK-CMV vector. A detailed restriction map of only the sequenced region is shown at the top. The bar indicates the open reading frame. The direction of transcription is indicated by an arrow under the open reading frame.
Figure 11 is a diagrammatic representation of the plasmid pHKM14 containing the *CYP52A2B* gene (SEQ ID NO: 87) present in the pBK-CMV vector. A detailed restriction map of only the sequenced region is shown at the top. The bar indicates the open reading frame. The direction of transcription is indicated by an arrow under the open reading frame.
Figure 12 is a diagrammatic representation of the plasmid pHKM15 containing the *CYP52A8B* gene (SEQ ID NO: 93) present in the pBK-CMV vector. A detailed restriction map of only the sequenced region is shown at the top. The bar indicates the open reading frame. The direction of transcription is indicated by an arrow under the open reading frame.
Figures 13A-13D show the complete DNA sequences including regulatory and coding regions for the *CPRA* gene (SEQ ID NO: 81) and *CPRB* gene (SEQ ID NO: 82) from C. *tropicalis* ATCC 20336. Figures 13A-13D show regulatory and coding region alignment of these sequences. Asterisks indicate conserved nucleotides. Bold indicates protein coding nucleotides; the start and stop codons are underlined.
Figure 14 shows the amino acid sequence of the *CPRA* (SEQ ID NO: 83) and *CPRB* (SEQ ID NO: 84) proteins from *C. tropicalis* ATCC 20336 and alignment of these amino acid sequences. Asterisks indicate residues which are not conserved.
Figures 15A-15M show the complete DNA sequences including regulatory and coding regions for the following genes from *C. tropicalis* ATCC 20366: *CYP52A1A* (SEQ ID NO: 85), *CYP52A2A* (SEQ ID NO: 86), *CYP52A2B* (SEQ ID NO: 87), *CYP52A3A* (SEQ ID NO: 88), *CYP52A3B* (SEQ ID NO: 89), *CYP52A5A* (SEQ ID NO. 90), *CYP52A5B* (SEQ ID NO: 91), *CYP52A8A* (SEQ ID NO: 92), *CYP52A8B* (SEQ ID NO: 93), and *CYP52D4A* (SEQ ID NO: 94). Figures 15A-15M show regulatory and coding region alignment of these sequences. Asterisks indicate conserved nucleotides. Bold indicates protein coding nucleotides; the start and stop codons are underlined.
Figures 16A-16C show the amino acid sequences encoding the *CYP52A1A* (SEQ ID NO: 95), *CYP52A2A* (SEQ ID NO: *96), CYP52A2B* (SEQ ID NO: 97), *CYP52A3A* (SEQ ID NO: 98), *CYP52A3B* (SEQ ID NO: 99), *CYP52A5A* (SEQ ID NO: 100), *CYP52A5B* (SEQ ID NO: 101), *CYP52A8A* (SEQ ID NO: 102), *CYP52A8B* (SEQ ID NO: 103) and *CYP52D4A* (SEQ ID NO. 104) proteins from C. *tropicalis* ATCC 20336. Asterisks indicate identical residues and dots indicate conserved residues.
Figure 17 is a diagrammatic representation of the pTAg PCR product cloning vector (commercially available from R&D Systems, Minneapolis, MN).
Figure 18 is a plot of the log ratio (U/C) of unknown target DNA product to competitor DNA product versus the concentration of competitor mRNA. The plot is used to calculate the target messenger RNA concentration in a quantitative competitive reverse transcription polymerase chain reaction (QC-RT-PCR).

Figure 19 is a graph showing the relative induction of *C. tropicalis* ATCC 20962 *CYP52A5A* (SEQ ID NO: 90) by the addition of the fatty acid substrate Emersol® 267 to the growth medium.

Figure 20 is a graph showing the induction of *C. tropicalis* ATCC 20962 *CYP52* and *CPR* genes by Emersol® 267. P450 genes *CYP52A3A* (SEQ ID NO: 88), *CYP52A3B* (SEQ ID NO: 89), and *CYP52D4A* (SEQ ID NO: 94) are expressed at levels below the detection level of the QC-RT-PCR assay.

Figure 21 is a scheme to integrate selected genes into the genome of *Candida tropicalis* strains and recovery of *URA3A* selectable marker.

Figure 22 is a schematic representation of the transformation of *C. tropicalis* H5343 ura3$^-$ with *CYP* and/or *CPR* genes. Only one *URA3* locus needs to be functional. There are a total of 6 possible *ura*3 targets (5*ura*3A loci-2 pox4 disruptions, 2 pox 5 disruptions, 1 *ura*3A locus; and 1 *ura*3B locus).

Figure 23 is the complete DNA sequence (SEQ ID NO: 105) encoding *URA3A* from *C. tropicalis* ATCC 20336 and the amino acid sequence of the encoded protein (SEQ ID NO: 106).

Figure 24 is a schematic representation of the plasmid pURAin, the base vector for integrating selected genes into the genome of *C. tropicalis.* The detailed construction of pURAin is described in the text.

Figure 25 is a schematic representation of the plasmid pNEB193 cloning vector (commercially available from New England Biolabs, Beverly, MA).

Figure 26 is a diagrammatic representation of the plasmid pPA15 containing the truncated *CYP52A2A* gene present in the pTriplEx vector. A detailed restriction map of only the sequenced region is shown at the top. The bar indicates the open reading frame. The direction of transcription is indicated by an arrow under the open reading frame.

Figure 27 is a schematic representation of pURA2in, the base vector is constructed in pNEB193 which contains the 8 bp recognition sequences for *Asc I, Pac I* and *Pme I*. *URA3A* (SEQ ID NO: 105) and *CYP52A2A* (SEQ ID NO: 86) do not contain these 8 bp recognition sites. *URA3A* is inverted so that the transforming fragment will attempt to recircularize prior to integration. An *Asc I*/*Pme I* fragment was used to transform H5343 ura$^-$.

Figure 28 shows a scheme to detect integration of *CYP52A2A* gene (SEQ ID NO: 86) into the genome of H5343 ura$^-$. In all cases, hybridization band intensity could reflect the number of integrations.

Figure 29 is a diagrammatic representation of the plasmid pPA57 containing the truncated *CYP52A3A* gene present in the pTriplEx vector. A detailed restriction map of only the sequenced region is shown at the top. The bar indicates the open reading frame. The direction of transcription is indicated by an arrow under the open reading frame.

Figure 30 is a diagrammatic representation of the plasmid pPA62 containing the truncated *CYP52A3B* gene present in the pTriplEx vector. A detailed restriction map of only the sequenced region is shown at the top. The bar indicates the open reading frame. The direction of transcription is indicated by an arrow under the open reading frame.

Figure 31 is a diagrammatic representation of the plasmid pPAL3 containing the truncated *CYP52A5A* gene present in the pTriplEx vector. A detailed restriction map of only the sequenced region is shown at the top. The bar indicates the open reading frame. The direction of transcription is indicated by an arrow under the open reading frame.

Figure 32 is a diagrammatic representation of the plasmid pPA5 containing the truncated *CYP52A5A* gene present in the pTriplEx vector. A detailed restriction map of only the sequenced region is shown at the top. The bar indicates the open reading frame. The direction of transcription is indicated by an arrow under the open reading frame.

Figure 33 is a diagrammatic representation of the plasmid pPA18 containing the truncated *CYP52D4A* gene present in the pTriplEx vector. A detailed restriction map of only the sequenced region is shown at the top. The bar indicates the open reading frame. The direction of transcription is indicated by an arrow under the open reading frame.

Figure 34 is a graph showing the expression of *CYP52A1* (SEQ ID NO: 85), *CYP52A2* (SEQ ID NO: 86) and *CYP52A5* genes (SEQ ID NOS: 90 and 91) from C. *tropicalis* 20962 in a fermentor run upon the addition of amounts of the substrate oleic acid or tridecane in a spiking experiment.

Figure 35 depicts a scheme used for the extraction and analysis of diacids and monoacids from fermentation broths.

Figure 36 is a graph showing the induction of expression of *CYP52A1A, CYP52A2A* and *CYP52A5A* in a fermentor run upon addition of the substrate octadecane. No induction of *CYP52A3A* or *CYP52A3B* was observed under these conditions.

## DESCRIPTION OF THE PREFERRED EMBODIMENTS

Diacid productivity is improved according to the present invention by selectively increasing enzymes which are known to be important to the oxidation of organic substrates such as fatty acids composing the desired feed. According to the present invention, ten *CYP* genes and two *CPR* genes of *C. tropicalis* have been identified and characterized that relate to participation in the ω-hydroxylase complex catalyzing the first step in the ω-oxidation pathway. In addition, a novel quantitative competitive reverse transcription polymerase chain reaction (QC-RT-PCR) assay is used to measure gene expression in the fermentor under conditions of induction by one or more organic substrates as defined herein. Based upon QC-RT-PCR results, three *CYP* genes, *CYP52A1, CYP52A2* and *CYP52A5,* have been identified as being of greater importance for the ω-oxidation of long chain fatty acids. Amplification of the *CPR* gene copy number improves productivity. The QC-RT-PCR assay indicates that both *CYP* and *CPR* genes appear to be under tight regulatory control.

In accordance with the present invention, a method for discriminating members of a gene family by quantifying the amount of target mRNA in a sample is provided which includes a) providing an organism containing a target gene; b) culturing the organism with an organic substrate which causes upregulation in the activity of the target gene; c) obtaining a sample of total RNA from the organism at a first point in time; d) combining at least a portion of the sample of the total RNA with a known amount of competitor RNA to form an RNA mixture, wherein the competitor RNA is substantially similar to the target mRNA but has a lesser number of nucleotides compared to the target mRNA; e) adding reverse transcriptase to the RNA mixture in a quantity sufficient to form corresponding target DNA and competitor DNA; (f) conducting a polymerase chain reaction in the presence of at least one primer specific for at least one substantially non-homologous region of the target DNA within the gene family, the primer also specific for the competitor DNA; g) repeating steps (c-f) using increasing amounts of the competitor RNA while maintaining a substantially constant amount of target RNA; h) determining the point at which the amount of target DNA is substantially equal to the amount of competitor DNA; i) quantifying the results by comparing the ratio of the concentration of unknown target to the known concentration of competitor; and j) obtaining a sample of total RNA from the organism at another point in time and repeating steps (d-i).

In addition, modification of existing promoters and/or the isolation of alternative promoters provides increased expression *of CYP* and *CPR* genes. Strong promoters are obtained from at least four sources: random or specific modifications of the *CYP52A2* promoter, *CYP52A5* promoter, *CYP52A1* promoter, the selection of a strong promoter from available *Candida* β-oxidation genes such as *POX4* and *POX5,* or screening to select another suitable *Candida* promoter.

Promoter strength can be directly measured using QT-RT-PCR to measure *CYP* and *CPR* gene expression in *Candida* cells isolated from fermentors. Enzymatic assays and antibodies specific for *CYP* and *CPR* proteins are used to verify that increased promoter strength is reflected by increased synthesis of the corresponding enzymes. Once a suitable promoter is identified, it is fused to the selected *CYP* and *CPR* genes and introduced into *Candida* for construction of a new improved production strain. It is contemplated that the coding region of the *CYP* and *CPR* genes can be fused to suitable promoters or other regulatory sequences which are well known to those skilled in the art.

In accordance with the present invention, studies on *C. tropicalis* ATCC 20336 have identified six unique *CYP* genes and four potential alleles. QC-RT-PCR analyses of cells isolated during the course of the fermentation bioconversions indicate that at least three of the *CYP* genes are induced by fatty acids and at least two of the *CYP* genes are induced by alkanes. See Figure 34. Two of the *CYP* genes are highly induced indicating participation in the ω-hydroxylase complex which catalyzes the rate limiting step in the oxidation of fatty acids to the corresponding diacids.

The biochemical characterizations of each P450 enzyme herein is used to tailor the *C. tropicalis* host for optimal diacid productivity and is used to select P450 enzymes to be amplified based upon the fatty acid content of the feedstream. *CYP* gene(s) encoding P450 enzymes that have a low specific activity for the fatty acid or alkane substrate of choice are targeted for inactivation, thereby reducing the physiological load on the cell.

Since it has been demonstrated that *CPR* can be limiting in yeast systems, the removal of non-essential P450s from the system can free electrons that are being used by non-essential P450s and make them available to the P450s important for diacid productivity. Moreover, the removal of non-essential P450s can make available other necessary but potentially limiting components of the P450 system (i.e., available membrane space, heme and/or NADPH).

Diacid productivity is thus improved by selective integration, amplification, and over expression of *CYP* and *CPR* genes in the C. *tropicalis* production host.

It should be understood that host cells into which one or more copies of desired *CYP* and/or *CPR* genes have been introduced can be made to include such genes by any technique known to those skilled in the art. For example, suitable host cells include procaryotes such as *Bacillus sp., Pseudomous sp., Actinomycetes sp., Eschericia sp., Mycobacterium sp.,* and eukaryotes such as yeast, algae, insect cells, plant cells and and filamentous fungi. Suitable host cells are preferably yeast cells such as *Yarrowia, Bebaromyces, Saccharomyces, Schizosaccharomyces,* and *Pichia* and more preferably those of the *Candida* genus. Preferred species of *Candida* are *tropicalis, maltosa, apicola, paratropicalis, albicans, cloacae, guillermondii, intermedia, lipolytica, parapsilosis* and *zeylenoides.* Certain preferred stains of *Candida tropicalis* are listed in U.S. Patent No. 5,254,466, incorporated herein by reference.

Vectors such as plasmids, phagemids, phages or cosmids can be used to transform or transfect suitable host cells. Host cells may also be transformed by introducing into a cell a linear DNA vector(s) containing the desired gene sequence. Such linear DNA may be advantageous when it is desirable to avoid introduction of non-native (foreign) DNA into the cell. For example, DNA consisting of a desired target gene(s) flanked by DNA sequences which are native to the cell can be introduced into the cell by electroporation, lithium acetate transformation, spheroplasting and the like. Flanking DNA sequences can include selectable markers and/or other tools for genetic engineering.

A suitable organic substrate herein can be any organic compound that is biooxidizable to a mono- or polycarboxylic acid. Such a compound can be any saturated or unsaturated aliphatic compound or any carbocyclic or heterocyclic aromatic compound having at least one terminal methyl group, a terminal carboxyl group and/or a terminal functional group which is oxidizable to a carboxyl group by biooxidation. A terminal functional group which is a derivative of a carboxyl group may be present in the substrate molecule and may be converted to a carboxyl group by a reaction other than biooxidation. For example, if the terminal group is an ester that neither the wild-type *C. tropicalis* nor the genetic modifications described herein will allow hydrolysis of the ester functionality to a carboxyl group, then a lipase can be added during the fermentation step to liberate free fatty acids. Suitable organic substrates include, but are not limited to, saturated fatty acids, unsaturated fatty acids, alkanes, alkenes, alkynes and combinations thereof.

Alkanes are a type of saturated organic substrate which are useful herein. The alkanes can be linear or cyclic, branched or straight chain, substituted or unsubstituted. Particularly preferred alkanes are those having from about 4 to about 25 carbon atoms, examples of which include but are not limited to butane, hexane, octane, nonane, dodecane, tridecane, tetradecane, octadecane and the like.

Examples of unsaturated organic substrates which can be used herein include but are not limited to internal olefins such as 2-pentene, 2-hexene, 3-hexene, 9-octadecene and the like; unsaturated carboxylic acids such as 2-hexenoic acid and esters thereof, oleic acid and esters thereof including triglyceryl esters having a relatively high oleic acid content, erucic acid and esters thereof including triglyceryl esters having a relatively high erucic acid content, ricinoleic acid and esters thereof including triglyceryl esters having a relatively high ricinoleic acid content, linoleic acid and esters thereof including triglyceryl esters having a relatively high linoleic acid content; unsaturated alcohols such as 3-hexen-1-ol, 9-octadecen-1-ol and the like; unsaturated aldehydes such as 3-hexen-1-al, 9-octadecen-1-al and the like. In addition to the above, an organic substrate which can be used herein include alicyclic compounds having at least one internal carbon-carbon double bond and at least one terminal methyl group, a terminal carboxyl group and/or a terminal functional group which is oxidizable to a carboxyl group by biooxidation. Examples of such compounds include but are not limited to 3,6-dimethyl, 1,4-cyclohexadiene; 3-methylcyclohexene; 3-methyl-1, 4-cyclohexadiene and the like.

Examples of the aromatic compounds that can be used herein include but are not limited to arenes such as o-, m-, p-xylene; o-, m-, p-methyl benzoic acid; dimethyl pyridine, and the like. The organic substrate can also contain other functional groups that are biooxidizable to carboxyl groups such as an aldehyde or alcohol group. The organic substrate can also contain other functional groups that are not biooxidizable to carboxyl groups and do not interfere with the biooxidation such as halogens, ethers, and the like.

Examples of saturated fatty acids which may be applied to cells incorporating the present *CYP* and *CPR* genes include caproic, enanthic, caprylic, pelargonic, capric, undecylic, lauric, myristic, pentadecanoic, palmitic, margaric, stearic, arachidic, behenic acids and combinations thereof. Examples of unsaturated fatty acids which may be applied to cells incorporating the present *CYP* and *CPR* genes include palmitoleic, oleic, erucic, linoleic, linolenic acids and combinations thereof. Alkanes and fractions of alkanes may be applied which include chain links from C12 to C24 in any combination. An example of a preferred fatty acid mixtures are Emersol® 267 and Tallow, both commercially available from Henkel Chemicals Group, Cincinnati, OH. The typical fatty acid composition of Emersol® 267 and Tallow is as follows:

| | TALLOW | E267 |
|---|---|---|
| C14:0 | 3.5% | 2.4% |
| C14:1 | 1.0% | 0.7% |
| C15:0 | 0.5% | ----- |
| C16:0 | 25.5% | 4.6% |
| C16:1 | 4.0% | 5.7% |
| C17:0 | 2.5% | ----- |
| C17:1 | ----- | 5.7% |
| C18:0 | 19.5% | 1.0% |
| C18:1 | 41.0% | 69.9% |
| C18:2 | 2.5% | 8.8% |
| C18:3 | ----- | 0.3% |
| C20:0 | 0.5% | ----- |
| C20:1 | ----- | 0.9% |

The following examples are meant to illustrate but not to limit the invention. All relevant microbial strains and plasmids are described in Table 1 and Table 2, respectively.

**Table 1.** List of *Escherichia coli* and *Candida tropicalis* strains

| *E. Coli* STRAIN | GENOTYPE | SOURCE |
|---|---|---|
| XL1Blue-MRF' | *endA1, gyrA96, hsdR17, lac$^-$, recA1, relA1, supE44, thi-1,* [F' *lacI$^q$Z M15, proAB,* Tn*10*] | Stratagene, La Jolla, CA |
| BM25.8 | *SupE44, thi (lac-proAB)* [F' *traD36, proAB$^+$, lacI$^q$Z M15*] *λimm434 (kan$^R$)P1 (cam$^R$) hsdR ($r_{k12}$- $m_{k12}$-)* | Clontech, Palo Alto, CA |
| XLOLR | *(mcrA)183 (mcrCB-hsdSMR-mrr)173 endA1 thi-1 recA1 gyrA96 relA1 lac* [F'*proAB lacI$^q$Z M15* Tn*10* (Tet$^r$) Su$^-$ (nonsuppressing λ$^r$(lambda resistant) | Stratagene, La Jolla, CA |

| *C. tropicalis* STRAIN | GENOTYPE | SOURCE |
|---|---|---|
| ATCC20336 | Wild-type | American Type Culture Collection, Rockville, MD |
| ATCC750 | Wild-type | American Type Culture Collection, Rockville, MD |
| ATCC 20962 | *ura3A/ura3B, pox4A::ura3A/pox4B::ura3A, pox5::ura3A/pox5::URA3A* | Henkel |
| H5343 ura- | *ura3A/ura3B, pox4A::ura3A/pox4B::ura3A, pox5::ura3A/pox5::URA3A, ura3-* | Henkel |
| HDC1 | *ura3A/ura3B, pox4A::ura3A/pox4B::ura3A, pox5::ura3A/pox5::URA3A, ura3::URA3A-CYP52A2A* | Henkel |
| HDC5 | *ura3A/ura3B, pox4A::ura3A/pox4B::ura3A, pox5::ura3A/pox5::URA3A, ura3::URA3A-CYP52A3A* | Henkel |
| HDC10 | *ura3A/ura3B, pox4A::ura3A/pox4B::ura3A, pox5::ura3A/pox5::URA3A, ura3::URA3A-CPRB* | Henkel |

| | | |
|---|---|---|
| HDC15 | *ura3A/ura3B, pox4A::ura3A/pox4B::ura3A, pox5::ura3A/pox5::URA3A, ura3::URA3A-CYP52A5A* | Henkel |
| HDC20 | *ura3A/ura3B, pox4A::ura3A/pox4B::ura3A, pox5::ura3A/pox5::URA3A, ura3::URA3A-CYP52A2A + CPR B* (*CYP* and *CPR* have opposite 5' to 3' orientation with respect to each other) | Henkel |
| HDC23 | *ura3A/ura3B, pox4A::ura3A/pox4B::ura3A, pox5::ura3A/pox5::URA3A, ura3::URA3A-CYP52A2A + CPR B* (*CYP* and *CPR* have same 5' to 3' orientation with respect to each other) | Henkel |

Table 2.

| List of plasmids isolated from genomic libraries and constructed for use in gene integrations. | | | | | |
|---|---|---|---|---|---|
| **Plasmid** | **Base vector** | **Insert** | **Insert Size** | **Plasmid size** | **Description** |
| pURAin | pNEB193 | *URA3A* | 1706 bp | 4399 bp | pNEB193 with the *URA3A* gene inserted in the *Asc*I - *PmeI* site, generating a *Pac*I site |
| pURA 2in | pURAin | *CYP52A2A* | 2230 bp | 6629 bp | pURAin containing a PCR *CYP52A2A* allele containing *Pac*I restriction sites |
| pURA REDB in | pURAin | *CPRB* | 3266 bp | 7665 bp | pURAin containing a PCR *CPRB* allele containing *Pac*I restriction sites |
| pHKM1 | pTriplEx | Truncated *CPRA* gene | Approx. 3.8 kb | Approx. 7.4 kb | A truncated *CPRA* gene obtained by first screening library containing the 5' untranslated region and 1.2 kb open reading frame |

Table 2. (continued)

| List of plasmids isolated from genomic libraries and constructed for use in gene integrations. | | | | | |
|---|---|---|---|---|---|
| **Plasmid** | **Base vector** | **Insert** | **Insert Size** | **Plasmid size** | **Description** |
| pHKM4 | PTriplEx | Truncated *CPRA* gene | Approx. 5 kb | Approx. 8.6 kb | A truncated *CPRA* gene obtained by screening second library containing the 3' untranslated region end sequence |
| pHKM9 | pBC-CMV | *CPRB* gene | Approx. 5.3 kb | Approx. 9.8 kb | *CPRB* allele isolated from the third library |
| pHKM11 | pBC-CMV | *CYP52A1A* | Approx. 5 kb | Approx. 9.5 kb | *CYP52A1A* isolated from the third library |
| pHKM12 | pBC-CMV | *CYP52A8A* | Approx. 7.5 kb | Approx. 12 kb | *CYP52A8A* isolated from the third library |
| pHKM13 | pBC-CMV | *CYP52D4A* | Approx. 7.3 kb | Approx. 11.8 kb | *CYP52D4A* isolated from the third library |
| pHKM14 | pBC-CMV | *CYP52A2B* | Approx. 6 kb | Approx. 10.5 kb | *CYP52A2B* isolated from the third library |
| pHKM15 | pBC-CMV | *CYP52A8B* | Approx. 6.6 kb | Approx. 11.1 kb | *CYP52A8B* isolated from the third library |
| pPAL3 | pTriplEx | *CYP52A5A* | *4.4 kb* | Approx. 8.1 kb | *CYP52A5A* isolated from the 1st library |
| pPA5 | pTriplEx | *CYP52A5B* | 4.1 kb | Approx. 7.8 kb | *CYP52A5B* isolated from the 2nd library |
| pPA15 | pTriplEx | *CYP52A2A* | 6.0 kb | Approx. 9.7 kb | *CYP52A2A* isolated from the 2nd library |
| pPA57 | pTriplEx | *CYP52A3A* | 5.5 kb | Approx. 9.2 kb | *CYP52A3A* isolated from the 2nd library |
| pPA62 | pTriplEx | *CYP52A3B* | 6.0 kb | Approx. 9.7 kb | *CYP52A3B* isolated from the 2nd library |

## EXAMPLE 1

### Purification of Genomic DNA from *Candida tropicalis* ATCC 20336

### A. Construction of Genomic Libraries

50 ml of YEPD broth (see Chart) was inoculated with a single colony of C. *tropicalis* 20336 from YEPD agar plate and grown overnight at 30°C. 5 ml of the overnight culture was inoculated into 100 ml of fresh YEPD broth and incubated at 30°C for 4 to 5 hr with shaking. Cells were harvested by centrifugation, washed twice with sterile distilled water and resuspended in 4 ml of spheroplasting buffer (1 M Sorbitol, 50 mM EDTA, 14 mM mercaptoethanol) and incubated for 30 min at 37°C with gentle shaking. 0.5 ml of 2 mg/ml zymolyase (ICN Pharmaceuticals, Inc., Irvine, CA) was added and incubated at 37°C with gentle shaking for 30 to 60 min. Spheroplast formation was monitored by SDS lysis. Spheroplasts were harvested by brief centrifugation (4,000 rpm, 3 min) and were washed once with the spheroplast buffer without mercaptoethanol. Harvested spheroplasts were then suspended in 4 ml of lysis buffer (0.2 M Tris/pH 8.0, 50 mM EDTA, 1% SDS) containing 100 µg/ml RNase (Qiagen Inc., Chatsworth, CA) and incubated at 37°C for 30 to 60 min.

Proteins were denatured and extracted twice with an equal volume of chloroform/isoamyl alcohol (24:1) by gently mixing the two phases by hand inversions. The two phases were separated by centrifugation at 10,000 rpm for 10 min and the aqueous phase containing the high-molecular weight DNA was recovered. To the aqueous layer NaCl was added to a final concentration of 0.2 M and the DNA was precipitated by adding 2 vol of ethanol. Precipitated DNA was spooled with a clean glass rod and resuspended in TE buffer (10 mM Tris/pH 8.0, 1 mM EDTA) and allowed to dissolve overnight at 4°C. To the dissolved DNA, RNase free of any DNase activity (Qiagen Inc., Chatsworth, CA) was added to a final concentration of 50 µg/ml and incubated at 37°C for 30 min. Then protease (Qiagen Inc., Chatsworth, CA) was added to a final concentration of 100 µg/ml and incubated at 55 to 60°C for 30 min. The solution was extracted once with an equal volume of phenol/chloroform/isoamyl alcohol (25:24:1) and once with equal volume of chloroform/isoamyl alcohol (24:1). To the aqueous phase 0.1 vol of 3 M sodium acetate and 2 volumes of ice cold ethanol (200 proof) were added and the high molecular weight DNA was spooled with a glass rod and dissolved in 1 to 2 ml of TE buffer.

### *B*. Genomic DNA Preparation for PCR Amplification of *CYP* and *CPR* Genes

Five 5 ml of YPD medium was inoculated with a single colony and grown at 30°C overnight. The culture was centrifuged for 5 min at 1200 x g. The supematant was removed by aspiration and 0.5 ml of a sorbitol solution (0.9 M sorbitol, 0.1 M Tris-Cl pH 8.0, 0.1 M EDTA) was added to the pellet. The pellet was resuspended by vortexing and 1 µl of 2-mercaptoethanol and 50 µl of a 10 µg/ml zymolyase solution were added to the mixture. The tube was incubated at 37°C for 1 hr on a rotary shaker (200 rpm). The tube was then centrifuged for 5 min at 1200 x g and the supernatant was removed by aspiration. The protoplast pellet was resuspended in 0.5 ml lx TE (10 mM Tris-Cl pH 8.0, 1 mM EDTA) and transferred to a 1.5 ml microcentrifuge tube. The protoplasts were lysed by the addition of 50 µl 10% SDS followed by incubation at 65°C for 20 min. Next, 200 µl of 5M potassium acetate was added and after mixing, the tube was incubated on ice for at least 30 min. Cellular debris was removed by centrifugation at 13,000 x g for 5 min. The supernatant was carefully removed and transferred to a new microfuge tube. The DNA was precipitated by the addition of 1 ml 100% (200 proof) ethanol followed by centrifugation for 5 min at 13,000 x g. The DNA pellet was washed with 1 ml 70 % ethanol followed by centrifugation for 5 min at 13,000 x g. After partially drying the DNA under a vacuum, it was resuspended in 200 µl of 1x TE. The DNA concentration was determined by ratio of the absorbance at 260 nm /280 nm ($A_{260/280}$).

## EXAMPLE 2

### Construction of *Candida tropicalis* 20336 Genomic Libraries

Three genomic libraries of *C. tropicalis* were constructed, two at Clontech Laboratories, Inc., (Palo Alto, CA) and one at Henkel Corporation (Cincinnati, OH).

### A. Clontech Libraries

The first Clontech library was made as follows: Genomic DNA was prepared from C. *tropicalis* 20336 as described above, partially digested with *Eco*RI and size fractionated by gel electrophoresis to eliminate fragments smaller than 0.6 kb. Following size fractionation, several ligations of the *Eco*RI genomic DNA fragments and lambda (λ) TriplEx™ vector (Figure 1) arms with *Eco*RI sticky ends were packaged into λ phage heads under conditions designed to obtain one million independent clones. The second genomic library was constructed as follows: Genomic DNA was digested partially with *Sau3A1* and size fractionated by gel electrophoresis. The DNA fragments were blunt ended using standard protocols as described, e.g., in Sambrook et al, *Molecular Cloning: A Laboratory Manual,* 2ed. Cold Spring Harbor Press, USA (1989), incorporated herein by reference. The strategy was to fill in the *Sau3A1* overhangs with Klenow polymerase (Life Technologies, Grand Island, NY) followed by digestion with S1 nuclease (Life Technologies, Grand Island, NY). After S1 nuclease digestion the fragments were end filled one more time with Klenow polymerase to obtain the final blunt-ended DNA fragments. *Eco*RI linkers were ligated to these blunt-ended DNA fragments followed by ligation into the λTriplEx vector. The resultant library contained approximately 2 X $10^6$ independent clones with an average insert size of 4.5 kb.

### B. Henkel Library

The third genomic library was constructed at Henkel Corporation using λZAP Express™ vector (Stratagene, La Jolla, CA) (Figure 2). Genomic DNA was partially digested with *Sau3A1* and fragments in the range of 6 to 12 kb were purified from an agarose gel after electrophoresis of the digested DNA. These DNA fragments were then ligated to *Bam*HI digested λZAP Express™ vector arms according to manufacturers protocols. Three ligations were set up to obtain approximately 9.8 X $10^5$ independent clones. All three libraries were pooled and amplified according to manufacturer instructions to obtain high-titre (>$10^9$ plaque forming units/ml) stock for long-term storage. The titre of packaged phage library was ascertained after infection of *E. coli* XL1Blue-MRF' cells. *E. coli* XL1Blue-MRF' were grown overnight in either in LB medium or NZCYM (Chart) containing 10 mM $MgSO_4$ and 0.2% maltose at 37°C or 30°C, respectively with shaking. Cells were then centrifuged and resuspended in 0.5 to 1 volume of 10 mM $MgSO_4$. 200 µl of this *E. coli* culture was mixed with several dilutions of packaged phage library and incubated at 37 °C for 15 min. To this mixture 2.5 ml of LB top agarose or NZCYM top agarose (maintained at 60°C) (see Chart) was added and plated on LB agar or NCZYM agar (see Chart) present in 82 mm petri dishes. Phage were allowed to propagate overnight at 37°C to obtain discrete plaques and the phage titre was determined.

## EXAMPLE 3

### Screening of Genomic Libraries

Both λTriplEx™ and λZAP Express™ vectors are phagemid vectors that can be propagated either as phage or plasmid DNA (after conversion of phage to plasmid). Therefore, the genomic libraries constructed in these vectors can be screened either by plaque hybridization (screening of lambda form of library) or by colony hybridization (screening plasmid form of library after phage to plasmid conversion). Both vectors are capable of expressing the cloned genes and the main difference is the mechanism of excision of plasmid from the phage DNA. The cloning site in λTriplEx™ is located within a plasmid which is present in the phage and is flanked by *loxP* site (Figure 1). When λTriplEx™ is introduced into *E. coli* strain BM25.8 (supplied by Clontech), the *Cre* recombinase present in BM25.8 promotes the excision and circularization of plasmid pTriplEx from the phage λTriplEx™ at the *loxP* sites. The mechanism of excision of plasmid pBK-CMV from phage λZAP Express™ is different. It requires the assistance of a helper phage such as ExAssist™ (Stratagene) and an *E. coli* strain such as XLOR (Stratagene). Both pTriplEx and pBK-CMVcan replicate autonomously in *E. coli.*

### A. Screening Genomic Libraries (Plasmid Form)

#### 1) Colony Lifts

A single colony of *E. coli* BM25.8 was inoculated into 5 ml of LB containing 50 µg/ml kanamycin, 10 mM $MgSO_4$ and 0.1% maltose and grown overnight at 31 °C, 250 rpm. To 200 µl of this overnight culture (~ 4 X $10^8$ cells) 1 µl of phage library (2 - 5 X $10^6$ plaque forming units) and 150 µl LB broth were added and incubated at 31°C for 30 min after which 400 µl of LB broth was added and incubated at 31°C, 225 rpm for 1 h. This bacterial culture was diluted and plated on LB agar containing 50 µg/ml ampicillin (Sigma Chemical Company, St. Louis, MO) and kanamycin (Sigma Chemical Company) to obtain 500 to 600 colonies/plate. The plates were incubated at 37°C for 6 to 7 hrs until the colonies became visible. The plates were then stored at 4°C for 1.5 h before placing a Colony/Plaque Screen™ Hybridization Transfer Membrane disc (DuPont NEN Research Products, Boston, MA) on the plate in contact with bacterial colonies. The transfer of colonies to the membrane was allowed to proceed for 3 to 5 min. The membrane was then lifted and placed on a fresh LB agar (see Chart) plate containing 200 µg/ml of chloramphenicol with the side exposed to the bacterial colonies facing up. The plates containing the membranes were then incubated at 37°C overnight in order to allow full development of the bacterial colonies. The LB agar plates from which colonies were initially lifted were incubated at 37°C overnight and stored at 4°C for future use. The following morning the membranes containing bacterial colonies were lifted and placed on two sheets of Whatman 3M (Whatman, Hillsboro, OR) paper saturated with 0.5 N NaOH and left at room temperature (RT) for 3 to 6 min to lyse the cells. Additional treatment of membranes was as described in the protocol provided by NEN Research Products.

#### 2) DNA Hybridizations

Membranes were dried overnight before hybridizing to oligonucleotide probes prepared using a non-radioactive ECL™ 3'-oligolabelling and detection system from Amersham Life Sciences (Arlington Heights, IL). DNA labeling, prehybridization and hybridizations were performed according to manufacturer's protocols. After hybridization, membranes were washed twice at room temperature in 5 X SSC, 0.1% SDS (in a volume equivalent to 2 ml/$cm^2$ of membrane) for 5 min each followed by two washes at 50°C in 1X SSC, 0.1% SDS (in a volume equivalent to 2 ml/$cm^2$ of membrane) for 15 min each. The hybridization signal was then generated and detected with Hyperfilm ECL™ (Amersham) according to manufacturer's protocols. Membranes were aligned to plates containing bacterial colonies from which colony lifts were performed and colonies corresponding to positive signals on X-ray were then isolated and propagated in LB broth. Plasmid DNA's were isolated from these cultures and analyzed by restriction enzyme digestions and by DNA sequencing.

**B. Screening Genomic Libraries (Plaque Form)**

**1) λ Library Plating**

*E. coli* XL1Blue-MRF' cells were grown overnight in LB medium (25 ml) containing 10 mM $MgSO_4$ and 0.2% maltose at 37°C, 250 rpm. Cells were then centrifuged (2,200 x g for 10 min) and resuspended in 0.5 volumes of 10 mM $MgSO_4$. 500 µl of this *E. coli* culture was mixed with a phage suspension containing 25,000 amplified lambda phage particles and incubated at 37°C for 15 min. To this mixture 6.5 ml of NZCYM top agarose (maintained at 60°C) (see Chart) was added and plated on 80 - 100 ml NCZYM agar (see Chart) present in a 150 mm petridish. Phage were allowed to propagate overnight at 37°C to obtain discrete plaques. After overnight growth plates were stored in a refrigerator for 1-2 hr before plaque lifts were performed.

**2) Plaque Lift and DNA Hybridizations**

Magna Lift™ nylon membranes (Micron Separations, Inc., Westborough, MA) were placed on the agar surface in complete contact with λ plaques and transfer of plaques to nylon membranes was allowed to proceed for 5 min at RT. After plaque transfer the membrane was placed on 2 sheets of Whatman 3M™ (Whatman, Hillsboro, OR) filter paper saturated with a 0.5 N NaOH, 1.0 M NaCl solution and left for 10 min at RT to denature DNA. Excess denaturing solution was removed by blotting briefly on dry Whatman 3M paper. Membranes were then transferred to 2 sheets of Whatman 3M™ paper saturated with 0.5 M Tris-HCl (pH 8.0), 1.5 M NaCl and left for 5 min to neutralize. Membranes were then briefly washed in 200 - 500 ml of 2 X SSC, dried by air and baked for 30 - 40 min at 80°C. The membranes were then probed with labelled DNA.

Membranes were prewashed with a 200 - 500 ml solution of 5 X SSC, 0.5% SDS, 1 mM EDTA (pH 8.0) for 1 - 2 hr at 42°C with shaking (60 rpm) to get rid of bacterial debris from the membranes. The membranes were prehybridized for 1 - 2 hr at 42°C with (in a volume equivalent to 0.125 - 0.25 ml/cm$^2$ of membrane) ECL Gold™ buffer (Amersham) containing 0.5 M NaCl and 5% blocking reagent. DNA fragments that were used as probes were purified from agarose gel using a QIAEX II™ gel extraction kit (Qiagen Inc., Chatsworth, CA) according to manufacturers protocol and labeled using an Amersham ECL™ direct nucleic acid labeling kit (Amersham). Labeled DNA (5 - 10 ng/ml hybridization solution) was added to the prehybridized membranes and the hybridization was allowed to proceed overnight. The following day membranes were washed with shaking (60 rpm) twice at 42°C for 20 min each time in (in a volume equivalent to 2 ml/cm$^2$ of membrane) a buffer containing either 0.1 (high stringency) or 0.5 (low stringency) X SSC, 0.4% SDS and 360 g/l urea. This was followed by two 5 min washes at room temperature in (in a volume equivalent to 2 ml/cm$^2$ of membrane) 2 X SSC. Hybridization signals were generated using the ECL™ nucleic acid detection reagent and detected using Hyperfilm ECL™ (Amersham).

Agar plugs which contained plaques corresponding to positive signals on the X-ray film were taken from the master plates using the broad-end of Pasteur pipet. Plaques were selected by aligning the plates with the x-ray film. At this stage, multiple plaques were generally taken. Phage particles were eluted from the agar plugs by soaking in 1 ml SM buffer (Sambrook et al., *supra)* overnight The phage eluate was then diluted and plated with freshly grown *E. coli* XL1Blue-MRF' cells to obtain 100 - 500 plaques per 85 mm NCZYM agar plate. Plaques were transferred to Magna Lift nylon membranes as before and probed again using the same probe. Single well-isolated plaques corresponding to signals on X - ray film were picked by removing agar plugs and eluting the phage by soaking overnight in 0.5 ml SM buffer.

**C. Conversion of λ Clones to Plasmid Form**

The lambda clones isolated were converted to plasmid form for further analysis. Conversion from the plaque to the plasmid form was accomplished by infecting the plaques into *E. coli* strain BM25.8. The *E. coli* strain was grown overnight at 31°C, 250 rpm in LB broth containing 10 mM $MgSO_4$ and 0.2% maltose until the $OD_{600}$ reached 1.1 - 1.4. Ten milliliters of the overnight culture was removed and mixed with 100 µl of 1 M $MgCl_2$. A 200 µl volume of cells was removed, mixed with 150 µl of eluted phage suspension and incubated at 31°C for 30 min. LB broth (400 µl) was added to the tube and incubation was continued at 31 °C for 1 hr with shaking, 250 rpm. 1 - 10 µl of the infected cell suspension was plated on LB agar containing 100 µg/ml ampicillin (Sigma, St. Louis, MO). Well-isolated colonies were picked and grown overnight in 5 ml LB broth containing 100 µg/ml ampicillin at 37°C, 250 rpm. Plasmid DNA was isolated from these cultures and analyzed. To convert the λZAP Express™ vector to plasmid form *E. coli* strains XLlBlue-MRF' and XLOR were used. The conversion was performed according to the manufacturer's (Stratagene) protocols for single-plaque excision.

## EXAMPLE 4

### Transformation of *C. tropicalis* H5343 ura-

### A. Transformation of *C. tropicalis* H5343 by Electroporation

5 ml of YEPD was inoculated with C. *tropicalis* H5343 *ura-* from a frozen stock and incubated overnight on a New Brunswick shaker at 30°C and 170 rpm. The next day, 10 µl of the overnight culture was inoculated into 100 ml YEPD and growth was continued at 30°C, 170 rpm. The following day the cells were harvested at an $OD_{600}$ of 1.0 and the cell pellet was washed one time with sterile ice-cold water. The cells were resuspended in ice-cold sterile 35 % Polyethylene glycol (4,000 MW) to a density of $5x10^8$ cells/ml. A 0.1 ml volume of cells were utilized for each electroporation. The following electroporation protocol was followed: 1.0 µg of transforming DNA was added to 0.1 ml cells, along with 5 µg denatured, sheared calf thymus DNA and the mixture was allowed to incubate on ice for 15 min. The cell solution was then transferred to an ice-cold 0.2 cm electroporation cuvette, tapped to make sure the solution was on the bottom of the cuvette and electroporated. The cells were electroporated using an Invitrogen electroporator (Carlsbad, CA) at 450 Volts, 200 Ohms and 250 µF. Following electroporation, 0.9 ml SOS media (1M Sorbitol, 30% YEPD, 10 mM $CaCl_2$) was added to the suspension. The resulting culture was grown for 1 hr at 30°C, 170 rpm. Following the incubation, the cells were pelleted by centrifugation at 1500 x g for 5 min. The electroporated cells were resuspended in 0.2 ml of 1M sorbitol and plated on synthetic complete media minus uracil (SC - uracil) (Nelson, *supra).* In some cases the electroporated cells were plated directly onto SC - uracil. Growth of transformants was monitored for 5 days. After three days, several transformants were picked and transferred to SC-uracil plates for genomic DNA preparation and screening.

### B. Transformation of *C. tropicalis* Using Lithium Acetate

The following protocol was used to transform C. *tropicalis* in accordance with the procedures described in *Current Protocols in Molecular Biology,* Supplement 5, 13.7.1 (1989), incorporated herein by reference.

5 ml of YEPD was inoculated with *C. tropicalis* H5343 *ura-* from a frozen stock and incubated overnight on a New Brunswick shaker at 30°C and 170 rpm. The next day, 10 µl of the overnight culture was inoculated into 50 ml YEPD and growth was continued at 30°C, 170 rpm. The following day the cells were harvested at an $OD_{600}$ of 1.0. The culture was transferred to a 50 ml polypropylene tube and centrifuged at 1000 X g for 10 min. The cell pellet was resuspended in 10 ml sterile TE (10mM Tris-Cl and 1mM EDTA, pH 8.0). The cells were again centrifuged at 1000 X g for 10 min and the cell pellet was resuspended in 10 ml of a sterile lithium acetate solution [LiAc (0.1 M lithium acetate, 10 mM Tris-Cl, pH 8.0, 1 mM EDTA)]. Following centrifugation at 1000 X g for 10 min., the pellet was resuspended in 0.5 ml LiAc. This solution was incubated for one hour at 30°C while shaking gently at 50 rpm. A 0.1 ml aliquot of this suspension was incubated with 5 µg of transforming DNA at 30°C with no shaking for 30 min. A 0.7 ml PEG solution (40 % wt/vol polyethylene glycol 3340, 0.1 M lithium acetate, 10 mM Tris-Cl, pH 8.0, 1 mM EDTA) was added and incubated at 30°C for 45 min. The tubes were then placed at 42°C for 5 min. A 0.2 ml aliquot was plated on synthetic complete media minus uracil (SC - uracil) (Kaiser et al. *Methods in Yeast Genetics,* Cold Spring Harbor Laboratory Press, USA, 1994, incorporated herein by reference). Growth of transformants was monitored for 5 days. After three days, several transformants were picked and transferred to SC-uracil plates for genomic DNA preparation and screening.

## EXAMPLE 5

### Plasmid DNA Isolation

Plasmid DNA were isolated from *E. coli* cultures using Qiagen plasmid isolation kit (Qiagen Inc., Chatsworth, CA) according to manufacturer's instructions.

## EXAMPLE 6

### DNA Sequencing and Analysis

DNA sequencing was performed at Sequetech Corporation (Mountain View, CA) using Applied Biosystems automated sequencer (Perkin Elmer, Foster City, CA). DNA sequences were analyzed with MacVector and GeneWorks software packages (Oxford Molecular Group, Campbell, CA).

## EXAMPLE 7

### PCR Protocols

PCR amplification was carried out in a Perkin Elmer Thermocycler using the Ampli*Taq*Gold enzyme (Perkin Elmer Cetus, Foster City, CA) kit according to manufacturer's specifications. Following successful amplification, in some cases, the products were digested with the appropriate enzymes and gel purified using QiaexII (Qiagen, Chatsworth, CA) as per manufacturer instructions. In specific cases the Ultma *Taq* polymerase (Perkin Elmer Cetus, Foster City, CA) or the Expand Hi-Fi *Taq* polymerase (Boehringer Mannheim, Indianapolis, IN) were used per manufacturer's recommendations or as defined in Table 3.

Table 3.

| PCR amplification conditions used with different primer combinations. | | | | | |
|---|---|---|---|---|---|
| **PRIMER COMBINATION** | ***Taq*** | **TEMPLATE DENATURING CONDITION** | **ANNEALING TEMP/TIME** | **EXTENSION TEMP/TIME** | **CYCLE Number** |
| 3674-41-1/ 41-2/ 41-4 + 3674-41-4 | Ampli-*Taq* Gold | 94 C/30 sec | 55 C/30 sec | 72 C/1 min | 30 |
| URA Primer 1a<br>URA Primer 1b | Ampli-*Taq* Gold | 95 C/1 min | 70 C/1 min | 72 C/2 min | 35 |
| URA Primer 2a<br>URA Primer 2b | Ampli-*Taq* Gold | 95 C/1 min | 70 C/1 min | 72 C/2 min | 35 |
| *CYP*2A#1<br>*CYP*2A#2 | Ampli-*Taq* Gold | 95 C/1 min | 70 C/1 min | 72 C/2 min | 35 |
| *CYP*3A#1<br>*CYP*3A#2 | Ultma *Taq* | 95 C/1 min | 70 C/1 min | 72 C/1 min | 30 |
| *CPR*B#1<br>*CPR B#2* | Expand Hi-Fi *Taq* | 94 C/15 sec<br>94 C/15 sec | 50 C/30 sec<br>50 C/30 sec | 68 C/3 min<br>68 C/3 min +20 sec/cycle | 10<br>15 |
| *CYP*5A#1<br>*CYP*5A#2 | Expand Hi-Fi *Taq* | 94 C/15 sec<br>94 C/15 sec | 50 C/30 sec<br>50 C/30 sec | 68 C/3 min<br>68 C/3 min +20 sec/cycle | 10<br>15 |

Table 4 below contains a list of primers (SEQ ID NOS: 1-35) used for PCR amplification to construct gene integration vectors or to generate probes for gene detection and isolation.

**Table 4.** Primer table for PCR amplification to construct gene integration vectors, to generate probes for gene isolation and detection and to obtain DNA sequence of constructs. (A- deoxyadenosine triphosphate [dATP], G- deoxyguanosine triphosphate [dGTP], C- deoxycytosine triphosphate [dCTP], T- deoxythymidine triphosphate [dTTP], Y- dCTP or dTTP, R- dATP or dGTP, W- dATP or dTTP, M- dATP or dCTP, N- dATP or dCTP or dGTP or dTTP).

| Target gene(s) | Patent Primer Name | Lab Primer Name | Sequence (5' to 3') | PCR Product Size |
|---|---|---|---|---|
| | | | | |
| *CYP52A2A* | CYP2A#1 | 3659-72M | *CCTTAATTAA*ATGCACGAAGCGGAGATAAAAG (SEQ ID NO: 1) | 2230 bp |
| | CYP2A#2 | 3659-72N | *CCTTAATTAA*GCATAAGCTTGCTCGAGTCT (SEQ ID NO: 2) | |
| | | | | |
| *CYP52A3A* | CYP3A#1 | 3659-72O | *CCTTAATTAA*ACGCAATGGGAACATGGAGTG (SEQ ID NO: 3) | 2154 bp |
| | CYP3A#2 | 3659-72P | *CCTTAATTAA*TCGCACTACGGTTATTGGTATCAG (SEQ ID NO: 4) | |
| | | | | |
| *CYP52A5A* | CYP5A#1 | 3659-72K | *CCTTAATTAA*TCAAAGTACGTTCAGGCGG (SEQ ID NO: 5) | 3298 bp |
| | CYP5A#2 | 3659-72L | *CCTTAATTAA*GGCAGACAACAACTTGGCAAAGTC (SEQ ID NO: 6) | |
| | | | | |
| *CPRB* | CPRB#1 | 3698-20A | *CCTTAATTAA*GAGGTCGTTGGTTGAGTTTTC (SEQ ID NO: 7) | 3266 bp |
| | CPRB#2 | 3698-20B | *CCTTAATTAA*TTGATAATGACGTTGCGGG (SEQ ID NO: 8) | |
| | | | | |
| *URA3A* | URA Primer 1a | 3698-7C | *AGGCGCGCC*GGAGTCCAAAAAGACCAACCTCTG (SEQ ID NO: 9) | 956 bp |
| | URA Primer 1b | 3698-7D | *CCTTAATTAA*TACGTGGATACCTTCAAGCAAGTG (SEQ ID NO: 10) | |
| | | | | |

| | | | | |
|---|---|---|---|---|
| *URA3A* | URA Primer 2a | 3698-7A | *CCTTAATTAA*GCTCACGAGTTTTGGGA TTTTCGAG (SEQ ID NO: 11) | 750 bp |
| | URA Primer 2b | 3698-7B | *GGGTTTAAAC*CGCAGAGGTTGGTCTT TTTGGACTC (SEQ ID NO: 12) | |
| | | | | |
| | | | *GGGTTTAAAC* - *Pme* I restriction site (SEQ ID NO: 13) | |
| | | | *AGGCGCGCC* - *Asc*I restriction site (SEQ ID NO: 14) | |
| | | | *CCTTAATTAA* - *Pac*I restriction site (SEQ ID NO: 15) | |
| | | | | |
| *CPR* | FMN1 | 3674-41-1 | TCYCAAACWGGTACWGCWGAA (SEQ ID NO: 16) | |
| *CPR* | FMN2 | 3674-41-2 | GGTTTGGGTAAYTCWACTTAT (SEQ ID NO: 17) | |
| *CPR* | FAD | 3674-41-3 | CGTTATTAYTCYATTTCTTC (SEQ ID NO: 18) | |
| *CPR* | NADPH | 3674-41-4 | GCMACACCRGTACCTGGACC (SEQ ID NO: 19) | |
| *CPR* | PRK1.F3 | PRK1.F3 | ATCCCAATCGTAATCAGC (SEQ ID NO: 20) | |
| *CPR* | PRK1.F5 | PRK1.F5 | ACTTGTCTTCGTTTAGCA (SEQ ID NO: 21) | |
| *CPR* | PRK4.R20 | PRK4.R20 | CTACGTCTGTGGTGATGC (SEQ ID NO: 22) | |
| *CYP* | UCup1 | UCup1 | CGNGAYACNACNGCNGG (SEQ ID NO: 23) | |
| *CYP* | UCup2 | UCup2 | AGRGAYACNACNGCNGG (SEQ ID NO: 24) | |
| *CYP* | UCdown1 | UCdown1 | AGNGCRAAYTGYTGNCC (SEQ ID NO: 25) | |
| *CYP* | UCdown2 | UCdown2 | YAANGCRAAYTGYTGNCC (SEQ ID NO: 26) | |
| *CYP* | HemeB1 | HemeB1 | ATTCAACGGTGGTCCAAGAATCTGTT TGG (SEQ ID NO: 27) | |
| *CYP* | 2,3,5P | 2,3,5P | GAGCTATGTTGAGACCACAGTTTGC (SEQ ID NO: 28) | |
| *CYP* | 2,3,5M | 2,3,5M | CTTCAGTTAAAGCAAATTGTTTGGCC (SEQ ID NO: 29) | |
| pTriplEx vector | Triplex5' | Triplex5' | CTCGGGAAGCGCGCCATTGTGTTGG (SEQ ID NO: 30) | |
| pTriplEx vector | Triplex3' | Triplex3' | TAATACGACTCACTATAGGGCGAAT TGGC (SEQ ID NO: 31) | |
| *CYP* | Cyp52a | Cyp52a | TGRYTCAAACCATCTYTCTGG (SEQ ID NO: 32) | |
| *CYP* | Cyp52b | Cyp52b | GGACCGGCGTTAAAGGG (SEQ ID NO: 33) | |
| *CYP* | Cyp52c | Cyp52c | CATAGTCGWATYATGCTTAGACC (SEQ ID NO: 34) | |
| *CYP* | Cyp52d | Cyp52d | GGACCACCATTGAATGG (SEQ ID NO: 35) | |

## EXAMPLE 8

### Yeast Colony PCR Procedure for Confirmation of Gene Integration into the Genome of *C. tropicalis*

Single yeast colonies were removed from the surface of transformation plates, suspended in *50* µl of spheroplasting buffer (50mM KCl, 10mM Tris-HCl, pH 8.3, 1.0 mg/ml Zymolyase, 5% glycerol) and incubated at 37°C for 30 min. Following incubation, the solution was heated for 10 min at 95°C to lyse the cells. Five µl of this solution was used as a template in PCR. Expand Hi-Fi *Taq* polymerase (Boehringer Mannheim, Indianapolis, IN) was used in PCR coupled with a gene-specific primer (gene to be integrated) and a *URA3* primer. If integration did occur, amplification would yield a PCR product of predicted size confirming the presence of an integrated gene.

## EXAMPLE 9

### Fermentation Method for Gene Induction Studies

A fermentor was charged with a semi-synthetic growth medium having the composition 75 g/l glucose (anhydrous), 6.7 g/l Yeast Nitrogen Base (Difco Laboratories), 3 g/l yeast extract, 3 g/l ammonium sulfate, 2 g/l monopotassium phosphate, 0.5 g/l sodium chloride. Components were made as concentrated solutions for autoclaving then added to the fermentor upon cooling: final pH approximately 5.2. This charge was inoculated with 5-10% of an overnight culture of *C. tropicalis* ATCC 20962 prepared in YM medium (Difco Laboratories) as described in the methods of Examples 17 and 20 of US Patent 5,254,466, which is incorporated herein by reference. *C. tropicalis* ATCC 20962 is a POX 4 and POX 5 disrupted C. *tropicalis* ATCC 20336. Air and agitation were supplied to maintain the dissolved oxygen at greater than about 40% of saturation versus air. The pH was maintained at about 5.0 to 8.5 by the addition of 5N caustic soda on pH control. Both a fatty acid feedstream (commercial oleic acid in this example) having a typical composition: 2.4% $C_{14}$; 0.7% $C_{14:1}$; 4.6% $C_{16}$; 5.7% $C_{16:1}$; 5.7% $C_{17:1}$; 1.0% $C_{18}$; 69.9% $C_{18:1}$; 8.8% $C_{18:2}$; 0.30% $C_{18:3}$; 0.90% $C_{20:1}$ and a glucose co-substrate feed were added in a feedbatch mode beginning near the end of exponential growth. Caustic was added on pH control during the bioconversion of fatty acids to diacids to maintain the pH in the desired range. Typically, samples for gene induction studies were collected just prior to starting the fatty acid feed and over the first 10 hours of bioconversion. Determination of fatty acid and diacid content was determined by a standard methyl ester protocol using gas liquid chromatography (GLC). Gene induction was measured using the QC-RT-PCR protocol described in this application.

## EXAMPLE 10

### RNA Preparation

The first step of this protocol involves the isolation of total cellular RNA from cultures of *C. tropicalis.* The cellular RNA was isolated using the Qiagen RNeasy Mini Kit (Qiagen Inc., Chatsworth, CA) as follows: 2 ml samples of *C. tropicalis* cultures were collected from the fermentor in a standard 2 ml screw capped Eppendorf style tubes at various times before and after the addition of the fatty acid or alkane substrate. Cell samples were immediately frozen in liquid nitrogen or a dry-ice/alcohol bath after their harvesting from the fermentor. To isolate total RNA from the samples, the tubes were allowed to thaw on ice and the cells pelleted by centrifugation in a microfuge for 5 minutes (min) at 4°C and the supernatant was discarded while keeping the pellet ice-cold. The microfuge tubes were filled 2/3 full with ice-cold Zirconia/Silica beads (0.5 mm diameter, Biospec Products, Bartlesville, OK) and the tube filled to the top with ice-cold RLT* lysis buffer (* buffer included with the Qiagen RNeasy Mini Kit). Cell rupture was achieved by placing the samples in a mini bead beater (Biospec Products, Bartlesville, OK) and immediately homogenized at full speed for 2.5 min. The samples were allowed to cool in a ice water bath for 1 minute and the homogenization/cool process repeated two more times for a total of 7.5 min homogenization time in the beadbeater. The homogenized cells samples were microfuged at full speed for 10 min and 700 µl of the RNA containing supernatant removed and transferred to a new eppendorf tube. 700 µl of 70% ethanol was added to each sample followed by mixing by inversion. This and all subsequent steps were performed at room temperature. Seven hundred microliters of each ethanol treated sample were transferred to a Qiagen RNeasy spin column, followed by centrifugation at 8,000 x g for 15 sec. The flow through was discarded and the column reloaded with the remaining sample (700 µl) and re-centrifuged at 8,000 x g for 15 sec. The column was washed once with 700 µl of buffer RW1*, and centrifuged at 8,000 x g for 15 sec and the flow through discarded. The column was placed in a new 2 ml collection tube and washed with 500 µl of RPE* buffer and the flow through discarded. The RPE* wash was repeated with centrifugation at 8,000 x g for 2 min and the flow through discarded. The spin column was transferred to a new 1.5 ml collection tube and 100 µl of RNase free water added to the column followed by centrifugation at 8,000 x g for 15 seconds. An additional 75 µl of RNase free water was added to the column followed by centrifugation at 8,000 x g for 2 min. RNA eluted in the water flow through was collected for further purification.

The RNA eluate was then treated to remove contaminating DNA. Twenty microliters of 10X DNase I buffer (0.5 M tris (pH 7.5), 50 mM $CaCl_2$, 100 mM $MgCl_2$), 10 µl of RNase-free DNase I (2 Units/µl, Ambion Inc., Austin, Texas) and 40 units Rnasin (Promega Corporation, Madison, Wisconsin) were added to the RNA sample. The mixture was then incubated at 37°C for 15 to 30 min. Samples were placed on ice and 250 µl Lysis buffer RLT* and 250 µl ethanol (200 proof) added. The samples were then mixed by inversion. The samples were transferred to Qiagen RNeasy spin columns and centrifuged at 8,000 x g for 15 sec and the flow through discarded. Columns were placed in new 2 ml collection tubes and washed twice with 500 µl of RPE* wash buffer and the flow through discarded. Columns were transferred to new 1.5 ml eppendorf tubes and RNA was eluated by the addition of 100 µl of DEPC treated water followed by centrifugation at 8,000 x g for 15 sec. Residual RNA was collected by adding an additional 50 µl of RNase free water to the spin column followed by centrifugation at full speed for 2 min. 10 µl of the RNA preparation was removed and quantified by the ($A_{260/280}$) method. RNA was stored at
-70°C. Yields were found to be 30-100 µg total RNA per 2.0 ml of fermentation broth.

## EXAMPLE 11

### Quantitative Competitive Reverse Transcription Polymerase Chain Reaction (QC-RT-PCR) Protocol

QC-RT-PCR is a technique used to quantitate the amount of a specific RNA in a RNA sample. This technique employs the synthesis of a specific DNA molecule that is complementary to an RNA molecule in the original sample by reverse transcription and its subsequent amplification by polymerase chain reaction. By the addition of various amounts of a competitor RNA molecule to the sample one can determine the concentration of the RNA molecule of interest (in this case the mRNA transcripts of the *CYP* and *CPR* genes). The levels of specific mRNA transcripts were assayed over time in response to the addition of fatty acid and/or alkane substrates to the growth medium of fermentation grown *C. tropicalis* cultures for the identification and characterization of the genes involved in the oxidation of these substrates. This approach can be used to identify the *CYP* and *CPR* genes involved in the oxidation of any given substrate based upon their transcriptional regulation.

### A. Primer Design

The first requirement for QC-RT-PCR is the design of the primer pairs to be used in the reverse transcription and subsequent PCR reactions. These primers need to be unique and specific to the gene of interest. As there is a family of genetically similar *CYP* genes present in *C. tropicalis* 20336, care had to be taken to design primer pairs that would be discriminating and only amplify the gene of interest, in this example the *CYP52A5* gene. In this manner, unique primers directed to substantially non-homologous (aka variable) regions within target members of a gene family are constructed. What constitutes substantially non-homologous regions is determined on a case by case basis. Such unique primers should be specific enough to anneal the non-homologous region of the target gene without annealing to other non-target members of the gene family. By comparing the known sequences of the members of a gene family, non-homologous regions are identified and unique primers are constructed which will anneal to those regions. It is contemplated that non-homologous regions herein would typically exhibit less than about 85% homology but can be more homologous depending on the positions which are conserved and stringency of the reaction. After conducting PCR, it may be helpful to check the reaction product to assure it represents the unique target gene product. If not, the reaction conditions can be altered in terms of stringency to focus the reaction to the desired target. Alternatively a new primer or new non-homologous region can be chosen. Due to the high level of homology between the genes of the *CYP52A* family, the most variable 5 prime region of the *CYP52A5* coding sequence was targeted for the design of the primer pairs. In Figure 3, a portion of the 5 prime coding region for the *CYP52A5A* (SEQ ID NO: 36) allele of *C. tropicalis* 20336 is shown. The boxed sequences in Figure 3 are the sequences of the forward and backwards primers (SEQ ID NOS: 47 and 48) used to quantitate expression of both alleles of this gene. The actual reverse primer (SEQ ID NO: 48) contains one less adenine than that shown in Figure 3. Primers used to measure the expression of specific *C. tropicalis* 20336 genes using the QC-RT-PCR protocol are listed in Table 5 (SEQ ID NOS: 37-58).

**Table 5.** Primer used to measure *C. tropicalis* gene expression in the QC-RT-PCR reactions.

| Primer Name | Direction | Target | Sequence |
|---|---|---|---|
| 3737-89F | F | *CYP52A1A* | CCGATGAAGTTTTCGACGAGTACCC (SEQ ID NO: 37) |
| 3737-89B | B | *CYP52A1A* | AAGGCTTTAACGTGTCCAATCTGGTC (SEQ ID NO: 38) |
| alk2aF1 | F | *CYP52A2A* | ATTATCGCCACATACTTCACCAAATGG (SEQ ID NO: 39) |
| alk2aB5 | B | *CYP52A2A* | CGAGATCGTGGATACGCTGGAGTG (SEQ ID NO: 40) |
| 7581-178-3 | F | *CYP52A3A* | GCCACTCGGTAACTTTGTCAGGGAC (SEQ ID NO: 41) |
| 7581-178-4 | B | *CYP52A3A* | CATTGAACTGAGTAGCCAAAACAGCC (SEQ ID NO: 42) |
| 3737-50F | F | *CYP52A3A & CYP52A3B* | CCTACGTTTGGTATCGCTACTCCGTTG (SEQ ID NO: 43) |
| 3737-50B | B | *CYP52A3A & CYP52A3B* | TTTCCAGCCAGCACCGTCCAAG (SEQ ID NO: 44) |
| 3737-175F | F | *CYP52D4A* | GCAGAGCCGATCTATGTTGCGTCC (SEQ ID NO: 45) |
| 3737-175B | B | *CYP52D4A* | TCATTGAATGCTTCCAGGAACCTCG (SEQ ID NO: 46) |
| 7581-97-F | F | *CYP52A5A& CYP52A5B* | AAGAGGGCAGGGCTCAAGAG (SEQ ID NO: 47) |
| 7581-97-M | B | *CYP52A5A& CYP52A5B* | TCCATGTGAAGATCCCATCAC (SEQ ID NO: 48) |
| 4P-2 | F | *CYP52A8A* | CTTGAAGGCCGTGTTGAACG (SEQ ID NO: 49) |
| 4M-1 | B | *CYP52A8A* | CAGGATTTGTCTGAGTTGCCG (SEQ ID NO: 50) |
| 3737-52F | F | *POX4A & POX4B* | CCATTGCCTTGAGATACGCCATTGGTAG (SEQ ID NO: 51) |
| 3737-52B | B | *POX4A & POX4B* | AGCCTTGGTGTCGTTCTTTTCAACGG (SEQ ID NO: 52) |
| 3737-53F | F | *POX5A* | TTGGGTTTGTTTGTTTCCTGTGTCCG (SEQ ID NO: 53) |
| 3737-53B | B | *POX5A* | CCTTTGACCTTCAATCTGGCGTAGACG (SEQ ID NO: 54) |
| F33 | F | *CPRA* | GGTTTGCTGAATACGCTGAAGGTGATG (SEQ ID NO: 55) |
| B63 | B | *CPRA* | TGGAGCTGAACAACTCTCTCGTCTCGG (SEQ ID NO: 56) |
| 3737-133F | F | *CPRA & CPRB* | TTCCTCAACACGGACAGCGG (SEQ ID NO: 57) |
| 3737-133B | B | *CPRA & CPRB* | AGTCAACCAGGTGTGGAACTCGTC (SEQ ID NO: 58) |

F=Forward B=Backward

### B. Design and Synthesis of the Competitor DNA Template

The competitor RNA is synthesized *in vitro* from a competitor DNA template that has the T7 polymerase promoter and preferably carries a small deletion of e.g., about 10 to 25 nucleotides relative to the native target RNA sequence. The DNA template for the *in-vitro* synthesis of the competitor RNA is synthesized using PCR primers that are between 46 and 60 nucleotides in length. In this example, the primer pairs for the synthesis of the *CYP52A5* competitor DNA are shown in Tables 6 and 7 (SEQ ID NOS: 59 AND 60).

**Table 6.** Forward and Reverse primers used to synthesize the competitor RNA template for the QC-RT-PCR measurement of *CYP52A5A* gene expression.

| | | |
|---|---|---|
| Forward Primer | *CYP52A5*A | GGATCCTAATACGACTCACTATAGGGAGGA AGAGGGCAGGGCTCAAGAG (SEQ ID NO: 59) |
| Reverse Primer | *CYP52A5*A | TCCATGTGAAGATCCCATCACGAGTGTGCC TCTTGCCCAAAG (SEQ ID NO: 60) |

**Table 7.** Primers for the synthesis of the QC-RT-PCR competitor RNA templates

| Primer Name | Direction | Target | Sequence 5'-3' |
|---|---|---|---|
| 3737-89C | F | *CYP52A1A* | GGATCCTAATACGACTCACTATAGGGAGGCCGATG AAGTTTTCGACGAGTACCC (SEQ ID NO: 61) |
| 3737-89D | B | *CYP52A1A* | AAGGCTTTAACGTGTCCAATCTGGTC AACATAGCTCTGGAGTGCTTCCAACC (SEQ ID NO: 62) |
| 7581-137-A | F | *CYP52A2A* | GGATCCTAATACGACTCACTATAGGGAGGATTATC GCCACATACTTCACCAAATGG (SEQ ID NO: 63) |
| 7581-137-B | B | *CYP52A2A* | CGAGATCGTGGATACGCTGGAGTGCGTCGCTCTTC TTCTTCAACAATTCAAG (SEQ ID NO: 64) |
| 7581-137-D | B | *CYP52A3A* | CATTGAACTGAGTAGCCAAAACAGCCCATGGTTTC AATCAATGGGAGGC (SEQ ID NO: 65) |
| 7581-137-C | F | *CYP52A3A* | GGATCCTAATACGACTCACTATAGGGAGGGCCACT CGGTAACTTTGTCAGGGAC (SEQ ID NO: 66) |

| | | | |
|---|---|---|---|
| 3737-50-D | F | *CYP52A3A & CYP52A3B* | GGATCCTAATACGACTCACTATAGGGAGGCCTACG TTTGGTATCGCTACTCCGTTG (SEQ ID NO: 67) |
| 3737-50-C | B | *CYP52A3A & CYP52A3B* | TTTCCAGCCAGCACCGTCCAAGCAACAAGGAGTAC AAGAAATCGTGTC (SEQ ID NO: 68) |
| 3737-175C | F | *CYP52D4A* | GGATCCTAATACGACTCACTATAGGGAGGGCAGAG CCGATCTATGTTGCGTCC (SEQ ID NO: 69) |
| 3737-175D | B | *CYP52D4A* | TCATTGAATGCTTCCAGGAACCTCGCCACATCCATC GAGAACCGG (SEQ ID NO: 70) |
| 7581-97-A | F | *CYP52A5A & CYP52A5B* | GGATCCTAATACGACTCACTATAGGGAGGAAGAGG GCAGGGCTCAAGAG (SEQ ID NO: 59) |
| 7581-97-B | B | *CYP52A5A & CYP52A5B* | TCCATGTGAAGATCCCATCACGAGTGTGCCTCTTGC CCAAAG (SEQ ID NO: 60) |
| 4P-2/T7 | F | *CYP52A8A* | GGATCCTAATACGACTCACTATAGGGAGGCTTGAA GGCCGTGTTGAACG (SEQ ID NO: 71) |
| 4M-3/4M-1 | B | *CYP52A8A* | CAGGATTTGTCTGAGTTGCCGCCTGATCAAGATAG GATCCTTGCCG (SEQ ID NO: 72) |
| 3737-26-D | F | *CPRA* | GGATCCTAATACGACTCACTATAGGGAGGGGTTTG CTGAATACGCTGAAGGTGATG (SEQ ID NO: 73) |
| 3737-26-C | B | *CPRA* | TGGAGCTGAACAACTCTCTCGTCTCGGGTGGTCGA ATGGACCCTTGGTCAAG (SEQ ID NO: 74) |
| 3737-133C | F | *CPRA & CPRB* | GGATCCTAATACGACTCACTATAGGGAGGTTCCTC AACACGGACAGCGG (SEQ ID NO: 75) |
| 3737-133D | B | *CPRA & CPRB* | AGTCAACCAGGTGTGGAACTCGTCGGTGGCAACAA TGAAAAACACCAAG (SEQ ID NO: 76) |
| 3737-52-C | F | *POX4A & POX4B* | GGATCCTAATACGACTCACTATAGGGAGGCCATTG CCTTGAGATACGCCATTGGTAG (SEQ ID NO: 77) |
| 3737-52-D | B | *POX4A & POX4B* | AGCCTTGGTGTCGTTCTTTTCAACGGAAGGTGGTCT CGATGGTGTGTTCAACC (SEQ ID NO: 78) |
| 3737-53-C | F | *POX5A* | GGATCCTAATACGACTCACTATAGGGAGGTTGGGT TTGTTTGTTTCCTGTGTCCG (SEQ ID NO: 79) |
| 3737-53-D | B | *POX5A* | CCTTTGACCTTCAATCTGGCGTAGACGCAGCACCA CCGATCCACCACTTG (SEQ ID NO: 80) |

F=Forward B=Backword

The forward primer (SEQ ID NO: 59) contains the T7 promoter consensus sequence "GGATCCTAATACGA CTCACTATAGGG AGG" fused to the primer 7581-97-F sequence (SEQ ID NO: 47). The Reverse Primer (SEQ ID NO: 60) contains the sequence of primer 7581-97M (SEQ ID NO: 48) followed by the 20 bases of upstream sequence with a 18 base pair deletion between the two blocks of the *CYP52A5* sequence. The forward primer was used with the corresponding reverse primer to synthesize the competitor DNA template. The primer pairs were combined in a standard *Taq* Gold polymerase PCR reaction according to the manufacturer's recommended conditions (Perkin-Elmer/Applied Biosystems, Foster City, CA). The PCR reaction mix contained a final concentration of 250 nM each primer and 10 ng C. *tropicalis* chromosomal DNA for template. The reaction mixture was placed in a thermocycler for 25 to 35 cycles using the highest annealing temperature possible during the PCR reactions to assure a homogeneous PCR product (in this case 62°C). The PCR products were either gel purified or filtered purified to remove un-incorporated nucleotides and primers. The competitor template DNA was then quantified using the ($A_{260/280}$) method. Primers used in QC-RT-PCR experiments for the synthesis of various competitive DNA templates are listed in Table 7 (SEQ ID NOS: 61-80).

### C. Synthesis of the Competitor RNA

Competitor template DNA was transcribed *In-Vitro* to make the competitor RNA using the Megascript T7 kit from Ambion Biosciences (Ambion Inc., Austin, Texas). 250 nanograms (ng) of competitor DNA template and the *in-vitro* transcription reagents are mixed according to the directions provided by the manufacturer. The reaction mixture was incubated for 4 hours at 37°C. The resulting RNA preparations were then checked by gel electrophoresis for the conditions giving the highest yields and quality of competitor RNA. This often required optimization according to the manufacturer's specifications. The DNA template was then removed using DNase I as described in the Ambion kit. The RNA competitor was then quantified by the ($A_{260/280}$) method. Serial dilution's of the RNA (1 ng/µl to 1 femtogram (fg)/µl) were made for use in the QC-RT-PCR reactions and the original stocks stored at -70°C.

### D. QC-RT-PCR Reactions

QC-RT-PCR reactions were performed using rTth polymerase from Perkin-Elmer(Perkin-Elmer/Applied Biosystems, Foster City, CA) according to the manufacturer's recommended conditions. The reverse transcription reaction was performed in a 10 µl volume with a final concentrations of 200 µM for each dNTP, 1.25 units rTth polymerase, 1.0 mM $MnCl_2$, 1X of the 10X buffer supplied with the Enzyme from the manufacturer, 100 ng of total RNA isolated from a fermentor grown culture of *C. tropicalis* and 1.25 µM of the appropriate reverse primer. To quantitate *CYP52A5* expression in *C. tropicalis* an appropriate reverse primer was 7581-97M (SEQ ID NO: 48). Several reaction mixes were prepared for each RNA sample characterized. To quantitate *CYP52A5* expression a series of 8 to 12 of the previously described QC-RT-PCR reaction mixes were aliquoted to different reaction tubes. To each tube 1 µl of a serial dilution containing from 100 pg to 100 fg *CYP52A5* competitor RNA per µl was added bringing the final reaction mixtures up to the final volume of 10 µl. The QC-RT-PCR reaction mixtures were mixed and incubated at 70°C for 15 min according to the manufacturer's recommended times for reverse transcription to occur. At the completion of the 15 minute incubation, the sample temperature was reduced to 4°C to stop the reaction and 40 µl of the PCR reaction mix added to the reaction to bring the total volume up to 50 µl. The PCR reaction mix consists of an aqueous solution containing 0.3125 µM of the forward primer 7581-97F (SEQ ID NO: 47), 3.125 mM $MgCl_2$ and 1X chelating buffer supplied with the enzyme from Perkin-Elmer. The reaction mixtures were placed in a thermocycler (Perkin-Elmer GeneAmp PCR System 2400, Perkin-Elmer/Applied Biosystems, Foster City, CA ) and the following PCR cycle performed: 94°C for 1 min. followed by 94°C for 10 seconds followed by 58°C for 40 seconds for 17 to 22 cycles. The PCR reaction was completed with a final incubation at 58°C for 2 min followed by 4°C. In some reactions where no detectable PCR products were produced the samples were returned the thermocycler for additional cycles, this process was repeated until enough PCR products were produced to quantify using HPLC. The number of cycles necessary to produce enough PCR product is a function of the amount of the target mRNA in the 100 ng of total cellular RNA. In cultures where the *CYP52A5* gene is highly expressed there is sufficient *CYP52A5* mRNA message present and less PCR cycles (≤17) are required to produce quantifiable amount of PCR product. The lower the concentrations of the target mRNA present the more PCR cycles are required to produce a detectable amount of product. These QC-RT-PCR procedures were applied to all the target genes listed in Table 5 using the respective primers indicated therein.

### E. HPLC Quantification

Upon completion of the QC-RT-PCR reactions the samples were analyzed and quantitated by HPLC. Five to fifteen microliters of the QC-RT-PCR reaction mix was injected into a Waters Bio-Compatible 625 HPLC with an attached Waters 484 tunable detector. The detector was set to measure a wave length of 254 nm. The HPLC contained a Sarasep brand DNASep™ column (Sarasep, Inc., San Jose, CA) which was placed within the oven and the temperature set for 52 °C. The column was installed according to the manufacturer's recommendation of having 30 cm. of heated PEEK tubing installed between the injector and the column. The system was configured with a Sarasep brand Guard column positioned before the injector. In addition, there was a 0.22 µm filter disk just before the column, within the oven. Two Buffers were used to create an elution gradient to resolve and quantitate the PCR products from the QC-RT-PCR reactions. Buffer-A consists of 0.1 M tri-ethyl ammonium acetate (TEAA) and 5% acetonitrile (volume to volume). Buffer-B consists of 0.1 M TEAA and 25% acetonitrile (volume to volume). The QC-RT-PCR samples were injected into the HPLC and the linear gradient of 75% buffer-A/ 25% buffer-B to 45% buffer-A/ 55% B was run over 6 min at a flow rate of 0.85 ml per minute. The QC-RT-PCR product of the competitor RNA being 18 base pairs smaller is eluted from the HPLC column before the QC-RT-PCR product from the *CYP52A5* mRNA(U). The amount of the QC-RT-PCR products are plotted and quantitated with an attached Waters Corporation 745 data module. The log ratios of the amount of *CYP52A5* mRNA QC-RT-PCR product (U) to competitor QC-RT-PCR product (C), as measured by peak areas, was plotted and the amount of competitor RNA required to equal the amount of *CYP52A5* mRNA product determined. In the case of each of the target genes listed in Table 5, the competitor RNA contained fewer base pairs as compared to the native target mRNA and eluted before the native mRNA in a manner similar to that demonstrated by *CYP52A5*. HPLC quantification of the genes was conducted as above.

## EXAMPLE 12

### Evaluation of New Strains in Shake Flasks

The *CYP* and *CPR* amplified strains such as strains HDC10, HDC15, HDC20 and HDC23 (Table 1) and H5343 were evaluated for diacid production in shake flasks. A single colony for each strain was transferred from a YPD agar plate into 5 ml of YPD broth and grown overnight at 30°C, 250 rpm. An inoculum was then transferred into 50 ml of DCA2 medium (Chart) and grown for 24 h at 30°C, 300 rpm. The cells were centrifuged at 5000 rpm for 5 min and resuspended in 50 ml of DCA3 medium (Chart) and grown for 24 h at 30°C, 300 rpm. 3% oleic acid w/v was added after 24 h growth in DCA3 medium and the cultures were allowed to bioconvert oleic acid for 48 h. Samples were harvested and the diacid and monoacid concentrations were analyzed as per the scheme given in Figure 35. Each strain was tested in duplicate and the results shown in Table 8 represent the average value from two flasks.

Table 8.

| Bioconversion of oleic acid by different recombinant strains of *Candida tropicalis* | | |
|---|---|---|
| Strain | Conversion to Oleic diacid (%) | Specific Conversion (g diacid/g biomass |
| H5343 | 41.9 | 0.53 |
| HDC 10-2 | 50.5 | 0.85 |
| HDC 15 | 54.4 | 0.85 |
| HDC 20-1 | 45.1 | 0.72 |
| HDC 20-2 | 45.3 | 0.58 |
| HDC 23-2 | 55.2 | 0.84 |
| HDC 23-3 | 58.8 | 0.89 |

## EXAMPLE 13

### Cloning and Characterization of *C. tropicalis* 20336 Cytochrome P450 Monooxygenase (*CYP*) and Cytochrome P450 NADPH Oxidoreductase *(CPR)* Genes

To clone *CYP* and *CPR* genes several different strategies were employed. Available CYP amino acid sequences were aligned and regions of similarity were observed (Figure 4). These regions corresponded to described conserved regions seen in other cytochrome P450 families (Goeptar et al., *supra* and Kalb et al. *supra).* Proteins from eight eukaryotic cytochrome P450 families share a segmented region of sequence similarity. One region corresponded to the HR2 domain containing the invariant cysteine residue near the carboxyl terminus which is required for heme binding while the other region corresponded to the central region of the I helix thought to be involved in substrate recognition (Figure 4). Degenerate oligonucleotide primers corresponding to these highly conserved regions of the *CYP52* gene family present in *Candida maltosa* and *Candida tropicalis* ATCC 750 were designed and used to amplify DNA fragments of *CYP* genes from *C. tropicalis* 20336 genomic DNA. These discrete PCR fragments were then used as probes to isolate full-length *CYP* genes from the *C. tropicalis* 20336 genomic libraries. In a few instances oligonucleotide primers corresponding to highly conserved regions were directly used as probes to isolate full-length *CYP* genes from genomic libraries. In the case of *CPR* a heterologous probe based upon the known DNA sequence for the *CPR* gene from *C. tropicalis* 750 was used to isolate the *C. tropicalis* 20336 *CPR* gene.

### A. Cloning of the *CPR* Gene from *C. tropicalis* 20336

#### 1) Cloning of the *CPRA* Allele

Approximately 25,000 phage particles from the first genomic library of *C. tropicalis* 20336 were screened with a 1.9 kb *Bam*HI-*Nde*I fragment from plasmid pCU3RED (See Picattagio et al., Bio/Technology 10:894-898 (1992), incorporated herein by reference) containing most of the *C. tropicalis* 750 *CPR* gene. Five clones that hybridized to the probe were isolated and the plasmid DNA from these lambda clones was rescued and characterized by restriction enzyme analysis. The restriction enzyme analysis suggested that all five clones were identical but it was not clear that a complete *CPR* gene was present.

PCR analysis was used to determine if a complete *CPR* gene was present in any of the five clones. Degenerate primers were prepared for highly conserved regions of known *CPR* genes (See Sutter et al., *J. Biol. Chem.* 265: 16428-16436 (1990), incorporated herein by reference) ( Figure 4). Two Primers were synthesized for the FMN binding region (FMN1, SEQ ID NO: 16 and FMN2, SEQ ID NO: 17). One primer was synthesized for the FAD binding region (FAD, SEQ ID NO: 18), and one primer for the NADPH binding region (NADPH, SEQ ID NO: 19) (Table 4). These four primers were used in PCR amplification experiments using as a template plasmid DNA isolated from four of the five clones described above. The FMN (SEQ ID NOS: 16 and 17) and FAD (SEQ ID NO: 18) primers served as forward primers and the NADPH primer (SEQ ID NO: 19) as the reverse primer in the PCR reactions. When different combinations of forward and reverse primers were used, no PCR products were obtained from any of the plasmids. However, all primer combinations amplified expected size products with a plasmid containing the *C. tropicalis* 750 *CPR* gene (positive control). The most likely reason for the failure of the primer pairs to amplify a product, was that all four of clones contained a truncated *CPR* gene. One of the four clones (pHKM1) was sequenced using the Triplex 5' (SEQ ID NO: 30) and the Triplex 3' (SEQ ID NO: 31) primers (Table 4) which flank the insert and the multiple cloning site on the cloning vector, and with the degenerate primer based upon the NADPH binding site described above. The NADPH primer (SEQ ID NO: 19) failed to yield any sequence data and this is consistent with the PCR analysis. Sequences obtained with Triplex primers were compared with *C. tropicalis* 750 *CPR* sequence using the MacVector™ program (Oxford Molecular Group, Campbell, CA). Sequence obtained with the Triplex 3' primer-(SEQ ID NO: 31) showed similarity to an internal sequence of the *C. tropicalis* 750 *CPR* gene confirming that pHKM1 contained a truncated version of a 20336 *CPR* gene. pHKM1 had a 3.8 kb insert which included a 1.2 kb coding region of the *CPR* gene accompanied by 2.5 kb of upstream DNA (Figure 5). Approximately 0.85 kb of the 20336 *CPR* gene encoding the C-terminal portion of the *CPR* protein is missing from this clone.

Since the first Clontech library yielded only a truncated *CPR* gene, the second library prepared by Clontech was screened to isolate a full-length *CPR* gene. Three putative *CPR* clones were obtained. The three clones, having inserts in the range of 5-7 kb, were designated pHKM2, pHKM3 and pHKM4. All three were characterized by PCR using the degenerate primers described above. Both pHKM2 and pHKM4 gave PCR products with two sets of internal primers. pHKM3 gave a PCR product only with the FAD (SEQ ID NO: 18) and NADPH (SEQ ID NO: 19) primers suggesting that this clone likely contained a truncated *CPR* gene. All three plasmids were partially sequenced using the two Triplex primers and a third primer whose sequence was selected from the DNA sequence near the truncated end of the *CPR* gene present in pHKM1. This analysis confirmed that both pHKM2 & 4 have sequences that overlap pHKM1 and that both contained the 3' region of *CPR* gene that is missing from pHKM1. Portions of inserts from pHKM1 and pHKM4 were sequenced and a full-length *CPR* gene was identified. Based on the DNA sequence and PCR analysis, it was concluded that pHKM1 contained the putative promoter region and 1.2 kb of sequence encoding a portion (5' end) of a *CPR* gene. pHKM4 had 1.1 kb of DNA that overlapped pHKM1 and contained the remainder (3' end) of a *CPR* gene along with a downstream untranslated region (Figure 6). Together these two plasmids contained a complete *CPRA* gene with an upstream promoter region. *CPRA* is 4206 nucleotides in length (SEQ ID NO: 81) and includes a regulatory region and a protein coding region (defined by nucleotides 1006-3042) which is 2037 base pairs in length and codes for a putative protein of 679 amino acids (SEQ ID NO: 83) (Figures 13 and 14). In Figure 13, the asterisks denote conserved nucleotides between *CPRA* and *CPRB,* bold denotes protein coding nucleotides, and the start and stop codons are underlined. The *CPRA* protein, when analyzed by the protein alignment program of the GeneWorks™ software package (Oxford Molecular Group, Campbell, CA), showed extensive homology to *CPR* proteins from *C. tropicalis 750* and *C. maltosa.*

**2) Cloning of the *CPRB* Allele**

To clone the second *CPRB* allele, the third genomic library, prepared by Henkel, was screened using DNA fragments from pHKM1 and pHKM4 as probes. Five clones were obtained and these were sequenced with the three internal primers used to sequence *CPRA*. These primers were designated PRK1.F3 (SEQ ID NO: 20), PRK1.F5 (SEQ ID NO: 21) and PRK4.R20 (SEQ ID NO: 22) (Table 4). and the two outside primers (M13 -20 and T3 [Stratagene]) for the polylinker region present in the pBK-CMV cloning vector. Sequence analysis suggested that four of these clones, designated pHKM5 to 8, contained inserts which were identical to the *CPRA* allele isolated earlier. All four seemed to contain a full length *CPR* gene. The fifth clone was very similar to the *CPRA* allele, especially in the open reading frame region where the identity was very high. However, there were significant differences in the 5' and 3' untranslated regions. This suggested that the fifth clone was the allele to *CPRA.* The plasmid was designated pHKM9 (Figure 7) and a 4.14 kb region of this plasmid was sequenced and the analysis of this sequence confirmed the presence of the *CPRB.* allele (SEQ ID NO: 82), which includes a regulatory region and a protein coding region (defined by nucleotides 1033-3069) (Figure 13). The amino acid sequence of the *CPRB* protein is set forth in SEQ ID NO: 84 (Figure 14).

**B. Cloning of C. *tropicalis* 20336 (*CYP*) Genes**

**1) Cloning of *CYP52A2A, CYP52A3A & 3B* and *CYP52A5A & 5B***

Clones carrying *CYP52A2A, A3A, A3B, A5A* and *A5B* genes were isolated from the first and second Clontech genomic libraries using an oligonucleotide probe (HemeB1, SEQ ID NO: 27) whose sequence was based upon the amino acid sequence for the highly conserved heme binding region present throughout the *CYP52* family. The first and second libraries were converted to the plasmid form and screened by colony hybridizations using the HemeB 1 probe (SEQ ID NO: 27) (Table 4). Several potential clones were isolated and the plasmid DNA was isolated from these clones and sequenced using the HemeB1 oligonucleotide (SEQ ID NO: 27) as a primer. This approach succeeded in identifying five *CYP52* genes. Three of the *CYP* genes appeared unique, while the remaining two were classified as alleles. Based upon an arbitrary choice of homology to *CYP52* genes from *Candida maltosa,* these five genes and corresponding plasmids were designated *CYP52A2A* (pPA15 [Figure 26]), *CYP52A3A* (pPA57 [Figure 29]), *CYP52A3B* (pPA62 [Figure 30]), *CYP52A5A* (pPAL3 [Figure 31]) and *CYP52A5B* (pPA5 [Figure 32]). The complete DNA sequence including regulatory and protein coding regions of these five genes was obtained and confirmed that all five were *CYP52* genes (Figure 15). In Figure 15, the asterisks denote conserved nucleotides among the *CYP* genes. Bold indicates the protein coding nucleotides of the *CYP* genes, and the start and stop codons are underlined. The *CYP52A2A* gene as represented by SEQ ID NO: 86 has a protein coding region defined by nucleotides 1199-2767 and the encoded protein has an amino acid sequence as set forth in SEQ ID NO: 96. The *CYP52A3A* gene as represented by SEQ ID NO: 88 has a protein encoding region defined by nucleotides 1126-2748 and the encoded protein has an amino acid sequence as set forth in SEQ ID NO: 98. The *CYP52A3B* gene as represented by SEQ ID NO: 89 has a protein coding defined by nucleotides 913-2535 and the encoded protein has an amino-acid sequence as set forth in SEQ ID NO: 99. The *CYP52A5A* gene as represented by SEQ ID NO: 90 has a protein coding region defined by nucleotides 1103-2656 and the encoded protein has an amino acid sequence as set forth in SEQ ID NO: 100. The *CYP52A5B* gene as represented by SEQ ID NO: 91 has a protein coding region defined by nucleotides 1142-2695 and the encoded protein has an amino acid sequence as set forth in SEQ ID NO: 101.

**2) Cloning of *CYP52A1A* and *CYP52A8A***

*CYP52A1A* and *CYP52A8A* genes were isolated from the third genomic library using PCR fragments as probes. The PCR fragment probe for *CYP52A1* was generated after PCR amplification of 20336 genomic DNA with oligonucleotide primers that were designed to amplify a region from the Helix I region to the HR2 region using all available *CYP52* genes from National Center for Biotechnology Information. Degenerate forward primers UCup1 (SEQ ID NO: 23) and UCup2 (SEQ ID NO: 24) were designed based upon an amino acid sequence (- RDTTAG-) from the Helix I region (Table 4). Degenerate primers UCdownl (SEQ ID NO: 25) and UCdown2 (SEQ ID NO: 26) were designed based upon an amino acid sequence (-GQQFAL) from the HR2 region (Table 4). For the reverse primers, the DNA sequence represents the reverse complement of the corresponding amino acid sequence. These primers were used in pairwise combinations in a PCR reaction with Stoffel *Taq* DNA polymerase (Perkin-Elmer Cetus, Foster City, CA) according to the manufacturer's recommended procedure. A PCR product of approximately 450 bp was obtained. This product was purified from agarose gel using Gene-clean™ (Bio 101, LaJolla, CA) and ligated to the pTAG™ vector (Figure 17) (R&D systems, Minneapolis, MN) according to the recommendations of the manufacturer. No treatment was necessary to clone into pTAG because it employs the use of the TA cloning technique. Plasmids from several transformants were isolated and their inserts were characterized. One plasmid contained the PCR clone intact. The DNA sequence of the PCR fragment (designated *44CYP3,* SEQ ID NO: 107) shared homology with the DNA sequences for the *CYP52A1* gene of *C. maltosa* and the *CYP52A3* gene of C. *tropicalis 750.* This fragment was used as a probe in isolating the *C. tropicalis* 20336 *CYP52A1* homolog. The third genomic library was screened using the *44CYP3* PCR probe (SEQ ID NO: 107) and a clone (pHKM11) that contained a full-length *CYP52* gene was obtained (Figure 8). The clone contained a gene having regulatory and protein coding regions. An open reading frame of 1572 nucleotides encoded a *CYP52* protein of 523 amino acids (Figures 15 and 16 ). This *CYP52* gene was designated *CYP52A1A* (SEQ ID NO: 85) since its putative amino acid sequence (SEQ ID NO: 95) was most similar to the *CYP52A1* protein of *C. maltosa*. The protein coding region of the *CYP52A1A* gene is defined by nucleotides 1177-2748 of SEQ ID NO: 85.

A similar approach was taken to clone *CYP52A8A.* A PCR fragment probe for *CYP52A8* was generated using primers for highly conserved sequences of *CYP52A3, CYP52A2* and *CYP52A5* genes of *C. tropicalis 750.* The reverse primer (primer 2,3,5,M) (SEQ ID NO: 29) was designed based on the highly conserved heme binding region (Table 4). The design of the forward primer (primer 2,3,5,P) (SEQ ID NO: 28) was based upon a sequence conserved near the N-terminus of the *CYP52A3, CYP52A2* and *CYP52A5* genes from *C. tropicalis 750* (Table 4). Amplification of 20336 genomic DNA with these two primers gave a mixed PCR product. One amplified PCR fragment was 1006 bp long (designated DCA1002). The DNA sequence for this fragment was determined and was found to have 85% identity to the DNA sequence for the *CYP52D4* gene of *C. tropicalis* 750. When this PCR product was used to screen the third genomic library one clone (pHKM12) was identified that contained a full-length *CYP52* gene along with 5' and 3' flanking sequences (Figure 9). The *CYP52* gene included regulatory and protein coding regions with an open reading frame of 1539 nucleotides long which encoded a putative CYP52 protein of 512 amino acids (Figures 15 and 16). This gene was designated as *CYP52A8A* (SEQ ID NO: 92) since its amino acid sequence (SEQ ID NO: 102) was most similar to the *CYP52A8* protein of C. *maltosa.* The protein coding region of the *CYP52A8A* gene is defined by nucleotides 464-2002 of SEQ ID NO: 92. The amino acid sequence of the *CYP52A8A* protein is set forth in SEQ ID NO: 102.

### 3) Cloning of *CYP52D4A*

The screening of the second genomic library with the HemeB1 (SEQ ID NO: 27) primer (Table 4) yielded a clone carrying a plasmid (pPA18) that contained a truncated gene having homology with the *CYP52D4* gene of *C. maltosa* (Figure 33). A 1.3 to 1.5-kb *Eco*RI-SstI fragment from pPA18 containing part of the truncated *CYP* gene was isolated and used as a probe to screen the third genomic library for a full length *CYP52* gene. One clone (pHKM13) was isolated and found to contain a full-length *CYP* gene with extensive 5' and 3' flanking sequences (Figure 10). This gene has been designated as *CYP52D4A* (SEQ ID NO: 94) and the complete DNA including regulatory and protein coding regions (coding region defined by nucleotides 767-2266) and putative amino acid sequence (SEQ ID NO: 104) of this gene is shown in Figures 15 and 16. *CYP52D4A* (SEQ ID NO: 94) shares the greatest homology with the *CYP52D4* gene of C. *maltosa.*

### 4) Cloning of *CYP52A2B* and *CYP52A8B*

A mixed probe containing *CYP52A1A, A2A, A3A, D4A, A5A* and *A8A* genes was used to screen the third genomic library and several putative positive clones were identified. Seven of these were sequenced with the degenerate primers Cyp52a (SEQ ID NO: 32), Cyp52b (SEQ ID NO: 33), Cyp52c (SEQ ID NO: 34) and Cyp52d (SEQ ID NO: 35) shown in Table 4. These primers were designed from highly conserved regions of the four *CYP52* subfamilies, namely *CYP52A, B, C & D.* Sequences from two clones, pHKM14 and pHKM15 (Figures 11 and 12), shared considerable homology with DNA sequence of the *C. tropicalis* 20336 *CYP52A2* and *CYP52A8* genes, respectively. The complete DNA (SEQ ID NO: 87) including regulatory and protein coding regions (coding region defined by nucleotides 1072-2640) and putative amino acid sequence (SEQ ID NO: 97) of the *CYP52* gene present in pHKM14 suggested that it is *CYP52A2B* (Figures 15 and 16). The complete DNA (SEQ ID NO: 93) including regulatory and protein coding regions (coding region defined by nucleotides 1017-2555) and putative amino acid sequence (SEQ ID NO: 103) of the *CYP52* gene present in pHKM15 suggested that it is *CYP52A8B* (Figures 15 and 16).

## EXAMPLE 14

### Identification of *CYP* and *CPR* Genes Induced by Selected Fatty Acid and Alkane Substrates

Genes whose transcription is turned on by the presence of selected fatty acid or alkane substrates have been identified using the QC-RT-PCR assay. This assay was used to measure (*CYP*) and (*CPR*) gene expression in fermentor grown cultures *C. tropicalis* ATCC 20962. This method involves the isolation of total cellular RNA from cultures of *C. tropicalis* and the quantification of a specific mRNA within that sample through the design and use of sequence specific QC-RT-PCR primers and an RNA competitor. Quantification is achieved through the use of known concentrations of highly homologous competitor RNA in the QC-RT-PCR reactions. The resulting QC-RT-PCR amplified cDNA's are separated and quantitated through the use of ion pairing reverse phase HPLC. This assay was used to characterize the expression of *CYP52* genes of *C. tropicalis* ATCC 20962 in response to various fatty acid and alkane substrates. Genes which were induced were identified by the calculation of their mRNA concentration at various times before and after induction. Figure 18 provides an example of how the concentration of mRNA for *CYP52A5* can be calculated using the QC-RT-PCR assay. The log ratio of unknown (U) to competitor product (C) is plotted versus the concentration of competitor RNA present in the QC-RT-PCR reactions. The concentration of competitor which results in a log ratio of U/C of zero, represents the point where the unknown messenger RNA concentration is equal to the concentration of the competitor. Figure 18 allows for the calculation of the amount of *CYP52A5* message present in 100 ng of total RNA isolated from cell samples taken at 0, 1, and 2 hours after the addition of Emersol® 267 in a fermentor run. From this analysis, it is possible to determine the concentration of the *CYP52A5* mRNA present in 100 ng of total cellular RNA. In the plot contained in Figure 18 it takes 0.46 pg of competitor to equal the number of mRNA's of *CYP52A5* in 100 ng of RNA isolated from cells just prior (time 0) to the addition of the substrate, Emersol® 267. In cell samples taken at one and two hours after the addition of Emersol® 267 it takes 5.5 and 8.5 pg of competitor RNA, respectively. This result demonstrates that *CYP52A5* (SEQ ID NOS: 90 and 91) is induced more than 18 fold within two hours after the addition of Emersol® 267. This type of analysis was used to demonstrate that *CYP52A5* (SEQ ID NO: 90 and 91) is induced by Emersol® 267. Figure 19 shows the relative amounts of *CYP52A5* (SEQ ID NOS: 90 and 91) expression in fermentor runs with and without Emersol® 267 as a substrate. The differences in the *CYP52A5* (SEQ. ID NOS: 90 and 91) expression patterns are due to the addition of Emersol® 267 to the fermentation medium.

This analysis clearly demonstrates that expression of *CYP52A5* (SEQ ID NOS: 90 and 91) in *C. tropicalis* 20962 is inducible by the addition of Emersol® 267 to the growth medium. This analysis was performed to characterize the expression of *CYP52A2A* (SEQ ID NO: 86), *CYP52A3AB* (SEQ ID NOS: 88 and 89), *CYP52A8A* (SEQ ID NO: 92), *CYP52A1A* (SEQ ID NO: 85), *CYP52D4A* (SEQ ID NO: 94) and *CPRB* (SEQ ID NO: 82) in response to the presence of Emersol® 267 in the fermentation medium (Figure 20). The results of these analysis' indicate, that like the *CYP52A5* gene (SEQ ID NOS: 90 and 91) of C. *tropicalis* 20962, the *CYP52A2A* gene (SEQ ID NO: 86) is inducible by Emersol® 267. A small induction is observed for *CYP52A1A* (SEQ ID NO: 85) and *CYP52A8A* (SEQ ID NO: 92). In contrast, any induction for *CYP52D4A* (SEQ ID NO: 94), *CYP52A3A* (SEQ ID NO: 88), *CYP52A3B* (SEQ ID NO: 89) is below the level of detection of the assay. *CPRB* (SEQ ID NO: 82) is moderately induced by Emersol® 267, four to five fold. The results of these analysis are summarized in Figure 20. Figure 34 provides an example of selective induction of *CYP52A* genes. When pure fatty acid or alkanes are spiked into a fermentor containing *C. tropicalis* 20962 or a derivative thereof, the transcriptional activation of *CYP52A* genes was detected using the QC-RT-PCR assay. Figure 34 shows that pure oleic acid (C18:1) strongly induces *CYP52A2A* (SEQ ID NO: 86) while inducing *CYP52A5* (SEQ ID NOS: 90 and 91). In the same fermentor addition of pure alkane (tridecane) shows strong induction of both *CYP52A2A* (SEQ ID NO: 86) and *CYP52A1A* (SEQ ID NO: 85). However, tridecane did not induce *CYP52A5* (SEQ ID NOS: 90 and 91). In a separate fermentation using ATCC 20962, containing pure octadecane as the substrate, induction of *CYP52A2A, CYP52A5A* and *CYP52A1A* is detected (see Figure 36). The foregoing demonstrates selective induction of particular *CYP* genes by specific substrates, thus providing techniques for selective metabolic engineering of cell strains. For example, if tridecane modification is desired, organisms engineered for high levels of *CYP52A2A* (SEQ ID NO: 86) and *CYP52A1A* (SEQ ID NO: 85) activity are indicated. If oleic acid modification is desired, organisms engineered for high levels of *CYP52A2A* (SEQ ID NO: 86) activity are indicated.

## EXAMPLE 15

### Integration of Selected *CYP* and *CPR* Genes into the Genome of *Candida tropicalis*

In order to integrate selected genes into the chromosome of *C. tropicalis* 20336 or its descendants, there has to be a target DNA sequence, which may or may not be an intact gene, into which the genes can be inserted. There must also be a method to select for the integration event. In some cases the target DNA sequence and the selectable marker are the same and, if so, then there must also be a method to regain use of the target gene as a selectable marker following the integration event. In *C. tropicalis* and its descendants, one gene which fits these criteria is *URA3A,* encoding orotidine-5'-phosphate decarboxylase. Using it as a target for integration, *ura*$^-$ variants of *C. tropicalis* can be transformed in such a way as to regenerate a *URA*$^+$ genotype via homologous recombination (Figure 21). Depending upon the design of the integration vector, one or more genes can be integrated into the genome at the same time. Using a split *URA3A* gene oriented as shown in Figure 22, homologous integration would yield at least one copy of the gene(s) of interest which are inserted between the split portions of the *URA3A* gene. Moreover, because of the high sequence similarity between *URA3A* and *URA3B* genes, integration of the construct can occur at both the *URA3A* and *URA3B* loci. Subsequently, an oligonucleotide designed with a deletion in a portion of the *URA* gene based on the identical sequence across both the *URA3A* and *URA3B* genes, can be utilized to yield *C. tropicalis* transformants which are once again *ura*⁻ but which still carry one or more newly integrated genes of choice (Figure 21). *ura*⁻ variants of *C. tropicalis* can also be isolated via other methods such as classical mutagenesis or by spontaneous mutation. Using well established protocols, selection of *ura*⁻ strains can be facilitated by the use of 5-fluoroorotic acid (5-FOA) as described, e.g., in Boeke et al., *Mol. Gen. Genet.* 197:345-346, (1984), incorporated herein by reference. The utility of this approach for the manipulation of *C. tropicalis* has been well documented as described, e.g., in Picataggio et al., *Mol. and Cell. Biol.* 11:4333-4339 (1991); Rohrer et al., *Appl. Microbiol. Biotechnol.* 36:650-654 (1992); Picataggio et al., *Bio/Technology* 10:894-898 (1992); U.S. Patent No. 5,648,247; U.S. Patent No. 5,620,878; U.S. Patent No. 5,204,252; U.S. Patent No. 5,254,466, all of which are incorporated herein by reference.

### *A.* Construction of a URA Integration Vector, pURAin.

Primers were designed and synthesized based on the 1712 bp sequence of the *URA3A* gene of *C. tropicalis* 20336 (see Figure 23). The nucleotide sequence of the *URA3A* gene of *C. tropicalis* 20336 is set forth in SEQ ID NO: 105 and the amino acid sequence of the encoded protein is set forth in SEQ ID NO: 106. *URA3A* Primer Set #1a (SEQ ID NO: 9) and #1b (SEQ ID NO: 10) (Table 4) was used in PCR with *C. tropicalis* 20336 genomic DNA to amplify *URA3A* sequences between nucleotide 733 and 1688 as shown in Figure 23. The primers are designed to introduce unique 5' *Asc*I and 3' *Pac*I restriction sites into the resulting amplified *URA3A* fragment. *Asc*I and *Pac*I sites were chosen because these sites are not present within *CYP* or *CPR* genes identified to date. *URA3A* Primer Set #2 was used in PCR with *C. tropicalis* 20336 genomic DNA as a template, to amplify *URA3A* sequences between nucleotide 9 and 758 as shown in Figure 23. *URA3A* Primer set #2a (SEQ ID NO: 11) and #2b (SEQ ID NO: 12) (Table 4) was designed to introduce unique 5' *Pac*I and 3' *Pme*I restriction sites into the resulting amplified *URA3A* fragment. The *Pme*I site is also not present within *CYP* and *CPR* genes identified to date. PCR fragments of the *URA3A* gene were purified, restricted with *Asc*I*, Pac*I and *Pme*I restriction enzymes and ligated to a gel purified, QiaexII cleaned *Asc*I-*Pme*I digest of plasmid pNEB 193 (Figure 25) purchased from New England Biolabs (Beverly, MA). The ligation was performed with an equimolar number of DNA termini at 16 °C for 16 hr using T4 DNA ligase (New England Biolabs). Ligations were transformed into *E. coli* XL1-Blue cells (Stratagene, LaJolla, CA) according to manufacturers recommendations. White colonies were isolated, grown, plasmid DNA isolated and digested with *Asc*I-*Pme*I to confirm insertion of the modified *URA3A* into pNEB193. The resulting base integration vector was named pURAin (Figure 24).

### B. Amplification of *CYP52A2A, CYP52A3A, CYP52A5A* and *CPRB* from C. *tropicalis* 20336 Genomic DNA

The genes encoding *CYP52A2A,* (SEQ ID NO: 86) and *CYP52A3A* (SEQ ID NO: 88) from *C. tropicalis* 20336 were amplified from genomic clones (pPA15 and pPA57, respectively) (Figures 26 and 29) via PCR using primers (Primer *CYP* 2A#1, SEQ ID NO: 1 and Primer *CYP* 2A#2, SEQ ID NO: 2 for CYP52A2A) (Primer *CYP* 3A#1, SEQ ID NO: 3 and Primer *CYP* 3A#2, SEQ ID NO: 4 for CYP52A3A) to introduce *Pac*I cloning sites. These PCR primers were designed based upon the DNA sequence determined for *CYP52A2A* (SEQ ID NO: 86) (Figure 15). The Ampli*Taq* Gold PCR kit (Perkin Elmer Cetus, Foster City, CA) was used according to manufacturers specifications. The *CYP52A2A* PCR amplification product was 2,230 base pairs in length , yielding 496 bp of DNA upstream of the *CYP52A2A* start codon and 168 bp downstream of the stop codon for the *CYP52A2A* ORF. The *CYP52A3A* PCR amplification product was 2154 base pairs in length, yielding 437bp of DNA upstream of the *CYP52A3A* start codon and 97bp downstream of the stop codon for the *CYP52A3A ORF*. The *CYP52A3A* PCR amplification product was 2154 base pairs in length, yielding 437bp of DNA upstream of the *CYP52A3A* start codon and 97bp downsteam of the stop codon for the *CYP52A3A* ORF.

The gene encoding *CYP52A5A* (SEQ ID NO: 90) from C. *tropicalis* 20336 was amplified from genomic DNA via PCR using primers (Primer *CYP* 5A#1, SEQ ID NO: 5 and Primer *CYP* 5A#2, SEQ ID NO: 6) to introduce *Pac*I cloning sites. These PCR primers were designed based upon the DNA sequence determined for *CYP52A5A* (SEQ ID NO: 90). The Expand Hi-Fi *Taq* PCR kit (Boehringer Mannheim, Indianapolis, IN) was used according to manufacturers specifications. The *CYP52A5A* PCR amplification product was 3,298 base pairs in length.

The gene encoding *CPRB* (SEQ ID NO: 82) from *C. tropicalis* 20336 was amplified from genomic DNA via PCR using primers (*CPR* B#1, SEQ ID NO: 7 and *CPR B#2,* SEQ ID NO: 8) based upon the DNA sequence determined for *CPRB* (SEQ ID NO: 82) (Figure 13). These primers were designed to introduce unique *Pac*I cloning sites. The Expand Hi-Fi *Taq* PCR kit (Boehringer Mannheim, Indianapolis, IN) was used according to manufacturers specifications. The *CPRB* PCR product was 3266 bp in length, yielding 747 bp pf DNA upstream of the *CPRB* start codon and 493 bp downstream of the stop codon for the *CPRB* ORF. The resulting PCR products were isolated via agarose gel electrophoresis, purified using QiaexII and digested with *Pac*I. The PCR fragments were purified, desalted and concentrated using a Microcon 100 (Amicon, Beverly, MA).

The above described amplification procedures are applicable to the other genes listed in Table 5 using the respectively indicated primers.

**C. Cloning of *CYP* and *CPR* Genes into pURAin.**

The next step was to clone the selected *CYP* and *CPR* genes into the pURAin integration vector. In a preferred aspect of the present invention, no foreign DNA other than that specifically provided by synthetic restriction site sequences are incorporated into the DNA which was cloned into the genome of *C. tropicalis,* i.e., with the exception of restriction site DNA only native *C. tropicalis* DNA sequences are incorporated into the genome. pURAin was digested with *Pac*I*,* Qiaex II cleaned, and dephosphorylated with Shrimp Alkaline Phosphatase (SAP) (United States Biochemical, Cleveland, OH) according the manufacturer's recommendations. Approximately 500 ng of *Pac*I linearized pURAin was dephosphorylated for 1 hr at 37°C using SAP at a concentration of 0.2 Units of enzyme per 1 pmol of DNA termini. The reaction was stopped by heat inactivation at 65°C for 20 min.

The *CYP52A2A Pac*I fragment derived using the primer shown in Table 4 was ligated to plasmid pURAin which had also been digested with *Pac*I. *Pac*I digested pURAin was dephosphorylated, and ligated to the *CYP52A2A* ULTMA PCR product as described previously. The ligation mixture was transformed into *E. coli* XL1 Blue MRF' (Stratagene) and 2 resistant colonies were selected and screened for correct constructs which should contain vector sequence, the inverted *URA3A* gene, and the amplified *CYP52A2A* gene (SEQ ID NO: 86) of 20336. *AscI-Pme*I digestion identified one of the two constructs, plasmid pURA2in, as being correct (Figure 27). This plasmid was sequenced and compared to *CYP52A2A* (SEQ ID NO: 86) to confirm that PCR did not introduce DNA base changes that would result in an amino acid change.

Prior to its use, the *CPRB Pac*I fragment derived using the primers shown in Table 4 was sequenced and compared to *CPRB* (SEQ ID NO: 82) to confirm that PCR did not introduce DNA base pair changes that would result in an amino acid change. Following confirmation, *CPRB* (SEQ ID NO: 82) was ligated to plasmid pURAin which had also been digested with *Pac*I. *Pac*I digested pURAin was dephosphorylated, and ligated to the *CPR* Expand Hi-Fi PCR product as described previously. The ligation mixture was transformed into *E. coli* XL1 Blue MRF' (Stratagene) and several resistant colonies were selected and screened for correct constructs which should contain vector sequence, the inverted *URA3A* gene, and the amplified *CPRB* gene (SEQ ID NO: 82) of 20336. AscI-*Pme*I digestion confirmed a successful construct, pURAREDBin.

In a manner similar to the above, each of the other *CYP* and *CPR* genes disclosed herein are cloned into pURAin. *Pac*I fragments of these genes, whose sequences are given in Figures 13 and 15, are derivable by methods known to those skilled in the art.

**1) Construction of Vectors Used to Generate HDC 20 and HDC 23**

A previously constructed integration vector containing *CPRB* (SEQ ID NO: 82), pURAREDBin, was chosen as the starting vector. This vector was partially digested with *Pac*I and the linearized fragment was gel-isolated. The active *Pac*I was destroyed by treatment with T4 DNA polymerase and the vector was re-ligated. Subsequent isolation and complete digestion of this new plasmid yielded a vector now containing only one active *Pac*I site. This fragment was gel-isolated, dephosphorylated and ligated to the *CYP52A2A Pac*I fragment. Vectors that contain the *CYP52A2A* (SEQ ID NO: 86) and *CPRB* (SEQ ID NO: 82) genes oriented in the same direction, pURAin *CPR* 2A S, as well as opposite directions (5' ends connected), pURAin *CPR* 2A O, were generated.

**D. Confirmation of *CYP* Integration (Figure 21 for Integration Scheme) into the Genome of *C. tropicalis***

Based on the construct, pURA2in, used to transform H5343 *ura*$^-$, a scheme to detect integration was devised. Genomic DNA from transformants was digested with *Dra* III and *Spe* I which are enzymes that cut within the *URA3A,* and *URA3B* genes but not within the integrated *CYP52A2A* gene. Digestion of genomic DNA where an integration had occurred at the *URA3A* or *URA3B* loci would be expected to result in a 3.5 kb or a 3.3 kb fragment, respectively (Figure 28). Moreover, digestion of the same genomic DNA with *Pac*I would yield a 2.2 kb fragment characteristic for the integrated *CYP52A2A* gene (Figure 28). Southern hybridizations of these digests with fragments of the *CYP52A2A* gene were used to screen for these integration events. Intensity of the band signal from the Southern using *Pac*I digestion was used as a measure of the number of integration events, ((i.e. the more copies of the *CYP52A2A* gene (SEQ ID NO: 86) which are present, the stronger the hybridization signal)).

*C. tropicalis* H5343 transformed *URA* prototrophs were grown at 30°C, 170 rpm, in 10 ml SC-uracil media for preparation of genomic DNA. Genomic DNA was isolated by the method described previously. Genomic DNA was digested with *Spe*I and *Dra*III. A 0.95% agarose gel was used to prepare a Southern hybridization blot. The DNA from the gel was transferred to a MagnaCharge nylon filter membrane (MSI Technologies, Westboro, MA) according to the alkaline transfer method of Sambrook et al., *supra.* For the Southern hybridization, a 2.2 kb *CYP52A2A* DNA fragment was used as a hybridization probe. 300 ng of *CYP52A2A* DNA was labeled using a ECL Direct labeling and detection system (Amersham) and the Southern was processed according to the ECL kit specifications. The blot was processed in a volume of 30 ml of hybridization fluid corresponding to 0.125 ml/$cm^2$. Following a prehybridization at 42 °C for 1 hr, 300 ng of *CYP52A2A* probe was added and the hybridization continued for 16 hr at 42°C. Following hybridization, the blots were washed two times for 20 min each at 42 °C in primary wash containing urea. Two 5 min secondary washes at RT were conducted, followed by detection according to directions. The blots were exposed for 16 hours (hr) as recommended.

Integration was confirmed by the detection of a *Spe*I-*Dra*III 3.5 kb fragment from the genomic DNA of the transformants but not with the *C. tropicalis* 20336 control. Subsequently, a *Pac*I digestion of the genomic DNA of the positive transformants, followed by a Southern hybridization using an *CYP52A2A* gene probe, confirmed integration by the detection of a 2.2 kb fragment. The resulting *CYP52A2A* integrated strain was named HDC1 (see Table 1).

In a manner similar to the above, each of the genes contained in the *Pac*I fragments which are described in Section 3c above were confirmed for integration into the genome of *C. tropicalis*.

Transformants generated by transformation with the vectors, pURAin *CPR* 2A S or pURAin CPR 2A O, were analyzed by Southern hybridization for integration of both the *CYP52A2A* (SEQ ID NO: 86) and *CPRB* (SEQ ID NO: 82) genes tandemly. Three strains were generated in which the *CYP52A2A* (SEQ ID NO: 86) and *CPRB* (SEQ ID NO: 82) genes integrated are in the opposite orientation (HDC 20-1, HDC 20-2 and HDC 20-3) and three were generated with the *CYP52A2A* (SEQ ID NO: 86) and *CPRB* (SEQ ID NO: 82) genes integrated in the same orientation (HDC 23-1, HDC 23-2 and HDC 23-3), Table 1.

### E. Confirmation of *CPRB* Integration into H5343 *ura*$^-$

Seven transformants were screened by colony PCR using *CPRB* primer #2 (SEQ ID NO: 8) and a *URA3A*-specific primer. In five of the transformants, successful integration was detected by the presence of a 3899 bp PCR product. This 3899 bp PCR product represents the *CPRB* gene adjacent to the *URA3A* gene in the genome of H5343 thereby confirming integration. The resulting *CPRB* integrated strains were named HDC10-1 and HDC10-2 (see Table 1).

### F. Strain Evaluation.

As determined by quantitative PCR, when compared to parent H5343, HDC10-1 contained three additional copies of the reductase gene and HDC10-2 contained four additional copies of the reductase gene. Evaluations of HDC20-1, HDC20-2 and HDC20-3 based on Southern hybridization data indicates that HDC20-1 contained multiple integrations, i.e., 2 to 3 times that of HDC20-2 or HDC20-3. Evaluations of HDC23-1, HDC23-2, and HDC23-3 based on Southern hybridization data indicates that HDC23-3 contained multiple integrations, i.e., 2 to 3 times that of HDC23-1 or HDC23-2. The data in Table 8 indicates that the integration of components of the ω-hydroxylase complex have a positive effect on the improvement of *Candida tropicalis* ATCC 20962 as a biocatalyst. The results indicate that *CYP52A5A* (SEQ ID NO: 90) is an important gene for the conversion of oleic acid to diacid. Surprisingly, tandem integrations of *CYP* and *CPR* genes oriented in the opposite direction (HDC 20 strains) seem to be less productive than tandem integrations oriented in the same direction (HDC 23 strains), Tables 1 and 8.

# CHART

## Media Composition

LB Broth

| | |
|---|---|
| Bacto Tryptone | 10 g |
| Bacto Yeast Extract | 5 g |
| Sodium Chloride | 10 g |
| Distilled Water | 1,000 ml |

LB Agar

| | |
|---|---|
| Bacto Tryptone | 10 g |
| Bacto Yeast Extract | 5 g |
| Sodium Chloride | 10 g |
| Agar | 15 g |
| Distilled Water | 1,000 ml |

LB Top Agarose

| | |
|---|---|
| Bacto Tryptone | 10 g |
| Bacto Yeast Extract | 5 g |
| Sodium Chloride | 10 g |
| Agarose | 7 g |
| Distilled Water | 1,000 ml |

NZCYM Broth

| | |
|---|---|
| Bacto Casein Digest | 10 g |
| Bacto Casamino Acids | 1 g |
| Bacto Yeast Extract | 5 g |
| Sodium Chloride | 5 g |
| Magnesium Sulfate (anhydrous) | 0.98 g |
| Distilled Water | 1,000 ml |

NZCYM Agar

| | |
|---|---|
| Bacto Casein Digest | 10 g |
| Bacto Casamino Acids | 1 g |
| Bacto Yeast Extract | 5 g |
| Sodium Chloride | 5 g |
| Magnesium Sulfate (anhydrous) | 0.98 g |
| Agar | 15 g |
| Distilled Water | 1,000 ml |

NZCYM Top Agarose

| | |
|---|---|
| Bacto Casein Digest | 10 g |
| Bacto Casamino Acids | 1 g |
| Bacto Yeast Extract | 5 g |
| Sodium Chloride | 5 g |
| Magnesium Sulfate (anhydrous) | 0.98 g |
| Agarose | 7 g |
| Distilled Water | 1,000 ml |

YEPD Broth

| | |
|---|---|
| Bacto Yeast Extract | 10 g |
| Bacto Peptone | 20 g |
| Glucose | 20 g |
| Distilled Water | 1,000 ml |

YEPD Agar*

| | |
|---|---|
| Bacto Yeast Extract | 10 g |
| Bacto Peptone | 20 g |
| Glucose | 20 g |
| Agar | 20 g |
| Distilled Water | 1,000 ml |

SC - uracil*

| | |
|---|---|
| Bacto-yeast nitrogen base without amino acids | 6.7g |
| Glucose | 20g |
| Bacto-agar | 20g |
| Drop-out mix | 2g |
| Distilled water | 1,000ml |

| DCA2 medium | g/l |
|---|---|
| Peptone | 3.0 |
| Yeast Extract | 6.0 |
| Sodium Acetate | 3.0 |
| Yeast Nitrogen Base (Difco) | 6.7 |
| Glucose (anhydrous) | 50.0 |
| Potassium Phosphate (dibasic, trihydrate) | 7.2 |
| Potassium Phosphate (monobasic, anhydrous) | 9.3 |

| DCA3 medium | g/l |
|---|---|
| 0.3 M Phosphate buffer containing, pH 7.5 | |
| Glycerol | 50 |
| Yeast Nitrogen base (Difco) | 6.7 |

Drop-out mix

| | | | |
|---|---|---|---|
| Adenine | 0.5g | Alanine | 2g |
| Arginine | 2g | Asparagine | 2g |
| Aspartic acid | 2g | Cysteine | 2g |
| Glutamine | 2g | Glutamic acid | 2g |
| Glycine | 2g | Histidine | 2g |
| Inositol | 2g | Isoleucine | 2g |
| Leucine | 10g | Lysine | 2g |
| Methionine | 2g | para-Aminobenzoic acid | 0.2g |
| Phenylalanine | 2g | Proline | 2g |
| Serine | 2g | Threonine | 2g |
| Tryptophan | 2g | Tyrosine | 2g |
| Valine | 2g | | |

*See Kaiser et al., Methods in Yeast Genetics, Cold Spring Harbor Laboratory Press, USA (1994), incorporated herein by reference.

Isolated nucleic acid encoding a *CPRA* protein having the amino acid sequence set forth in SEQ ID NO: 83.

Isolated nucleic acid comprising a coding region defined by nucleotides 1006-3042 as set forth in SEQ ID NO: 81.

Isolated nucleic acid as above comprising the nucleotide sequence as set forth in SEQ ID NO: 81.

Isolated protein comprising an amino acid sequence as set forth in SEQ ID NO: 83.

A vector comprising a nucleotide sequence encoding *CPRA* protein including an amino acid sequence as set forth in SEQ ID NO: 83.

A vector as above wherein the nucleotide sequence is set forth in nucleotides 1006-3042 of SEQ ID NO: 81

A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis.*
A host cell as above wherein the *Candida tropicalis* is *Candida tropicalis* 20336.
A host cell as above wherein the *Candida tropicalis* is H5343 ura-.
A method of producing a *CPRA* protein including an amino acid sequence as set forth in SEQ ID NO: 83 comprising:

a) transforming a suitable host cell with a DNA sequence that encodes the protein having the amino acid sequence as set forth in SEQ ID NO: 83; and
b) culturing the cell under conditions favoring the expression of the protein.

The method as above wherein the step of culturing the cell comprises adding an organic substrate to media containing the cell.
Isolated nucleic acid encoding a CPRB protein having the amino acid sequence set forth in SEQ ID NO: 84.
Isolated nucleic acid comprising a coding region defined by nucleotides 1033-3069 as set forth in SEQ ID NO: 82.
Isolated nucleic acid as above comprising the nucleotide sequence as set forth in SEQ ID NO: 82.
Isolated protein comprising an amino acid sequence as set forth in SEQ ID NO: 84.
A vector comprising a nucleotide sequence encoding *CPRB* protein including an amino acid sequence as set forth in SEQ ID NO: 84.
A vector as above wherein the nucleotide sequence is set forth in nucleotides 1033-3069 of SEQ ID NO: 82.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp*.
A host cell as above wherein the *Candida sp.* is *Candida tropicalis*.
A host cell as above wherein the *Candida tropicalis* is *Candida tropicalis* 20336.
A host cell as above wherein the *Candida tropicalis* is H5343 ura-.
A method of producing a *CPRB* protein including an amino acid sequence as set forth in SEQ ID NO: 84 comprising:

a) transforming a suitable host cell with a DNA sequence that encodes the protein having the amino acid sequence as set forth in SEQ ID NO: 84; and
b) culturing the cell under conditions favoring the expression of the protein.

The method as above wherein the step of culturing the cell comprises adding an organic substrate to media containing the cell.
Isolated nucleic acid encoding a *CYP52A1A* protein having the amino acid sequence set forth in SEQ ID NO: 95.
Isolated nucleic acid comprising a coding region defined by nucleotides 1177-2748 as set forth in SEQ ID NO: 85.
Isolated nucleic acid as above comprising the nucleotide sequence as set forth in SEQ ID NO: 85.
Isolated protein comprising an amino acid sequence as set forth in SEQ ID NO: 95.
A vector comprising a nucleotide sequence encoding *CYP52A1A* protein including an amino acid sequence as set forth in SEQ ID NO: 95.
A vector as above wherein the nucleotide sequence is set forth in nucleotides 1177-2748 of SEQ ID NO: 85.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis.*
A host cell as above wherein the *Candida tropicalis* is *Candida tropicalis* 20336.

A host cell as above wherein the *Candida tropicalis* is H5343 ura-.
A method of producing a *CYP52A1A* protein including an amino acid sequence as set forth in SEQ ID NO: 95 comprising:

a) transforming a suitable host cell with a DNA sequence that encodes the protein having the amino acid sequence as set forth in SEQ ID NO: 95; and
b) culturing the cell under conditions favoring the expression of the protein.

The method as above wherein the step of culturing the cell comprises adding an organic substrate to media containing the cell.
Isolated nucleic acid encoding a *CYP52A2A* protein having the amino acid sequence set forth in SEQ ID NO: 96.
Isolated nucleic acid comprising a coding region defined by nucleotides 1199-2767 as set forth in SEQ ID NO: 86.
Isolated nucleic acid as above comprising the nucleotide sequence as set forth in SEQ ID NO: 86.
Isolated protein comprising an amino acid sequence as set forth in SEQ ID NO: 96.
A vector comprising a nucleotide sequence encoding *CYP52A2A* protein including an amino acid sequence as set forth in SEQ ID NO: 96.
A vector as above wherein the nucleotide sequence is set forth in nucleotides 1199-2767 of SEQ ID NO: 86.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis.*
A host cell as above wherein the *Candida tropicalis* is *Candida tropicalis* 20336.
A host cell as above wherein the *Candida tropicalis* is H5343 ura-.
A method of producing a *CYP52A2A* protein including an amino acid sequence as set forth in SEQ ID NO: 96 comprising:

a) transforming a suitable host cell with a DNA sequence that encodes the protein having the amino acid sequence as set forth in SEQ ID NO: 96; and
b) culturing the cell under conditions favoring the expression of the protein.

The method as above wherein the step of culturing the cell comprises adding an organic substrate to media containing the cell.
Isolated nucleic acid encoding a *CYP52A2B* protein having the amino acid sequence set forth in SEQ ID NO: 97.
Isolated nucleic acid comprising a coding region defined by nucleotides 1072-2640 as set forth in SEQ ID NO: 87.
Isolated nucleic acid as above comprising the nucleotide sequence as set forth in SEQ ID NO: 87.
Isolated protein comprising an amino acid sequence as set forth in SEQ ID NO: 97.
A vector comprising a nucleotide sequence encoding *CYP52A2B* protein including an amino acid sequence as set forth in SEQ ID NO: 97.
A vector as above wherein the nucleotide sequence is set forth in nucleotides 1072-2640 of SEQ ID NO: 87.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp*.
A host cell as above wherein the *Candida sp.* is *Candida tropicalis.*
A host cell as above wherein the *Candida tropicalis* is *Candida tropicalis* 20336.
A host cell as above wherein the *Candida tropicalis* is H5343 ura-.
A method of producing a *CYP52A2B* protein including an amino acid sequence as set forth in SEQ ID NO: 97 comprising:

a) transforming a suitable host cell with a DNA sequence that encodes the protein having the amino acid sequence as set forth in SEQ ID NO: 97; and

b) culturing the cell under conditions favoring the expression of the protein.

The method as above wherein the step of culturing the cell comprises adding an organic substrate to media containing the cell.
Isolated nucleic acid encoding a *CYP52A3A* protein having the amino acid sequence set forth in SEQ ID NO: 98.
Isolated nucleic acid comprising a coding region defined by nucleotides 1126-2748 as set forth in SEQ ID NO: 88.
Isolated nucleic acid as above comprising the nucleotide sequence as set forth in SEQ ID NO: 88.
Isolated protein comprising an amino acid sequence as set forth in SEQ ID NO: 98.
A vector comprising a nucleotide sequence encoding *CYP52A3A* protein including an amino acid sequence as set forth in SEQ ID NO: 98.
A vector as above wherein the nucleotide sequence is set forth in nucleotides 1126-2748 of SEQ ID NO: 88.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis*.
A host cell as above wherein the *Candida tropicalis* is *Candida tropicalis* 20336.
A host cell as above wherein the *Candida tropicalis* is H5343 ura-.
A method of producing a *CYP52A3A* protein including an amino acid sequence as set forth in SEQ ID NO: 98 comprising:

a) transforming a suitable host cell with a DNA sequence that encodes the protein having the amino acid sequence as set forth in SEQ ID NO: 98; and
b) culturing the cell under conditions favoring the expression of the protein.

The method as above wherein the step of culturing the cell comprises adding an organic substrate to media containing the cell.
Isolated nucleic acid encoding a *CYP52A3B* protein having the amino acid sequence as set forth in SEQ ID NO: 99.
Isolated nucleic acid comprising a coding region defined by nucleotides 913-2535 as set forth in SEQ ID NO: 89.
Isolated nucleic acid as above comprising the nucleotide sequence as set forth in SEQ ID NO: 89.
Isolated protein comprising an amino acid sequence as set forth in SEQ ID NO: 99.
A vector comprising a nucleotide sequence encoding *CYP52A3B* protein including an amino acid sequence as set forth in SEQ ID NO: 99.
A vector as above wherein the nucleotide sequence is set forth in nucleotides 913-2535 of SEQ ID NO: 89.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis*.
A host cell as above wherein the *Candida tropicalis* is *Candida tropicalis* 20336.
A host cell as above wherein the *Candida tropicalis* is H5343 ura-.
A method of producing a *CYP52A3B* protein including an amino acid sequence as set forth in SEQ ID NO: 99 comprising:

a) transforming a suitable host cell with a DNA sequence that encodes the protein having the amino acid sequence as set forth in SEQ ID NO: 99; and
b) culturing the cell under conditions favoring the expression of the protein.

The method as above wherein the step of culturing the cell comprises adding an organic substrate to media containing the cell.
Isolated nucleic acid encoding a *CYP52A5A* protein having the amino acid sequence set forth in SEQ ID NO: 100.

Isolated nucleic acid comprising a coding region defined by nucleotides 1103-2656 as set forth in SEQ ID NO: 90.
Isolated nucleic acid as above comprising the nucleotide sequence as set forth in SEQ ID NO: 90.
Isolated protein comprising an amino acid sequence as set forth in SEQ ID NO: 100.
A vector comprising a nucleotide sequence encoding *CYP52A5A* protein including an amino acid sequence as set forth in SEQ ID NO: 100.
A vector as above wherein the nucleotide sequence is set forth in nucleotides 1103-2656 OF SEQ ID NO: 90.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis.*
A host cell as above wherein the *Candida tropicalis* is *Candida tropicalis* 20336.
A host cell as above wherein the *Candida tropicalis* is H5343 ura-.
A method of producing a *CYP52A5A* protein including an amino acid sequence as set forth in SEQ ID NO: 100 comprising:

a) transforming a suitable host cell with a DNA sequence that encodes the protein having the amino acid sequence as set forth in SEQ ID NO: 100; and
b) culturing the cell under conditions favoring the expression of the protein.

The method as above wherein the step of culturing the cell comprises adding an organic substrate to media containing the cell.
Isolated nucleic acid encoding a *CYP52A5B* protein having the amino acid sequence as set forth in SEQ ID NO: 101.
Isolated nucleic acid comprising a coding region defined by nucleotides 1142-2695 as set forth in SEQ ID NO: 91.
Isolated nucleic acid as above comprising the nucleotide sequence as set forth in SEQ ID NO: 91.
Isolated protein comprising an amino acid sequence as set forth in SEQ ID NO: 101.
A vector comprising a nucleotide sequence encoding *CYP52A5B* protein including the amino acid sequence as set forth in SEQ ID NO: 101.
A vector as above wherein the nucleotide sequence is set forth in nucleotides 1142-2695 of SEQ ID NO: 91.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp*. is *Candida tropicalis*.
A host cell as above wherein the *Candida tropicalis* is *Candida tropicalis* 20336.
A host cell as above wherein the *Candida tropicalis* is H5343 ura-.
A method of producing a *CYP52A5B* protein including an amino acid sequence as set forth in SEQ ID NO: 101 comprising:

a) transforming a suitable host cell with a DNA sequence that encodes the protein having the amino acid sequence as set forth in SEQ ID NO: 101; and
b) culturing the cell under conditions favoring the expression of the protein.

The method as above wherein the step of culturing the cell comprises adding an organic substrate to media containing the cell.
Isolated nucleic acid encoding a *CYP52A8A* protein having the amino acid sequence set forth in SEQ ID NO: 102.
Isolated nucleic acid comprising a coding region defined by nucleotides 464-2002 as set forth in SEQ ID NO: 92.
Isolated nucleic acid as above comprising the nucleotide sequence as set forth in SEQ ID NO: 92.
Isolated protein comprising an amino acid sequence as set forth in SEQ ID NO: 102.
A vector comprising a nucleotide sequence encoding *CYP52A8A* protein including an amino acid sequence as set forth in SEQ ID NO: 102.

A vector as above wherein the nucleotide sequence is set forth in nucleotides 464-2002 of SEQ ID NO: 92.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis.*
A host cell as above wherein the *Candida tropicalis* is *Candida tropicalis* 20336.
A host cell as above wherein the *Candida tropicalis* is H5343 ura-.
A method of producing a *CYP52A8A* protein including an amino acid sequence as set forth in SEQ ID NO: 102 comprising:

a) transforming a suitable host cell with a DNA sequence that encodes the protein having the amino acid sequence as set forth in SEQ ID NO: 102; and
b) culturing the cell under conditions favoring the expression of the protein.

The method as above wherein the step of culturing the cell comprises adding an organic substrate to media containing the cell.
Isolated nucleic acid encoding a *CYP52A8B* protein having the amino acid sequence set forth in SEQ ID NO: 103.
Isolated nucleic acid comprising a coding region defined by nucleotides 1017-2555 as set forth in SEQ ID NO: 93.
Isolated nucleic acid as above comprising the nucleotide sequence as set forth in SEQ ID NO: 93.
Isolated protein comprising an amino acid sequence as set forth in SEQ ID NO: 103.
A vector comprising a nucleotide sequence encoding *CYP52A8B* protein including an amino acid sequence as set forth in SEQ ID NO: 103.
A vector as above wherein the nucleotide sequence is set forth in nucleotides 1017-2555 of SEQ ID NO: 93.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*
A host cell as above wherein the *Candida sp.* is *Candida tropicalis.*
A host cell as above wherein the *Candida tropicalis* is *Candida tropicalis* 20336.
A host cell as above wherein the *Candida tropicalis* is H5343 ura-.
A method of producing a *CYP52A8B* protein including an amino acid sequence as set forth in SEQ ID NO: 103 comprising:

a) transforming a suitable host cell with a DNA sequence that encodes the protein having the amino acid sequence as set forth in SEQ ID NO: 103; and
b) culturing the cell under conditions favoring the expression of the protein.

The method as above wherein the step of culturing the cell comprises adding an organic substrate to media containing the cell.
Isolated nucleic acid encoding a *CYP52D4A* protein having the amino acid sequence set forth in SEQ ID NO: 104.
Isolated nucleic acid comprising a coding region defined by nucleotides 767-2266 as set forth in SEQ ID NO: 94.
Isolated nucleic acid as above comprising the nucleotide sequence as set forth in SEQ ID NO: 94.
Isolated protein comprising an amino acid sequence as set forth in SEQ ID NO: 104.
A vector comprising a nucleotide sequence encoding *CYP52D4A* protein including an amino acid sequence as set forth in SEQ ID NO: 104.
A vector as above wherein the nucleotide sequence is set forth in nucleotides 767-2266 of SEQ ID NO: 94.
A vector as above wherein the vector is selected from the group consisting of plasmid, phagemid, phage and cosmid.
A host cell transfected or transformed with the nucleic acid as above.
A host cell as above wherein the host cell is a yeast cell.
A host cell as above wherein the yeast cell is a *Candida sp.*

A host cell as above wherein the *Candida sp.* is *Candida tropicalis*.
A host cell as above wherein the *Candida tropicalis* is *Candida tropicalis* 20336.
A host cell as above wherein the *Candida tropicalis* is H5343 ura-.
A method of producing a *CYP52D4A* protein including an amino acid sequence as set forth in SEQ ID NO: 104 comprising:

a) transforming a suitable host cell with a DNA sequence that encodes the protein having the amino acid sequence as set forth in SEQ ID NO: 104; and
b) culturing the cell under conditions favoring the expression of the protein.

The method as above wherein the step of culturing the cell comprises adding an organic substrate to media containing the cell.
A method for discriminating members of a gene family by quantifying the amount of target mRNA in a sample comprising:

a) providing an organism containing a target gene;
b) culturing the organism with an organic substrate which causes upregulation in the activity of the target gene;
c) obtaining a sample of total RNA from the organism at a first point in time;
d) combining at least a portion of the sample of the total RNA with a known amount of competitor RNA to form an RNA mixture, wherein the competitor RNA is substantially similar to the target mRNA but has a lesser number of nucleotides compared to the target mRNA;
e) adding reverse transcriptase to the RNA mixture in a quantity sufficient to form corresponding target DNA and competitor DNA;
f) conducting a polymerase chain reaction in the presence of at least one primer specific for at least one substantially non-homologous region of the target DNA within the gene family, the primer also specific for the competitor DNA;
g) repeating steps (c-f) using increasing amounts of the competitor RNA while maintaining a substantially constant amount of target RNA;
(h) determining the point at which the amount of target DNA is substantially equal to the amount of competitor DNA;
(i) quantifying the results by comparing the ratio of the concentration of unknown target to the known concentration of competitor; and
(j) obtaining a sample of total RNA from the organism at another point in time and repeating steps (d-i).

A method as above wherein the target gene is selected from the group consisting of a *CPR* gene and a *CYP* gene.
A method as above wherein the *CPR* gene is selected from the group consisting of a *CPRA* gene (SEQ ID NO: 81) and a *CPRB* gene (SEQ ID NO: 82).
A method as above wherein the *CYP* gene is selected from the group consisting of *CYP52A1A* gene (SEQ ID NO: 85), *CYP52A2A* gene (SEQ ID NO: 86), *CYP52A2B* gene (SEQ ID NO: 87), *CYP52A3A* gene (SEQ ID NO: 88), *CYP52A3B* gene (SEQ ID NO. 89), *CYP52A5A* gene (SEQ ID NO: 90), *CYP52A5B* gene (SEQ ID NO: 91), *CYP52A8A* gene (SEQ ID NO: 92), *CYP52A8B* gene (SEQ ID NO: 93) and *CYP52D4A* gene (SEQ ID NO: 94).
A method for increasing production of a dicarboxylic acid comprising:

a) providing a host cell having a naturally occurring number of *CPRA* genes;
b) increasing, in the host cell, the number of *CPRA* genes which encode a *CPRA* protein having the amino acid sequence as set forth in SEQ ID NO: 83;
c) culturing the host cell in media containing an organic substrate which upregulates the *CPRA* gene, to effect increased production of dicarboxylic acid.

A method for increasing the production of a *CPRA* protein having an amino acid sequence as set forth in SEQ ID NO: 83 comprising:

a) transforming a host cell having a naturally occurring amount of *CPRA* protein with an increased copy number of a *CPRA* gene that encodes the *CPRA* protein having the amino acid sequence as set forth in SEQ ID NO: 83; and
b) culturing the cell and thereby increasing expression of the protein compared with that of a host cell containing a naturally occurring copy number of the *CPRA* gene.

A method for increasing production of a dicarboxylic acid comprising:

a) providing a host cell having a naturally occurring number of *CPRB* genes;
b) increasing, in the host cell, the number of *CPRB* genes which encode a *CPRB* protein having the amino acid sequence as set forth in SEQ ID NO: 84;
c) culturing the host cell in media containing an organic substrate which upregulates the *CPRB* gene, to effect increased production of dicarboxylic acid.

A method for increasing the production of a *CPRB* protein having an amino acid sequence as set forth in SEQ ID NO: 84 comprising:

a) transforming a host cell having a naturally occurring amount of *CPRB* protein with an increased copy number of a *CPRB* gene that encodes the *CPRB* protein having the amino acid sequence as set forth in SEQ ID NO: 84; and
b) culturing the cell and thereby increasing expression of the protein compared with that of a host cell containing a naturally occurring copy number of the *CPRB* gene.

A method for increasing production of a dicarboxylic acid comprising:

a) providing a host cell having a naturally occurring number of *CYP52A1A* genes;
b) increasing, in the host cell, the number of *CYP52A1A* genes which encode a *CYP52A1A* protein having the amino acid sequence as set forth in SEQ ID NO: 95;
c) culturing the host cell in media containing an organic substrate which upregulates the *CYP52A2A* gene, to effect increased production of dicarboxylic acid.

A method for increasing the production of a *CYP52A1A* protein having an amino acid sequence as set forth in SEQ ID NO: 95 comprising:

a) transforming a host cell having a naturally occurring amount of *CYP52A1A* protein with an increased copy number of a *CYP52A1A* gene that encodes the *CYP52A1A* protein having the amino acid sequence as set forth in SEQ ID NO: 95; and
b) culturing the cell and thereby increasing expression of the protein compared with that of a host cell containing a naturally occurring copy number of the *CYP52A1A* gene.

A method for increasing production of a dicarboxylic acid comprising:

a) providing a host cell having a naturally occurring number of *CYP52A2A* genes;
b) increasing, in the host cell, the number of *CYP52A2A* genes which encode a *CYP52A2A* protein having the amino acid sequence as set forth in SEQ ID NO: 96;
c) culturing the host cell in media containing an organic substrate which upregulates the *CYP52A2A* gene, to effect increased production of dicarboxylic acid.

A method for increasing the production of a *CYP52A2A* protein having an amino acid sequence as set forth in SEQ ID NO: 96 comprising:

a) transforming a host cell having a naturally occurring amount of *CYP52A2A* protein with an increased copy number of a *CYP52A2A* gene that encodes the *CYP52A2A* protein having the amino acid sequence as set forth in SEQ ID NO: 96; and
b) culturing the cell and thereby increasing expression of the protein compared with that of a host cell containing a naturally occurring copy number of the *CYP52A2A* gene.

A method for increasing production of a dicarboxylic acid comprising:

a) providing a host cell having a naturally occurring number of *CYP52A2B* genes;
b) increasing, in the host cell, the number of *CYP52A2B* genes which encode a *CYP52A2B* protein having the amino acid sequence as set forth in SEQ ID NO: 97;
c) culturing the host cell in media containing an organic substrate which upregulates the *CYP52A2B* gene, to effect increased production of dicarboxylic acid.

A method for increasing the production of a *CYP52A2B* protein having an amino acid sequence as set forth in SEQ ID NO: 97 comprising:

a) transforming a host cell having a naturally occurring amount of *CYP52A2B* protein with an increased copy number of a *CYP52A2B* gene that encodes the *CYP52A2B* protein having the amino acid sequence as set forth in SEQ ID NO: 97; and
b) culturing the cell and thereby increasing expression of the protein compared with that of a host cell containing a naturally occurring copy number of the *CYP52A2B* gene.

A method for increasing production of a dicarboxylic acid comprising:

a) providing a host cell having a naturally occurring number of *CYP52A3A* genes;
b) increasing, in the host cell, the number of *CYP52A3A* genes which encode a *CYP52A3A* protein having the amino acid sequence as set forth in SEQ ID NO: 98;
c) culturing the host cell in media containing an organic substrate which upregulates the *CYP52A3A* gene, to effect increased production of dicarboxylic acid.

A method for increasing the production of a *CYP52A3A* protein having an amino acid sequence as set forth in SEQ ID NO: 98 comprising:

a) transforming a host cell having a naturally occurring amount of *CYP52A3A* protein with an increased copy number of a *CYP52A3A* gene that encodes the *CYP52A3A* protein having the amino acid sequence as set forth in SEQ ID NO: 98; and
b) culturing the cell and thereby increasing expression of the protein compared with that of a host cell containing a naturally occurring copy number of the *CYP52A3A* gene.

A method for increasing production of a dicarboxylic acid comprising:

a) providing a host cell having a naturally occurring number of *CYP52A3B* genes;
b) increasing, in the host cell, the number of *CYP52A3B* genes which encode a *CYP52A3B* protein having the amino acid sequence as set forth in SEQ ID NO: 99;
c) culturing the host cell in media containing an organic substrate which upregulates the *CYP52A3B* gene, to effect increased production of dicarboxylic acid.

A method for increasing the production of a *CYP52A3B* protein having an amino acid sequence as set forth in SEQ ID NO: 99 comprising:

a) transforming a host cell having a naturally occurring amount of *CYP52A3B* protein with an increased copy number of a *CYP52A3B* gene that encodes the *CYP52A3B* protein having the amino acid sequence as set forth in SEQ ID NO: 99; and
b) culturing the cell and thereby increasing expression of the protein compared with that of a host cell containing a naturally occurring copy number of the *CYP52A3B* gene.

A method for increasing production of a dicarboxylic acid comprising:

a) providing a host cell having a naturally occurring number of *CYP52A5A* genes;
b) increasing, in the host cell, the number of *CYP52A5A* genes which encode a *CYP52A5A* protein having the amino acid sequence as set forth in SEQ ID NO: 100;
c) culturing the host cell in media containing an organic substrate which upregulates the *CYP52A5A* gene, to effect increased production of dicarboxylic acid.

A method for increasing the production of a *CYP52A5A* protein having an amino acid sequence as set forth in SEQ ID NO: 100 comprising:

a) transforming a host cell having a naturally occurring amount of *CYP52A5A* protein with an increased copy number of a *CYP52A5A* gene that encodes the *CYP52A5A* protein having the amino acid sequence as set forth in SEQ ID NO: 100; and
b) culturing the cell and thereby increasing expression of the protein compared with that of a host cell containing a naturally occurring copy number of the *CYP52A5A* gene.

A method for increasing production of a dicarboxylic acid comprising:

a) providing a host cell having a naturally occurring number of *CYP52A5B* genes;
b) increasing, in the host cell, the number of *CYP52A5B* genes which encode a *CYP52A5B* protein having the amino acid sequence as set forth in SEQ ID NO: 101;
c) culturing the host cell in media containing an organic substrate which upregulates the *CYP52A5B* gene, to effect increased production of dicarboxylic acid.

A method for increasing the production of a *CYP52A5B* protein having an amino acid sequence as set forth in SEQ ID NO: 101 comprising:

a) transforming a host cell having a naturally occurring amount of *CYP52A5B* protein with an increased copy number of a *CYP52A5B* gene that encodes the *CYP52A5B* protein having the amino acid sequence as set forth in SEQ ID NO: 101; and
b) culturing the cell and thereby increasing expression of the protein compared with that of a host cell containing a naturally occurring copy number of the *CYP52A5B* gene.

A method for increasing production of a dicarboxylic acid comprising:

a) providing a host cell having a naturally occurring number of *CYP52A8A* genes;
b) increasing, in the host cell, the number of *CYP52A8A* genes which encode a *CYP52A8A* protein having the amino acid sequence as set forth in SEQ ID NO: 102;
c) culturing the host cell in media containing an organic substrate which upregulates the *CYP52A8A* gene, to effect increased production of dicarboxylic acid.

A method for increasing the production of a *CYP52A8A* protein having an amino acid sequence as set forth in SEQ ID NO: 102 comprising:

a) transforming a host cell having a naturally occurring amount of *CYP52A8A* protein with an increased copy number of a *CYP52A8A* gene that encodes the *CYP52A8A* protein having the amino acid sequence as set forth in SEQ ID NO: 102; and
b) culturing the cell and thereby increasing expression of the protein compared with that of a host cell containing a naturally occurring copy number of the *CYP52A8A* gene.

A method for increasing production of a dicarboxylic acid comprising:

a) providing a host cell having a naturally occurring number of *CYP52A8B* genes;
b) increasing, in the host cell, the number of *CYP52A8B* genes which encode a *CYP52A8B* protein having the amino acid sequence as set forth in SEQ ID NO: 103;
c) culturing the host cell in media containing an organic substrate which upregulates the *CYP52A8B* gene, to effect increased production of dicarboxylic acid.

A method for increasing the production of a *CYP52A8B* protein having an amino acid sequence as set forth in SEQ ID NO: 103 comprising:

a) transforming a host cell having a naturally occurring amount of *CYP52A8B* protein with an increased copy number of a *CYP52A8B* gene that encodes the *CYP52A8B* protein having the amino acid sequence as set forth in SEQ ID NO: 103; and
b) culturing the cell and thereby increasing expression of the protein compared with that of a host cell containing a naturally occurring copy number of the *CYP52A8B* gene.

A method for increasing production of a dicarboxylic acid comprising:

a) providing a host cell having a naturally occurring number of *CYP52D4A* genes;
b) increasing, in the host cell, the number of *CYP52D4A* genes which encode a *CYP52D4A* protein having the amino acid sequence as set forth in SEQ ID NO: 104;
c) culturing the host cell in media containing an organic substrate which upregulates the *CYP52D4A* gene, to effect increased production of dicarboxylic acid.

A method for increasing the production of a *CYP52D4A* protein having an amino acid sequence as set forth

in SEQ ID NO: 104 comprising:

a) transforming a host cell having a naturally occurring amount of *CYP52D4A* protein with an increased copy number of a *CYP52D4A* gene that encodes the *CYP52D4A* protein having the amino acid sequence as set forth in SEQ ID NO: 104; and
b) culturing the cell and thereby increasing expression of the protein compared with that of a host cell containing a naturally occurring copy number of the *CYP52D4A* gene.

A method for discriminating members of a gene family as above wherein culturing the organism with the organic substrate is accomplished in a fermentor.

## Claims

1. Isolated nucleic acid encoding a *CYP52A3A* protein having the amino acid sequence set forth in SEQ ID NO: 98.

2. Isolated nucleic acid comprising a coding region defined by nucleotides 1126-2748 as set forth in SEQ ID NO: 88.

3. Isolated nucleic acid according to claim 2 comprising the nucleotide sequence as set forth in SEQ ID NO: 88.

4. Isolated protein comprising an amino acid sequence as set forth in SEQ ID NO: 98.

5. A vector comprising a nucleotide sequence encoding *CYP52A3A* protein including an amino acid sequence as set forth in SEQ ID NO: 98.

6. A vector according to claim 5 wherein the nucleotide sequence is set forth in nucleotides 1126-2748 of SEQ ID NO: 88.

7. A vector according to claim 5 wherein the vector is selected from the group consisting of plasmid, phagemid, phage and cosmid.

8. A host cell transfected or transformed with the nucleic acid of claim 1.

9. A host cell according to claim 8 wherein the host cell is a yeast cell.

10. A host cell according to claim 9 wherein the yeast cell is a *Candida sp.*

11. A host cell according to claim 10 wherein the *Candida sp.* is *Candida tropicalis*.

12. A host cell according to claim 11 wherein the *Candida tropicalis* is *Candida tropicalis* 20336.

**13.** A host cell according to claim 12 wherein the *Candida tropicalis* is H5343 ura-.

**14.** A method of producing a *CYP52A3A* protein including an amino acid sequence as set forth in SEQ ID NO: 98 comprising:

a) transforming a suitable host cell with a DNA sequence that encodes the protein having the amino acid sequence as set forth in SEQ ID NO: 98; and
b) culturing the cell under conditions favoring the expression of the protein.

**15.** The method according to claim 14 wherein the step of culturing the cell comprises adding an organic substrate to media containing the cell.

**16.** A method for increasing production of a dicarboxylic acid comprising:

a) providing a host cell having a naturally occurring number of *CYP52A3A* genes;
b) increasing, in the host cell, the number of *CYP52A3A* genes which encode a *CYP52A3A* protein having the amino acid sequence as set forth in SEQ ID NO: 98;
c) culturing the host cell in media containing an organic substrate which upregulates the *CYP52A3A* gene, to effect increased production of dicarboxylic acid.

**17.** A method for increasing the production of a *CYP52A3A* protein having an amino acid sequence as set forth in SEQ ID NO: 98 comprising:

a) transforming a host cell having a naturally occurring amount of *CYP52A3A* protein with an increased copy number of a *CYP52A3A* gene that encodes the *CYP52A3A* protein having the amino acid sequence as set forth in SEQ ID NO: 98; and
b) culturing the cell and thereby increasing expression of the protein compared with that of a host cell containing a naturally occurring copy number of the *CYP52A3A* gene.

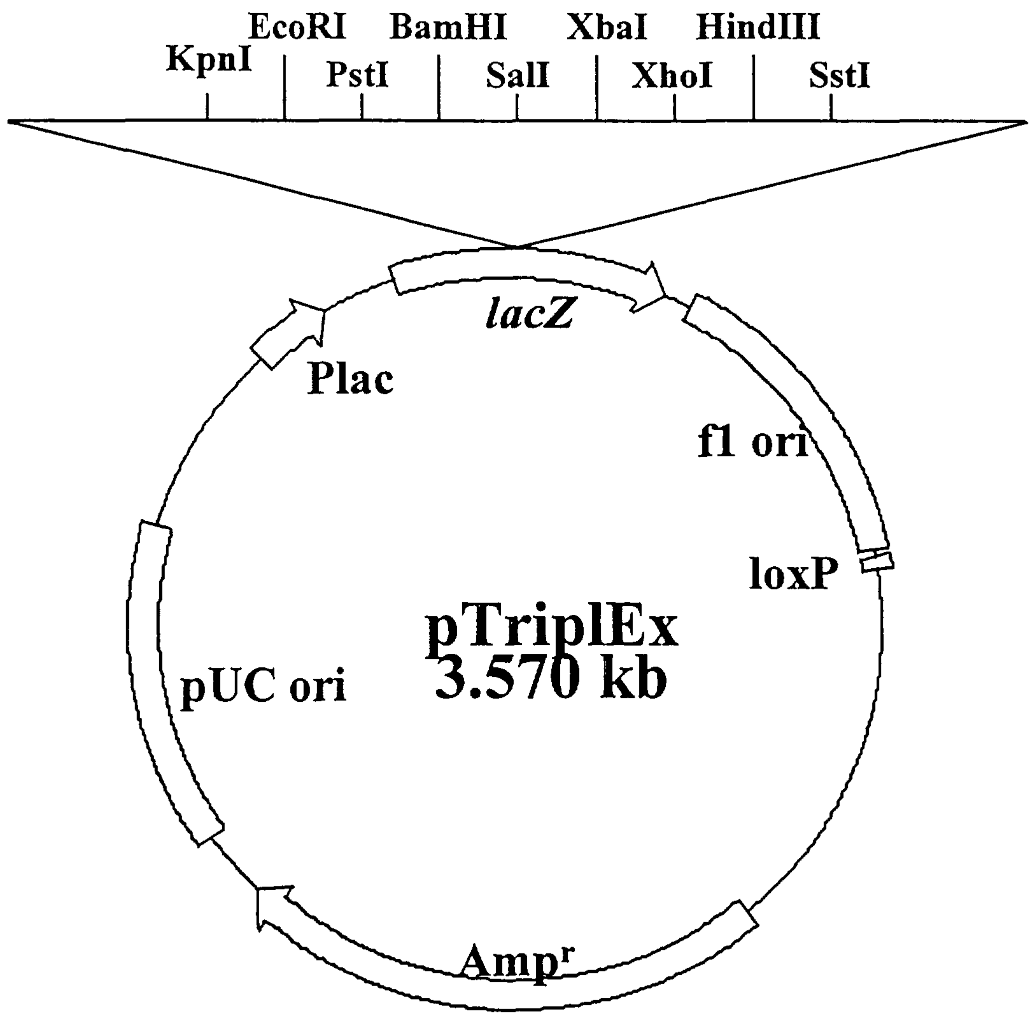
KpnI
EcoRI
PstI
BamHI
SalI
XbaI
XhoI
HindIII
SstI
lacZ
Plac
f1 ori
loxP
pTriplEx
3.570 kb
pUC ori
Ampr

Figure 1

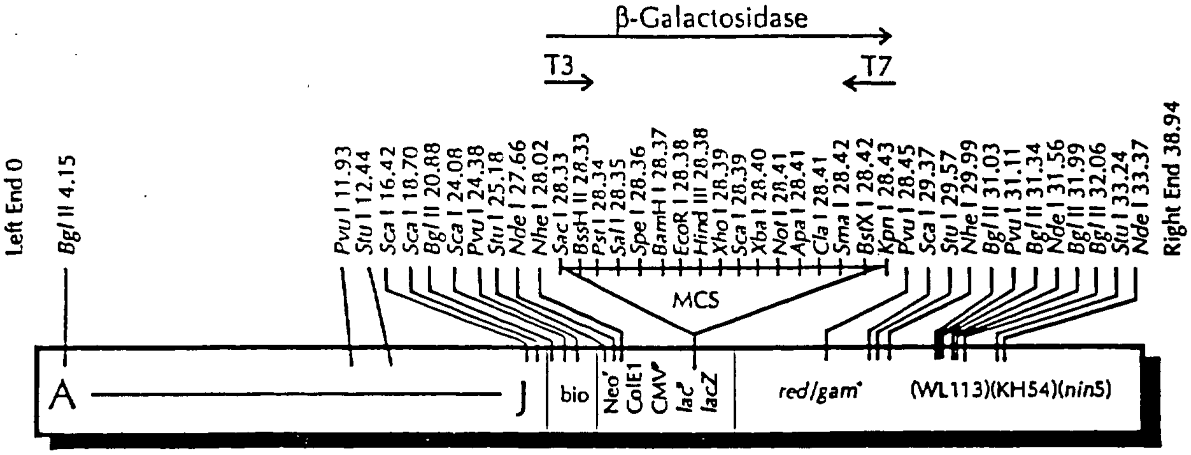


Figure 2A

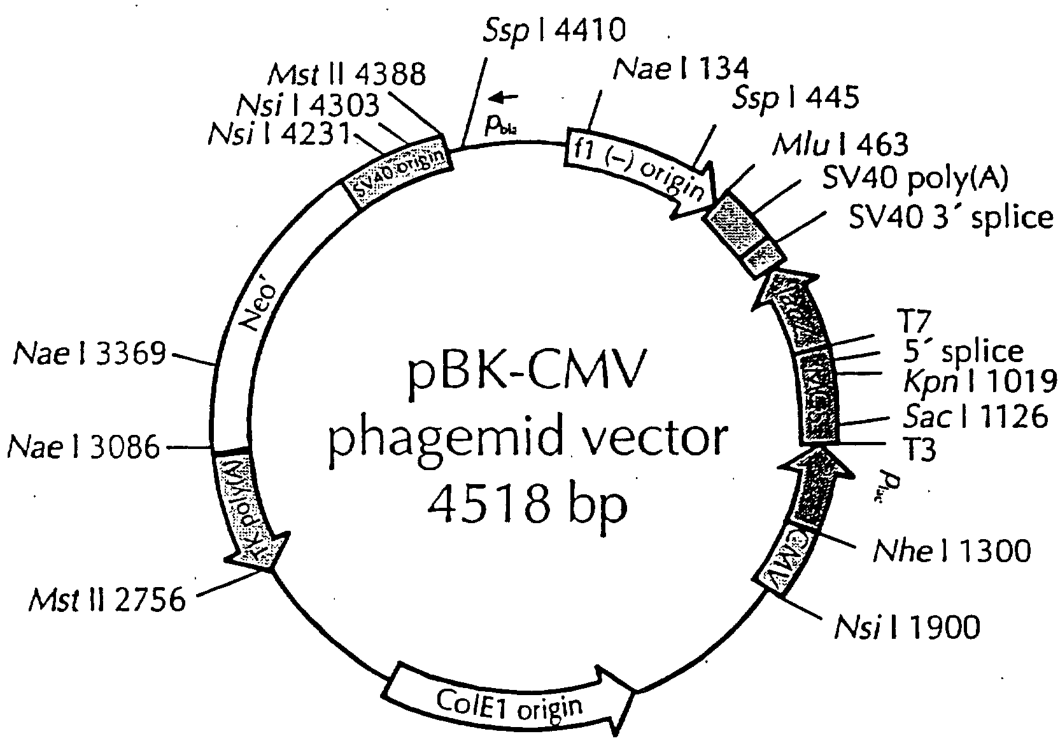


Figure 2B

# QC-RT-PCR primers for the 5' coding sequence of *Candida tropicalis* 20336 P450*CYP52A5A*

5' ATGATTGAACAACTCCTAGAATATTGGTAT GTCGTTGTGCCAGTGTTGTACATCATCAAA CAACTCCTTGCATACACAAAGACTCGCGTC 3' 90
3' TACTAACTTGTTGAGGATCTTATAACCATA CAGCAACACGGTCACAACATGTAGTAGTTT GTTGAGGAACGTATGTGTTTCTGAGCGCAG 5'

5' TTGATGAAAAAGTTGGGTGCTGCTCCAGTC ACAAACAAGTTGTACGACAACGCTTTCGGT ATCGTCAATGGATGGAAGGCTCTCCAGTTC 3' 180
3' AACTACTTTTTCAACCCACGACGAGGTCAG TGTTTGTTCAACATGCTGTTGCGAAAGCCA TAGCAGTTACCTACCTTCCGAGAGGTCAAG 5'

Forward Primer 7581-97F

5' AAGAAAGAGGGCAGGGCTCAAGAGTACAAC GATTACAAGTTTGACCACTCCAAGAACCCA AGCGTGGGCACCTACGTCAGTATTCTTTTC 3' 270
3' TTCTTTCTCCCGTCCCGAGTTCTCATGTTG CTAATGTTCAAACTGGTGAGGTTCTTGGGT TCGCACCCGTGGATGCAGTCATAAGAAAAG 5'

5' GGCACCAGGATCGTCGTGACCAAAGATCCA GAGAATATCAAAGCTATTTTGGCAACCCAG TTTGGTGATTTTTCTTTGGGCAAGAGGCAC 3' 360
3' CCGTGGTCCTAGCAGCACTGGTTTCTAGGT CTCTTATAGTTTCGATAAAACCGTTGGGTC AAACCACTAAAAAGAAACCCGTTCTCCGTG 5'

5' ACTCTTTTTAAGCCTTTGTTAGGTGATGGG ATCTTCACATTGGACGGCGAAGGCTGGAAG CACAGCAGAGCCATGTTGAGACCACAGTTT 3' 450
3' TGAGAAAAATTCGGAAACAATCCACTACCC TAGAAGTGTAACCTGCCGCTTCCGACCTTC GTGTCGTCTCGGTACAACTCTGGTGTCAAA 5'

Reverse Primer 7581-97M

5' GCCAGAGAACAAGTTGCTCATGTGACGTCG TTGGAACCACACTTCCAGTTGTTGAAGAAG CATATTCTTAAGCACAAGGGTGAATACTTT 3' 540
3' CGGTCTCTTGTTCAACGAGTACACTGCAGC AACCTTGGTGTGAAGGTCAACAACTTCTTC GTATAAGAATTCGTGTTCCCACTTATGAAA 5'

Figure 3

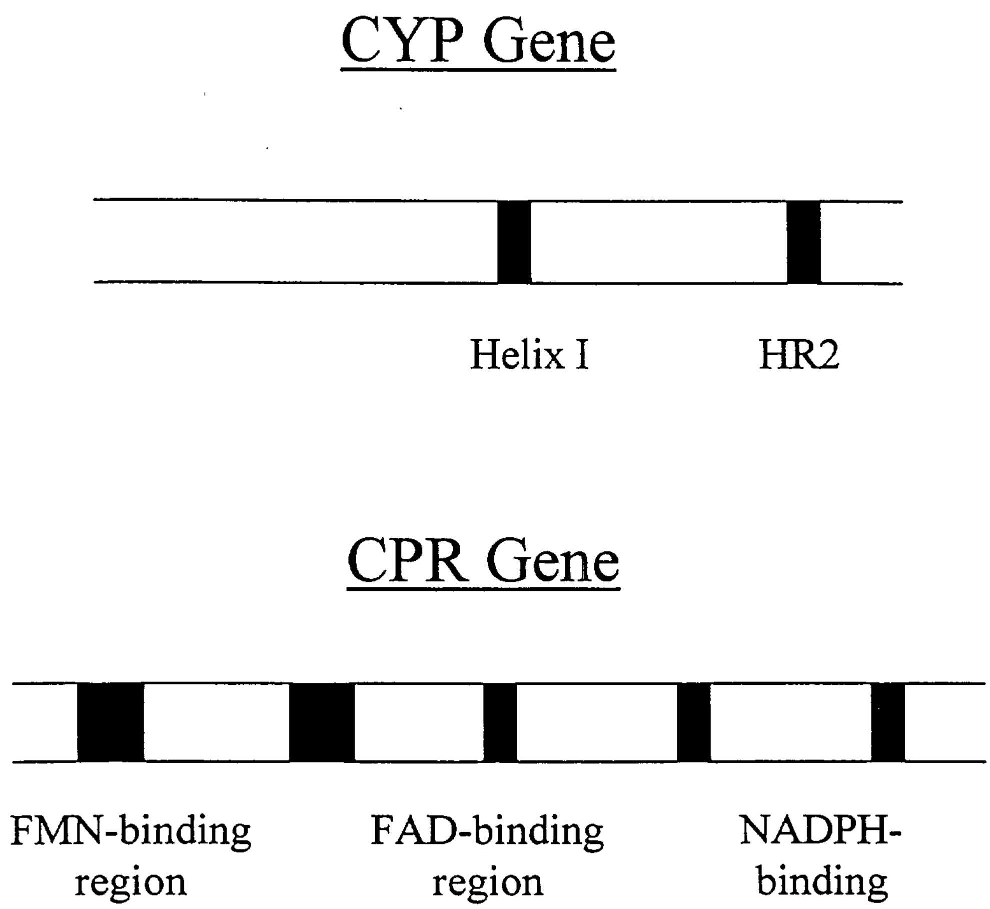
CYP Gene
Helix I
HR2
CPR Gene
FMN-binding region
FAD-binding region
NADPH-binding

Figure 4

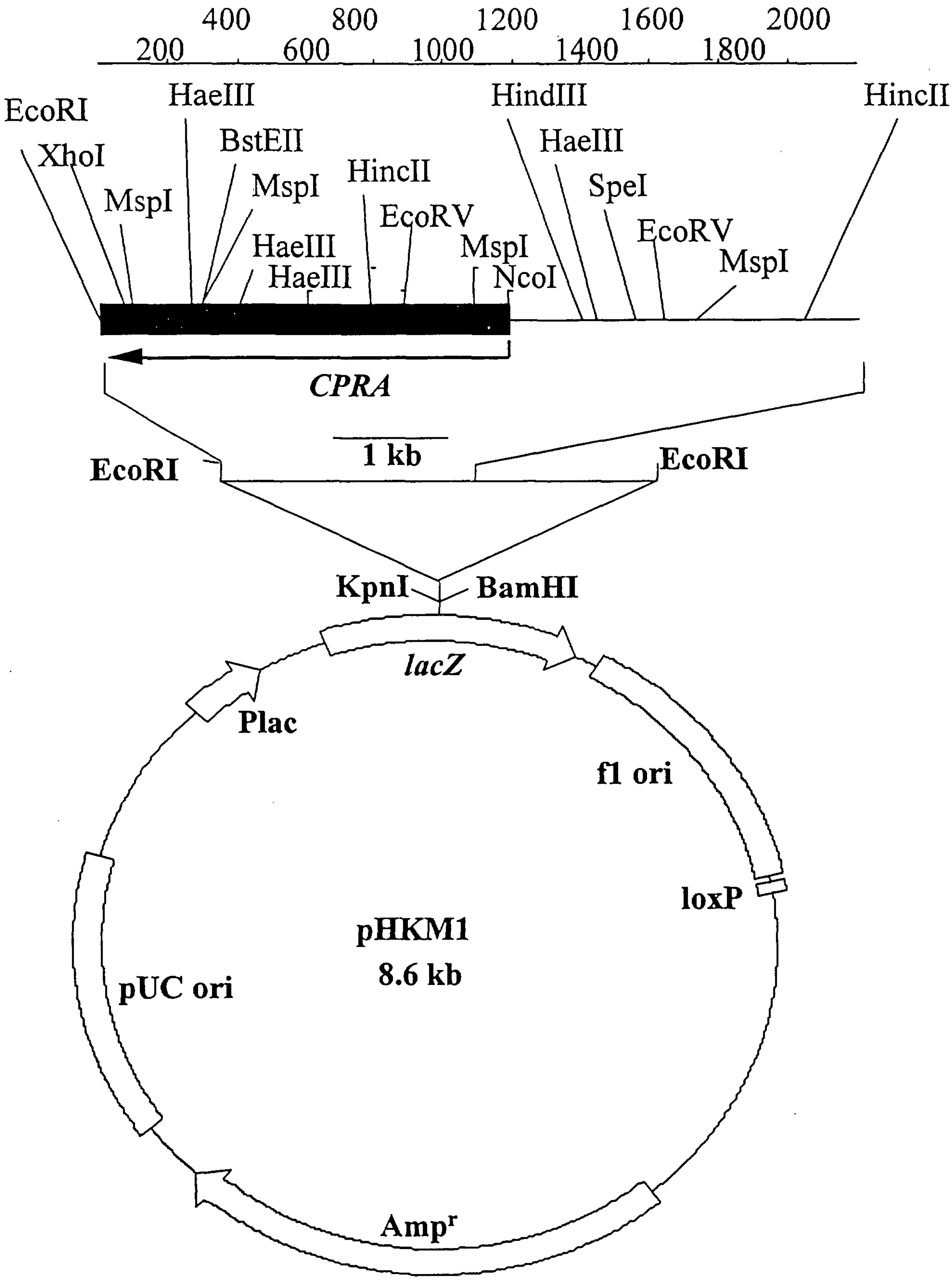

400
800
1200
1600
2000
200
600
1000
1400
1800
EcoRI
XhoI
MspI
HaeIII
BstEII
MspI
HincII
EcoRV
HaeIII
HaeIII
MspI
NcoI
HindIII
HaeIII
SpeI
EcoRV
MspI
HincII
CPRA
1 kb
EcoRI
EcoRI
KpnI
BamHI
lacZ
Plac
f1 ori
loxP
pHKM1
8.6 kb
pUC ori
Amp^r


Figure 5

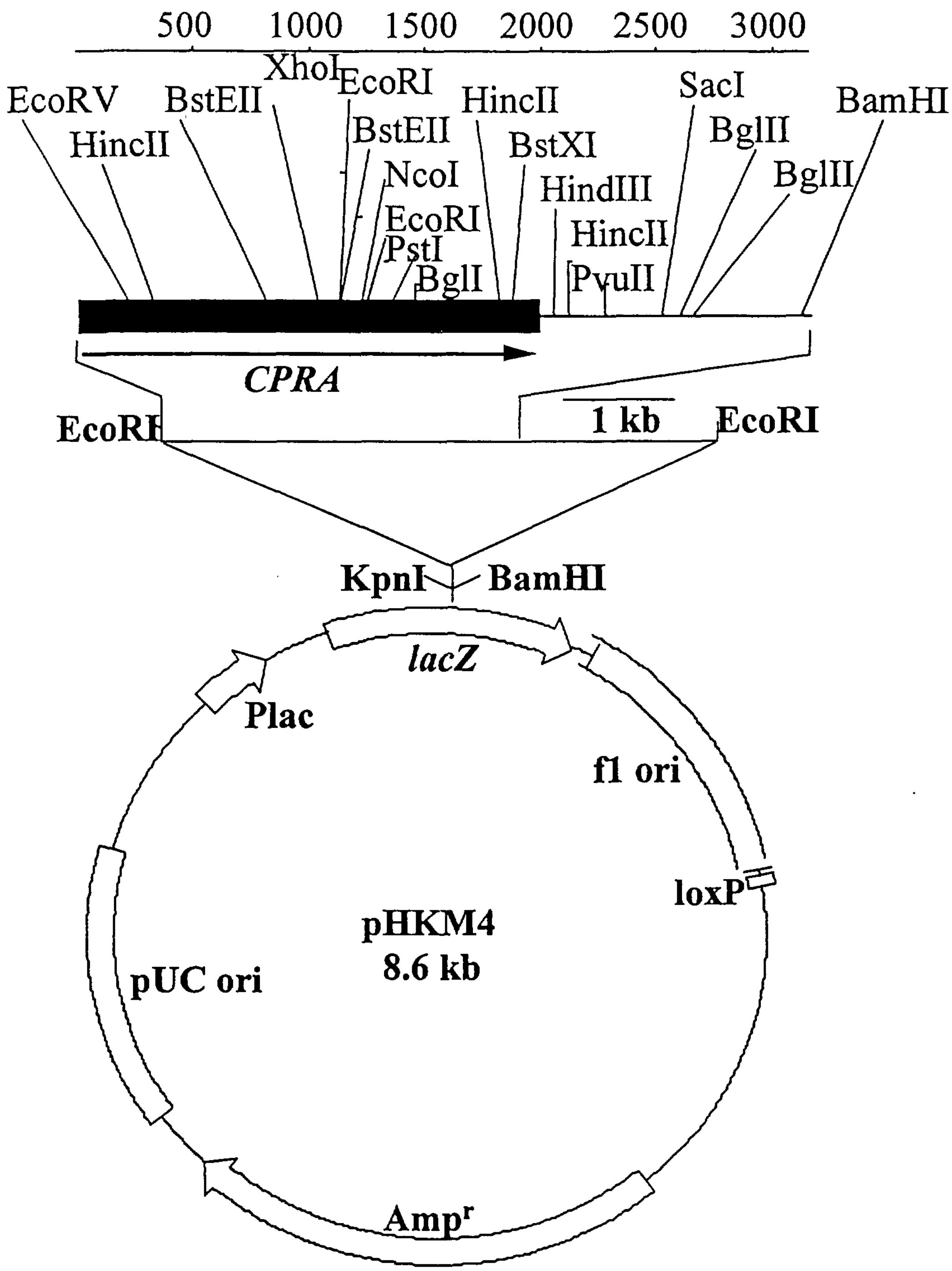
500
1000
1500
2000
2500
3000
EcoRV
HincII
BstEII
XhoI
EcoRI
BstEII
NcoI
EcoRI
PstI
BglI
HincII
BstXI
HindIII
HincII
PvuII
SacI
BglII
BglII
BamHI
CPRA
EcoRI
1 kb
EcoRI
KpnI
BamHI
lacZ
Plac
f1 ori
loxP
pHKM4
8.6 kb
pUC ori
Ampr

Figure 6

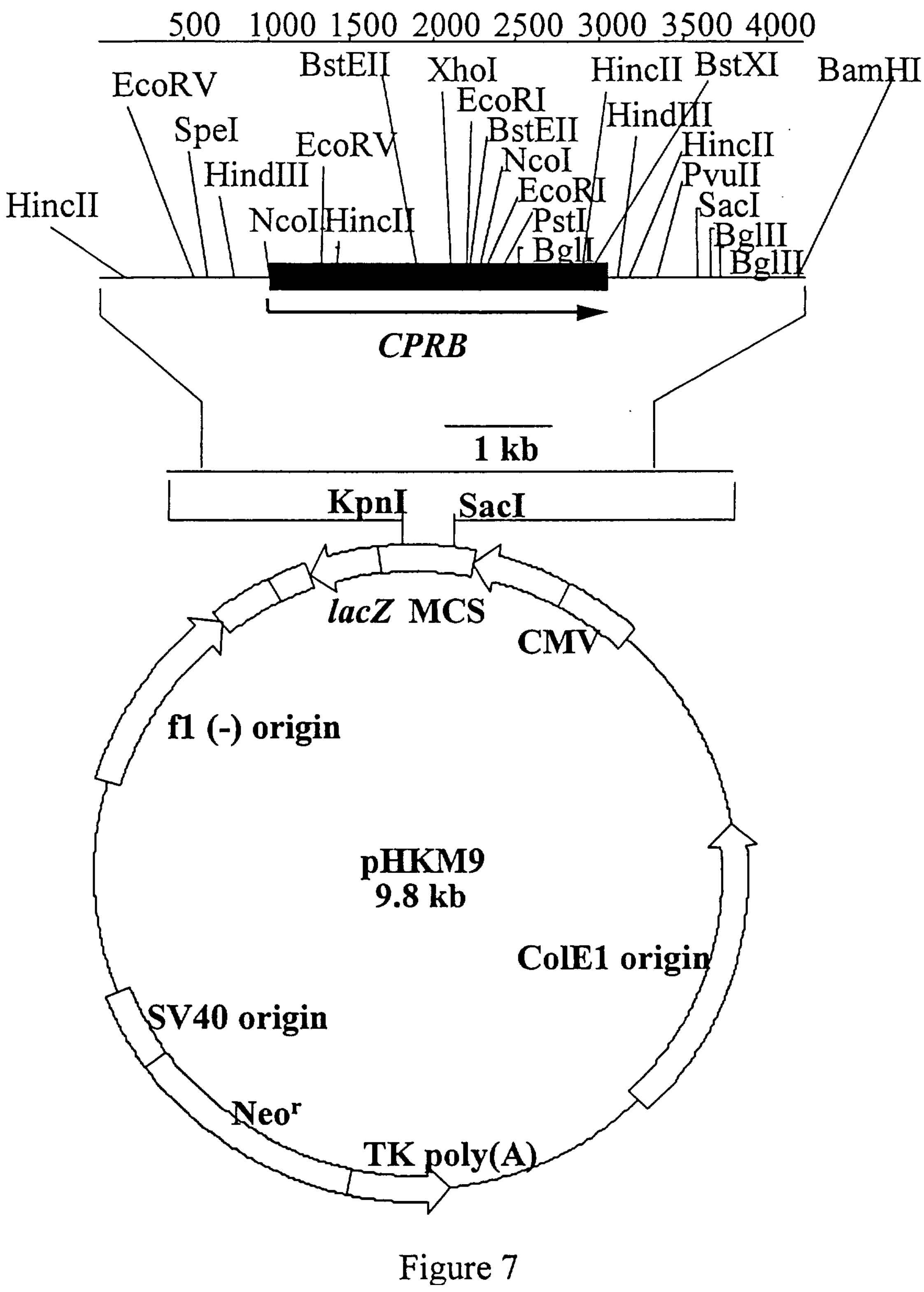
500 1000 1500 2000 2500 3000 3500 4000
HincII
EcoRV
SpeI
HindIII
NcoI
EcoRV
HincII
BstEII
XhoI
EcoRI
BstEII
NcoI
EcoRI
PstI
BglI
HincII
HindIII
BstXI
HincII
PvuII
SacI
BglII
BglII
BamHI
CPRB
1 kb
KpnI
SacI
lacZ MCS
CMV
f1 (-) origin
pHKM9
9.8 kb
ColE1 origin
SV40 origin
Neor
TK poly(A)

Figure 7

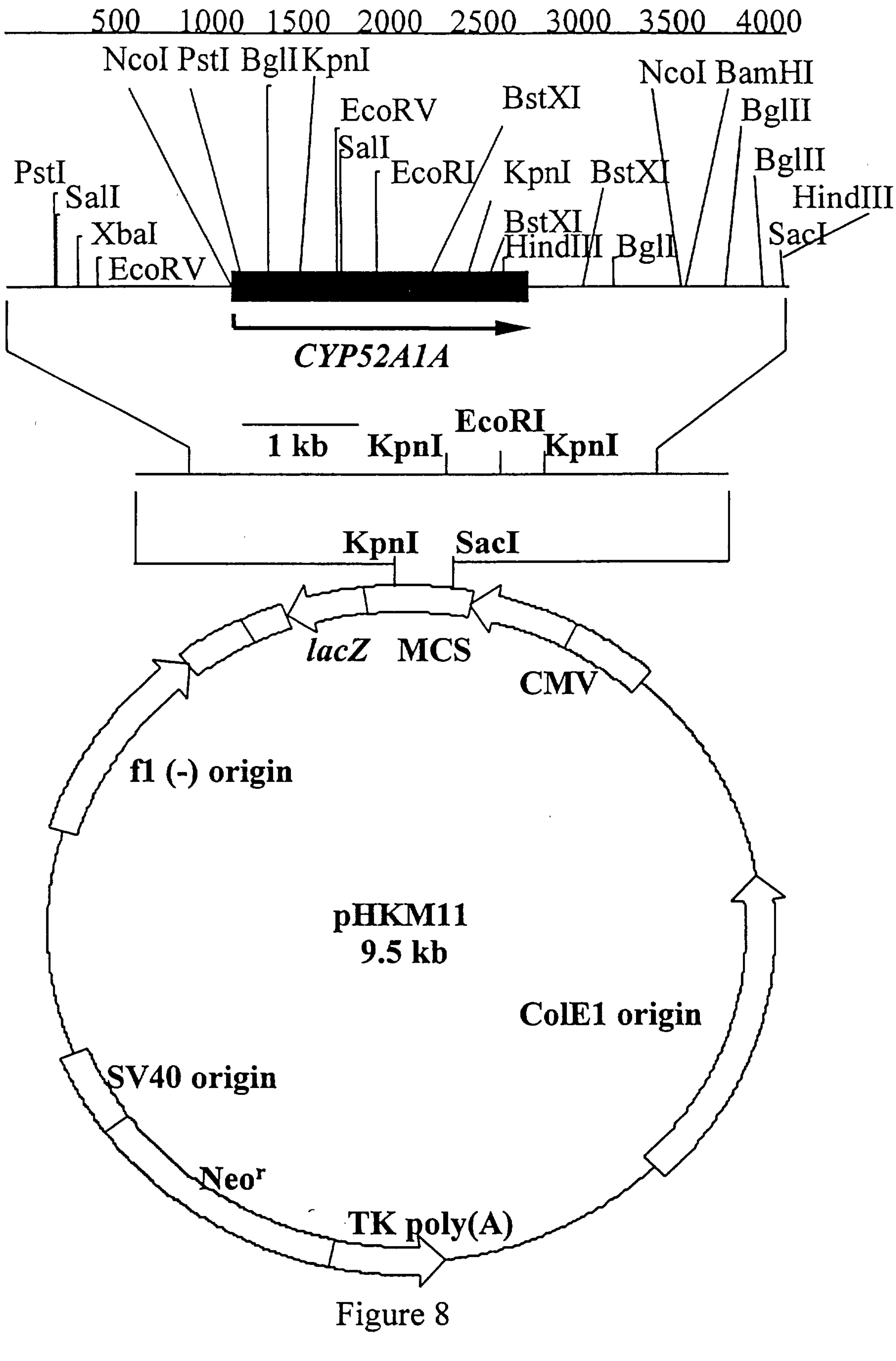
500 1000 1500 2000 2500 3000 3500 4000
NcoI PstI BglI KpnI
EcoRV
SalI
EcoRI
BstXI
KpnI
BstXI
NcoI BamHI
BglII
BglII
HindIII
SacI
PstI
SalI
XbaI
EcoRV
BstXI
HindIII
BglI
CYP52A1A
1 kb
KpnI
EcoRI
KpnI
KpnI
SacI
lacZ
MCS
CMV
f1 (-) origin
pHKM11
9.5 kb
ColE1 origin
SV40 origin
Neor
TK poly(A)

Figure 8

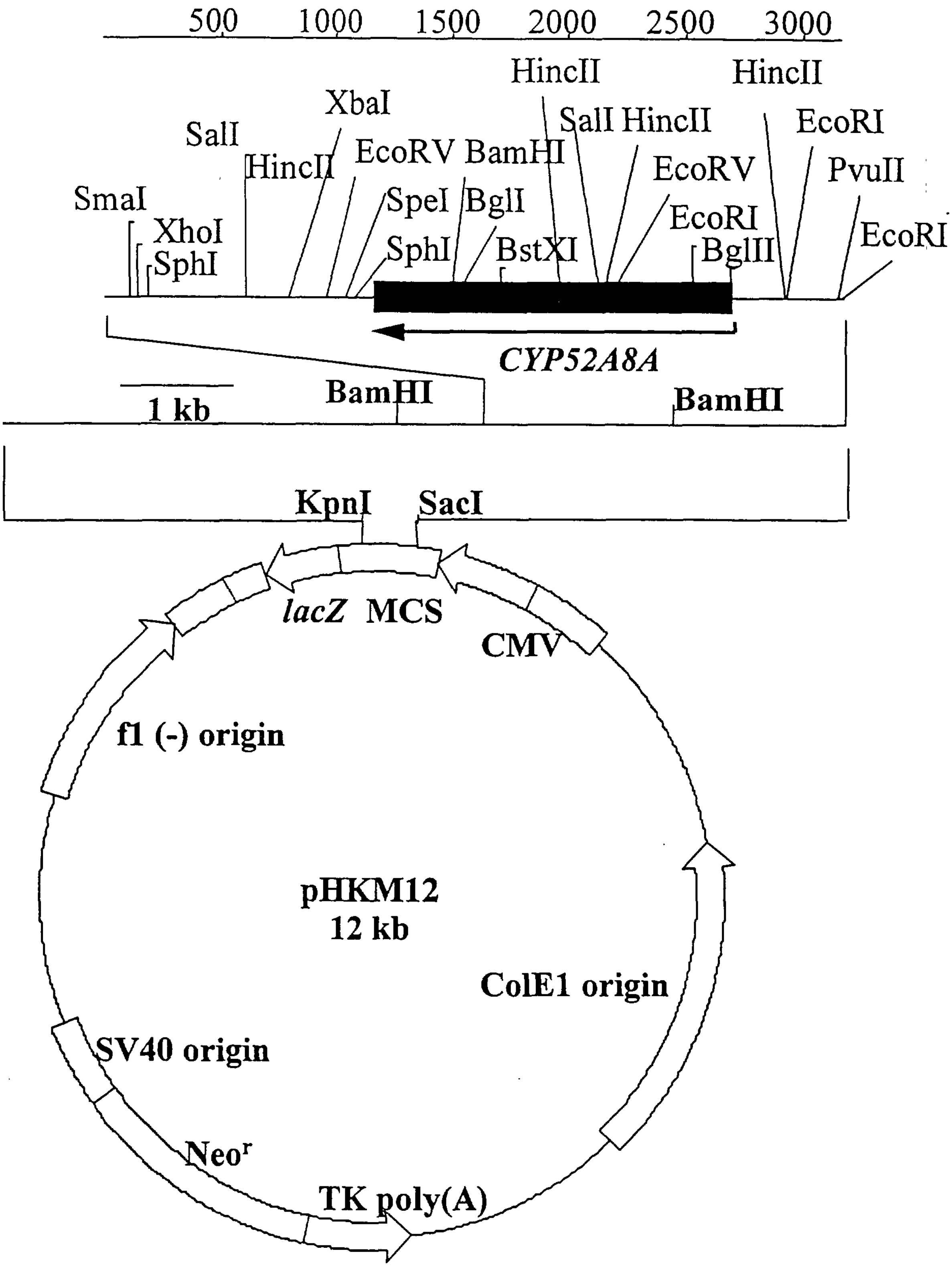
500
1000
1500
2000
2500
3000
HincII
HincII
XbaI
SalI HincII
EcoRI
SalI
HincII
EcoRV BamHI
EcoRV
PvuII
SmaI
SpeI
BglI
EcoRI
XhoI
EcoRI
SphI
SphI
BstXI
BglII
CYP52A8A
1 kb
BamHI
BamHI
KpnI
SacI
lacZ
MCS
CMV
f1 (-) origin
pHKM12
12 kb
ColE1 origin
SV40 origin
Neo^r
TK poly(A)

Figure 9

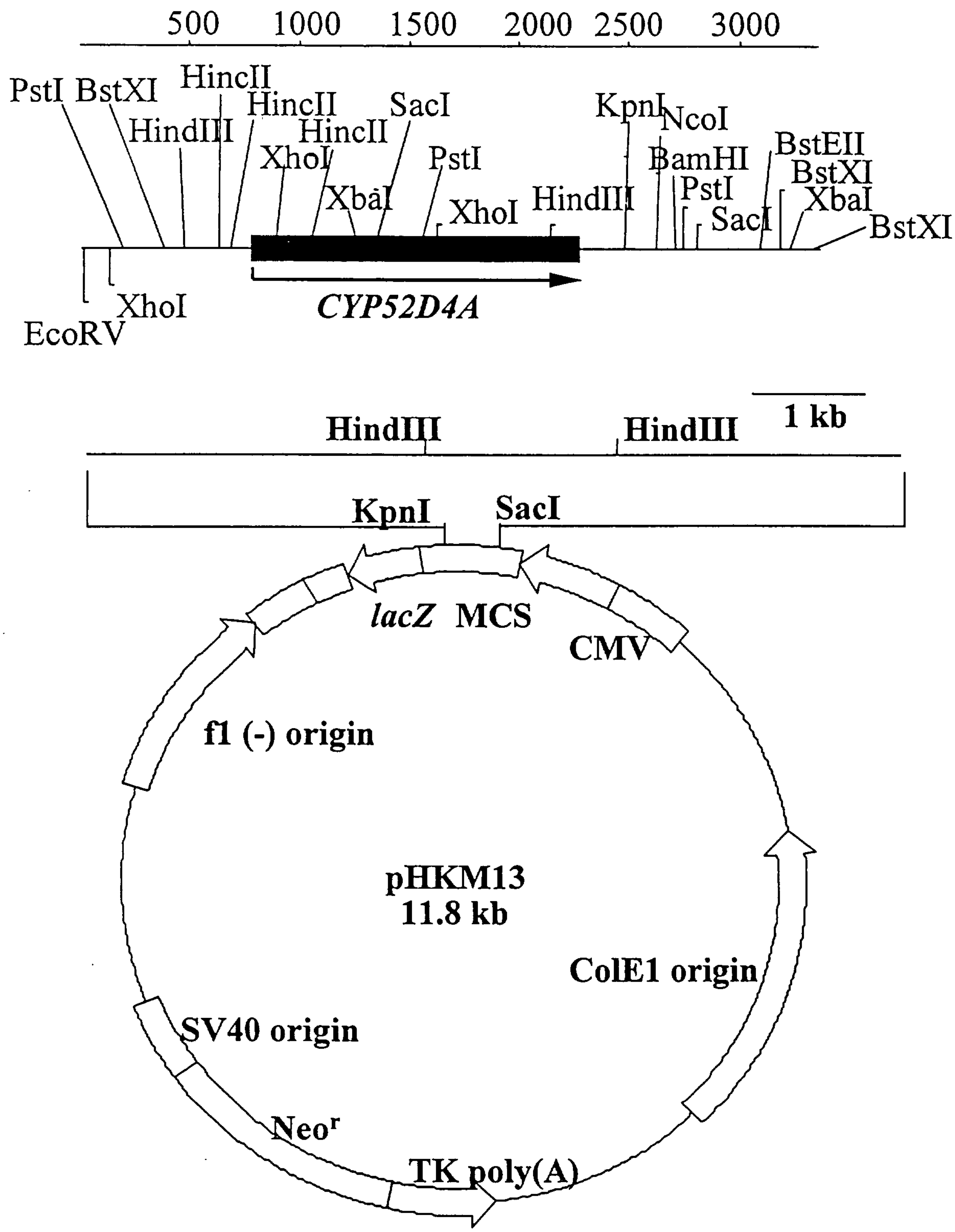
500
1000
1500
2000
2500
3000
PstI
BstXI
HincII
HindIII
HincII
HincII
SacI
XhoI
PstI
XbaI
XhoI
HindIII
KpnI
NcoI
BamHI
PstI
SacI
BstEII
BstXI
XbaI
BstXI
XhoI
EcoRV
CYP52D4A
HindIII
HindIII
1 kb
KpnI
SacI
lacZ
MCS
CMV
f1 (-) origin
pHKM13
11.8 kb
ColE1 origin
SV40 origin
Neo^r
TK poly(A)

Figure 10

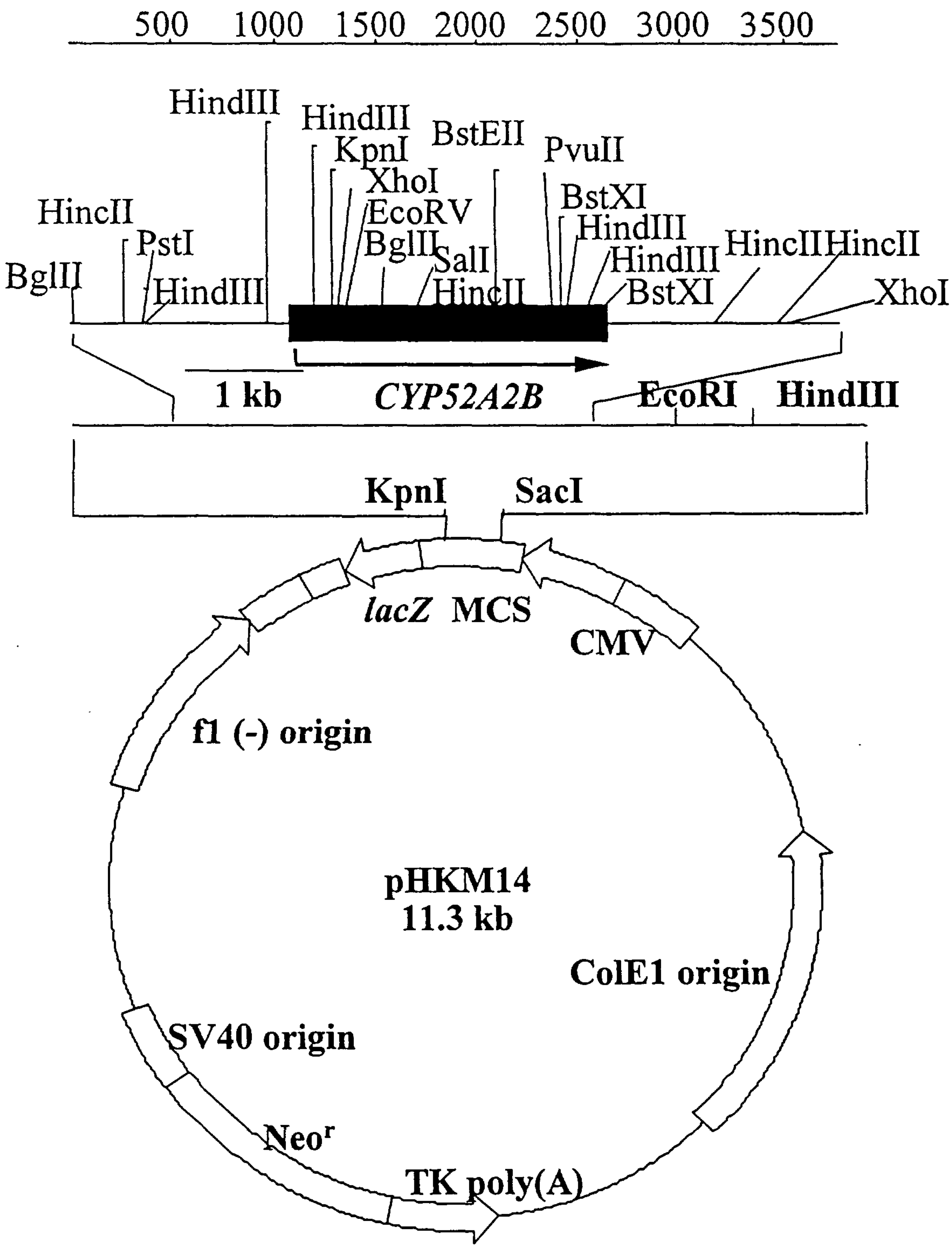
500 1000 1500 2000 2500 3000 3500
HindIII
HindIII
KpnI
XhoI
EcoRV
BglII
SalI
HincII
BstEII
PvuII
BstXI
HindIII
HindIII
BstXI
HincII
PstI
HindIII
BglII
HincII
HincII
XhoI
1 kb
CYP52A2B
EcoRI
HindIII
KpnI
SacI
lacZ
MCS
CMV
f1 (-) origin
pHKM14
11.3 kb
ColE1 origin
SV40 origin
Neo^r
TK poly(A)

Figure 11

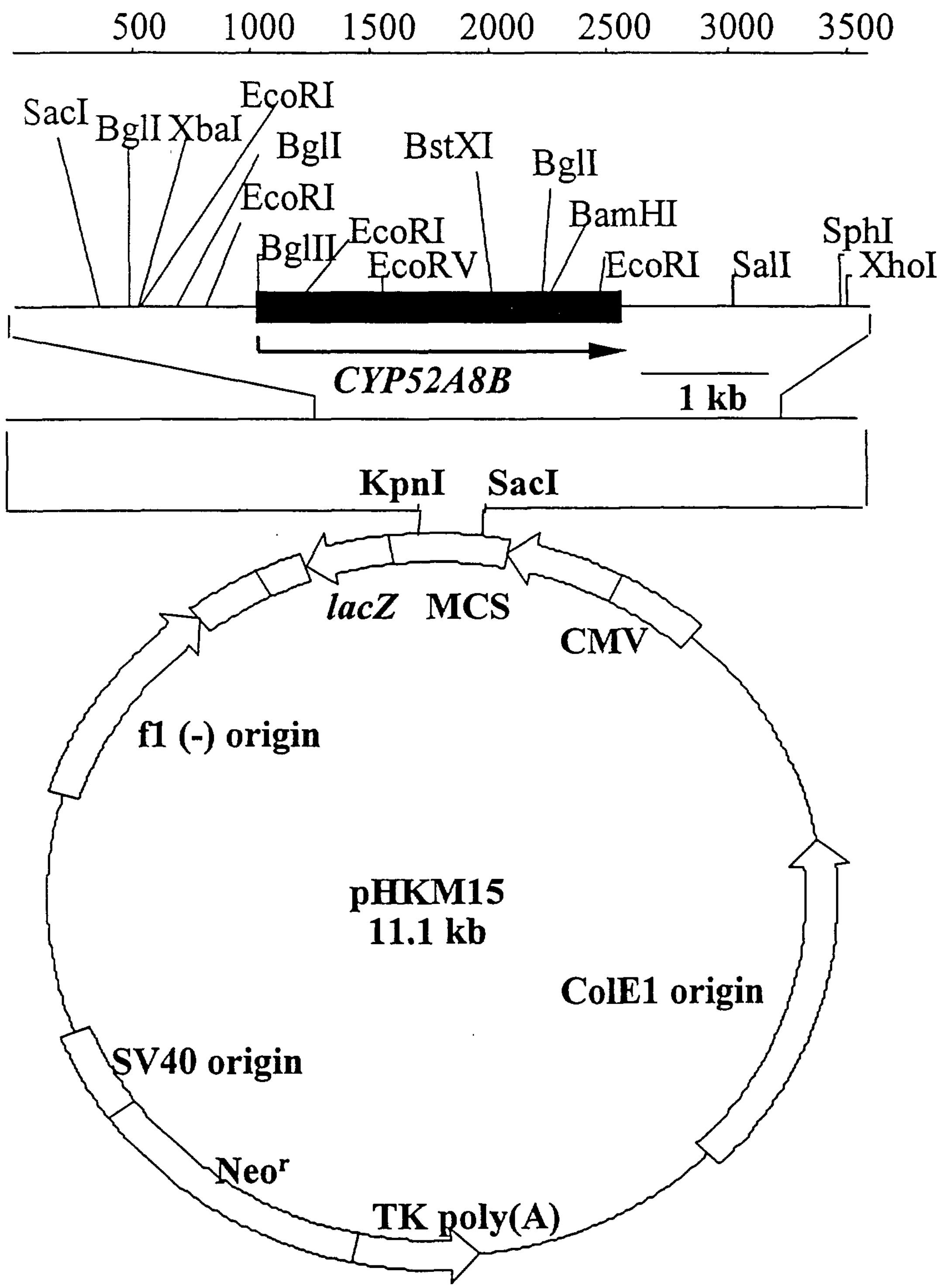
500
1000
1500
2000
2500
3000
3500
SacI
BglI
XbaI
EcoRI
BglI
EcoRI
BglII
EcoRI
EcoRV
BstXI
BglI
BamHI
EcoRI
SalI
SphI
XhoI
CYP52A8B
1 kb
KpnI
SacI
lacZ
MCS
CMV
f1 (-) origin
pHKM15
11.1 kb
ColE1 origin
SV40 origin
Neo[r]
TK poly(A)

Figure 12

*C. tropicalis* 20336 *CPR* Allele DNA Alignment of DS Sequence

```
CPRA     1                                                                  CATCA    5
CPRB     1 TATATGATATATGATATATCTTCCTGTGTAATTATTATTCGTATTCGTTAATACTTACTACATTTTTTT   70
                                                                              *

CPRA     6 AGATCATCTATGGGGATAATTA-----CGACAGCAACATTGCAGAAAGAGCGTTGGTCACAATCGAAAGA   70
CPRB    71 TCTTTATTTATGAAGAAAAGGAGAGTTCGTAAGTTGAGTTGAGTAGAATAGGCTGTTGTGCATACGGGGA  140
              * ** ****  ** **  *     **  **     ***   * *    * ** *    **     **

CPRA    71 GCCTATG-GCGTTGCCGTCGTTGAGGCAAATGACAGCAC--CAACAATAACGATGGTCCCAGTGAAGAGC  137
CPRB   141 GCAGAGGAGAGTATCCGACGAGGAGGAACTGGGTGAAATTTCATCTATGCTGTTGCGTCCTGTACTGTAC  210
           **  * * * **  *** **  **** *   *     *   ** * **   * **   ** **   *  *

CPRA   138 CTTCAGAACAGTCCATTGTTGACGCT--TAAGGCACGGATAATTACGTGGGGCAAAGGAACGCGGAATTA  205
CPRB   211 TGTAAATCTTAGATTTCCTAGAGGTTGTTCTAGCAAATAAAGTGTTTCAAGATACAATTTTACAGGCAAG  280
             * *          *  * ** * *  *   ***   * * *       *  * *      * *

CPRA   206 GTTATGGGGGGATCAAA--AGCGGAAGATTTGTGTTGCTTGTGGGTTTTTTCCTTTATTTTTCATATGAT  273
CPRB   281 GGTAAAGGATCAACTGATTAGCGGAAGATTGGTGTTGCCTGTGGGGTTCTT---TTATTTTTCATATGAT  347
           * **  **   * *  *  *********** ******* ****** ** **   ****************

CPRA   274 TTCTTTGCGCAAGTAACATGTGCCAATTTAGTTTGTGATTAGCGTGCC-CCACAATTGGCATCGTGGACG  342
CPRB   348 TTCTTTGCGCGAGTAACATGTGCCAATCTAGTTTATGATTAGCGTACCTCCACAATTGGCATCTTGGACG  417
           ********** **************** ****** ********** ** ************** ******

CPRA   343 GGCGTGTTTTGTCATACCCCAAGTCTTAACTAGCTCCACAGTCTCGACGGTGTCTCGACGATGTCTTCTT  412
CPRB   418 GGCGTGTTTTGTCTTACCCCAAGCCTTATTTAGTTCCACAGTCTCGACGGTGTCTCGCCGATGTCTTCTC  487
           ************* ********* ****  *** *********************** ***********

CPRA   413 CCACCCCTCCCATGAATCATTCAAAGTTGTTGGGGGATCTCCACCAAGGGCACCGGAGTTAATGCTTATG  482
CPRB   488 CCACCCCTCGCAGGAATCATTCGAAGTTGTTGGGGGATCTCCTCC---------GCAGTTTATGTTCATG  548
           ********* ** ********* ******************* **         * **** *** * ***

CPRA   483 TTTCTCCCACTTTGGTTGTGATTGGGGTAGTCTAGTGAGTTGGAGATTTTCTTTTTTTCGCAGGTGTCTC  552
CPRB   549 TCTTTCCCACTTTGGTTGTGATTGGGGTAGCGTAGTGAGTTGGTGATTTTCTTTTTT-CGCAGGTGTCTC  617
           * * **************************  *********** ************* ************

CPRA   553 CGATATCGAAATTTGATGAATATAGAGAGAAGCCAGATCAGCACAGTAGATTGCCTTTGTAGTTAGAGAT  622
CPRB   618 CGATATCGAAGTTTGATGAATATAG----GAGCCAGATCAGCATGGTATATTGCCTTTGTAGATAGAGAT  683
           ********** **************     *************  *** ************* *******

CPRA   623 GTTGAACAGCAACTAGTTGAATTACACGCCACCACTTGACAGCAAGTGCAGTGAGCTGTAAACGATGCAG  692
CPRB   684 GTTGAACAACAACTAGCTGAATTACACACCACCGCT-----------------------AAACGATGCGC  730
           ******** ******* ********** ***** **                       *********

CPRA   693 CCAGAGTGTCACCACCAACTGACGTTGGGTGGAGTTGTTGTTGTTGTTGTTGGCAGGGCCATATTGCTAA  762
CPRB   731 ACAGGGTGTCACCGCCAACTGACGTTGGGTGGAGTTG---------TTGTTGGCAGGGCCATATTGCTAA  791
            *** ******** ***********************         ************************

CPRA   763 ACGAAGACAAGTAGCACAAAACCCAAGCTTAAGAACAAAAATAAAAAAAATTCATACGACAATTCCAAAG  832
CPRB   792 ACGAAGAGAAGTAGCACAAAACCCAAGGTTAAGAACAA---TTAAAAAAATTCATACGACAATTCCACAG  858
           ******* ******************* **********   * ************************ **

CPRA   833 CCATTGATTTACATAAT--CAACAG-TAAGACAGAAAAAACTTTCAACATTTCAAAGTTCCCTTTTTCCT  899
CPRB   859 CCATTTACATAATCAACAGCGACAAATGAGACAGAAAAAACTTTCAACATTTCAAAGTTCCCTTTTTCCT  928
           ***** *  **   **   * ***  * ******************************************

CPRA   900 ATTACTTCTTTTTTTTCTTCTTTCCTT-------CTTTCCTTCTGTTTTTCTTACTTTATCAGTCTTTTA  962
CPRB   929 ATTACTTCTTTTTTTCTTTCCTTCCTTTCATTTCCTTTCCTTCTGCTTTTATTACTTTACCAGTCTTTTG  998
           ***************  *** ******       *********** **** ******** *********

CPRA   963 CTTGTTTTTGCAATTCCTCATCCTCCTCCTACTCCTCCTCACCATGGCTTTAGACAAGTTAGATTTGTAT 1032
CPRB   999 CTTGTTTTTGCAATTCCTCATCCTCCTCCT---------CACCATGGCTTTAGACAAGTTAGATTTGTAT 1059
           ******************************         *******************************
```

Figure 13A

```
CPRA  1033 GTCATCATAACATTGGTGGTCGCTGTAGCCGCCTATTTTGCTAAGAACCAGTTCCTTGATCAGCCCCAGG 1102
CPRB  1060 GTCATCATAACATTGGTGGTCGCTGTGGCCGCCTATTTTGCTAAGAACCAGTTCCTTGATCAGCCCCAGG 1129
           ************************** *******************************************

CPRA  1103 ACACCGGGTTCCTCAACACGGACAGCGGAAGCAACTCCAGAGACGTCTTGCTGACATTGAAGAAGAATAA 1172
CPRB  1130 ACACCGGGTTCCTCAACACGGACAGCGGAAGCAACTCCAGAGACGTCTTGCTGACATTGAAGAAGAATAA 1199
           **********************************************************************

CPRA  1173 TAAAAACACGTTGTTGTTGTTTGGGTCCCAGACGGGTACGGCAGAAGATTACGCCAACAAATTGTCCAGA 1242
CPRB  1200 TAAAAACACGTTGTTGTTGTTTGGGTCCCAGACCGGTACGGCAGAAGATTACGCCAACAAATTGTCAAGA 1269
           ********************************* ******************************** ***

CPRA  1243 GAATTGCACTCCAGATTTGGCTTGAAAACGATGGTTGCAGATTTCGCTGATTACGATTGGGATAACTTCG 1312
CPRB  1270 GAATTGCACTCCAGATTTGGCTTGAAAACCATGGTTGCAGATTTCGCTGATTACGATTGGGATAACTTCG 1339
           ***************************** ****************************************

CPRA  1313 GAGATATCACCGAAGACATCTTGGTGTTTTTCATTGTTGCCACCTATGGTGAGGGTGAACCTACCGATAA 1382
CPRB  1340 GAGATATCACCGAAGATATCTTGGTGTTTTTCATCGTTGCCACCTACGGTGAGGGTGAACCTACCGACAA 1409
           **************** ***************** *********** ******************** **

CPRA  1383 TGCCGACGAGTTCCACACCTGGTTGACTGAAGAAGCTGACACTTTGAGTACCTTGAAATACACCGTGTTC 1452
CPRB  1410 TGCCGACGAGTTCCACACCTGGTTGACTGAAGAAGCTGACACTTTGAGTACTTTGAGATATACCGTGTTC 1479
           *************************************************** **** *** *********

CPRA  1453 GGGTTGGGTAACTCCACGTACGAGTTCTTCAATGCCATTGGTAGAAAGTTTGACAGATTGTTGAGCGAGA 1522
CPRB  1480 GGGTTGGGTAACTCCACCTACGAGTTCTTCAATGCTATTGGTAGAAAGTTTGACAGATTGTTGAGTGAGA 1549
           ***************** ***************** ***************************** ****

CPRA  1523 AAGGTGGTGACAGGTTTGCTGAATACGCTGAAGGTGATGACGGTACTGGCACCTTGGACGAAGATTTCAT 1592
CPRB  1550 AAGGTGGTGACAGATTTGCTGAATATGCTGAAGGTGACGACGGCACTGGCACCTTGGACGAAGATTTCAT 1619
           ************* *********** *********** ***** **************************

CPRA  1593 GGCCTGGAAGGACAATGTCTTTGACGCCTTGAAGAATGATTTGAACTTTGAAGAAAAGGAATTGAAGTAC 1662
CPRB  1620 GGCCTGGAAGGATAATGTCTTTGACGCCTTGAAGAATGACTTGAACTTTGAAGAAAAGGAATTGAAGTAC 1689
           ************ ************************** ******************************

CPRA  1663 GAACCAAACGTGAAATTGACTGAGAGAGACGACTTGTCTGCTGCTGACTCCCAAGTTTCCTTGGGTGAGC 1732
CPRB  1690 GAACCAAACGTGAAATTGACTGAGAGAGATGACTTGTCTGCTGCCGACTCCCAAGTTTCCTTGGGTGAGC 1759
           ***************************** ************** *************************

CPRA  1733 CAAACAAGAAGTACATCAACTCCGAGGGCATCGACTTGACCAAGGGTCCATTCGACCACACCCACCCATA 1802
CPRB  1760 CAAACAAGAAGTACATCAACTCCGAGGGCATCGACTTGACCAAGGGTCCATTCGACCACACCCACCCATA 1829
           **********************************************************************

CPRA  1803 CTTGGCCAGAATCACCGAGACGAGAGAGTTGTTCAGCTCCAAGGACAGACACTGTATCCACGTTGAATTT 1872
CPRB  1830 CTTGGCCAGGATCACCGAGACCAGAGAGTTGTTCAGCTCCAAGGAAAGACACTGTATTCACGTTGAATTT 1899
           ********* *********** *********************** *********** ************

CPRA  1873 GACATTTCTGAATCGAACTTGAAATACACCACCGGTGACCATCTAGCTATCTGGCCATCCAACTCCGACG 1942
CPRB  1900 GACATTTCTGAATCGAACTTGAAATACACCACCGGTGACCATCTAGCCATCTGGCCATCCAACTCCGACG 1969
           ********************************************** ***********************

CPRA  1943 AAAACATTAAGCAATTTGCCAAGTGTTTCGGATTGGAAGATAAACTCGACACTGTTATTGAATTGAAGGC 2012
CPRB  1970 AAAACATCAAGCAATTTGCCAAGTGTTTCGGATTGGAAGATAAACTCGACACTGTTATTGAATTGAAGGC 2039
           ******* **************************************************************

CPRA  2013 GTTGGACTCCACTTACACCATCCCATTCCCAACCCCAATTACCTACGGTGCTGTCATTAGACACCATTTA 2082
CPRB  2040 ATTGGACTCCACTTACACCATTCCATTCCCAACTCCAATTACTTACGGTGCTGTCATTAGACACCATTTA 2109
            ******************** *********** ******** ***************************

CPRA  2083 GAAATCTCCGGTCCAGTCTCGAGACAATTCTTTTTGTCAATTGCTGGGTTTGCTCCTGATGAAGAAACAA 2152
CPRB  2110 GAAATCTCCGGTCCAGTCTCGAGACAATTCTTTTTGTCGATTGCTGGGTTTGCTCCTGATGAAGAAACAA 2179
           ************************************** *******************************

CPRA  2153 AGAAGGCTTTACCAGACTTGGTGGTGACAAGCAAGAATTCGCCGCCAAGGTCACCCGCAGAAAGTTCAA 2222
CPRB  2180 AGAAGACTTTCACCAGACTTGGTGGTGACAAACAAGAATTCGCCACCAAGGTTACCCGCAGAAAGTTCAA 2249
           ***** **** ******************* ************ ******* ******************
```

Figure 13B

```
CPRA  2223 CATTGCCGATGCCTTGTTATATTCCTCCAACAACGCTCCATGGTCCGATGTTCCTTTTGAATTCCTTATT 2292
CPRB  2250 CATTGCCGATGCCTTGTTATATTCCTCCAACAACACTCCATGGTCCGATGTTCCTTTTGAGTTCCTTATT 2319
           ********************************** ************************* *********

CPRA  2293 GAAAACGTTCCACACTTGACTCCACGTTACTACTCCATTTCGTCTTCGTCATTGAGTGAAAAGCAACTCA 2362
CPRB  2320 GAAAACATCCAACACTTGACTCCACGTTACTACTCCATTTCTTCTTCGTCGTTGAGTGAAAAACAACTCA 2389
           ****** * * ****************************** ******** *********** *******

CPRA  2363 TCAACGTTACTGCAGTTGTTGAAGCCGAAGAAGAAGCTGATGGCAGACCAGTCACTGGTGTTGTCACCAA 2432
CPRB  2390 TCAATGTTACTGCAGTCGTTGAGGCCGAAGAAGAAGCCGATGGCAGACCAGTCACTGGTGTTGTTACCAA 2459
           **** *********** ***** ************** ************************** *****

CPRA  2433 CTTGTTGAAGAACGTTGAAATTGTGCAAAACAAGACTGGCGAAAAGCCACTTGTCCACTACGATTTGAGC 2502
CPRB  2460 CTTGTTGAAGAACATTGAAATTGCGCAAAACAAGACTGGCGAAAAGCCACTTGTTCACTACGATTTGAGC 2529
           ************* ********* ****************************** ***************

CPRA  2503 GGCCCAAGAGGCAAGTTCAACAAGTTCAAGTTGCCAGTGCATGTGAGAAGATCCAACTTTAAGTTGCCAA 2572
CPRB  2530 GGCCCAAGAGGCAAGTTCAACAAGTTCAAGTTGCCAGTGCACGTGAGAAGATCCAACTTTAAGTTGCCAA 2599
           ***************************************** ****************************

CPRA  2573 AGAACTCCACCACCCCAGTTATCTTGATTGGTCCAGGTACTGGTGTTGCCCCATTGAGAGGTTTTGTCAG 2642
CPRB  2600 AGAACTCCACCACCCCAGTTATCTTGATTGGTCCAGGTACTGGTGTTGCCCCATTGAGAGGTTTCGTTAG 2669
           **************************************************************** ** **

CPRA  2643 AGAAAGAGTTCAACAAGTCAAGAATGGTGTCAATGTTGGCAAGACTTTGTTGTTTTATGGTTGCAGAAAC 2712
CPRB  2670 AGAAAGAGTTCAACAAGTCAAGAATGGTGTCAATGTTGGCAAGACTTTGTTGTTTTATGGTTGCAGAAAC 2739
           **********************************************************************

CPRA  2713 TCCAACGAGGACTTTTTGTACAAGCAAGAATGGGCCGAGTACGCTTCTGTTTTGGGTGAAAACTTTGAGA 2782
CPRB  2740 TCCAACGAGGACTTTTTGTACAAGCAAGAATGGGCCGAGTACGCTTCTGTTTTGGGTGAAAACTTTGAGA 2809
           **********************************************************************

CPRA  2783 TGTTCAATGCCTTCTCCAGACAAGACCCATCCAAGAAGGTTTACGTCCAGGATAAGATTTAGAAAACAG 2852
CPRB  2810 TGTTCAATGCCTTCTCTAGACAAGACCCATCCAAGAAGGTTTACGTCCAGGATAAGATTTAGAAAACAG 2879
           **************** *****************************************************

CPRA  2853 CCAACTTGTGCACGAGTTGTTGACTGAAGGTGCCATTATCTACGTCTGTGGTGATGCCAGTAGAATGGCT 2922
CPRB  2880 CCAACTTGTGCACGAATTGTTGACCGAAGGTGCCATTATCTACGTCTGTGGTGACGCCAGTAGAATGGCC 2949
           *************** ******** ***************************** ************** 

CPRA  2923 AGAGACGTGCAGACCACAATTTCCAAGATTGTTGCTAAAAGCAGAGAAATTAGTGAAGACAAGGCTGCTG 2992
CPRB  2950 AGAGACGTCCAGACCACGATCTCCAAGATTGTTGCCAAAAGCAGAGAAATCAGTGAAGACAAGGCCGCTG 3019
           ******** ******** ** ************** ************** ************** ****

CPRA  2993 AATTGGTCAAGTCCTGGAAGGTCCAAAATAGATACCAAGAAGATGTTTGGTAGACTCAAACGAATCTCTC 3062
CPRB  3020 AATTGGTCAAGTCCTGGAAAGTCCAAAATAGATACCAAGAAGATGTTTGGTAGACTCAAACGAATCTCTC 3089
           ******************* **************************************************

CPRA  3063 TTTCTCCCAACGCATTTATGAATCTTTATTCTCATTGAAGCTTTACATATGTTCTACACTTTATTTTTTT 3132
CPRB  3090 TTTCTCCCAACGCATTTATGAA----TATTCTCATTGAAGTTTTACATATGTTCTATATTTCATTTTTTT 3155
           **********************    ************** *************** * ** ********

CPRA  3133 TTTTTTTTTTATTATTATATTACGAAACATAGGTCAACTATATATACTTGATTAAATGTTATAGAAACAA 3202
CPRB  3156 TTT----------ATTATATTACGAAACATAGGTCAACTATATATACTTGATTAAATGTTATAGAAACAA 3215
           ***          *********************************************************

CPRA  3203 TAACTATTATCTACTCGTCTACTTCTTTGGCATTGACATCAACATTACCGTTCCCATTACCGTTGCCGTT 3272
CPRB  3216 TAATTATTATCTACTCGTCTACTTCTTTGGCATTGGCATTGGCATTGGCATTGGCATTGCCGTTGCCGTT 3285
           *** ******************************* ***   ****  * **  **** ***********

CPRA  3273 GGCAATGCCGGGATATTTAGTACAGTATCTCCAATCCGGATTTGAGCTATTGTAGATCAGCTGCAAGTCA 3342
CPRB  3286 GGTAATGCCGGGATATTTAGTACAGTATCTCCAATCCGGATTTGAGCTATTGTAAATCAGCTGCAAGTCA 3355
           ** *************************************************** ***************

CPRA  3343 TTCTCCACCTTCAACCAGTACTTATACTTCATCTTTGACTTCAAGTCCAAGTCATAAATATTACAAGTTA 3412
CPRB  3356 TTCTCCACCTTCAACCAGTACTTATACTTCATCTTTGACTTCAAGTCCAAGTCATAAATATTACAAGTTA 3425
           **********************************************************************
```

Figure 13C

CPRA 3413 GCAAGAACTTCTGGCCATCCACGATATAGACGTTATTCACGTTATTATGCGACGTATGGATGTGGTTATC 3482
CPRB 3426 GCAAGAACTTCTGGCCATCCACAATATAGACGTTATTCACGTTATTATGCGACGTATGGATATGGTTATC 3495
********************** ************************************** ********

CPRA 3483 CTTATTGAACTTCTCAAACTTCAAAAACAACCCCACGTCCCGCAACGTCATTATCAACGACAAGTTCTGG 3552
CPRB 3496 CTTATTGAACTTCTCAAACTTCAAAAACAACCCCACGTCCCGCAACGTCATTATCAACGACAAGTTCTGA 3565
*********************************************************************

CPRA 3553 CTCACGTCGTCGGAGCTCGTCAAGTTCTCAATTAGATCGTTCTTGTTATTGATCTTCTGGTACTTTCTCA 3622
CPRB 3566 CTCACGTCGTCGGAGCTCGTCAAGTTCTCAATTAGATCGTTCTTGTTATTGATCTTCTGGTACTTTCTCA 3635
**********************************************************************

CPRA 3623 ATTGCTGGAACACATTGTCCTCGTTGTTCAAATAGATCTTGAACAACTTTTTCAACGGGATCAACTTCTC 3692
CPRB 3636 ACTGCTGGAACACATTGTCCTCGTTGTTCAAATAGATCTTGAACAACTTCTTCAAGGGAATCAACTTTTC 3705
* *********************************************** ***** ** ******** **

CPRA 3693 AATCTGGGCCAAGATCTCCGCCGGGATCTTCAGAAACAAGTCCTGCAACCCCTGGTCGATGGTCTCCGGG 3762
CPRB 3706 GATCTGGGCCAAGATTTCCGCCGGGATCTTCAGAAACAAGTCCTGCAACCCCTGGTCGATGGTCTCGGGG 3775
 ************** ************************************************** ***

CPRA 3763 TACAACAAGTCCAAGGGGCAGAAGTGTCTAGGCACGTGTTTCAACTGGTTCAACGAACATGTTCGACAGT 3832
CPRB 3776 TACAACAAGTCTAAGGGGCAGAAGTGTCTAGGCACGTGTTTCAACTGGTTCAAGGAACATGTTCGACAGT 3845
*********** ***************************************** ****************

CPRA 3833 AGTTCGAGTTATAGTTATCGTACAACCATTTTGGTTTGATTTCGAAAATGACGGAGCTGATGCCATCATT 3902
CPRB 3846 AGTTCGAGTTATAGTTATCGTACAACCACTTTGGCTTGATTTCGAAAATGACGGAGCTGATCCCATCATT 3915
**************************** ***** ************************** ********

CPRA 3903 CTCCTGGTTCCTCTCATAGTACAACTGGCACTTCTTCGAGAGGCTCAATTCCTCGTAGTTCCCGTCCAAG 3972
CPRB 3916 CTCCTGGTTCCTTTCATAGTACAACTGGCATTTCTTCGAGAGACTCAACTCCTCGTAGTTCCCGTCCAAG 3985
************ ***************** *********** ***** *********************

CPRA 3973 ATATTCGGCAACAAGAGCCCGTACCGCTCACGGAGCATCAAGTCGTGGCCCTGGTTGTTCAACTTGTTGA 4042
CPRB 3986 ATATTCGGCAACAAGAGCCCGTAGCGCTCACGGAGCATCAAGTCGTGGCCCTGGTTGTTCAACTTGTTGA 4055
*********************** **********************************************

CPRA 4043 TGAAGTCCGAGGTCAAGACAATCAACTGGATGTCGATGATCTGGTGCGGGAACAAGTTCTTGCATTTTAG 4112
CPRB 4056 TGAAGTCCGATGTCAAGACAATCAACTGGATGTCGATGATCTGGTGCGGAAACAAGTTCTTGCACTTTAG 4125
********** ************************************** ************** *****

CPRA 4113 CTCGATGAAGTCGTACAACTCACACGTCGAGATATACTCCTGTTCCTCCTTCAAGAGCCGGATCCGCAAG 4182
CPRB 4126 CTCGATGAAGTCGTACAACT 4145
********************

CPRA 4183 AGCTTGTGCTTCAAGTAGTCGTTG 4206
CPRB 4146 4145

Figure 13D

```
CPRA   MALDKLDLYVIITLVVAVAAYFAKNQFLDQPQDTGFLNTDSGSNSRDVLLTLKKNNKNTL   60
CPRB   MALDKLDLYVIITLVVAVAAYFAKNQFLDQPQDTGFLNTDSGSNSRDVLLTLKKNNKNTL   60

CPRA   LLFGSQTGTAEDYANKLSRELHSRFGLKTMVADFADYDWDNFGDITEDILVFFIVATYGE  120
CPRB   LLFGSQTGTAEDYANKLSRELHSRFGLKTMVADFADYDWDNFGDITEDILVFFIVATYGE  120

                               *
CPRA   GEPTDNADEFHTWLTEEADTLSTLKYTVFGLGNSTYEFFNAIGRKFDRLLSEKGGDRFAE  180
CPRB   GEPTDNADEFHTWLTEEADTLSTLRYTVFGLGNSTYEFFNAIGRKFDRLLSEKGGDRFAE  180

CPRA   YAEGDDGTGTLDEDFMAWKDNVFDALKNDLNFEEKELKYEPNVKLTERDDLSAADSQVSL  240
CPRB   YAEGDDGTGTLDEDFMAWKDNVFDALKNDLNFEEKELKYEPNVKLTERDDLSAADSQVSL  240

                                               *
CPRA   GEPNKKYINSEGIDLTKGPFDHTHPYLARITETRELFSSKDRHCIHVEFDISESNLKYTT  300
CPRB   GEPNKKYINSEGIDLTKGPFDHTHPYLARITETRELFSSKERHCIHVEFDISESNLKYTT  300

CPRA   GDHLAIWPSNSDENIKQFAKCFGLEDKLDTVIELKALDSTYTIPFPTPITYGAVIRHHLE  360
CPRB   GDHLAIWPSNSDENIKQFAKCFGLEDKLDTVIELKALDSTYTIPFPTPITYGAVIRHHLE  360

                               *            *                   *
CPRA   ISGPVSRQFFLSIAGFAPDEETKKAFTRLGGDKQEFAAKVTRRKFNIADALLYSSNNAPW  420
CPRB   ISGPVSRQFFLSIAGFAPDEETKKTFTRLGGDKQEFATKVTRRKFNIADALLYSSNNTPW  420

                  **
CPRA   SDVPFEFLIENVPHLTPRYYSISSSSLSEKQLINVTAVVEAEEEADGRPVTGVVTNLLKN  480
CPRB   SDVPFEFLIENIQHLTPRYYSISSSSLSEKQLINVTAVVEAEEEADGRPVTGVVTNLLKN  480

       *  *
CPRA   VEIVQNKTGEKPLVHYDLSGPRGKFNKFKLPVHVRRSNFKLPKNSTTPVILIGPGTGVAP  540
CPRB   IEIAQNKTGEKPLVHYDLSGPRGKFNKFKLPVHVRRSNFKLPKNSTTPVILIGPGTGVAP  540

CPRA   LRGFVRERVQQVKNGVNVGKTLLFYGCRNSNEDFLYKQEWAEYASVLGENFEMFNAFSRQ  600
CPRB   LRGFVRERVQQVKNGVNVGKTLLFYGCRNSNEDFLYKQEWAEYASVLGENFEMFNAFSRQ  600

CPRA   DPSKKVYVQDKILENSQLVHELLTEGAIIYVCGDASRMARDVQTTISKIVAKSREISEDK  660
CPRB   DPSKKVYVQDKILENSQLVHELLTEGAIIYVCGDASRMARDVQTTISKIVAKSREISEDK  660

CPRA   AAELVKSWKVQNRYQEDVW                                           680
CPRB   AAELVKSWKVQNRYQEDVW                                           680
```

Figure 14

# *C. tropicalis* 20336 *CYP52* DNA Alignment of DS Sequence

```
CYP52A1A      1                                                                         0
CYP52A2A      1 GACCTGTGACGCTTCCGGTGTCTTGCCACCAGTCTCCAAGTTGACCGACGCCCAAGTCATGTACCACTTT  70
CYP52A2B      1                                                                         0
CYP52A3A      1                                                             GACATCATAAT  11
CYP52A3B      1                                                                         0
CYP52A5A      1                                                                         0
CYP52A5B      1                                                            TTACAATCATGG  12
CYP52A8A      1                                                                         0
CYP52A8B      1                                                                         0
CYP52D4A      1                                                                         0

CYP52A1A      1             CATATGCGCTAATCTTCTTTTTCTTTTTATCACAGGAGAAACTATCCCACCCCCACTTC  59
CYP52A2A     71 ATTTCCGGTTACACTTCCAAGATGGCTGGTACTGAAGAAGGTGTCACGGAACCACAAGCTACTTTCTCCG 140
CYP52A2B      1                                                                         0
CYP52A3A     12 GACCCGGTTATTTCGCCCTCAGGTTGCTTATTTGAGCCGTAAAGTGCAGTAGAAACTTTGCCTTGGGTTC  81
CYP52A3B      1                                                                         0
CYP52A5A      1                                                                 TGGAGTC   7
CYP52A5B     13 AGCTCGCTAGGAACCCAGATGTCTGGGAGAAGCTCCGCGAAGAGGTCAACACGAACTTTGGCATGGAGTC  82
CYP52A8A      1                                                                         0
CYP52A8B      1                                                                         0
CYP52D4A      1                                                                         0

CYP52A1A     60 GAAACACAATGACAACTCCTGCGTAACTTGCAAATTCTTGTCTGACTAATTGAAAACTCCGGACGAGTCA 129
CYP52A2A    141 CTTGTTTCGGTCAACCATTCTTGGTGTTGCACCCAATGAAGTACGCTCAACAATTGTCTGACAAGATCTC 210
CYP52A2B      1                                             GCTCAACAATTGTCTGACAAGATCTC  26
CYP52A3A     82 AAACTCTAGTATAATGGTGATAACTGGTTGCACTCTTGCCATAGGCATGAAAATAGGCCGTTATAGTACT 151
CYP52A3B      1                                                                         0
CYP52A5A      8 GCCAGACTTGCTCACTTTTGACTCCCTTCGAAACTCAAAGTACGTTCAGGCGGTGCTCAACGAAACGCTC  77
CYP52A5B     83 GCCAGACTTGCTCACTTTTGACTCTCTTAGAAGCTCAAAGTACGTTCAGGCGGTGCTCAACGAAACGCTT 152
CYP52A8A      1                                                                         0
CYP52A8B      1                                            AAAACCGATACAAGAAGAAGACAGTCAA  28
CYP52D4A      1                                                                         0

CYP52A1A    130 GACCTCCAGTCAAACGGACAGACAGACAAACACTTGGTGCGATGTTCATACCTACAGACATGTCAACGGG 199
CYP52A2A    211 GCAACACAAGGCTAACGCCTGGTTGTTGAACACCGGTTGGGTTGGTTCTTCTGCTGCTAGAGGTGGTAAG 280
CYP52A2B     27 GCAACACAAGGCTAACGCCTGGTTGTTGAACACTGGTTGGGTTGGTTCTTCTGCTGCTAGAGGTGGTAAG  96
CYP52A3A    152 ATATTTAATAAGCGTAGGAGTATAGGATGCATATGACCGGTTTTTCTATATTTTTAAGATAATCTCTAGT 221
CYP52A3B      1                                                                CCTGCAGA   8
CYP52A5A     78 CGTATCTACCCGGGGGTACCACGAAACATGAAGACAG--CTACGTGCAACACGACGTTGCCACGCGGAGG 145
CYP52A5B    153 CGTATCTACCCGGGGGTGCCACGAAACATGAAGACAG--CTACGTGCAACACGACGTTGCCGCGTGGAGG 220
CYP52A8A      1                                                                         0
CYP52A8B     29 CAAGAACGTTAATGTCAACCAGGCGCCAAGAAGACGG--TTTGGCGGACTTGGAAGAATGTGGCATTTGC  96
CYP52D4A      1                                                                         0

CYP52A1A    200 TGTTAGACGACGGTTTCTTGCAAAGAC-AGGTGTTGGCATCTCGTACGATGGCAACTGCAGGAGGTGTCG 268
CYP52A2A    281 AGATGCTCATTGAAGTACACCAGAGCCATTTTGGACGCTATCCACTCTGGTGAATTGTCCAAGGTTGAAT 350
CYP52A2B     97 AGATGTTCATTGAAGTACACCAGAGCCATTTTGGACGCTATCCACTCTGGTGAATTGTCCAAGGTTGAAT 166
CYP52A3A    222 AAATTTTGTATTCTCAGTAGGATTTCATCAAATTTCGCAACCAATTCTGGCGAAAAAATGATTCTTTTAC 291
CYP52A3B      9 ATTCGCGGCCGCGTCGACAGAGTAGCAGTTATGCAAGCATGTGATTGTGGTTTTTGCAACCTGTTTGCAC  78
CYP52A5A    146 AGGCA-AAGACGGCAAGGAACCTATCT-TGGTGCAGAAGGGACAGTCCGTTGGGTTGATTACTATTGCCA 213
CYP52A5B    221 AGGCA-AAGACGGTAAGGAACCTATTT-TGGTGCAGAAGGGCCAGTCCGTTGGGTTGATTACTATTGCCA 288
CYP52A8A      1                                                                         0
CYP52A8B     97 CCATG-ATGTTTATGTTCTGGAGAGGT-TTTTCAAGGAATCGTCATCCTCCGCCACCACAAGAACCACCA 164
CYP52D4A      1                                                                         0
```

Figure 15A

```
CYP52A1A   269 ACTTCTCCTTTAGGCAATAGAAAAAGACTAAGAGAACAGCGTTTTTACAGGTTGCATTGGTTAATGTAGT  338
CYP52A2A   351 ACGAAACTTTCCCAGTCTTCAACTTGAATGTCCCAACCTCCTGTCCAGGTGTCCCAAGTGAAATCTTGAA  420
CYP52A2B   167 ACGAGACTTTCCCAGTCTTCAACTTGAATGTCCCAACCTCCTGCCCAGGTGTCCCAAGTGAAATCTTGAA  236
CYP52A3A   292 GTCAAAAGCTGA-ATAGTGCAGTTTAAAGCACCTAAAATCACATATACAGCCTCTAGATACGACAGAGAA  360
CYP52A3B    79 GACAAATGATCG-ACAGT-CGATT--ACGTAATCCATATTATTTAGAGGGGTAATAAAAAATAAATGGCA  144
CYP52A5A   214 CGCAGACGGACCCAGAGTATTTTGGGGCCGACGCTGGTGAGTTTAAGCCGGAGAGATGGTTTGATTCA--  281
CYP52A5B   289 CGCAGACGGACCCAGAGTATTTTGGGGCAGATGCTGGTGAGTTCAAACCGGAGAGATGGTTTGATTCA--  356
CYP52A8A     1                                                                          0
CYP52A8B   165 GTTAACGAGATCCATATTCACAACCCACCGCAAGGTGACAATGCTCAACAACAACAGCAACAACAACA--  232
CYP52D4A     1                                                                          0

CYP52A1A   339 ATTTTTTTAGTCCCAGCATTCTGTGGGTTGCTCTGGGTTTCTAGAATAGGAAATCACAGGAGAATGCAAA  408
CYP52A2A   421 CCCAACCAAGGCCTGGACCGG-AAGGTGTTGACTCCTTCAACAAGGAAATCAAGTCTTTGGCTGGTAAGT  489
CYP52A2B   237 CCCAACCAAGGCCTGGACCG--AAGGTGTTGACTCCTTCAACAAGGAAATCAAGTCTTTGGCTGGTAAGT  304
CYP52A3A   361 GCTCTTTATGATCTGAAGAAGCATTAGAATAGCT---ACTATGAGCCACTATTGGTGTATATATTAGGGA  427
CYP52A3B   145 GCC----AGAATTTCAAACATTTTGCAAACAATGCAAAAGATGAGAAACTCCAACAGAAAAAATAAAAAA  210
CYP52A5A   282 AGCATGAAGAACTTGGGGTGTAAATACTTGCCGTTCAATGCTGGGCCACGGACTTGCTTGGGGCAGCAGT  351
CYP52A5B   357 AGCATGAAGAACTTGGGGTGTAAGTACTTGCCGTTCAATGCTGGGCCCCGGACTTGTTTGGGGCAGCAGT  426
CYP52A8A     1                                                                          0
CYP52A8B   233 ACCCCCACAAGAACAGTGGAATAATGCCAGTCAA-CAAAGAGTGGTGACAGACGAGGGAGAAAACGCAAG  301
CYP52D4A     1                                                                          0

CYP52A1A   409 TTCAGATGGAAGAACAAAGAGATAAAAAACAAAAAAAAACTGAGTTTTGCACCAATAGAATGTTTG----  474
CYP52A2A   490 TTGCTGAAAAC--TTCAAGACCTATGCTGACCAAGCTACCGCTGA--AGTGAGAGCTGCAGGTCCAGAAG  555
CYP52A2B   305 TTGCTGAAAAC--TTCAAGACCTATGCTGACCAAGCTACCGCTGA--AGTTAGAGCTGCAGGTCCAGAAG  370
CYP52A3A   428 TTGGTGCAATTAAGTACGTACTAATAAACAGAAGAAAATACTTAACCAATTTCTGGTGTATACTTAGTGG  497
CYP52A3B   211 ACTCCGCAGC--ACTCCGAACCAACAAAACAATGGGGGGCGCCAG--AATTATTGAC---TATT------  267
CYP52A5A   352 ACACTTTGATTGAAGCGAGCTACTTGCTAGTCCGGTTGGCCCAGACCTAC-CGGGCAATAGATTTG----  416
CYP52A5B   427 ACACTTTGATTGAAGCGAGCTATTTGCTAGTCAGGTTGGCGCAGACCTAC-CGGGTAATCGATTTG----  491
CYP52A8A     1                                                                          0
CYP52A8B   302 CAACAGTGGTTCTGATGCAAGATCAGCTACACCGCTTCATCAGGAAAAGC-AGGAGCTCCCACCAC----  366
CYP52D4A     1         GATGTGGTGCTTGATTTCTCGAGACACATCCTTGTGAGGTGCCATGAATCTGTACCTG----   58

CYP52A1A   475 -ATGATATCATCCACTCGCTAAACGAATCATGTGGGTGATCTTCTCTTTAGTTTTGGTCTATCATAAAAC  543
CYP52A2A   556 CTTAAAGATATTTATTCATTATTTAGTTTGCCTATTTATTTCTCATTACCCATC-ATCATTCAACACTAT  624
CYP52A2B   371 CTTAAAGATATTTATTCACTATTTAGTTTGCCTATTTATTTCTCATCACCCATC-ATCATTCAACAATAT  439
CYP52A3A   498 -TGAGGGACCTTTTCTGAACATTCGGGTCAAACTTTTTTTTGGAGTGCGACATCGATTTTTCGTTTGTGT  566
CYP52A3B   268 ----GTGACTTTTTTTTATTTTTTCCGTTAA--CTTTCATTGCAGTGAAGTGT--GTTACACGGGGTGGT  329
CYP52A5A   417 -CAGCCAGGATCGGCGTACC-CACCAAGAAAGAAGTCGTTGATCAACATGAGTGCTGCCGACGGGGTGTT  484
CYP52A5B   492 -CTGCCAGGGTCGGCGTACC-CACCAAGAAAGAAGTCGTTGATCAATATGAGTGCTGCCGATGGGGTGGT  559
CYP52A8A     1                                                                          0
CYP52A8B   367 -CATATGCCCATCACGAGCAACACCAGCAGGTTAGTGTATAGTAGTCTGTAGTTAAGTCAATGCAATGTA  435
CYP52D4A    59 -TCTGTAAGCACAGGGAACTGCTTCAACACCTTATTGCATATTCTGTCTATTGCAAGCGTGTGCTGCAAC  127

CYP52A1A   544 ACATGAAAGTGAAATCCAAA-TACACTACACTCCGGGTATTGTCCTTCGTTTTACAGATGTCTCATTGTC  612
CYP52A2A   625 ATATAAAGTTACTTCGGA-----------TATCATTGTAATCGTGCGTGTCGCAATTGGATGATTTGGAA  683
CYP52A2B   440 ATATAAAGTTATTTCGGAAC-TCATA---TATCATTGTAATCGTGCGTGTTGCAATTGGGTAATTTGAAA  505
CYP52A3A   567 AATAATAGTGAACCTTTGTG-TAATAAATCTTCATGCAAGACTTGCATAATTCGAGCTTGGGAGTTCACG  635
CYP52A3B   330 GATGGTGTTGGTTTCTACAA-TGCAAGGGCACAGTTGAAGGTTTCCACATAACGT-TGCACCATATCAAC  397
CYP52A5A   485 TGT--AAAGCTTTATAAGGA-TGTAACGGTAGATGGATAGTTGTGTAGGAGGAGCGGAGATAAATTAGAT  551
CYP52A5B   560 TGT--AAAGTTTCACAAGGA-TCTAGATGGATATGTA-AGGTGTGTAGGAGGAGCGGAGATAAATTAGAT  625
CYP52A8A     1                                                                          0
CYP52A8B   436 CCA--ATAAGACTATCCCTT-CTTACAACCAAGTTTTCTGCCGCGCCTGTCTGGCA-ACAGATGCTGGCC  501
CYP52D4A   128 GATATCTGCCAAGGTATATAGCAGAACGTGCTGATGGTTCCTCCGGTCATATTCTGTTGGTAGTTCTGCA  197
```

Figure 15B

```
CYP52A1A   613 TTACTTTTGAGGTCATAGGAGTTGCCTGTGAGAGATCACAGAGATTATCACACTCACATTTATCGTAGTT  682
CYP52A2A   684 CTGCGCTTGAAACGGATTCATGCACGAAGCGGAGA-TAAAAGATTACGT---AATTTATCTCCTGAGACA  749
CYP52A2B   506 CTGTAGTTGGAACGGATTCATGCACGATGCGGAGA-TAACACG---------AGATTATCTCCTAAGACA  565
CYP52A3A   636 C--CAATTTGACCTCGTTCATGTGATAAAAGAAAAGCCAAAAGGTAATT---AGCAGACGC---AATGGG  697
CYP52A3B   398 T--CAATTTATCCTCATTCATGTGATAAAAGAAGAGCCAAAAGGTAATT---GGCAGACCCCCCAAGGGG  462
CYP52A5A   552 TTGATTTTG---TGTAAGGTTTTGGATGTCAACCTACTCCGCACTTCATGCA-GTGTGTGTGACACAAGG  617
CYP52A5B   626 TTGATTTTG---TGTAAGGTTTAGCACGTCAAGCTACTCCGCACTTTGT----GTGTAGGGAGCACA---  685
CYP52A8A     1                                                                          0
CYP52A8B   502 GACACACTT---TCAACTGAGTTTGGTCTAGAATTCTTGCACATGCACGACA-AGGAAACTCTTACAAAG  567
CYP52D4A   198 GGTAAATTTGGATGTCAGGTAGTGGAGGGAGGTTTGTATCGGTTGTGTT-TTCTTCTTCCTCTCTCTCTG  266

CYP52A1A   683 TCCTATCTCATGCTGTGTGTCTCTGGTTGGTTCATGAGTTTGGATT--GTTGTACATTAAAGGAATCGCT  750
CYP52A2A   750 ATTTTAGCCGTGTTCACACGCCCTTCTTTGTT-CTGAGCGAAGGAT--AAATAATTAGACTTCCACAGCT  816
CYP52A2B   566 ATTTTGGCCTCATTCACACGCCCTTCTT-----CTGAGCTAAGGAT--AAATAATTAGACTTCACAAGTT  628
CYP52A3A   698 AACATGGAGTGGAAAGCAATGGAAGCACGCCC-AGGACGGAGTAATTTAGTCCACACTACATCTGGGGGT  766
CYP52A3B   463 AACACGGAGTAGAAAGCAATGGAAACACGCCC-ATGACAGTGCCATTTAGCCCACAACACATCTAGTATT  531
CYP52A5A   618 GTGTACTACGTGTGCGTGTGCGCCAAGAGACA---GCCCAAGGGGG--TGGTAGTGT-GTGTTGGCGGAA  681
CYP52A5B   686 ---TACTCCGTCTGCGCCTGTGCCAAGAGACG---GCCCAGGGG-------TAGTGT-GTGGTGGTGGAA  741
CYP52A8A     1    GAATTCTTTGGATCTAATTCCAGCTGATC---TTGCTAATCCT--TATCAACGTAGTTGTGATCATT   62
CYP52A8B   568 --ACAACACTTGTGCTCTGATGCCACTTGATC---TTGCTAAGCCT--TATCAACGTAATTGAGATCATT  630
CYP52D4A   267 ATTCAACCTCCACGTCTCCTTCGGGTTCTGTGTCTGTGTCTGAGTC--GTACTGTTGGATTAAGTCCATC  334

CYP52A1A   751 GGAAAGCAAAGCTAACTAAATTTTCTTTGTCACAGGTACACTAACCTGTAAAACTTCACTGCCACGCCAG  820
CYP52A2A   817 CATTCTAATTTCCGT---CACGCGAATATTGAA-------------GGGGGGTACATGTGGCCGCTGAA-  869
CYP52A2B   629 CATTAAAATATCCGT---CACGCGAAAACTGCAACAATAAGGAAGGGGGGGGTAGACGTAGCCGATGAA-  694
CYP52A3A   767 ----TTTTTTTTTGTGCGCAAGTACACACCTGGACT-TTAGTTTTTGCCCCATAAAGTTAACAATCTAA-  830
CYP52A3B   532 CTTTTTTTTTTTTGTGCGCAGGTGCACACCTGGACT-TTAGTTATTGCCCCATAAAGTTAACAATCTCA-  599
CYP52A5A   682 GTGCATGTGACACA---ACGCGTGGGTTCTGGCCAATGGTGGACTAAGTGCAGGTAAGCAGCGACCTGAA  748
CYP52A5B   742 GTGCATGTGACACA---ATACCCTGGTTCTGGCCAATTGGGGATTTAGTGTAGGTAAGCTGCGACCTGAA  808
CYP52A8A    63 GTTTGTCTGAATTAT--ACACACCAGTGGAAGAATATGGTCTAATTTGCACGTCCCACTGGCATTGTG--  128
CYP52A8B   631 GTTTGTCTGAATTAT--ACACACCAGTGGAAGAATCTGGTCTAATCTGCACGCCTCATGGGCATTGTG--  696
CYP52D4A   335 GCATGTGTGAAAAAAAGTAGCGCTTATTTAGACAACCAGTTCGTTGGGCGGGTATCAGAAATAGTCTGTT  404

CYP52A1A   821 TCTTTCCTGATTGGGCAAGTGCACAAACTACA-ACCTGCAAAACAG----CACTCCGCTTGTCACAGGTT  885
CYP52A2A   870 -TGTGGGGG--CAGTAAACGCAGTCTCTC-------------------CTCTCCCAGGAATAGTGCAACGG  918
CYP52A2B   695 -TGTGGGGTGCCAGTAAACGCAGTCTCTCTCTCCCCCCCCCCCCCCCCCCCCTCAGGAATAGTACAACGG  763
CYP52A3A   831 -CCTTTGGC-TCTCCAACTCTCTCCGCCCCCAAATATTCGTTTTT-ACACCCTCAAGCTAGCGACAGCAC  897
CYP52A3B   600 -CCTTTGGC-TCTCCCAGTGTCTCCGCCTCCAGATGCTCGTTTT--ACACCCTCGAGCTAACGACAACAC  665
CYP52A5A   749 ACATTCCTCAACGCTTAAGACACTGGTGG-TAGAGATGCGGACCAGG-----CTATTCTTGTCGT-GCTA  811
CYP52A5B   809 ACACTCCTCAACGCTTGAGACACTGGTGGGTAGAGATGCGGGCCAGGA--GGCTATTCTTGTCGT-GCTA  875
CYP52A8A   129 -TGTTT-----GTGGGGGGGGGGGGGTGCACACATTTTTAGTGCCA----TTCTTTGTTGATTAC-CCCT  187
CYP52A8B   697 -TGTTTT--GGGGGGGGGGGGGGGGGTGCACACATTTTTAGTGCGAATGTTTGTTTGCTGGTTCC-CCCT  762
CYP52D4A   405 GTGCACGACCATGAGTATGCAACTTGACGAGACGTCGTTAGGA-----ATCCACAGAATGATAGCAGGAA  469

CYP52A1A   886 GTCTCCTCTCAACCAACAAAAAAATAAGATTAAACTTTCTTTGCTCATGCATCAATCGGAGTTATCTCTG  955
CYP52A2A   919 AGGAAGGATAACGGATAGAAAGCGGAATGCGAGGAAAAT--TTTGAACGCGCAAGAAAAGCAATATCCGG  986
CYP52A2B   764 GGGAAGGATAACGGATAGCAAGTGGAATGCGAGGAAAAT--TTTGAATGCGCAAGGAAAGCAATATCCGG  831
CYP52A3A   898 AACACCCATTAGAGGAATGGGGCAAAGTTAAACACTTTTGGCTTCAATGATTCCTATTCGCTACTACATT  967
CYP52A3B   666 AACACCCATGAGGGGAATGGG-CAAAGTTAAACACTTTTGGTTTCAATGATTCCTATTTGCTACT-----  729
CYP52A5A   812 CCCGGCGCATGGA-AAATCAACTGCGGGAAGAA--TAAATTTATCCGTAGAATCCACAGAGCG------G  872
CYP52A5B   876 CCCG-TGCACGGA-AAATCGATTGAGGGAAGAA--CAAATTTATCCGTGAAATCCACAGAGCG------G  935
CYP52A8A   188 CCCCCCTATCAT---TCATTCCCACAGGATTAG--TTTTTTCCTCACTGGAATTCGCTGTCC--------  244
CYP52A8B   763 CCCCCCTCCCCCCTATCATGCCCACAGGATTAG--TTTTTTCCTCACTGGAATTCGCTGTCC--------  822
CYP52D4A   470 GCTTACTACGTGAGAGATTCTGCTTAGAGGATG--TTCTCTTCTTGTTGATTCCATTAGGTGGGTATCAT  537
```

Figure 15C

```
CYP52A1A   956 A--AAGAGTTGCCTTTGTGTAATGTGTGCCAAA-CTCAAACTGCAAAACTAACCACAGAATGAT------ 1016
CYP52A2A   987 GCTACCAGGTTTTGAGCCAGGGAACACACTCCTATTTCTGCTCAATGACTGAACATAGAAAAAA------ 1050
CYP52A2B   832 GCTATCAGGTTTTGAGCCAGGGGACACACTCCT-CTTCTGCACAAAAACTTAACGTAGACAAAAAAAAAA  900
CYP52A3A   968 CTTCTCTTGTTTTGTGCTTTGAATTGCACCATGTGAAATAAACGACAATTATATATACCTTTTCATC--- 1034
CYP52A3B   730 ---CTCTTGTTTTGTGTTTTGATTTGCACCATGTGAAATAAACGACAATTATATATACCTTTTCGTC---  793
CYP52A5A   873 A--TAAATTTGCCCACCTCCATCATCAACCACG-CCGCCACTAACTACATCACTCCCCTATTTT------  933
CYP52A5B   936 A--TAAATTTGTCACATTGCTGCGTTGCCCAC------------CCACAGCATTCTC-------------  978
CYP52A8A   245 ------ACCTGTCAACCCCCCCCCCCCCCCCC-CCACTGCC--CTACCCTGCCCTGC------------  293
CYP52A8B   823 ------ACCTGTCAACCCCCTCAC---------------------TGCCCTGCCCTGC------------  853
CYP52D4A   538 CTCCGGTGGTGACAACTTGACACAAGCAGTTCCGAGAACCACCCACAACAATCACCATTCCAGC------  601
                        *

CYP52A1A  1017 TTCCCTCACAATTATATAAACTCACCCACATTTCCACAGACCGTAATTTCATGTCTCAC-TTTCTCTTTT 1085
CYP52A2A  1051 -----CACCAAGACGCAATGAAACGCACATGGACATTTAGACCTCCCCACATGTGATAGTTTGTCTTAAC 1115
CYP52A2B   901 AACTCCACCAAGACACAATGAATCGCACATGGACATTTAGACCTCCCCACATGTGAAAGCTTCTCTGGCG  970
CYP52A3A  1035 CCTCCTCCTATATCTCTTTTTGCTAC-ATTTTGTTTTTTACGTTTCTTGCTTTTGCACTCTCCCACTCCC 1103
CYP52A3B   794 TGTCCTCCAATGTCTCTTTTTGCTGCCATTTTGCTTTTTGCTTTTTGCTTTTGCACTCTCTCCCACTCCC  863
CYP52A5A   934 CTCTCTCTCTCTTTGTCTTACTCCGCTCCCGTTTCCTTAGCCACAGATACACACCCACT-GCAAACAGCA 1002
CYP52A5B   979 TTTTCTCTCTCTTTGTCTTACTCCGCTCCTGTTTCCTTATCCAGAAATACACACCAACTCATATAAAGAT 1048
CYP52A8A   294 CCTGCACGTCCTGTGTTTTGTGCTGTGTCTTTCCCACGCTATAAAAGCCCTGGCGTCCGGCCAAGGTTTT  363
CYP52A8B   854 CCTGCACGCCCTGTGTTTTGTGCTGTGGCACTCCCACGCTATAAAAGCCCTGGCGTACGGCCAAGGTTTT  923
CYP52D4A   602 TATCACTTCTACATGTCAACCTACGATGTATCTCATCACCATCTAGTTTCTTGGCAATCGTTTATTTGTT  671

CYP52A1A  1086 GCTCTTCTTTTACTTAGTCAGGTTTGATAACTTCCTTTTTTATTACCCTATCTTATTTATTTATTTATTC 1155
CYP52A2A  1116 AGA------AAAGTATAATAAGAACCCATGCCGTCCCTTTTCTTTCGCCGCTTCAACTTTTTTTTTTTTA 1179
CYP52A2B   971 AAAGCAAAAAAAGTATAATAAGGACCCATGCCTTCCCTCTTCCTGGGCCGTTTCAACTTTTTCTTTTTCT 1040
CYP52A3A  1104 ACAA---------------------------AGAAAAAAAAACTACACTATGTCGTCTTCTCCATCGTTT 1146
CYP52A3B   864 ACAATCAGTGCAGCAACACACAAAGAAGAAAAATAAAAAAACCTACACTATGTCGTCTTCTCCATCGTTT  933
CYP52A5A  1003 GCA--ACAATTATAAAGATACGCC-----AGGCCCACCTTCTTTCTTTTTCTTCACTTTTTTGACTGC-A 1064
CYP52A5B  1049 ACG--CTAGCCCAGCTGTCTTTCT-----TTTTCTTCACTTTTTTGGTGTGTTGCTTTTTTGGCTGC-T 1110
CYP52A8A   364 TCCACCCAGCCAAAAAAACAGTCTAAAAAATTTGGTTGATCCTTTTTGGTTGCAAGGTTTT---CCAC-C  429
CYP52A8B   924 TCCTCACAGCCAAAAAAA----------AATTTGGCTGATCCTTTTGGGCTGCAAGGTTTTTCACCAC-C  982
CYP52D4A   672 ATGGGTCAACATCCAATACAACTCCACCAA--TGAAGAAGAAAAACGGAAAGCAGAATACCAGAATGACA  739
                                                                        *

CYP52A1A  1156 ATTTATACCAACCAACC--AACCATGGCCACACAAGAAATCATCGATTCTGTACTTCCGTACTTGACCAA 1223
CYP52A2A  1180 TCTT------------ACACACATCACGACCA-TGACTGTACACGATATTATCGCCACATACTTCACCAA 1236
CYP52A2B  1041 TTGTCTATCAACACACACACACCTCACGACCA-TGACTGCACAGGATATTATCGCCACATACATCACCAA 1109
CYP52A3A  1147 GCCC-------AAGAGGTTCTCGCTACCACTAGTCCTTACATCGAGTACTTTCTTGACA-ACTACACCAG 1208
CYP52A3B   934 GCTC-------AGGAGGTTCTCGCTACCACTAGTCCTTACATCGAGTACTTTCTTGACA-ACTACACCAG  995
CYP52A5A  1065 ACTTTCTACAATCCACCACAGCCACCACCACAGCCGCTATGATTGAACAACTCCTAGAATATT------- 1127
CYP52A5B  1111 ACTTTCTACAACC-------ACCACCACCACCACCACCATGATTGAACAAATCCTAGAATATT------- 1166
CYP52A8A   430 ACCACTTCCACCA--CCTCAACTATTCGAACAA--AAGATGCTCGATCAGATCTTACATTACT-------  488
CYP52A8B   983 ACCACCACCACCA--CCTCAACTATTCAAACAA--AGGATGCTCGACCAGATCTTCCATTACT------- 1041
CYP52D4A   740 GTGTG----AGTTCCTGACCATTGCTAATCTA-TGGCTATATCTAGTTTGCTATCGTGGATG-------  797
                                                                  *        *

CYP52A1A  1224 ATGGTACACTGTGATTACTGCAGCAGTATTAGTCTTCCTTATCTCCACAAACATCAAGAACTACGTCAAG 1293
CYP52A2A  1237 ATGGTACGTGATAGTACCACTCGCTTTGATTGCTTATAGAGTCCTCGACTACTTCTATGGCAGATACTTG 1306
CYP52A2B  1110 ATGGTACGTGATAGTACCACTCGCTTTGATTGCTTATAGGGTCCTCGACTACTTTTACGGCAGATACTTG 1179
CYP52A3A  1209 ATGGTACTACTTCATACCTTTGGTGCTTCTTTCGTTGAACTTTATAAGTTTGCTCCACACAAGGTACTTG 1278
CYP52A3B   996 ATGGTACTACTTCATCCCTTTGGTGCTTCTTTCGTTGAACTTCATCAGCTTGCTCCACACAAAGTACTTG 1065
CYP52A5A  1128 --GGTATGTCGTTGTGCCAGTGTTGTACATCATCAAACAACTCCTTGCATACACAAAGACTCGCGTCTTG 1195
CYP52A5B  1167 --GGTATATTGTTGTGCCTGTGTTGTACATCATCAAACAACTCATTGCCTACAGCAAGACTCGCGTCTTG 1234
CYP52A8A   489 --GGTACATTGTCTTGCCATTGTTGGCCATTATCAACCAGATCGTGGCTCATGTCAGGACCAATTATTTG  556
CYP52A8B  1042 --GGTACATTGTCTTGCCATTGTTGGTCATTATCAAGCAGATCGTGGCTCATGCCAGGACCAATTATTTG 1109
CYP52D4A   798 -TGATCTGTGTCGTCTTCATTTGCGTTTGTGTTTATTTCGGGTAT-GAATATTGTTATACTAAATACTTG  865
                  * *            *          *                                       *
```

Figure 15D

```
CYP52A1A  1294 GCAAAGAAATTGAAATGTGTCGATCCACCATACTTGAAGGATGCCGGTCTCACTGGTATTCTGTCTTTGA 1363
CYP52A2A  1307 ATGTACAAGCTTGGTGCTAAACCATTTTTCCAGAAACAGACAGACGGCTGTTTCGGATTCAAAGCTCCGC 1376
CYP52A2B  1180 ATGTACAAGCTTGGTGCTAAACCGTTTTTCCAGAAACAAACAGACGGTTATTTCGGATTCAAAGCTCCAC 1249
CYP52A3A  1279 GAACGCAGGTTCCACGCCAAGCCACTCGGTAACTTTGTCAGGGACCCTACGTTTGGTATCGCTACTCCGT 1348
CYP52A3B  1066 GAACGCAGGTTCCACGCCAAGCCGCTCGGTAACGTCGTGTTGGATCCTACGTTTGGTATCGCTACTCCGT 1135
CYP52A5A  1196 ATGAAAAAGTTGGGTGCTGCTCCAGTCACAAACAAGTTGTACGACAACGCTTTCGGTATCGTCAATGGAT 1265
CYP52A5B  1235 ATGAAACAGTTGGGTGCTGCTCCAATCACAAACCAGTTGTACGACAACGTTTTCGGTATCGTCAACGGAT 1304
CYP52A8A   557 ATGAAGAAATTGGGTGCTAAGCCATTCACACACGTCCAACGTGACGGGTGGTTGGGCTTCAAATTCGGCC  626
CYP52A8B  1110 ATGAAGAAGTTGGGCGCTAAGCCATTCACACATGTCCAACTAGACGGGTGGTTTGGCTTCAAATTTGGCC 1179
CYP52D4A   866 ATGCACAAACATGGCGCTCGAGAAATCGAGAATGTGATCAACGATGGGTTCTTTGGGTTCCGCTTACCTT  935
                                              *          *           **  *

CYP52A1A  1364 TCGCCGCCATCAAGGCCAAGAACGACGGTAG-ATTGGCTAACTTTGCC------GATGAAGTTTT----- 1421
CYP52A2A  1377 TTGAATTGTTGAAGAAGAAGAGCGACGGTAC-CCTCATAGACTTCACA------CTCCAGCGTATC---C 1436
CYP52A2B  1250 TTGAATTGTTAAAAAAGAAGAGTGACGGTAC-CCTCATAGACTTCACT------CTCGAGCGTATC---C 1309
CYP52A3A  1349 TGCTTTTGATCTACTTGAAGTCGAAAGGTAC-GGTCATGAAGTTTGCTTGGGGCCTCTGGAACAACAAGT 1417
CYP52A3B  1136 TGATCTTGATCTACTTAAAGTCGAAAGGTAC-AGTCATGAAGTTTGCCTGGAGCTTCTGGAACAACAAGT 1204
CYP52A5A  1266 GGAAGGCTCTCCAGTTCAAGAAAGAGGGCAGGGCTCAAGAGTACAACG------ATTACAAGTTTG---- 1325
CYP52A5B  1305 GGAAGGCTCTCCAGTTCAAGAAAGAGGGCAGAGCTCAAGAGTACAACG------ATCACAAGTTTG---- 1364
CYP52A8A   627 GTGAATTCCTCAAAGCAAAAAGTGCTGGGAG-ACTGGTTGATTTAATC------ATCTCCCGTTT-----  684
CYP52A8B  1180 GTGAATTCCTCAAAGCTAAAAGTGCTGGGAG-GCAGGTTGATTTAATC------ATCTCCCGTTT----- 1237
CYP52D4A   936 TGCTACTCATGCGAGCCAGCAATGAGGGCCG-ACTTATCGAGTTCAGT------GTCAAGAGATTCGAGT  998
                        *       *        **

CYP52A1A  1422 ----CGACGAGTACCCAAACCACACCTTCTACTTGTCTGTTGCCGGTGCTTTGAAGATTGTCATGACTGT 1487
CYP52A2A  1437 ACGATCTCGATCGTCCCGATATCCCAACTTTCACATTCCCGGTCTTTTCCATCAACCTTGTCAATACCCT 1506
CYP52A2B  1310 AAGCGCTCAATCGTCCAGATATCCCAACTTTACATTCCCAATCTTTTCCATCAACCTTATCAGCACCCT 1379
CYP52A3A  1418 ACATCGTCAGAGACCCAAAGTACAAGACAACTGGGCTCAGGATTGTTGGCCTCCCATTGATTGAAACCAT 1487
CYP52A3B  1205 ACATTGTCAAAGACCCAAAGTACAAGACCACTGGCCTTAGAATTGTCGGCCTCCCATTGATTGAAACCAT 1274
CYP52A5A  1326 ACCACTCCAAGAACCCAAGCGTGGGCACCTACGTCAGTATTCTTTTCGGCACCAGGATCGTCGTGACCAA 1395
CYP52A5B  1365 ACAGCTCCAAGAACCCAAGCGTCGGCACCTATGTCAGTATTCTTTTTGGCACCAAGATTGTCGTGACCAA 1434
CYP52A8A   685 ------CCACGA----TAATGAGGACACTTTCTCCAGCTATGCTTTTGGCAACCATGTGGTGTTCACCAG  744
CYP52A8B  1238 ------CCACGA----TAATGAGGACACTTTCTCCAGCTATGCTTTTGGCAACCATGTGGTGTTCACCAG 1297
CYP52D4A   999 -CGGCGCCACAT--CCACAGAACAAGACATTGGTCAACCGGGCATTGAGCGTTCCTGTGATACTCACCAA 1065
                      *                                                 *  *    **

CYP52A1A  1488 TGACCCAGAAAACATCAAGGCTGTCTTGGCCACCCAATTCACTGACTTCTCCTTGGGTACCAGACACGCC 1557
CYP52A2A  1507 TGAGCCGGAGAACATCAAGGCCATCTTGGCCACTCAGTTCAACGATTTCTCCTTGGGTACCAGACACTCG 1576
CYP52A2B  1380 TGAGCCGGAGAACATCAAGGCTATCTTGGCCACCCAGTTCAACGATTTCTCCTTGGGCACCAGACACTCG 1449
CYP52A3A  1488 GGACCCAGAGAACATCAAGGCTGTTTTGGCTACTCAGTTCAATGATTTCTCTTTGGGAACCAGACACGAT 1557
CYP52A3B  1275 AGACCCAGAGAACATCAAAGCTGTGTTGGCTACTCAGTTCAACGATTTCTCCTTGGGAACTAGACACGAT 1344
CYP52A5A  1396 AGATCCAGAGAATATCAAAGCTATTTTGGCAACCCAGTTTGGTGATTTTTCTTTGGGCAAGAGGCACACT 1465
CYP52A5B  1435 GGATCCAGAGAATATCAAAGCTATTTTGGCAACCCAGTTTGGCGATTTTTCTTTGGGCAAGAGACACGCT 1504
CYP52A8A   745 GGACCCCGAGAATATCAAGGCGCTTTTGGCAACCCAGTTTGGTGATTTTTCATTGGGCAGCAGGGTCAAG  814
CYP52A8B  1298 GGACCCCGAGAATATCAAGGCGCTTTTGGCAACCCAGTTTGGTGATTTTTCATTGGGAAGCAGGGTCAAA 1367
CYP52D4A  1066 GGACCCAGTGAATATCAAAGCGATGCTATCGACCCAGTTTGATGACTTTTCCCTTGGGTTGAGACTACAC 1135
                ** ** *  ** ***** **  *  *  * ** ** **    ** ** **  * **    **

CYP52A1A  1558 CACTTTGCTCCTTTGTTGGGTGACGGTATCTTCACCTTGGACGGAGAAGGTTGGAAGCACTCCAGAGCTA 1627
CYP52A2A  1577 CACTTTGCTCCTTTGTTGGGTGATGGTATCTTTACGTTGGATGGCGCCGGCTGGAAGCACAGCAGATCTA 1646
CYP52A2B  1450 CACTTTGCTCCTTTGTTGGGCGATGGTATCTTTACCTTGGACGGTGCCGGCTGGAAGCACAGCAGATCTA 1519
CYP52A3A  1558 TTCTTGTACTCCTTGTTGGGTGACGGTATTTTCACCTTGGACGGTGCTGGCTGGAAACATAGTAGAACTA 1627
CYP52A3B  1345 TTCTTGTACTCCTTGTTGGGCGATGGTATTTTTACCTTGGACGGTGCTGGCTGGAAACACAGTAGAACTA 1414
CYP52A5A  1466 CTTTTTAAGCCTTTGTTAGGTGATGGGATCTTCACATTGGACGGCGAAGGCTGGAAGCACAGCAGAGCCA 1535
CYP52A5B  1505 CTTTTTAAACCTTTGTTAGGTGATGGGATCTTCACCTTGGACGGCGAAGGCTGGAAGCATAGCAGATCCA 1574
CYP52A8A   815 TTCTTCAAACCATTATTGGGGTACGGTATCTTCACATTGGACGCCGAAGGCTGGAAGCACAGCAGAGCCA  884
CYP52A8B  1368 TTCTTCAAACCATTGTTGGGGTACGGTATCTTCACCTTGGACGGCGAAGGCTGGAAGCACAGCAGAGCCA 1437
CYP52D4A  1136 CAGTTTGCGCCGTTGTTGGGGAAAGGCATCTTTACTTTGGACGGCCCAGAGTGGAAGCAGAGCCGATCTA 1205
                  **     * ** ** **  * ** ** ** ** ***** *     *  ***** **     ** * *
```

Figure 15E

```
CYP52A1A  1628 TGTTGAGACCACAGTTTGCTAGAGACCAGATTGGACACGTTAAAGCCTTGGAACCACACATCCAAATCAT 1697
CYP52A2A  1647 TGTTGAGACCACAGTTTGCCAGAGAACAGATTTCCCACGTCAAGTTGTTGGAGCCACACGTTCAGGTGTT 1716
CYP52A2B  1520 TGTTGAGACCACAGTTTGCCAGAGAACAGATTTCCCACGTCAAGTTGTTGGAGCCACACATGCAGGTGTT 1589
CYP52A3A  1628 TGTTGAGACCACAGTTTGCTAGAGAACAGGTTTCTCACGTCAAGTTGTTGGAGCCACACGTTCAGGTGTT 1697
CYP52A3B  1415 TGTTGAGACCACAGTTTGCTAGAGAACAGGTTTCCCACGTCAAGTTGTTGGAACCACACGTTCAGGTGTT 1484
CYP52A5A  1536 TGTTGAGACCACAGTTTGCCAGAGAACAAGTTGCTCATGTGACGTCGTTGGAACCACACTTCCAGTTGTT 1605
CYP52A5B  1575 TGTTAAGACCACAGTTTGCCAGAGAACAAGTTGCTCATGTGACGTCGTTGGAACCACACTTCCAGTTGTT 1644
CYP52A8A   885 TGTTGAGACCACAGTTTGCCAGAGAACAAGTTGCTCATGTGACGTCGTTGGAACCACACTTCCAGTTGTT  954
CYP52A8B  1438 TGTTGAGACCACAGTTTGCCAGAGAGCAAGTTGCTCATGTGACGTCGTTGGAACCACATTTCCAGTTGTT 1507
CYP52D4A  1206 TGTTGCGTCCGCAATTTGCCAAAGATCGGGTTTCTCATATCCTGGATCTAGAACCGCATTTTGTGTTGCT 1275
               ****  * ** ** ***** * *** *   **    **  *        * ** ** **  *     *  *

CYP52A1A  1698 GGCTAAGCAGATCAAGTTGAACCAGGGAAAGACTTTCGATATCCAAGAATTGTTCTTTAGATTTACCGTC 1767
CYP52A2A  1717 CTTCAAACACGTCAGAAAGGCACAGGGCAAGACTTTTGACATCCAGGAATTGTTTTTCAGATTGACCGTC 1786
CYP52A2B  1590 CTTCAAGCACGTCAGAAAGGCACAGGGCAAGACTTTTGACATCCAAGAATTGTTTTTCAGATTGACCGTC 1659
CYP52A3A  1698 CTTCAAGCACGTTAGAAAGCACCGCGGTCAAACGTTCGACATCCAAGAATTGTTCTTCAGGTTGACCGTC 1767
CYP52A3B  1485 CTTCAAGCACGTTAGAAAACACCGCGGTCAGACTTTTGACATCCAAGAATTGTTCTTCAGATTGACCGTC 1554
CYP52A5A  1606 GAAGAAGCATATTCTTAAGCACAAGGGTGAATACTTTGATATCCAGGAATTGTTCTTTAGATTTACCGTT 1675
CYP52A5B  1645 GAAGAAGCATATCCTTAAACACAAGGGTGAGTACTTTGATATCCAGGAATTGTTCTTTAGATTTACTGTC 1714
CYP52A8A   955 GAAGAAGCATATCCTTAAACACAAGGGTGAGTACTTTGATATCCAGGAATTGTTCTTTAGATTTACTGTC 1024
CYP52A8B  1508 GAAGAAGCATATTCTTAAGCACAAGGGTGAATACTTTGATATCCAGGAATTGTTCTTTAGATTTACCGTT 1577
CYP52D4A  1276 TCGGAAGCACATTGATGGCCACAATGGAGACTACTTCGACATCCAGGAGCTCTACTTCCGGTTCTCGATG 1345
                    ** **   *               **  *    ** ** ***** **   *  *   **   *  **  *  *

CYP52A1A  1768 GACACCGCTACTGAGTTCTTGTTTGGTGAATCCGTTCACTCCTTGTACGATGAAAAATTGGGCATCCCAA 1837
CYP52A2A  1787 GACTCCGCCACCGAGTTTTTGTTTGGTGAATCCGTTGAGTCCTTGAGAGATGAATCTATCGGCATGTCCA 1856
CYP52A2B  1660 GACTCCGCCACTGAGTTTTTGTTTGGTGAATCCGTTGAGTCCTTGAGAGATGAATCTATTGGGATGTCCA 1729
CYP52A3A  1768 GACTCCGCCACCGAGTTCTTGTTTGGTGAGTCTGCTGAATCCTTGAGGGACGAATCTATTGGATTGACCC 1837
CYP52A3B  1555 GACTCCGCCACCGAGTTCTTGTTTGGTGAGTCTGCTGAATCCTTGAGAGACGACTCTGTTGGTTTGACCC 1624
CYP52A5A  1676 GATTCGGCCACGGAGTTCTTATTTGGTGAGTCCGTGCACTCCTTAAAGGACGAATCTATTGGTATCAACC 1745
CYP52A5B  1715 GACTCGGCCACGGAGTTCTTATTTGGTGAGTCCGTGCACTCCTTAAAGGACGAAACTATCGGTATCAACC 1784
CYP52A8A  1025 GACTCGGCCACGGAGTTCTTATTTGGTGAGTCCGTGCACTCCTTAAAGGACGAGGAAATTGGCTACGACA 1094
CYP52A8B  1578 GATTCAGCGACGGAGTTCTTATTTGGTGAGTCCGTGCACTCCTTAAGGGACGAGGAAATTGGCTACGATA 1647
CYP52D4A  1346 GATGTGGCGACGGGGTTTTTGTTTGGCGAGTCTGTGGGGTCGTTGAAAGACGAAGATGCGAGG------- 1408
               **     ** **  * *** ** ***** ** ** *      ** **    ** **        *

CYP52A1A  1838 CTCCAAACGAAA---TCCCAGGAAGAGAAAACTTTGCCGCTGCTTTCAACGTTTCCCAACACTACTTGGC 1904
CYP52A2A  1857 TCAATGCGCTTGACTTTGACGGCAAGGCTGGCTTTGCTGATGCTTTTAACTATTCGCAGAATTATTTGGC 1926
CYP52A2B  1730 TCAATGCACTTGACTTTGACGGCAAGGCTGGCTTTGCTGATGCTTTTAACTACTCGCAGAACTATTTGGC 1799
CYP52A3A  1838 CAACCACCAAGGATTTCGATGGCAGAAGAGATTTCGCTGACGCTTTCAACTATTCGCAGACTTACCAGGC 1907
CYP52A3B  1625 CAACCACCAAGGATTTCGAAGGCAGAGGAGATTTCGCTGACGCTTTCAACTACTCGCAGACTTACCAGGC 1694
CYP52A5A  1746 AAGACGATATAGATTTTGCTGGTAGAAAGGACTTTGCTGAGTCGTTCAACAAAGCCCAGGAATACTTGGC 1815
CYP52A5B  1785 AAGACGATATAGATTTTGCTGGTAGAAAGGACTTTGCTGAGTCGTTCAACAAAGCCCAGGAGTATTTGTC 1854
CYP52A8A  1095 CGAAAGACATGT---CTGAAGAAAGACGCAGATTTGCCGACGCGTTCAACAAGTCGCAAGTCTACGTGGC 1161
CYP52A8B  1648 CGAAGGACATGG---CTGAAGAAAGACGCAAATTTGCCGACGCGTTCAACAAGTCGCAAGTCTATTTGTC 1714
CYP52D4A  1409 ---------------------------------TTCCTGGAAGCATTCAATGAGTCGCAGAAGTATTTGGC 1446
                                                **    *   * ** **     * **    **  * *

CYP52A1A  1905 CACCAGAAGTTACTCCCAGACTTTTTACTTTTTGACCAACCCTAAGGAATTCAGAGACTGTAACGCCAAG 1974
CYP52A2A  1927 TTCGAGAGCGGTTATGCAACAATTGTACTGGGTGTTGAACGGGAAAAAGTTTAAGGAGTGCAACGCTAAA 1996
CYP52A2B  1800 TTCGAGAGCGGTTATGCAACAATTGTACTGGGTGTTGAACGGGAAAAAGTTTAAGGAGTGCAACGCTAAA 1869
CYP52A3A  1908 CTACAGATTTTTGTTGCAACAAATGTACTGGATCTTGAATGGCTCGGAATTCAGAAAGTCGATTGCTGTC 1977
CYP52A3B  1695 CTACAGATTTTTGTTGCAACAAATGTACTGGATTTTGAATGGCGCGGAATTCAGAAAGTCGATTGCCATC 1764
CYP52A5A  1816 TATTAGAACCTTGGTGCAGACGTTCTACTGGTTGGTCAACAACAAGGAGTTTAGAGACTGTACCAAGCTG 1885
CYP52A5B  1855 TATTAGAATTTTGGTGCAGACCTTCTACTGGTTGATCAACAACAAGGAGTTTAGAGACTGTACCAAGCTG 1924
CYP52A8A  1162 CACCAGAGTTGCTTTACAGAACTTGTACTGGTTGGTCAACAACAAAGAGTTCAAGGAGTGCAATGACATT 1231
CYP52A8B  1715 CACCAGAGTTGCTTTACAGACATTGTACTGGTTGGTCAACAACAAAGAGTTCAAGGAGTGCAACGACATT 1784
CYP52D4A  1447 AACTAGGGCAACGTTGCACGAGTTGTACTTCTTTGTGACGGGTTTAGGTTTCGCCAGTACAACAAGGTT 1516
                    **           **     * ****    *      *            **     * *  *
```

Figure 15F

```
CYP52A1A  1975 GTCCACCACTTGGCCAAGTACTTTGTCAACAAGGCCTTGAACTTTACTCCTGAAGAACTCGAAGAGAAAT 2044
CYP52A2A  1997 GTGCACAAGTTTGCTGACTACTACGTCAACAAGGCTTTGGACTTGACGCCTGAACAATTGGAAAAGCAGG 2066
CYP52A2B  1870 GTGCACAAGTTTGCTGACTATTACGTCAGCAAGGCTTTGGACTTGACACCTGAACAATTGGAAAAGCAGG 1939
CYP52A3A  1978 GTGCACAAGTTTGCTGACCACTATGTGCAAAAGGCTTTGGAGTTGACCGACGATGACTTGCAGAAACAAG 2047
CYP52A3B  1765 GTGCACAAGTTTGCTGACCACTATGTGCAAAAGGCTTTGGAGTTGACCGACGATGACTTGCAGAAACAAG 1834
CYP52A5A  1886 GTGCACAAGTTCACCAACTACTATGTTCAGAAAGCTTTGGATGCTAGCCCAGAAGAGCTTGAAAAGCAAA 1955
CYP52A5B  1925 GTGCACAAGTTTACCAACTACTATGTTCAGAAAGCTTTGGATGCTACCCCAGAGGAACTTGAAAAGCAAG 1994
CYP52A8A  1232 GTCCACAAGTTTACCAACTACTATGTTCAGAAAGCCTTGGATGCTACCCCAGAGGAACTTGAAAAGCAAG 1301
CYP52A8B  1785 GTCCACAAGTTCACCAACTACTATGTTCAGAAAGCCTTGGATGCTACCCCAGAGGAACTTGAAAAACAAG 1854
CYP52D4A  1517 GTGCGAAAGTTCTGCAGCCAGTGTGTCCACAAGGCGTTAGATGTTGCACCGGAAGACACC---------A 1577
               ** *   * **        * *  **    ** ** **  *          **  *

CYP52A1A  2045 CCAAGTCCGGTTACGTTTCTTGTACGAATTGGTTAAGCAAACCAGAGATCCAAAGGTCTTGCAAGATCA 2114
CYP52A2A  2067 ATGGTT------ATGTGTTTTTGTACGAATTGGTCAAGCAAACCAGAGACAAGCAAGTGTTGAGAGACCA 2130
CYP52A2B  1940 ATGGTT------ATGTGTTCTTGTACGAGTTGGTCAAGCAAACCAGAGACAGGCAAGTGTTGAGAGACCA 2003
CYP52A3A  2048 ACGGCT------ATGTGTTCTTGTACGAGTTGGCTAAGCAAACCAGAGACCCAAAGGTCTTGAGAGACCA 2111
CYP52A3B  1835 ACGGCT------ATGTGTTCTTGTACGAGTTGGCTAAGCAAACTAGAGACCCAAAGGTCTTGAGAGACCA 1898
CYP52A5A  1956 GTGGGT------ATGTGTTCTTGTACGAGCTTGTCAAGCAGACAAGAGACCCCAATGTGTTGCGTGACCA 2019
CYP52A5B  1995 GCGGGT------ATGTGTTCTTGTATGAGCTTGTCAAGCAGACGAGAGACCCCAAGGTGTTGCGTGACCA 2058
CYP52A8A  1302 GCGGGT------ATGTGTTCTTGTATGAGCTTGTCAAGCAGACGAGAGACCCCAAGGTGTTGCGTGACCA 1365
CYP52A8B  1855 GCGGGT------ATGTGTTCTTGTACGAGCTTGCCAAGCAGACGAAAGACCCCAATGTGTTGCGTGACCA 1918
CYP52D4A  1578 GCGAGT------ACGTGTTTCTCCGCGAGTTGGTCAAACACACTCGAGATCCCGTTGTTTACAAGACCA 1641
                    *      * ** **  *    ** * ** ** ** ***       ** **    ** **

CYP52A1A  2115 ATTGTTGAACATTATGGTTGCCGGAAGAGACACCACTGCCGGTTTGTTGTCCTTTGCTTTGTTTGAATTG 2184
CYP52A2A  2131 ATTGTTGAACATCATGGTTGCTGGTAGAGACACCACCGCCGGTTTGTTGTCGTTTGTTTTCTTTGAATTG 2200
CYP52A2B  2004 GTTGTTGAACATCATGGTTGCCGGTAGAGACACCACCGCCGGTTTGTTGTCGTTTGTTTTCTTTGAATTG 2073
CYP52A3A  2112 GTTATTGAACATTTTGGTTGCCGGTAGAGACACGACCGCCGGTTTGTTGTCATTTGTTTTCTACGAGTTG 2181
CYP52A3B  1899 GTTGTTGAACATTTTGGTTGCCGGTAGAGACACGACCGCCGGTTTGTTGTCGTTTGTGTTCTACGAGTTG 1968
CYP52A5A  2020 GTCTTTGAACATCTTGTTGGCCGGAAGAGACACCACTGCTGGGTTGTTGTCGTTTGCTGTCTTTGAGTTG 2089
CYP52A5B  2059 GTCTTTGAACATCTTGTTGGCAGGAAGAGACACCACTGCTGGGTTGTTGTCCTTTGCTGTGTTTGAGTTG 2128
CYP52A8A  1366 GTCTTTGAACATCTTGTTGGCAGGAAGAGACACCACTGCTGGGTTGTTGTCCTTTGCTGTGTTTGAGTTG 1435
CYP52A8B  1919 GTCTTTGAACATCTTGTTGGCTGGAAGGGACACCACTGCTGGGTTGTTGTCCTTTGCTGTGTTTGAGTTG 1988
CYP52D4A  1642 AGCGTTGAACGTCTTGCTTGCTGGACGCGACACCACCGCGTCGTTATTATCGTTTGCAACATTTGAGCTA 1711
                   ****** *  ** * ** **  * ***** ** **    ** ** ** ****     *  **  *

CYP52A1A  2185 GCTAGACACCCAGAGATGTGGTCCAAGTTGAGAGAAGAAATCGAAGTTAACTTTGGTGTTGGTGAAGACT 2254
CYP52A2A  2201 GCCAGAAACCCAGAAGTTACCAACAAGTTGAGAGAAGAAATTGAGGACAAGTTTGGACTCGGTGAGAATG 2270
CYP52A2B  2074 GCCAGAAACCCAGAGGTGACCAACAAGTTGAGAGAAGAAATCGAGGACAAGTTTGGTCTTGGTGAGAATG 2143
CYP52A3A  2182 TCAAGAAACCCTGAGGTGTTTGCTAAGTTGAGAGAGGAGGTGGAAAACAGATTTGGACTCGGTGAAGAAG 2251
CYP52A3B  1969 TCGAGAAACCCTGAAGTGTTTGCCAAGTTGAGAGAGGAGGTGGAAAACAGATTTGGACTCGGCGAAGAGG 2038
CYP52A5A  2090 GCCAGACACCCAGAGATCTGGGCCAAGTTGAGAGAGGAAATTGAACAACAGTTTGGTCTTGGAGAAGACT 2159
CYP52A5B  2129 GCCAGAAACCCACACATCTGGGCCAAGTTGAGAGAGGAAATTGAACAGCAGTTTGGTCTTGGAGAAGACT 2198
CYP52A8A  1436 GCCAGAAACCCACACATCTGGGCCAAGTTGAGAGAGGAAATTGAACAGCAGTTTGGTCTTGGAGAAGACT 1505
CYP52A8B  1989 GCCAGGAACCCACACATCTGGGCCAAGTTGAGAGAGGAAATTGAATCACACTTTGGGCTGGGTGAGGACT 2058
CYP52D4A  1712 GCCCGGAATGACCACATGTGGAGGAAGCTACGAGAGGAGGTT-------ATCCTGA---CGATGGACCG 1771
                *  *  *     *  *       *** *  **** **  *            **     *  *

CYP52A1A  2255 CCCGCGTTGAAGAAATTACCTTCGAAGCCTTGAAGAGATGTGAATACTTGAAGGCTATCCTTAACGAAAC 2324
CYP52A2A  2271 CTAGTGTTGAAGACATTTCCTTTGAGTCGTTGAAGTCCTGTGAATACTTGAAGGCTGTTCTCAACGAAAC 2340
CYP52A2B  2144 CTCGTGTTGAAGACATTTCCTTTGAGTCGTTGAAGTCATGTGAATACTTGAAGGCTGTTCTCAACGAAAC 2213
CYP52A3A  2252 CTCGTGTTGAAGAGATCTCGTTTGAGTCCTTGAAGTCTTGTGAGTACTTGAAGGCTGTCATCAATGAAAC 2321
CYP52A3B  2039 CTCGTGTTGAAGAGATCTCTTTTGAGTCCTTGAAGTCCTGTGAGTACTTGAAGGCTGTCATCAATGAAGC 2108
CYP52A5A  2160 CTCGTGTTGAAGAGATTACCTTTGAGAGCTTGAAGAGATGTGAGTACTTGAAAGCGTTCCTTAATGAAAC 2229
CYP52A5B  2199 CTCGTGTTGAAGAGATTACCTTTGAGAGCTTGAAGAGATGTGAGTACTTGAAAGCGTTCCTTAACGAAAC 2268
CYP52A8A  1506 CTCGTGTTGAAGAGATTACCTTTGAGAGCTTGAAGAGATGTGAGTACTTGAAGGCCGTGTTGAACGAAAC 1575
CYP52A8B  2059 CTCGTGTTGAAGAGATTACCTTTGAGAGCTTGAAGAGATGTGAGTACTTGAAAGCCGTGTTGAACGAAAC 2128
CYP52D4A  1772 TCCAG--TGATGAAATAACCGTGGCCGGGTTGAAGAGTTGCCGTTACCTCAAAGCAATCCTAAACGAAAC 1839
                      *** ** **  *  *  *     ******   **    *** * ** **  *  * ** *** *
```

Figure 15G

```
CYP52A1A  2325 CTTGCGTATGTACCCATCTGTTCCTGTCAACTTTAGAACCGCCACCAGAGACACCACTTTGCCAAGAGGT 2394
CYP52A2A  2341 CTTGAGATTGTACCCATCCGTGCCACAGAATTTCAGAGTTGCCACCAAGAACACTACCCTCCCAAGAGGT 2410
CYP52A2B  2214 TTTGAGATTGTACCCATCCGTGCCACAGAATTTCAGAGTTGCCACCAAAAACACTACCCTTCCAAGGGGA 2283
CYP52A3A  2322 CTTGAGATTGTACCCATCGGTTCCACACAACTTTAGAGTTGCTACCAGAAACACTACCCTCCCAAGAGGT 2391
CYP52A3B  2109 CTTGAGATTGTACCCATCTGTTCCACACAACTTCAGAGTTGCCACCAGAAACACTACCCTTCCAAGAGGC 2178
CYP52A5A  2230 CTTGCGTATTTACCCAAGTGTCCCAAGAAACTTCAGAATCGCCACCAAGAACACGACATTGCCAAGGGGC 2299
CYP52A5B  2269 CTTGCGTGTTTACCCAAGTGTCCCAAGAAACTTCAGAATCGCCACCAAGAATACAACATTGCCAAGGGGT 2338
CYP52A8A  1576 TTTGAGATTACACCCAAGTGTCCCAAGAAACGCAAGATTTGCGATTAAAGACACGACTTTACCAAGAGGC 1645
CYP52A8B  2129 GTTGAGATTACACCCAAGTGTCCCAAGAAACGCAAGATTTGCGATTAAAGACACGACTTTACCAAGAGGC 2198
CYP52D4A  1840 TCTTCGACTATACCCAAGTGTGCCTAGGAACGCGAGATTTGCTACGAGGAATACGACGCTTCCTCGTGGC 1909
                 *  *  *  *****   ** **    **    ***   ** *  *   * ** **  * **  * **

CYP52A1A  2395 GGTGGTGCTAACGGTACCGACCCAATCTACATTCCTAAAGGCTCCACTGTTGCTTACGTTGTCTACAAGA 2464
CYP52A2A  2411 GGTGGTAAGGACGGGTTGTCTCCTGTTTTGGTGAGAAAGGGTCAGACCGTTATTTACGGTGTCTACGCAG 2480
CYP52A2B  2284 GGTGGTAAGGACGGGTTATCTCCTGTTTTGGTCAGAAAGGGTCAAACCGTTATGTACGGTGTCTACGCTG 2353
CYP52A3A  2392 GGTGGTGAAGATGGATACTCGCCAATTGTCGTCAAGAAGGGTCAAGTTGTCATGTACACTGTTATTGCTA 2461
CYP52A3B  2179 GGTGGTAAAGACGGATGCTCGCCAATTGTTGTCAAGAAGGGTCAAGTTGTCATGTACACTGTCATTGGTA 2248
CYP52A5A  2300 GGTGGTTCAGACGGTACCTCGCCAATCTTGATCCAAAAGGGAGAAGCTGTGTCGTATGGTATCAACTCTA 2369
CYP52A5B  2339 GGTGGTCCAGACGGTACCCAGCCAATCTTGATCCAAAAGGGAGAAGGTGTGTCGTATGGTATCAACTCTA 2408
CYP52A8A  1646 GGTGGCCCCAACGGCAAGGATCCTATCTTGATCAGGAAGGATGAGGTGGTGCAGTACTCCATCTCGGCAA 1715
CYP52A8B  2199 GGTGGCCCCAACGGCAAGGATCCTATCTTGATCAGAAAGAATGAGGTGGTGCAATACTCCATCTCGGCAA 2268
CYP52D4A  1910 GGAGGTCCAGATGGATCGTTTCCGATTTTGATAAGAAAGGGCCAGCCAGTGGGGTATTTCATTTGTGCTA 1979
               ** **     * **       **  *     *    **          **    **     *

CYP52A1A  2465 CCCACCGTTTGGAAGAATACTACGGTAAGGACGCTAACGACTTCAGACCAGAAAGATGGTTTGAACCATC 2534
CYP52A2A  2481 CCCACAGAAACCCAGCTGTTTACGGTAAGGACGCTCTTGAGTTTAGACCAGAGAGATGGTTTGAGCCAGA 2550
CYP52A2B  2354 CCCACAGAAACCCAGCTGTCTACGGTAAGGACGCCCTTGAGTTTAGACCAGAGAGGTGGTTTGAGCCAGA 2423
CYP52A3A  2462 CCCACAGAGACCCAAGTATCTACGGTGCCGACGCTGACGTCTTCAGACCAGAAAGATGGTTTGAACCAGA 2531
CYP52A3B  2249 CCCACAGAGACCCAAGTATCTACGGTGCCGACGCCGACGTCTTCAGACCAGAAAGATGGTTCGAGCCAGA 2318
CYP52A5A  2370 CTCATTTGGACCCTGTCTATTACGGCCCTGATGCTGCTGAGTTCAGACCAGAGAGATGGTTTGAGCCATC 2439
CYP52A5B  2409 CCCACTTAGATCCTGTCTATTATGGCCCTGATGCTGCTGAGTTCAGACCAGAGAGATGGTTTGAGCCATC 2478
CYP52A8A  1716 CTCAGACAAATCCTGCTTATTATGGCGCCGATGCTGCTGATTTTAGACCGGAAAGATGGTTTGAACCATC 1785
CYP52A8B  2269 CTCAGACAAATCCTGCTTATTATGGCGCCGATGCTGCTGATTTTAGACCGGAAAGATGGTTTGAGCCATC 2338
CYP52D4A  1980 CACACTTGAATGAGAAGGTATATGGGAATGATAGCCATGTGTTTCGACCGGAGAGATGGGCTGCGTTAGA 2049
               * **                ** **     **       *  **  **** ** ** ***   *    *

CYP52A1A  2535 TACTAAGAAGTTGGGCTGGGCTTATGTTCCATTCAACGGTGGTCCAAGAGTCTGCTTGGGTCAACAATTC 2604
CYP52A2A  2551 GACAAAGAAGCTTGGCTGGGCCTTCCTCCCATTCAACGGTGGTCCAAGAATCTGTTTGGGACAGCAGTTT 2620
CYP52A2B  2424 GACAAAGAAGCTTGGCTGGGCCTTCCTTCCATTCAACGGTGGTCCAAGAATTTGCTTGGGACAGCAGTTT 2493
CYP52A3A  2532 AACTAGAAAGTTGGGCTGGGCATACGTTCCATTCAATGGTGGTCCAAGAATCTGTTTGGGTCAACAGTTT 2601
CYP52A3B  2319 AACTAGAAAGTTGGGCTGGGCATATGTTCCATTCAATGGTGGTCCAAGAATCTGTTTGGGTCAGCAGTTT 2388
CYP52A5A  2440 AACCAAAAAGCTCGGCTGGGCTTACTTGCCATTCAACGGTGGTCCAAGAATCTGTTTGGGTCAGCAGTTT 2509
CYP52A5B  2479 AACCAGAAAGCTCGGCTGGGCTTACTTGCCATTCAACGGTGGGCCACGAATCTGTTTGGGTCAGCAGTTT 2548
CYP52A8A  1786 AACTAGAAACTTGGGATGGGCTTTCTTGCCATTCAACGGTGGTCCAAGAATCTGTTTGGGACAACAGTTT 1855
CYP52A8B  2339 AACTAGAAACTTGGGATGGGCTTACTTGCCATTCAACGGTGGTCCAAGAATCTGCTTGGGACAACAGTTT 2408
CYP52D4A  2050 GGGCAAGAGTTTGGGCTGGTCGTATCTTCCATTCAACGGCGGCCCGAGAAGCTGCCTTGGTCAGCAGTTT 2119
                   *  *   * ** *** * *   * ******** ** ** **  **   **  * ** ** ** **

CYP52A1A  2605 GCCTTGACTGAAGCTTCTTATGTGATCACTAGATTGGCCCAGATGTTTGAAACTGTCTCATCTGATCCAG 2674
CYP52A2A  2621 GCCTTGACAGAAGCTTCGTATGTCACTGTCAGGTTGCTCCAGGAGTTTGCACACTTGTCTATGGACCCAG 2690
CYP52A2B  2494 GCCTTGACAGAAGCTTCGTATGTCACTGTCAGATTGCTCCAAGAGTTTGGACACTTGTCTATGGACCCCA 2563
CYP52A3A  2602 GCCTTGACCGAAGCTTCATACGTCACTGTCAGATTGCTCCAGGAGTTTGCACACTTGTCTATGGACCCAG 2671
CYP52A3B  2389 GCCTTGACTGAAGCTTCATACGTCACTGTCAGATTGCTCCAAGAGTTTGGAAACTTGTCCCTGGATCCAA 2458
CYP52A5A  2510 GCCTTGACGGAAGCTGGCTATGTGTTGGTTAGATTGGTGCAAGAGTTCTCCCACGTTAGGCTGGACCCAG 2579
CYP52A5B  2549 GCCTTGACCGAAGCTGGTTACGTTTTGGTCAGATTGGTGCAAGAGTTCTCCCACATTAGGCTGGACCCAG 2618
CYP52A8A  1856 GCTTTGACTGAAGCCGGTTACGTTTTGGTTAGACTTGTTCAGGAGTTTCCAAACTTGTCACAAGACCCCG 1925
CYP52A8B  2409 GCTTTGACCGAAGCCGGTTACGTTTTGGTTAGACTTGTTCAGGAATTCCCTAGCTTGTCACAGGACCCCG 2478
CYP52D4A  2120 GCAATCCTTGAAGCTTCGTATGTTTTGGCTCGATTGACACAGTGCTACACGACGATACAGCTTAG---AA 2186
               **  *    *****    ** **        *  *    **    *         *
```

Figure 15H

```
CYP52A1A  2675 GTCTCGAATACCCTCCACCAAAGTGTATTCACTTGACCATGAGTCACAACGATGGTGTCTTTGTCAAGAT 2744
CYP52A2A  2691 ACACCGAATATCCACCTAAGAAAATGTCGCATTTGACCATGTCGCTTTTCGACGGTGCCAATATTGAGAT 2760
CYP52A2B  2564 ACACCGAATATCCACCTAGGAAAATGTCGCATTTGACCATGTCCCTTTTCGACGGTGCCAACATTGAGAT 2633
CYP52A3A  2672 ACACCGAATATCCACCAAAATTGCAGAACACCTTGACCTTGTCGCTCTTTGATGGTGCTGATGTTAGAAT 2741
CYP52A3B  2459 ACGCTGAGTACCCACCAAAATTGCAGAACACCTTGACCTTGTCACTCTTTGATGGTGCTGACGTTAGAAT 2528
CYP52A5A  2580 ACGAGGTGTACCCGCCAAAGAGGTTGACCAACTTGACCATGTGTTTGCAGGATGGTGCTATTGTCAAGTT 2649
CYP52A5B  2619 ATGAAGTGTATCCACCAAAGAGGTTGACCAACTTGACCATGTGTTTGCAGGATGGTGCTATTGTCAAGTT 2688
CYP52A8A  1926 AAACCAAGTACCCACCACCTAGATTGGCACACTTGACGATGTGCTTGTTTGACGGTGCACACGTCAAGAT 1995
CYP52A8B  2479 AAACTGAGTACCCACCACCTAGATTGGCACACTTGACGATGTGCTTGTTTGACGGGGCATACGTCAAGAT 2548
CYP52D4A  2187 CTACCGAGTACCCACCAAAGAAACTCGTTCATCTCACGATGAGTCTTCTCAACGGGGTGTACATCCGAAC 2256
                       ** ** **                *  **  **          * ** *      *

CYP52A1A  2745 GTAA-AGTAGTCGATGCTGGGTATTCGATTACATGT--GTATAGGAAGATTTTGGTTTTTATTCGTTCT 2811
CYP52A2A  2761 GTATTAGAGGGTCATGTGTTATTTT-GATTGTTTA-----GTTTGTAATTACTGATTAGGTTAATTCATG 2824
CYP52A2B  2634 GTATTAGAGGATCATGTGTTATTTTTGATTGGTTTAGTCTGTTTGTAGCTATTGATTAGGTTAATTCACG 2703
CYP52A3A  2742 GTACTAAGGTTGCTTTTCCTTGCTAATTTTCTTCTGTATAGCTTGTGTATTTAAATTGAATCGGCAATTG 2811
CYP52A3B  2529 GTTCTAAGGTTGCTTATCCTTGCTAGTGTTATT---TATAGTTTGTGTATTTAAATTGAATCGGCGATTG 2595
CYP52A5A  2650 TGACTAGCGGCGTGGTGAATGCGTTTGATTTTGTA---GTTTCTGTTTGCAGTAATGAGATAACTATTCA 2716
CYP52A5B  2689 TGACTAGTA-CGTA-TGAGTGCGTTTGATTTTGTA---GTTTCTGTTTGCAGTAATGAGATAACTATTCA 2753
CYP52A8A  1996 GTCATAGGTTTCCC---CATACAAGTAGTTCAGTA---ATTATACACTGTTTTTACTTTCTCTTCATACC 2059
CYP52A8B  2549 GCAATAGGTTT-------------------------------------TGGTTTGACTTTGTTTCCATA-- 2580
CYP52D4A  2257 TAGAACTTGATTATGTGTTTATGGTTAATCGGGGCAAAGCACTGCAAGTCATTGATGTTTGTGGAAGCCC 2326

CYP52A1A  2812 TTTTTTTAATTTTTGTTAAATTAG-TTTAGAGATTTCATTAATACATAGATGGGTGCTATTTCCGAAACT 2880
CYP52A2A  2825 GATTGTTATTTATTGATAGGGGTT---------TGCGCGTGTTGCATTCACTTGGGATCGTTCCAGGTTG 2885
CYP52A2B  2704 GATTGTTATTTATTGATAGGGGGTGCGTGTGTGTGTGTGTGTTGCATTCACATGGGATCGTTCCAGGTTG 2773
CYP52A3A  2812 ATTTTTCTGATACCAATAACCGTA--------GTGCGATTTGACCAAAACCGTTCAAACTTTTTGTTCTC 2873
CYP52A3B  2596 ATTTTTCTGGTACTAATAACTGTA--------GTGGGTTTTGACCAAAACCGTTCAAACTTTTTTTTTTT 2657
CYP52A5A  2717 GATAAGGCGAGTGGATGTACGTTT-TGTAAGAGTTT--CCT-TACAACCTTGGTGGGG-TGTGTGAGGTT 2781
CYP52A5B  2754 GATAAGGCGGGTGGATGTACGTTT-TGTAAGAGTTT--CCT-TACAACCCTGGTGGG--TGTGTGAGGTT 2817
CYP52A8A  2060 AAATGGACAAAAGTTTAAGCATG-CCTAACAACGTGACCG-GACAATTGTGTCGCACTAGTATGTAACA 2127
CYP52A8B  2581 ---------------------------------------------------------------TGCAAGT 2587
CYP52D4A  2327 AGCATTGGTGTTCCGGAGCATCAATAACCAATGTCTTGAAGGGTTTGATTTTCTTGACCTTCTTCTTCCT 2396

CYP52A1A  2881 TTACTTCTATCC--CCTGTATCCCTTATTATCCCTCTCAGTCACATGATTGCTGTAATTGTCGTGCAGGA 2948
CYP52A2A  2886 ATGTTTCCTTCCATCCT--GTCGAGTCAAAAGGAGTTTTGTTTTGTAACTCCGGACGATGTTTTAAATAG 2953
CYP52A2B  2774 TTGTTTCCTTCCATCCT--GTTGAGTCAAAAGGAGTTTTGTTTTGTAACTCCGGACGATGTCTTAGATAG 2841
CYP52A3A  2874 TCGTTGACG------------TGCTCGCTCATCAGCACTGTTTGAAGACGAAAGA-GAAAATTTTTTGTA 2930
CYP52A3B  2658 TTTTCTTCCCCCTACCTTCGTTGCTCGCTCATCAGCACTGTTTGAAAACGAAAAAAGAAAATTTTTTGTA 2727
CYP52A5A  2782 GAGGTTGCATCTT-GGGGAGATTACACCTTTTG-CAGCTCTCCGTATACACTTGTACTCTTTGTAACCTC 2849
CYP52A5B  2818 G----CATCTTAG-GGAGAGATAGCACCTTTTG-CAGCTCTCCGTATACAGTTTTACTCTTTGTAACCTA 2881
CYP52A8A  2128 ATTGTAAAAATAG-TGTACACTAATTTGTGGTGGCCGGAGATAAATTACAGTTTGGTTTTGTGTAAACTC 2196
CYP52A8B  2588 AGTTCAGTAAT---TACACACTAATTTGTGGTGGCCGGCGATAAATTACCGTTTGGTTTTGTGTAAAAAT 2654
CYP52D4A  2397 GAGCTTCTTTCCG--TCAAACTTGTACAGAATGGCCATCATTTCAGGAACAACCA-CGTACGACGGCCGG 2463
                                                              *

CYP52A1A  2949 CACAAACTCCCTAACGGACTTAAACCATAAACAAGCTCAGAACCATAAGCCGACATCACTCCTTCTTCTC 3018
CYP52A2A  2954 AAGGTCGATCTCCATGTGATTGTTTTGACTGTTACTGTGATTATGTAATCTGCG-------GACGTTATA 3016
CYP52A2B  2842 AAGGTCGATCTCCATGTGATTGTTT-GACTGCTACTCTGATTATGTAATCTGTAAAGCCTAGACGTTATG 2910
CYP52A3A  2931 AACAACACTGTCCAAATTTACCCAACGTGAACCATTATG--CAAATGAGCGGCC---------CTTTCAA 2989
CYP52A3B  2728 AACAACATTGCCCAAACTTACCCAACGTGAACCATTATAACCAAATGAGCGGCG---------CTTTCAA 2788
CYP52A5A  2850 TATCAATCATGTGGGGGGGGGGTTCATTGTTTGGC-CATGGTGGTGCATGTTAAATCCGCC-AACTACC 2917
CYP52A5B  2882 TGCCAATCATGTGG------GGATTCATTGTTTGCC-CATGGTGGTGCATGCAAAATCCCCCCAACTACC 2944
CYP52A8A  2197 GCGGATATCTCTGGC-----AGTTTCTCTTCTCCGC-AGCAGCTTTGCCACGGGTTTGCTCTGGGGCCAA 2260
CYP52A8B  2655 TCGGACATCTCTGGT-----GGTTTCCCTTCTCCGC-AGCAGCTTTGCCACGGGTTTGCTCTGCGGCCAA 2718
CYP52D4A  2464 TACCGCATCTGGAGTA---TCTCGCCGTCGTTCAAGTAG--CACGAAAACAGCAACGACGTCACCATCTG 2528
```

Figure 15I

```
CYP52A1A  3019 TCTTCTCCAACCAATAGCATGGACAGACCCACCCTCCTATCCGAATCGAAGACCCTTATTGACTCCATAC 3088
CYP52A2A  3017 CAAGCATGTGATTGTGGTTTT------GCAGCCT-TTTGCACGACAAATGATCGTCAGACGATTACGTAA 3079
CYP52A2B  2911 CAAGCATGTGATTGTGGTTTTT-----GCAACCTGTTTGCACGACAAATGATCGACAGTCGATTACGTAA 2975
CYP52A3A  2990 CTGGTCGCTGGAAGCATTCGGG-----GATATCTACAACGCCCTTAAGTTTGAAACAGACATTGATTTAG 3054
CYP52A3B  2789 CTGGTCACTGGAGGCATTCGGG-----GATATCTACAACACCCTTAAGTTTGAGGAAGACATTGATTTAG 2853
CYP52A5A  2918 CAATCTCACATGAAACTCAAGCACACTAAAAAAAAAAAAGATGTTGGGGGAAAACTT-TGGTTTCCCTTC 2986
CYP52A5B  2945 CAATCTCACATGAAACTCAAGCACACTAGAAAAAAAA--GATGTTGCGTGGGTTCTT-TTGATG------ 3005
CYP52A8A  2261 CAAATTCAAAAGGGGG-----------AGAAACTTAACACCCCTTATCTCTCCACTC-TAGGTTGTAGCT 2318
CYP52A8B  2719 CAAATTCGAAAGGGGGGGGGGGGGGGGAGAAAGTTAACACCCCCTGTTCC--CACCG-TAGGCTGTAGCT 2785
CYP52D4A  2529 CTTCCCAATCTTGACACCC--------ACAGATACCCCTGCGGCTTCATGGATCAAAAACGTCGGCAACC 2590

CYP52A1A  3089 CCACCTGGAAGCCCCTCAAGCCACACACGTCATCCAGCCCACCCATCACCACATCCCTCTACTCGACAAC 3158
CYP52A2A  3080 TCTTTGTTA-----GAGGGGTAAAAAAAAACAAAATGGCAGCCAGAATTTCAAACATTCTGCAAACAATG 3144
CYP52A2B  2976 TCCATATTAT-TTAGAGGGGTAATAAAAAATAAA-TGGCAGCCAGAATTTCAAACATTTTGCAAACAATG 3043
CYP52A3A  3055 ACACCATAGA-TTTCAGCGGCATCAAGAATGACC----TTGCCCACATTTTGACGACCCCAACACCACTG 3119
CYP52A3B  2854 ACACCATAGA-TTTCAGCGGCATCAAGAATGACC----TTGTCCACATTTTGACAACCCCAACACCACTG 2918
CYP52A5A  2987 TTAGTAATT--AAACACTCTCACTCTCACTCTCACTCTCTCCACTCAGACAAACCAACCACCTGGGCTGC 3054
CYP52A5B  3006 TTGGGGAAA--ACTTTCGTTTCCTTTCTCAGTAATTAAACGTTCTCACTCAGACAAACCACCTGGGCTGC 3073
CYP52A8A  2319 CTTGTGGGG--ATGCAATTGTCGTACGTTTTTTATGTTTTGTCTAGACTTTGATGATTACGTTGGATTTC 2386
CYP52A8B  2786 CTTGTGGGGGGATGTAATTGTCGTACGTTTTC-ATGTTTGGCCCAGACTTTGATGATTACGTAGGCTTTC 2854
CYP52D4A  2591 CCGCGTATATGTCCATGTAATTCTCCATGGCCACCT--CCATCAACACACTGATGGAGCGACTGACGGTG 2658
                                                                  *

CYP52A1A  3159 GTCCAAAGACGGCGAGTTCTGGTGTGCCCGGAAATCAGCCATCCCGGCCACATACAAGCAGCCGTTGATT 3228
CYP52A2A  3145 CAAAAAATGGGAAACTC--CAACAGACAAAA-AAAAAAACTCCGCAGCACTCCGAACCCACAGAACAATG 3211
CYP52A2B  3044 CAAAAGATGAGAAACTC--CAACAGAAAAAATAAAAAAACTCCGCAGCACTCCGAACCAACAAAACAATG 3111
CYP52A3A  3120 GAAGAATCACGCCAGA----AACTAGGCGATGGATCCAAGCCTGTGACCTTGCCCAATGGAGACGAAGTG 3185
CYP52A3B  2919 GAAGAATCGCGCCAGA----AACTAGGCGATGGATCCAAGCCTGTGGCCTTGCCCAATGGAGACGAAGTG 2984
CYP52A5A  3055 AGACAACCAGAAAAAAAAAGAACAAAATCCAGATAGAAAAACAAAGGGCT-GGACAACCATAAAT-AAAC 3122
CYP52A5B  3074 AGACAACCAGAAAAAA------CAAAATCCAGATAGAAGAAGAAAGGGCT-GGACAACCATAAAT-AAAC 3135
CYP52A8A  2387 TTATGTCTGAGGCGTG----CTTGAAAGAAGTGTCAAAATGTGACAGGCG-ACGCTATTCGACAT-GAAC 2450
CYP52A8B  2855 TTATGTCTAAGGCGTG----CTTGACACAAGTGTCAAAAGGTGACAGGCG-ACGTTATTCGACAT-GAAC 2918
CYP52D4A  2659 CCACCACTGCCCTCGG------TTGAGTCAAGGCAGTATGATGCCGGGATCCAGTACTCCAATGGGAACC 2722

CYP52A1A  3229 GCGTGCATACTCGGCGAGCCCACAATGGGAGCCACGCATTCGGACCATGAAGCAAAGTACATTCACGAGA 3298
CYP52A2A  3212 GGG----CGCCAGAATTATTGACTATTGTGACTTTTTTA-----------CGCTAACGCTCATTGCAGTG 3266
CYP52A2B  3112 GGGG--GCGCCAGAATTATTGACTATTGTGACTTTTTTTT--ATTTTTTCCGTTAACTTTCATTGCAGTG 3177
CYP52A3A  3186 GAGTTGAACCAAGCGTTCCTAGAAGTTACCACATTATTGTCGAATGAGTTTGACTTGGACCAATTGAACG 3255
CYP52A3B  2985 GAGTTGAACCAAGCGTTCCTAGAAGTTACCACATTATTGTCGAACGAGTTTGACTTGGACCAATTGAACG 3054
CYP52A5A  3123 AATCTAGGGTCTACTCCATCTTCCACTGTTTCTTCTTCTTCAGACTTAGCT-AACAAACAACTCACTTCA 3191
CYP52A5B  3136 AACCTAGGGTCCACTCCATCTTTCACT---TCTTCTTCTTCAGACTTATCT-AACAAACGACTCACTTCA 3201
CYP52A8A  2451 GCGAAAGGGTTATTTGCATCAATACGAG--GGGCTGACTCTAGTCTAGG---ATGGCAGTCCTAGGTTGC 2515
CYP52A8B  2919 GCAAAAGGGTAATTTGCATCGATACGAG--GGGTTGCCTCTGGTCTAAG---AAGGACCCCCCAGGTTGC 2983
CYP52D4A  2723 TCT-----GCACGGTGTCGCTGCAGTTTTTGAGGCGTATTTCGA--------TCCATGATCGTTCTTTGG 2779

CYP52A1A  3299 TCACGGGTGTTTCAG-TGTCGCAGATTGAGAAGTTCGACGATGGATGGAAGTACGATCTCGTTGCGGATT 3367
CYP52A2A  3267 TAGTGCGTCTTACACGG-------GGTATTGCTTTCTACAATGCAAGGGCA-CAGTTGAAGGTTTGCACC 3328
CYP52A2B  3178 AAGTGTGTTACACGGGGTGGTGATGGTGTTGGTTTCTACAATGCAAGGGCA-CAGTTGAAGGTTTCCACA 3246
CYP52A3A  3256 CGGCAGAGTTGTTATACTA-CGCTGGCGACATATCCTACAAGAAGGGCACATCAATCGCAGACAGTGCCA 3324
CYP52A3B  3055 CGGCCGAGTTGTTATACTA-CGCCGGCGACATATCCTACAAGAAGGGCACATCAATTGCCGACAGTGCCA 3123
CYP52A5A  3192 CCATGGATTACGCAGGCATCACGCGTGGCTCCATCAGAGG-CGAGGCCTTGAAGAAACTCG--CAGAATT 3258
CYP52A5B  3202 CCATGGATTACGCAGGTATCACGCGTGGGTCCATCAGAGG-CGAAGCCTTGAAGAAACTCG--CCGAGTT 3268
CYP52A8A  2516 AAACATGTTGCACCA-TATCCCTCCTGGAGTTGGTCGAC--CTCGCCTACGCC-ACCCTCA--GCGATCG 2579
CYP52A8B  2984 AAACATGTTGCACTG-CATCCCACTCAGAGTTGGTCGAC--CACGCCTACGCTTACCCTCA--GCGATCG 3048
CYP52D4A  2780 TGCTGTAGTATAACGAGCT--CTTGGTGTCCTTGAAATGGAACAGGTTGGATGTGTTGTTGAGTTTGTCT 2847
```

Figure 15J

```
CYP52A1A  3368 ACGACTTCGGTGGGTTGTTATCTAAACGAAGATTCTATGAGACGCAGCATGTGTTTCGGTTCGAGGATTG 3437
CYP52A2A  3329 TAACGTTGCCCCGTGTCAACTCAATTTGAC-----------G--AGTAACTTCCTAAGCTCGAATTATGC 3385
CYP52A2B  3247 TAACGTTGCACCATATCAACTCAATTTATC-----------CTCATTCATGTGATAAAAGAAGAGCCAAA 3305
CYP52A3A  3325 GATTGTCTTATTATTTGAGAGCAAACTAC---------------ATCTTGAACATACTTGGGTATTTGAT 3379
CYP52A3B  3124 GATTGTCTTACTATTTGAGAGCAAACTAC---------------ATCTTGAACATACTTGGGTACTTTAT 3178
CYP52A5A  3259 G-ACCATCCAGAACCAGCCATCCAGCT------TGAAAGAAATCAACACCGGCATCCAGAAGGACGACTT 3321
CYP52A5B  3269 G-ACCATCCAGAACCAGCCATCCAGCT------TGAAAGAAATCAACACCGGCATCCAGAAGGACGACTT 3331
CYP52A8A  2580 GCACTTTCCGTTGTTCAATATTTCTC------------------CTTCCCATTGTTCCAGGGGTTA--TC 2629
CYP52A8B  3049 GCACTTTCCGTTGCTCAATATTTCTCT------CCCCCCTGCTTCCCCCCATTGTTCCAGGGATTA--TC 3110
CYP52D4A  2848 GCGTGCTTGGTTTGCAAGTCTTCGATCG----------------AGCGTAGTGAGTAGACAGTTGGCGGG 2901

CYP52A1A  3438 TGCGTACGTCATGAGTGTGCCTTTTGATGGACCCAAGGAGGAAGGTTACGTGGTTGGGACGTACAGATCC 3507
CYP52A2A  3386 AGCT-CGTGCGTCAACCTATGTGCAGGAAAGAAAAAATCCAAAAA--AATCGAAA-ATGCGACTTTCGAT 3451
CYP52A2B  3306 AGGT-AAT-TGGCAGACCCCCCAAGGGGAACACGGAGTAGAAAGC--AATGGAAACACGCCCATGACAGT 3371
CYP52A3A  3380 TTCG-AAGCAGCGATTGGATTTGATAGTCACGGACAACGACGCGT--TGTTTGATAGTATTTTGAAAAGT 3446
CYP52A3B  3179 TTCG-AAGCAGCGATTGGATGTGATAGTCACCGACAACAACGCGT--TGTTTGATAATATTTTGAAAAGT 3245
CYP52A5A  3322 TGCC-AAGTTGTTGTCTGCCACCCCGAAAATCCCCACCAAGCACA--AGTTGAACGGCAACCACGAATT- 3387
CYP52A5B  3332 TGCC-AAGTTGTTGTCTTCCACCCCGAAAATCCACACCAAGCACA--AGTTGAATGGCAACCACGAATT- 3397
CYP52A8A  2630 AACA-ACGTTGCCGGCCTCCTC------------CCCAAATTA-----------CAAGAAAAATAAATT- 2674
CYP52A8B  3111 AACA-ACGTTGCCGGTCTCCTCTCCCCCCCCTCCCCCCAGTTAT-------GTACAAGAAAATTAAATT- 3171
CYP52D4A  2902 GGTGGTGGCTCGGGCTTTATTCTGTGTTTGTGTTTCCTTCTTAGT--CTTGGAATGACGCTGTTATCGAC 2969

CYP52A1A  3508 ATTGAAAGGTTGAGCTGGGGTAAAGACGGGGACGTGGA-GTGGACCATGG---CGACGACGTCGGATCCT 3573
CYP52A2A  3452 TTTGAATAAACCAAAAAGAAAAATGTCGCACTTTTTTC-----TCGCTCTCGCTCTCTCGACCCAAATCA 3516
CYP52A2B  3372 GCCATTTAGCCCACA---ACACATCTAGTATTCTTTTT-----TTTTTTTGTGCGCAGGTGCACACCTGG 3433
CYP52A3A  3447 TTTGAAAAGATCTAC----AAGTTGATAAGCGTGTTGA-----ACGATATGATTGACAAGCAAAAGGTGA 3507
CYP52A3B  3246 TTTGAAAAGATCTAC----AAGTTGATAAGCGCGTTGA-----ACGATATGATTGACAAGCAAAAGGTGA 3306
CYP52A5A  3388 GTCTGAGGTCGCCATTGCCAAAAAGGAGTACGAGGTGTTGATTGCCTTGAGCGACGCCACAAAAGACCCA 3457
CYP52A5B  3398 GTCCGAAGTCGCCATTGCCAAAAAGGAGTACGAGGTGTTGATTGCCTTGAGCGACGCCACGAAAGAACCA 3467
CYP52A8A  2675 GTCGCACGGCACCGATCTGTCAAAGATACAGATAA--------ACCTTAAATCTGCAAAAACAAGACCCC 2736
CYP52A8B  3172 GTCGCACGGCACCGATACGTCAAAGATACAGAGAA--------ACCTTAA-----------------TCC 3216
CYP52D4A  2970 GGTTCGTAGTATAAGTAGCGCCAATATGAGAATGTATA-----TCCGCATCACCCAAGACTCTTCAGCCT 3034

CYP52A1A  3574 GGTGGGTTTATCCCGCA-ATGGATAACTCGATTGAGCA-TCCCTGGAGCAATCGCAAAAGATGTGCCTAG 3641
CYP52A2A  3517 CAACAAATCCTCGCGCGCAGTATTTCGACGAAAC--CACAACAAATAAAAAAAACAAATTCTACACCACT 3584
CYP52A2B  3434 ACTTTAGTTATTGCCC-CATAAAGTTAACAATCT--CACCTTTGGCTCTCCCAGTGTCTCCGCCTCCAGA 3500
CYP52A3A  3508 CAAGCGACATCAACAGTCTAGCATTCATCAATTG--CATCAACTACTCGAGAGGTCAACTATTCTCCGCA 3575
CYP52A3B  3307 CAAGCGACATCAACAGTCTAGCATTTATCAACTG--CATCAACTACTCGAGGGGTCAACTATTCTCCGCA 3374
CYP52A5A  3458 ATCAAAGTGACCTCCCAGATCAAGATCTTGATTGACAAGTTCAAGGTGTACTTGT---TTGAGTTGCCTG 3524
CYP52A5B  3468 ATCAAAGTCACCTCCCAGATCAAGATCTTGATTGACAAGTTCAAGGTGTACTTGT---TTGAGTTGCCCG 3534
CYP52A8A  2737 TCCCCATAGCCTAGAAGCACCAGCAAGATGATGGAGCAACTCCTCCAGTACTGGTACATCGCACTCTCTG 2806
CYP52A8B  3217 CTCCCATAGCCTAGAAGCATCAAAAAGATGATTGAGCAACTCCTCCAGTACTGGTACATTGCACTCCCTG 3286
CYP52D4A  3035 GTTACAACGACTGAGGCTGTTGGCCGTGTGACCAATTGGTTTCTTGGTGACCTAGATTGGTCCCGCAGG 3104
                                         *

CYP52A1A  3642 TG---TATTAAACTACATACAGAAATAAAAACGTGTCTTGATTCATTGGTTT---GGTTCTTGTTGGGTT 3705
CYP52A2A  3585 T----CTTTTTCTTCACCAGTCAACAAAAAACAACAAATTATACACCATTTCAACGATTTTTGCTCTTAT 3650
CYP52A2B  3501 TG---CTCGTTTTACACCCTCGAGCTAACGACAACACAACACCCATGAGGGGAATGGGCAAAGTT----- 3562
CYP52A3A  3576 CA---CGAACTTTTGGG-ACTGGTTTTGTTTGGATTGGTCGACATCTATTTCAACCAGTTTGGCACATTA 3641
CYP52A3B  3375 CA---CGAACTTTTGGG-ACTGGTTTTGTTTGGATTGGTTGACAACTATTTCAACCAGTTTGGCTCATTA 3440
CYP52A5A  3525 AC---CAGAAGTTCTCCTACTCCATCGTGTCCAACTCCGTCAACATCGCCCCC-TGGACCTTGCTCGGGG 3590
CYP52A5B  3535 AC---CAGAAGTTCTCCTACTCCATCGTGTCCAACTCCGTTAACATTGCCCCC-TGGACCTTGCTCGGTG 3600
CYP52A8A  2807 TA---TGGTTCATCCTTCGCTACTTGGCTTCCCACGCACGAGCCGTCTACTTG-CGCCACAAGCTCGGCG 2872
CYP52A8B  3287 TA---TGGTTCATTCTCCGCTACGTGGCTTCCCACGCACGAACCATCTACTTG-CGCCACAAGCTCGGCG 3352
CYP52D4A  3105 GAAAGCAAGGGCTGCTAGGGGGGCATACCAAACAAGGTCGTGTAATCAGTATCTATGGTGCTACCATGTG 3174
```

Figure 15K

```
CYP52A1A  3706 CCGAGCCAATATTTCACATCATCTCCTAAATTCTCCAAGAATCCCAACGTAGCGTAGTCCAGCACGCCCT 3775
CYP52A2A  3651 AAATGCTATATAATGGTTTAATTCAACTCAGGTATGTTTAT-TTTACTGTTTTCAGCTCAAGTATGT--T 3717
CYP52A2B  3563 AAACACTTTTGGTTTCAATGATTCCTATTTGCTACTCTCTTGTTTTGTGTTTTGATTTGCACCATGT--G 3630
CYP52A3A  3642 GACAACTACAAGAAGGTATTGGCATTGATACTGAAGAACATCAGCGATGAAGACATCTTGATCATAC--A 3709
CYP52A3B  3441 GACAACTACAAGAAAGTATTGGCATTGATACTGAAGAACATCAGTGATGAAGATATCTTGATCGTAC--G 3508
CYP52A5A  3591 AGAAGTTGACCACGGGCTTGATCAACTTGGCCTTCCAGAACAACAAGCAGCACTTGGACGAGGTCATT-G 3659
CYP52A5B  3601 AGAAGTTGACCACGGGCTTGATCAACTTGGCGTTCCAGAACAACAAGCAGCACTTGGACGAAGTCATC-G 3669
CYP52A8A  2873 CGGCGCCATTCACGCACACCCAGTACGACGGCTGGTATGGGTTCAAGTTTGGGCGGGAGTTTCTCAA--G 2940
CYP52A8B  3353 CGGCGCCGTTCACGCACACCCAGTACGACGGATGGTATGGGTTCAAGTTTGGGCGGGAGTTTCTCAA--G 3420
CYP52D4A  3175 TGTGGTTGGGGGGAAATTCCCGCATTTTTGTGTAACGAAAGTTCTAGAAAGTTCTCGTGGGTTCTGAG-A 3243

CYP52A1A  3776 CTGAGATCTTATTTAATATCGACTTCTCAACCACCGGTGGAATC--CCGTTCAGACCATTGTTACCTGTA 3843
CYP52A2A  3718 CAAATACTAACTACTTTTGATGTTTGTCGCTTTTCTAGAATCAAAACAACGCCCACAACACGCCGAGCTT 3787
CYP52A2B  3631 AAATAAACGACAATTATATATACCTTT---TCGTCTGTCCTC----CAATGTCT-CTTTTTGCTGCCATT 3692
CYP52A3A  3710 CTTCCTCCCATCGACACTACAATTGTTTAAGCTGGTGTTGGACAA-GAAAGACGACGCTGCAGTTGAACA 3778
CYP52A3B  3509 CTTCCTCCCATCGACACTACAATTGTTTAAGCTGGTGTTGGATAA-GAAAGACGACGCCACTGTTGACCA 3577
CYP52A5A  3660 ACATCTTCAACGAGTTCATCGACAAGTTCTTTGGCAACACGGAG--CCGCAATTGAC-----CAACTTCT 3722
CYP52A5B  3670 ACATCTTCAACGAGTTCATCGACAAGTTCTTTGGCAACACAGAG--CCGCAATTGAC-----CAACTTCT 3732
CYP52A8A  2941 GCGAAGAAGATCGGGCGGCAGACGGACTTGGTGCATGCGCGGTT--CCGTGGCGG-------CATGGACA 3001
CYP52A8B  3421 GCGAAGAAGATTGGAAGGCAGACGGACTTGGTGCATGCGCGGTT--CCGTGGAGGGGG----CATGGATA 3484
CYP52D4A  3244 ATCTGCTGGAACCATCCACCCGCATTTCCGTTGCCAAAGTGGGAA-GAGCAATCAACCCACCCTGCTTTG 3312

CYP52A1A  3844 GTGTGTTTGCTCTTGTTCTTGATGACAATGATGTATTTGTCACGATACCTGAAATAATAAAACATCCAGT 3913
CYP52A2A  3788 GTCGAATAGACGGTTTGTTTACTCATTAGATGGTCCCAGATTACTTTTCAAGCCAAAGTCTCT-CGAGTT 3856
CYP52A2B  3693 TTGCTTTTTGCTTTTTGCTTTTGCACT---CTCTCCCACTCCCACAATCAGTGCAGCAACACA-CAA    3755
CYP52A3A  3779 GTTCTACAAGTACATCACTTCAACAGT--GTCACGAGACTACAACTCCAACATCGGCTCCACAGCCAAAG 3846
CYP52A3B  3578 GTTCTACAAGTACATCACCTCAACAGT--GTCGCAAGACTACAACTCCAACATCGGAGCCACAGCCAAAG 3645
CYP52A5A  3723 TGACCTTGTGCGGTGTGTTGGACGGGTTGATTGACCATGCC-AACTTCTTGAGCGTGTCCTCGCGGACCT 3791
CYP52A5B  3733 TGACCTTGTCCGGTGTGTTGGACGGGTTGATTGACCATGCC-AACTTCTTGAGCGTGTCCTCCAGGACCT 3801
CYP52A8A  3002 CCTTCTCGAGCTACACTTTCGGCATCCATATCATCCTTACC-CGGGACCCGGAGAACATCAAGGCGGTCT 3070
CYP52A8B  3485 CTTTCTCGAGCTATACTTTCGGCATCCATATCATTCTTACT-CGGGACCCGGAGAACATCAAGGCGGTCT 3553
CYP52D4A  3313 CCCAATCAGCCATTCCCCTGGGAATATAAATTCAAC                                   3348

CYP52A1A  3914 CATTGAGCTTATTACTCGTGAACTTATGAAAGAACTCATTCAAGCCGTTCCCAAAAAACCCAGAATTGAA 3983
CYP52A2A  3857 TTGTTTGCTGTTTCCCCAATTCCTAACTATGAAGGGTTTTATAAGGTCCAAAGACCCCAAGGCATAGTT 3926
CYP52A2B  3756                                                                        3755
CYP52A3A  3847 ATGATATCGATTTGTCCAAAACCAAACTCAGTGGCTTTGAGGTGTTGACGAGTT                 3900
CYP52A3B  3646 ATGATATCGATTTGTCCAAAGCC                                                3668
CYP52A5A  3792 TCAAGATCTTCTTGAACTTGGACTCGTATGTGGAC                                    3826
CYP52A5B  3802 TCAAGATCTTCTTGAACTTGGACTCGTTTGTGGACAACTCGGACTTCTTGAACGACGTGGAGAACTACTC 3871
CYP52A8A  3071 TGGCGACGCAGTTCGATGACTTCTCGCTCGGTGGCAGGATCAGGTTCTTGAAGCCGTTGTTGGGGTATGG 3140
CYP52A8B  3554 TGGCGACGCAGTTCGATGACTTTTCG                                             3579
CYP52D4A  3349                                                                        3348

CYP52A1A  3984 GATCTTGCTCAACTGGTCATGCAAGTAGTAGATCGCCATGATCTGATACTTTACCAAGCTATCCTCTCCA 4053
CYP52A2A  3927 TTTTTGGTTCCTTCTTGTCGTG                                                 3948
CYP52A2B  3756                                                                        3755
CYP52A3A  3901                                                                        3900
CYP52A3B  3669                                                                        3668
CYP52A5A  3827                                                                        3826
CYP52A5B  3872 CGACTTTTTGTACGACGAGCCGAACGAGTACCAGAACTT                                3910
CYP52A8A  3141 GATATTCACGTT                                                           3152
CYP52A8B  3580                                                                        3579
CYP52D4A  3349                                                                        3348
```

Figure 15L

| | | | |
|---|---|---|---|
| CYP52A1A | 4054 | AGTTCTCCCACGTACGGCAAGTACGGCAACGAGCTCTGGAAGCTTTGTTGTTTGGGGTCATA | 4115 |
| CYP52A2A | 3949 | | 3948 |
| CYP52A2B | 3756 | | 3755 |
| CYP52A3A | 3901 | | 3900 |
| CYP52A3B | 3669 | | 3668 |
| CYP52A5A | 3827 | | 3826 |
| CYP52A5B | 3911 | | 3910 |
| CYP52A8A | 3153 | | 3152 |
| CYP52A8B | 3580 | | 3579 |
| CYP52D4A | 3349 | | 3348 |

Figure 15M

```
CYP52A1A    1        MATQEIIDSVLPYL-------TKWYTVITAAVLVFLISTNIKNYV  38
CYP52A2A    1        MTVHDIIATY----------FTKWYVIVPLALIAYRVLDYFYGRY  35
CYP52A2B    1        MTAQDIIATY----------ITKWYVIVPLALIAYRVLDYFYGRY  35
CYP52A3A    1 MSSSPSFAQEVLATTSPYIEYFLDNYTRWYYFIPLVLLSLNFISLLHTRY  50
CYP52A3B    1 MSSSPSFAQEVLATTSPYIEYFLDNYTRWYYFIPLVLLSLNFISLLHTKY  50
CYP52A5A    1           MIEQLLEY----------WYVVVPVLYIIKQLLAYTKTRV  30
CYP52A5B    1           MIEQILEY----------WYIVVPVLYIIKQLIAYSKTRV  30
CYP52A8A    1           MLDQILHY----------WYIVLPLLAIINQIVAHVRTNY  30
CYP52A8B    1           MLDQIFHY----------WYIVLPLLVIIKQIVAHARTNY  30
CYP52D4A    1          MAISSLLSWD---------VICVVFICVCVYFGYEYCYTKY  32
                         .                        .

CYP52A1A   39 KAKKLKCVDPPYLKDAGLTGILSLIAAIKAKNDGRLANFAD---EVFDEY  85
CYP52A2A   36 LMYKLGAKPFFQKQTDGCFGFKAPLELLKKKSDGTLIDFTL---QRIHDL  82
CYP52A2B   36 LMYKLGAKPFFQKQTDGYFGFKAPLELLKKKSDGTLIDFTL---ERIQAL  82
CYP52A3A   51 LERRFHAKPLGNFVRDPTFGIATPLLLIYLKSKGTVMKFAWGLWNNKYIV 100
CYP52A3B   51 LERRFHAKPLGNVVLDPTFGIATPLILIYLKSKGTVMKFAWSFWNNKYIV 100
CYP52A5A   31 LMKKLGAAPVTNKLYDNAFGIVNGWKALQFKKEGRAQEYND---YKFDHS  77
CYP52A5B   31 LMKQLGAAPITNQLYDNVFGIVNGWKALQFKKEGRAQEYND---HKFDSS  77
CYP52A8A   31 LMKKLGAKPFTHVQRDGWLGFKFGREFLKAKSAGRLVDLII---SRFHDN  77
CYP52A8B   31 LMKKLGAKPFTHVQLDGWFGFKFGREFLKAKSAGRQVDLII---SRFHDN  77
CYP52D4A   33 LMHKHGAREIENVINDGFFGFRLPLLLMRASNEGRLIEFSV---KRFESA  79
                  .  .           *       .       *

CYP52A1A   86 PN--HTFYLSVAGALKIVMTVDPENIKAVLATQFTDFSLGTRHAHFAPLL 133
CYP52A2A   83 DRPDIPTFTFPVFSINLVNTLEPENIKAILATQFNDFSLGTRHSHFAPLL 132
CYP52A2B   83 NRPDIPTFTFPIFSINLISTLEPENIKAILATQFNDFSLGTRHSHFAPLL 132
CYP52A3A  101 RDPKYKTTGLRIVGLPLIETMDPENIKAVLATQFNDFSLGTRHDFLYSLL 150
CYP52A3B  101 KDPKYKTTGLRIVGLPLIETIDPENIKAVLATQFNDFSLGTRHDFLYSLL 150
CYP52A5A   78 KNPSVGTYVSILFGTRIVVTKDPENIKAILATQFGDFSLGKRHTLFKPLL 127
CYP52A5B   78 KNPSVGTYVSILFGTKIVVTKDPENIKAILATQFGDFSLGKRHALFKPLL 127
CYP52A8A   78 ED----TFSSYAFGNHVVFTRDPENIKALLATQFGDFSLGSRVKFFKPLL 123
CYP52A8B   78 ED----TFSSYAFGNHVVFTRDPENIKALLATQFGDFSLGSRVKFFKPLL 123
CYP52D4A   80 PHPQNKTLVNRALSVPVILTKDPVNIKAMLSTQFDDFSLGLRLHQFAPLL 129
                                  .. * .* ****.*.*** ***** *      **

CYP52A1A  134 GDGIFTLDGEGWKHSRAMLRPQFARDQIGHVKALEPHIQIMAKQIKLNQG 183
CYP52A2A  133 GDGIFTLDGAGWKHSRSMLRPQFAREQISHVKLLEPHVQVFFKHVRKAQG 182
CYP52A2B  133 GDGIFTLDGAGWKHSRSMLRPQFAREQISHVKLLEPHMQVFFKHVRKAQG 182
CYP52A3A  151 GDGIFTLDGAGWKHSRTMLRPQFAREQVSHVKLLEPHVQVFFKHVRKHRG 200
CYP52A3B  151 GDGIFTLDGAGWKHSRTMLRPQFAREQVSHVKLLEPHVQVFFKHVRKHRG 200
CYP52A5A  128 GDGIFTLDGEGWKHSRAMLRPQFAREQVAHVTSLEPHFQLLKKHILKHKG 177
CYP52A5B  128 GDGIFTLDGEGWKHSRSMLRPQFAREQVAHVTSLEPHFQLLKKHILKHKG 177
CYP52A8A  124 GYGIFTLDAEGWKHSRAMLRPQFAREQVAHVTSLEPHFQLLKKHILKHKG 173
CYP52A8B  124 GYGIFTLDGEGWKHSRAMLRPQFAREQVAHVTSLEPHFQLLKKHILKHKG 173
CYP52D4A  130 GKGIFTLDGPEWKQSRSMLRPQFAKDRVSHILDLEPHFVLLRKHIDGHNG 179
              * ******   **.**.*******.... *.  ****  .  *..    *
```

Figure 16A

```
CYP52A1A   184 KTFDIQELFFRFTVDTATEFLFGESVHSLYDEKLGIPTP-NEIPGRENFA 232
CYP52A2A   183 KTFDIQELFFRLTVDSATEFLFGESVESLRDESIGMSINALDFDGKAGFA 232
CYP52A2B   183 KTFDIQELFFRLTVDSATEFLFGESVESLRDESIGMSINALDFDGKAGFA 232
CYP52A3A   201 QTFDIQELFFRLTVDSATEFLFGESAESLRDESIGLTPTTKDFDGRRDFA 250
CYP52A3B   201 QTFDIQELFFRLTVDSATEFLFGESAESLRDDSVGLTPTTKDFEGRGDFA 250
CYP52A5A   178 EYFDIQELFFRFTVDSATEFLFGESVHSLKDESIGINQDDIDFAGRKDFA 227
CYP52A5B   178 EYFDIQELFFRFTVDSATEFLFGESVHSLKDETIGINQDDIDFAGRKDFA 227
CYP52A8A   174 EYFDIQELFFRFTVDSATEFLFGESVHSLKDEEIGYDTKDMSEERRR-FA 222
CYP52A8B   174 EYFDIQELFFRFTVDSATEFLFGESVHSLRDEEIGYDTKDMAEERRK-FA 222
CYP52D4A   180 DYFDIQELYFRFSMDVATGFLFGESVGSLKDE-------D-------ARFL 216
                 ******.** ..* ** ******  ** *.                *

CYP52A1A   233 AAFNVSQHYLATRSYSQTFYFLTNPKEFRDCNAKVHHLAKYFVNKALNFT 282
CYP52A2A   233 DAFNYSQNYLASRAVMQQLYWVLNGKKFKECNAKVHKFADYYVNKALDLT 282
CYP52A2B   233 DAFNYSQNYLASRAVMQQLYWVLNGKKFKECNAKVHKFADYYVSKALDLT 282
CYP52A3A   251 DAFNYSQTYQAYRFLLQQMYWILNGSEFRKSIAVVHKFADHYVQKALELT 300
CYP52A3B   251 DAFNYSQTYQAYRFLLQQMYWILNGAEFRKSIAIVHKFADHYVQKALELT 300
CYP52A5A   228 ESFNKAQEYLAIRTLVQTFYWLVNNKEFRDCTKLVHKFTNYYVQKALDAS 277
CYP52A5B   228 ESFNKAQEYLSIRILVQTFYWLINNKEFRDCTKLVHKFTNYYVQKALDAT 277
CYP52A8A   223 DAFNKSQVYVATRVALQNLYWLVNNKEFKECNDIVHKFTNYYVQKALDAT 272
CYP52A8B   223 DAFNKSQVYLSTRVALQTLYWLVNNKEFKECNDIVHKFTNYYVQKALDAT 272
CYP52D4A   217 EAFNESQKYLATRATLHELYFLCDGFRFRQYNKVVRKFCSQCVHKALDVA 266
                .** .* * . *   .  * .     *.     *.. .   * ***  .

CYP52A1A   283 PEELEEKSKSGYVFLYELVKQTRDPKVLQDQLLNIMVAGRDTTAGLLSFA 332
CYP52A2A   283 PEQLE-K-QDGYVFLYELVKQTRDKQVLRDQLLNIMVAGRDTTAGLLSFV 330
CYP52A2B   283 PEQLE-K-QDGYVFLYELVKQTRDRQVLRDQLLNIMVAGRDTTAGLLSFV 330
CYP52A3A   301 DDDLQ-K-QDGYVFLYELAKQTRDPKVLRDQLLNILVAGRDTTAGLLSFV 348
CYP52A3B   301 DDDLQ-K-QDGYVFLYELAKQTRDPKVLRDQLLNILVAGRDTTAGLLSFV 348
CYP52A5A   278 PEELE-K-QSGYVFLYELVKQTRDPNVLRDQSLNILLAGRDTTAGLLSFA 325
CYP52A5B   278 PEELE-K-QGGYVFLYELVKQTRDPKVLRDQSLNILLAGRDTTAGLLSFA 325
CYP52A8A   273 PEELE-K-QGGYVFLYELVKQTRDPKVLRDQSLNILLAGRDTTAGLLSFA 320
CYP52A8B   273 PEELE-K-QGGYVFLYELAKQTKDPNVLRDQSLNILLAGRDTTAGLLSFA 320
CYP52D4A   267 PEDTS-----EYVFLRELVKHTRDPVVLQDQALNVLLAGRDTTASLLSFA 311
                .         **** ** *.*.*  **.** **...******* ****

CYP52A1A   333 LFELARHPEMWSKLREEIEVNFGVGEDSRVEEITFEALKRCEYLKAILNE 382
CYP52A2A   331 FFELARNPEVTNKLREEIEDKFGLGENASVEDISFESLKSCEYLKAVLNE 380
CYP52A2B   331 FFELARNPEVTNKLREEIEDKFGLGENARVEDISFESLKSCEYLKAVLNE 380
CYP52A3A   349 FYELSRNPEVFAKLREEVENRFGLGEEARVEEISFESLKSCEYLKAVINE 398
CYP52A3B   349 FYELSRNPEVFAKLREEVENRFGLGEEARVEEISFESLKSCEYLKAVINE 398
CYP52A5A   326 VFELARHPEIWAKLREEIEQQFGLGEDSRVEEITFESLKRCEYLKAFLNE 375
CYP52A5B   326 VFELARNPHIWAKLREEIEQQFGLGEDSRVEEITFESLKRCEYLKAFLNE 375
CYP52A8A   321 VFELARNPHIWAKLREEIEQQFGLGEDSRVEEITFESLKRCEYLKAVLNE 370
CYP52A8B   321 VFELARNPHIWAKLREEIESHFGLGEDSRVEEITFESLKRCEYLKAVLNE 370
CYP52D4A   312 TFELARNDHMWRKLREEVILTMGPSSD----EITVAGLKSCRYLKAILNE 357
                .**.*.  .  *****.    *        .*.   ** * **** .**
```

Figure 16B

```
CYP52A1A  383 TLRMYPSVPVNFRTATRDTTLPRGGGANGTDPIYIPKGSTVAYVVYKTHR 432
CYP52A2A  381 TLRLYPSVPQNFRVATKNTTLPRGGGKDGLSPVLVRKGQTVIYGVYAAHR 430
CYP52A2B  381 TLRLYPSVPQNFRVATKNTTLPRGGGKDGLSPVLVRKGQTVMYGVYAAHR 430
CYP52A3A  399 TLRLYPSVPHNFRVATRNTTLPRGGGEDGYSPIVVKKGQVVMYTVIATHR 448
CYP52A3B  399 ALRLYPSVPHNFRVATRNTTLPRGGGKDGCSPIVVKKGQVVMYTVIGTHR 448
CYP52A5A  376 TLRIYPSVPRNFRIATKNTTLPRGGGSDGTSPILIQKGEAVSYGINSTHL 425
CYP52A5B  376 TLRVYPSVPRNFRIATKNTTLPRGGGPDGTQPILIQKGEGVSYGINSTHL 425
CYP52A8A  371 TLRLHPSVPRNARFAIKDTTLPRGGGPNGKDPILIRKDEVVQYSISATQT 420
CYP52A8B  371 TLRLHPSVPRNARFAIKDTTLPRGGGPNGKDPILIRKNEVVQYSISATQT 420
CYP52D4A  358 TLRLYPSVPRNARFATRNTTLPRGGGPDGSFPILIRKGQPVGYFICATHL 407
              .**. **** * * * . ********  *  *. . *   * * .  ..

CYP52A1A  433 LEEYYGKDANDFRPERWFEPSTKKLGWAYVPFNGGPRVCLGQQFALTEAS 482
CYP52A2A  431 NPAVYGKDALEFRPERWFEPETKKLGWAFLPFNGGPRICLGQQFALTEAS 480
CYP52A2B  431 NPAVYGKDALEFRPERWFEPETKKLGWAFLPFNGGPRICLGQQFALTEAS 480
CYP52A3A  449 DPSIYGADADVFRPERWFEPETRKLGWAYVPFNGGPRICLGQQFALTEAS 498
CYP52A3B  449 DPSIYGADADVFRPERWFEPETRKLGWAYVPFNGGPRICLGQQFALTEAS 498
CYP52A5A  426 DPVYYGPDAAEFRPERWFEPSTKKLGWAYLPFNGGPRICLGQQFALTEAG 475
CYP52A5B  426 DPVYYGPDAAEFRPERWFEPSTRKLGWAYLPFNGGPRICLGQQFALTEAG 475
CYP52A8A  421 NPAYYGADAADFRPERWFEPSTRNLGWAFLPFNGGPRICLGQQFALTEAG 470
CYP52A8B  421 NPAYYGADAADFRPERWFEPSTRNLGWAYLPFNGGPRICLGQQFALTEAG 470
CYP52D4A  408 NEKVYGNDSHVFRPERWAALEGKSLGWSYLPFNGGPRSCLGQQFAILEAS 457
                  ** *.  ******     . ***...******* *******. **

CYP52A1A  483 YVITRLAQMFETVSSDPGLEYPPPKCIHLTMSHNDGVFVKM   523
CYP52A2A  481 YVTVRLLQEFAHLSMDPDTEYPPKKMSHLTMSLFDGANIEMY  522
CYP52A2B  481 YVTVRLLQEFGHLSMDPNTEYPPRKMSHLTMSLFDGANIEMY  522
CYP52A3A  499 YVTVRLLQEFAHLSMDPDTEYPPKLQNTLTLSLFDGADVRMY  540
CYP52A3B  499 YVTVRLLQEFGNLSLDPNAEYPPKLQNTLTLSLFDGADVRMF  540
CYP52A5A  476 YVLVRLVQEFSHVRLDPDEVYPPKRLTNLTMCLQDGAIVKFD  517
CYP52A5B  476 YVLVRLVQEFSHIRLDPDEVYPPKRLTNLTMCLQDGAIVKFD  517
CYP52A8A  471 YVLVRLVQEFPNLSQDPETKYPPPRLAHLTMCLFDGAHVKMS  512
CYP52A8B  471 YVLVRLVQEFPSLSQDPETEYPPPRLAHLTMCLFDGAYVKMQ  512
CYP52D4A  458 YVLARLTQCYTTIQLR-TTEYPPKKLVHLTMSLLNGVYIRTRT 499
              **  ** * .  .      ***     **..   *  .
```

Figure 16C

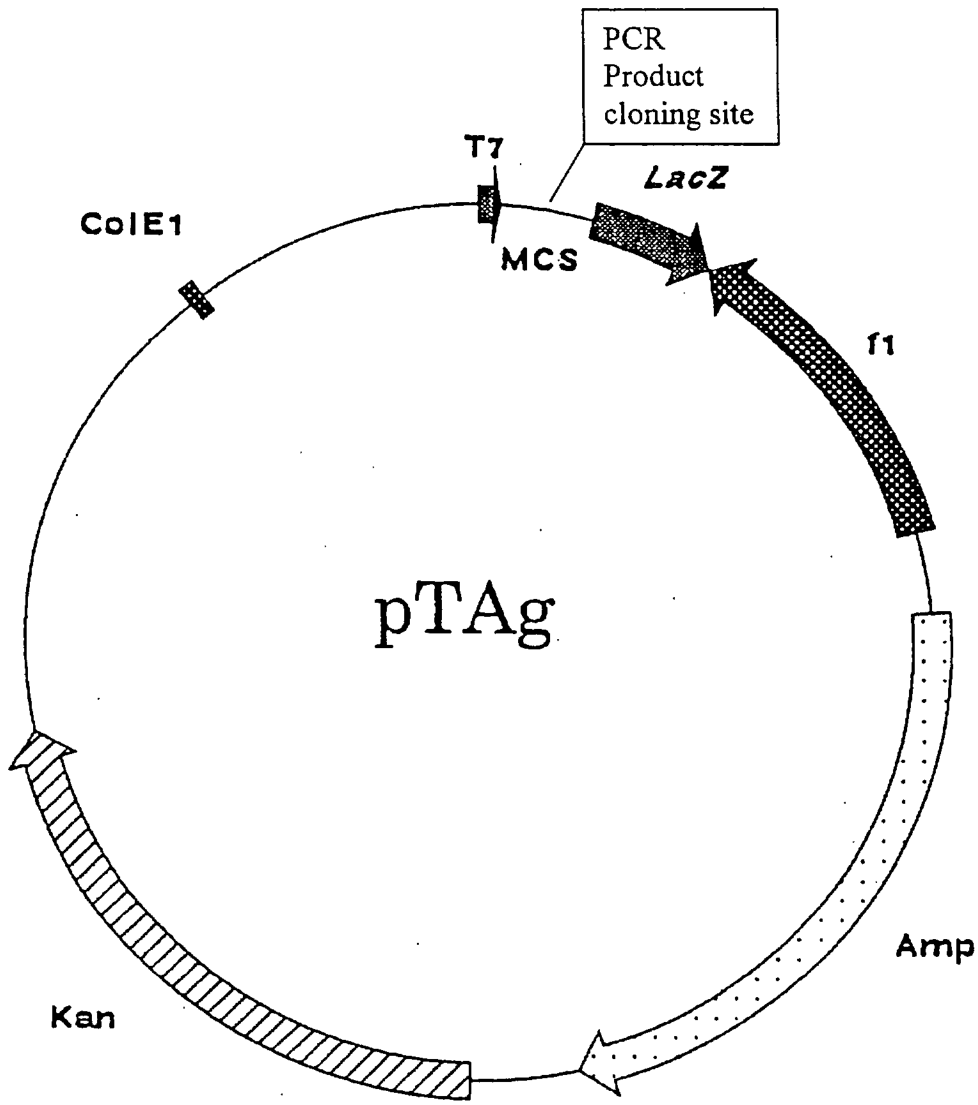


Figure 17

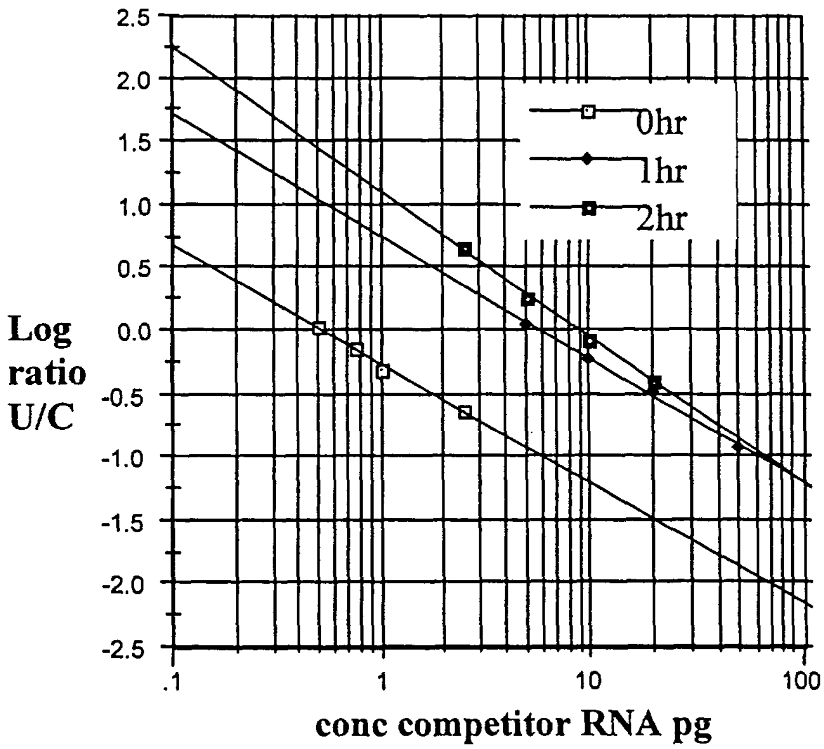
2.5
2.0
1.5
1.0
0.5
0.0
-0.5
-1.0
-1.5
-2.0
-2.5
Log ratio U/C
0hr
1hr
2hr
.1
1
10
100
conc competitor RNA pg

Figure 18

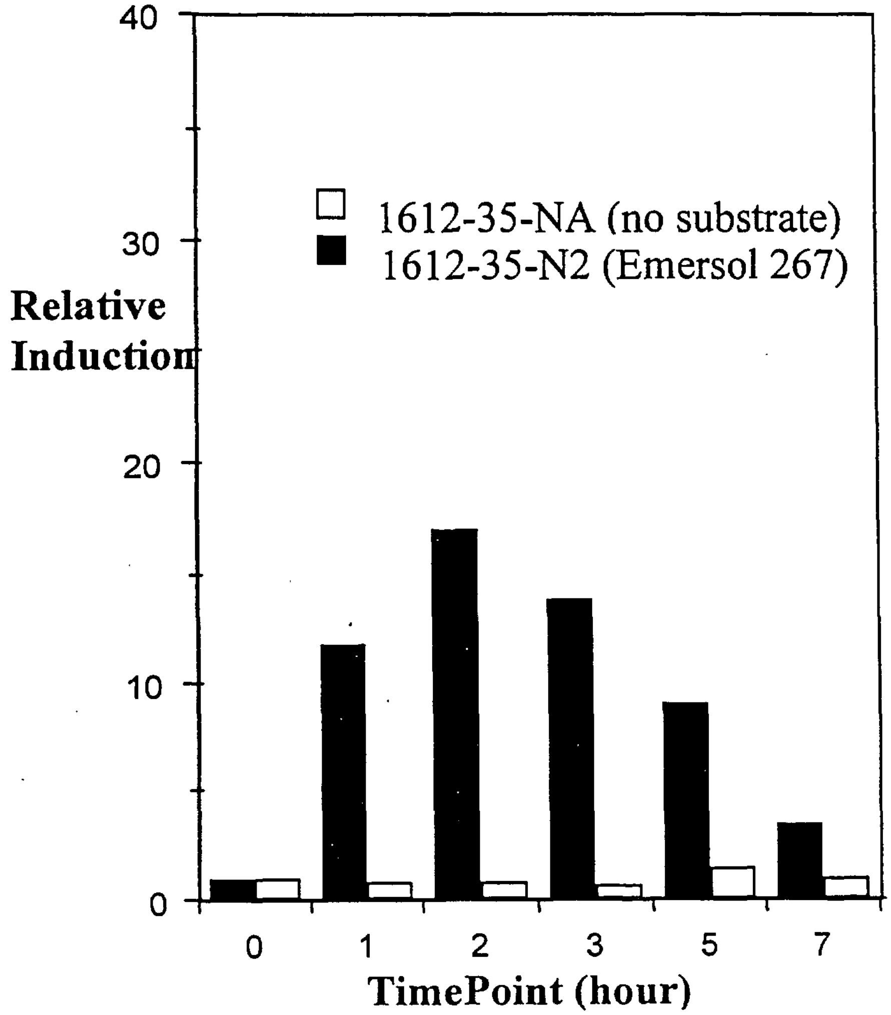
40
30
20
10
0
Relative Induction
1612-35-NA (no substrate)
1612-35-N2 (Emersol 267)
0
1
2
3
5
7
TimePoint (hour)

Figure 19

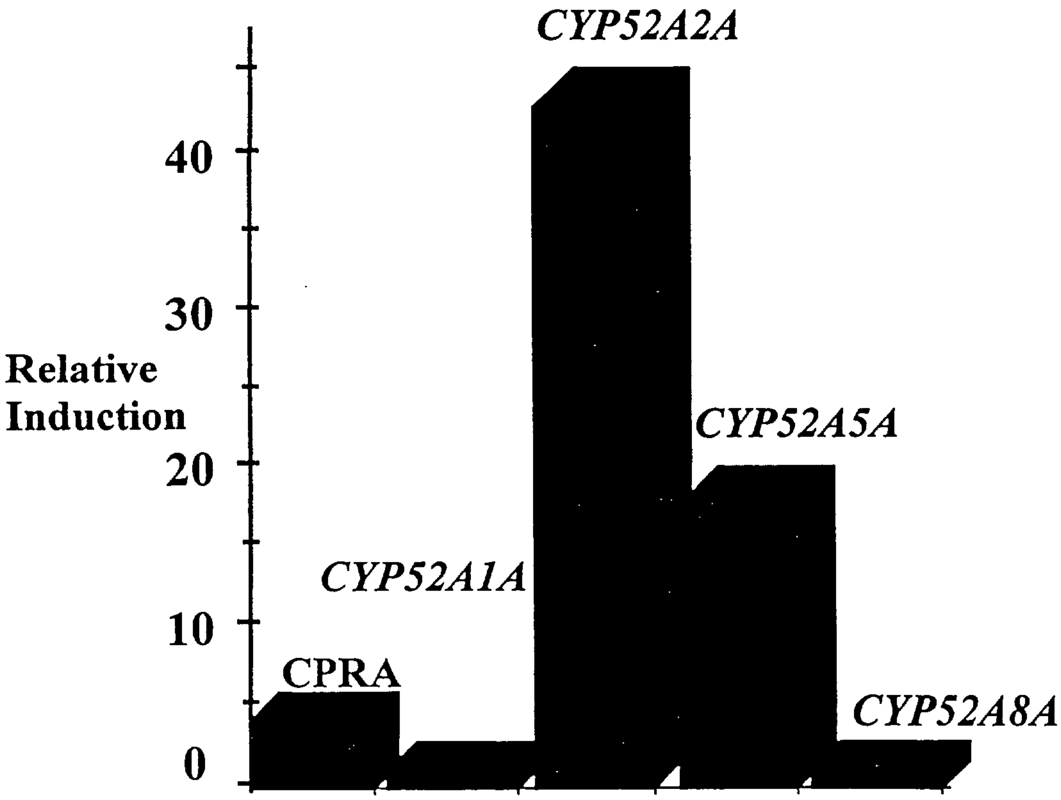
CYP52A2A
40
30
Relative
Induction
20
CYP52A5A
CYP52A1A
10
CPRA
CYP52A8A
0

Figure 20

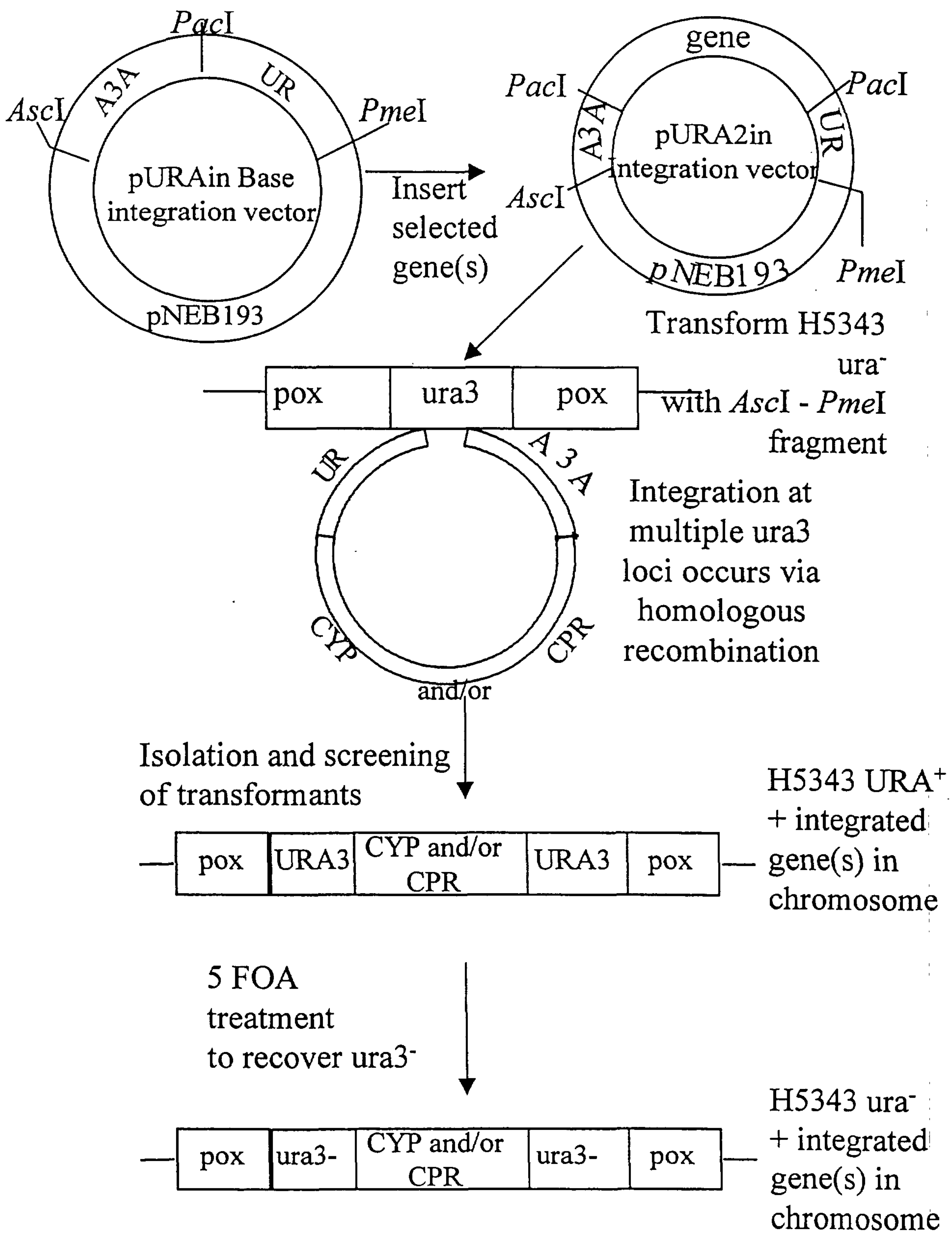
PacI
AscI
A3A
UR
PmeI
pURAin Base integration vector
pNEB193
Insert selected gene(s)
gene
PacI
A3A
UR
pURA2in integration vector
AscI
pNEB193
PmeI
Transform H5343 ura-
with AscI - PmeI fragment
pox
ura3
pox
UR
A3A
CYP
CPR
and/or
Integration at multiple ura3 loci occurs via homologous recombination
Isolation and screening of transformants
pox
URA3
CYP and/or CPR
URA3
pox
H5343 URA+ + integrated gene(s) in chromosome
5 FOA treatment to recover ura3-
pox
ura3-
CYP and/or CPR
ura3-
pox
H5343 ura- + integrated gene(s) in chromosome

Figure 21

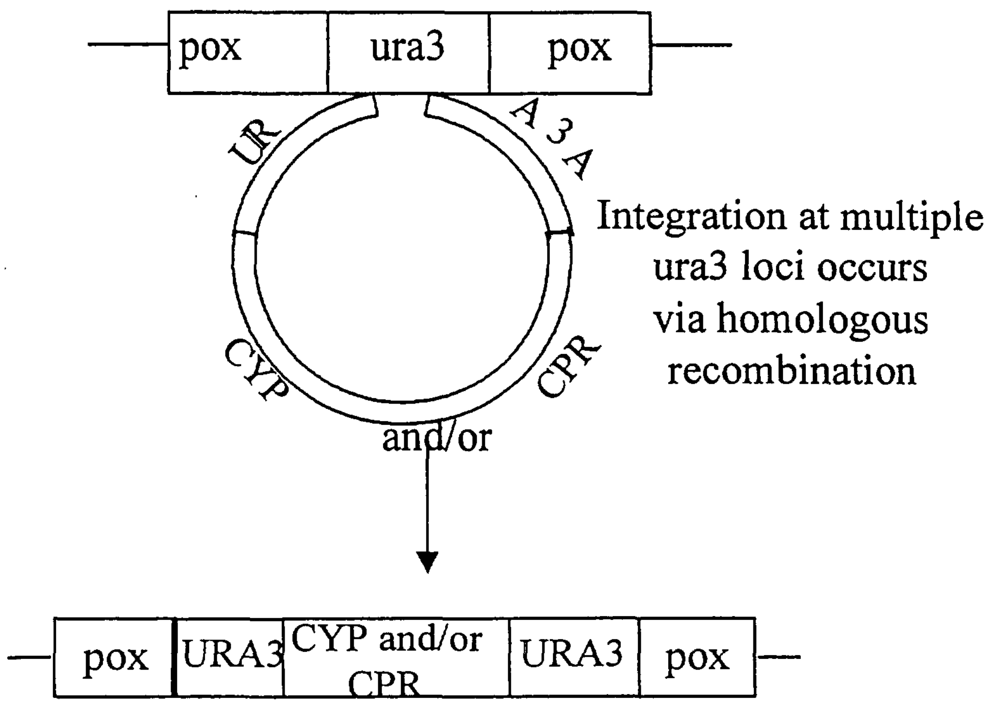
pox
ura3
pox
UR
A 3 A
Integration at multiple ura3 loci occurs via homologous recombination
CYP
CPR
and/or
pox
URA3
CYP and/or CPR
URA3
pox

Figure 22

Sequence Range: 1 to 1712

```
        10         20         30         40         50         60         70         80         90        100
GGTACCGAGC TCACGAGTTT TGGGATTTTC GAGTTTGGAT TGTTTCCTTT GTTGATTGAA TTGACGAAAC CAGAGGTTTT CAAGACAGAT AAGATTGGGT

       110        120        130        140        150        160        170        180        190        200
TTATCAAAAC GCAGTTTGAA ATATTCCAGT TGGTTTCCAA GATATCTTGA AGAAGATTGA CGATTTGAAA TTTGAAGAAG TGGAGAAGAT CTGGTTTGGA

       210        220        230        240        250        260        270        280        290        300
TTGTTGGAGA ATTTCAAGAA TCTCAAGATT TACTCTAACG ACGGGTACAA CGAGAATTGT ATTGAATTGA TCAAGAACAT GATCTTGGTG TTACAGAACA

       310        320        330        340        350        360        370        380        390        400
TCAAGTTCTT GGACCAGACT GAGAATGCCA CAGATATACA AGGCGTCATG TGATAAAATG GATGAGATTT ATCCCACAAT TGAAGAAAGA GTTTATGGAA

       410        420        430        440        450        460        470        480        490        500
AGTGGTCAAC CAGAAGCTAA ACAGGAAGAA GCAAACGAAG AGGTGAAACA AGAAGAAGAA GGTAAATAAG TATTTTGTAT TATATAACAA ACAAAGTAAG

       510        520        530        540        550        560        570        580        590        600
GAATACAGAT TTATACAATA AATTGCCATA CTAGTCACGT GAGATATCTC ATCCATTCCC CAACTCCCAA GAAAAAAAAA AAGTGAAAAA AAAAATCAAA

       610        620        630        640        650        660        670        680        690        700
CCCAAAGATC AACCTCCCCA TCATCATCGT CATCAAACCC CCAGCTCAAT TCGCAATGGT TAGCACAAAA ACATACACAG AAAGGGCATC AGCACACCCC
                                                            M V  S T K  T Y T  E R A S  A H P>

       710        720        730        740        750        760        770        780        790        800
TCCAAGGTTG CCCAACGTTT ATTCCGCTTA ATGGAGTCCA AAAAGACCAA CCTCTGCGCC TCGATCGACG TGACCACAAC CGCCGAGTTC CTTTCGCTCA
 S K V  A Q R L  F R L  M E S  K K T N  L C A  S I D  V T T T  A E F  L S L>

       810        820        830        840        850        860        870        880        890        900
TCGACAAGCT CGGTCCCCAC ATCTGTCTCG TGAAGACGCA CATCGATATC ATCTCAGACT TCAGCTACGA GGGCACGATT GAGCCGTTGC TTGTGCTTGC
I D K L  G P H  I C L  V K T H  I D I  I S D  F S Y E  G T I  E P L  L V L A>

       910        920        930        940        950        960        970        980        990       1000
AGAGCGCCAC GGGTTCTTGA TATTCGAGGA CAGGAAGTTT GCTGATATCG GAAACACCGT GATGTTGCAG TACACCTCGG GGGTATACCG GATCGCGGCG
  E R H  G F L  I F E D  R K F  A D I  G N T V  M L Q  Y T S  G V Y R  I A A>

      1010       1020       1030       1040       1050       1060       1070       1080       1090       1100
TGGAGTGACA TCACGAACGC GCACGGAGTG ACTGGGAAGG GCGTCGTTGA AGGGTTGAAA CGCGGTGCGG AGGGGGTAGA AAAGGAAAGG GGCGTGTTGA
W S D  I T N A  H G V  T G K  G V V E  G L K  R G A  E G V E  K E R  G V L>

      1110       1120       1130       1140       1150       1160       1170       1180       1190       1200
TGTTGGCGGA GTTGTCGAGT AAAGGCTCGT TGGCGCATGG TGAATATACC CGTGAGACGA TCGAGATTGC GAAGAGTGAT CGGGAGTTCG TGATTGGGTT
M L A E  L S S  K G S  L A H G  E Y T  R E T  I E I A  K S D  R E F  V I G F>

      1210       1220       1230       1240       1250       1260       1270       1280       1290       1300
CATCGCGCAG CGGGACATGG GGGGTAGAGA AGAAGGGTTT GATTGGATCA TCATGACGCC TGGTGTGGGG TTGGATGATA AAGGCGATGC GTTGGGCCAG
  I A Q  R D M  G G R E  E G F  D W I  I M T P  G V G  L D D  K G D A  L G Q>

      1310       1320       1330       1340       1350       1360       1370       1380       1390       1400
CAGTATAGGA CTGTTGATGA GGTGGTTCTG ACTGGTACCG ATGTGATTAT TGTCGGGAGA GGGTTGTTTG GAAAAGGAAG AGACCCTGAG GTGGAGGGAA
 Q Y R  T V D E  V V L  T G T  D V I I  V G R  G L F  G K G R  D P E  V E G>

      1410       1420       1430       1440       1450       1460       1470       1480       1490       1500
AGAGATACAG GGATGCTGGA TGGAAGGCAT ACTTGAAGAG AACTGGTCAG TTAGAATAAA TATTGTAATA AATAGGTCTA TATACATACA CTAAGCTTCT
K R Y R  D A G  W K A  Y L K R  T G Q  L E *>

      1510       1520       1530       1540       1550       1560       1570       1580       1590       1600
AGGACGTCAT TGTAGTCTTC GAAGTTGTCT GCTAGTTTAG TTCTCATGAT TTCGAAAACC AATAACGCAA TGGATGTAGC AGGGATGGTG GTTAGTGCGT

      1610       1620       1630       1640       1650       1660       1670       1680       1690       1700
TCCTGACAAA CCCAGAGTAC GCCGCCTCAA ACCACGTCAC ATTCGCCCTT TGCTTCATCC GCATCACTTG CTTGAAGGTA TCCACGTACG AGTTGTAATA

      1710
CACCTTGAAG AA
```

Figure 23

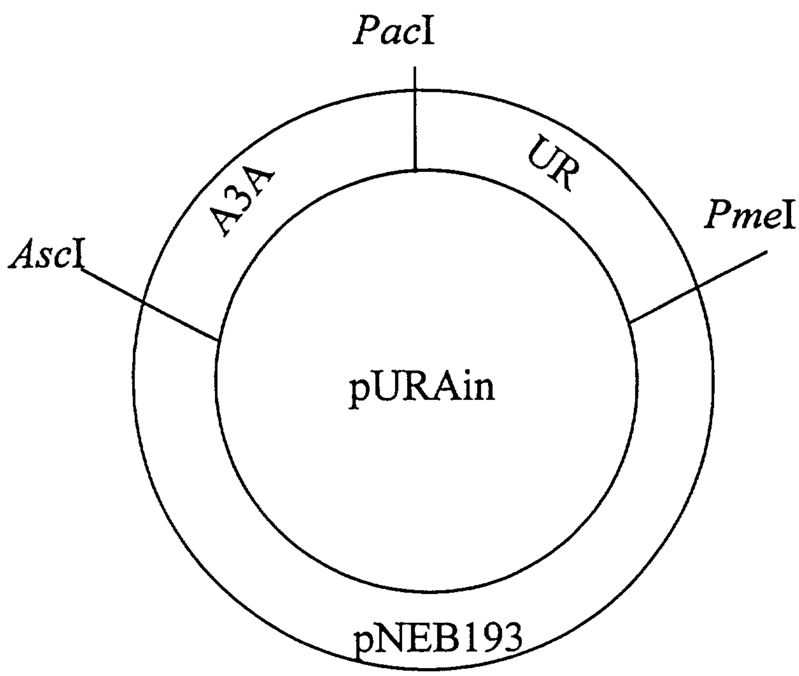


Figure 24

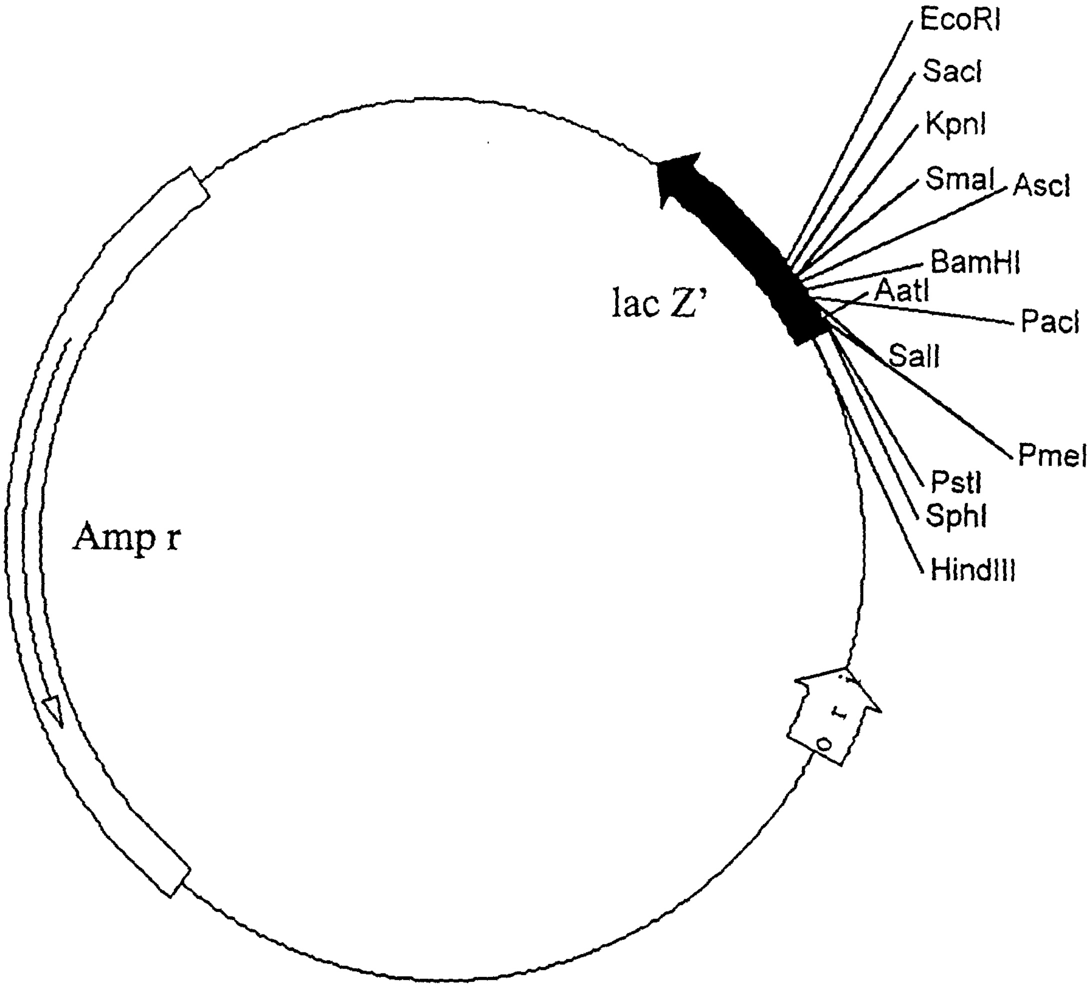
EcoRI
SacI
KpnI
SmaI
AscI
BamHI
AatI
PacI
SalI
PmeI
PstI
SphI
HindIII
lac Z'
Amp r
ori

Figure 25

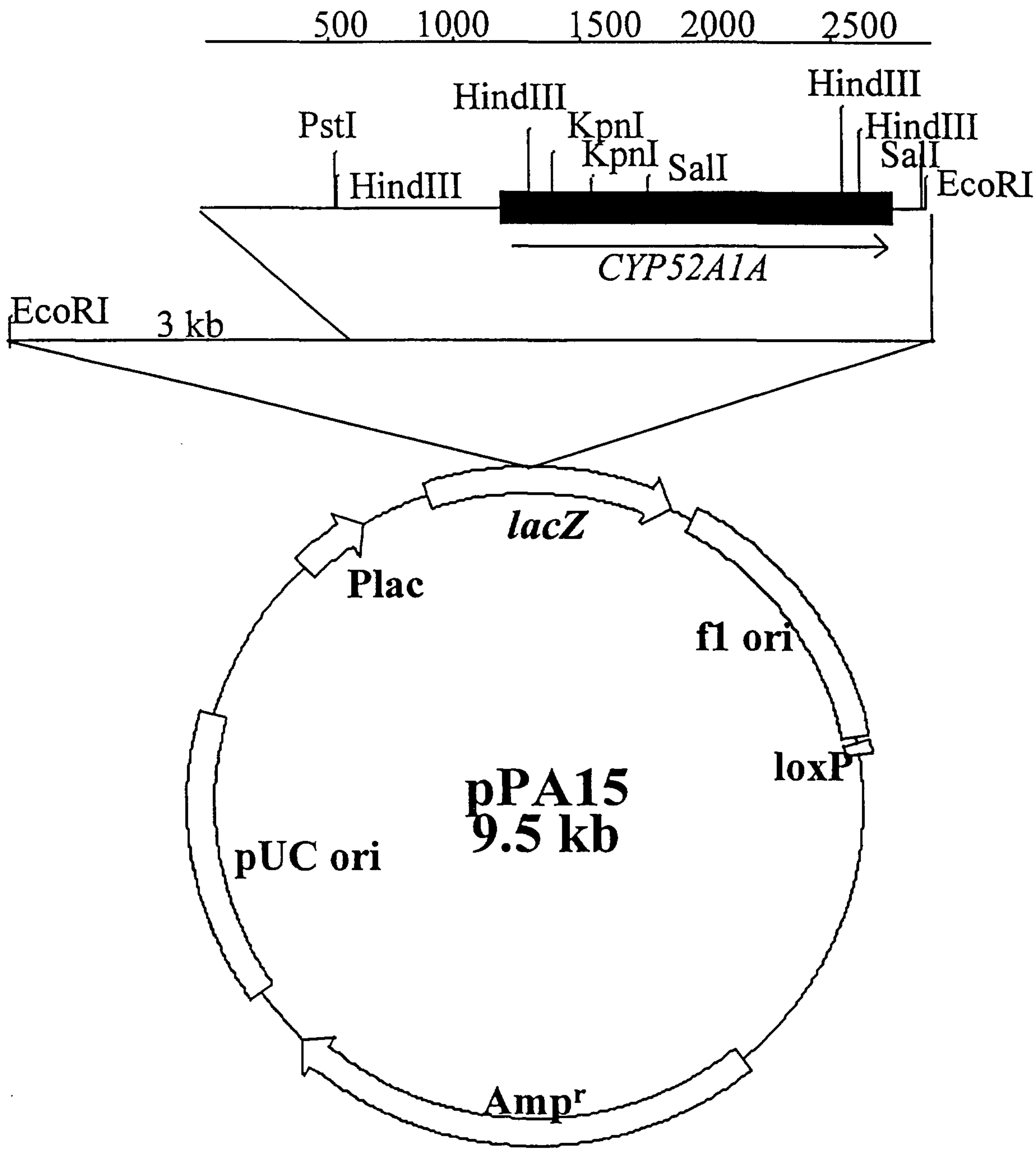
500
1000
1500
2000
2500
PstI
HindIII
HindIII
KpnI
KpnI
SalI
HindIII
HindIII
SalI
EcoRI
CYP52A1A
EcoRI
3 kb
lacZ
Plac
f1 ori
loxP
pPA15
9.5 kb
pUC ori
Amp^r

Figure 26

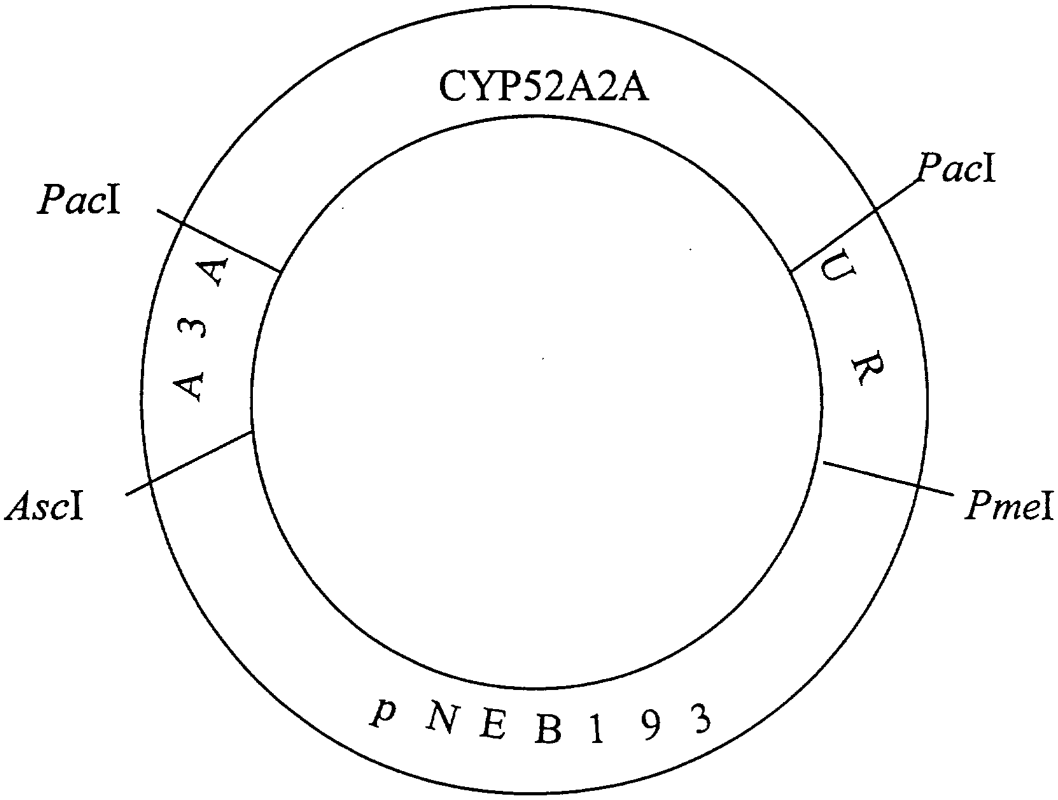


Figure 27

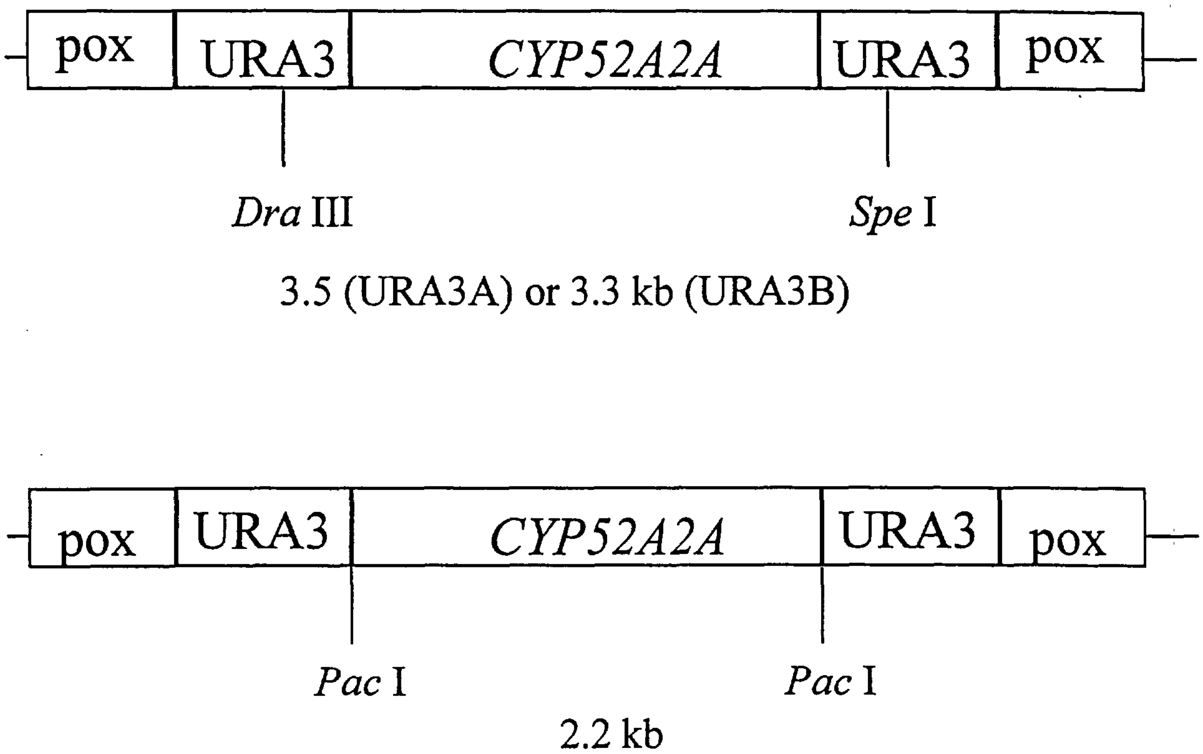
pox
URA3
CYP52A2A
URA3
pox
Dra III
Spe I
3.5 (URA3A) or 3.3 kb (URA3B)
pox
URA3
CYP52A2A
URA3
pox
Pac I
Pac I
2.2 kb

Figure 28

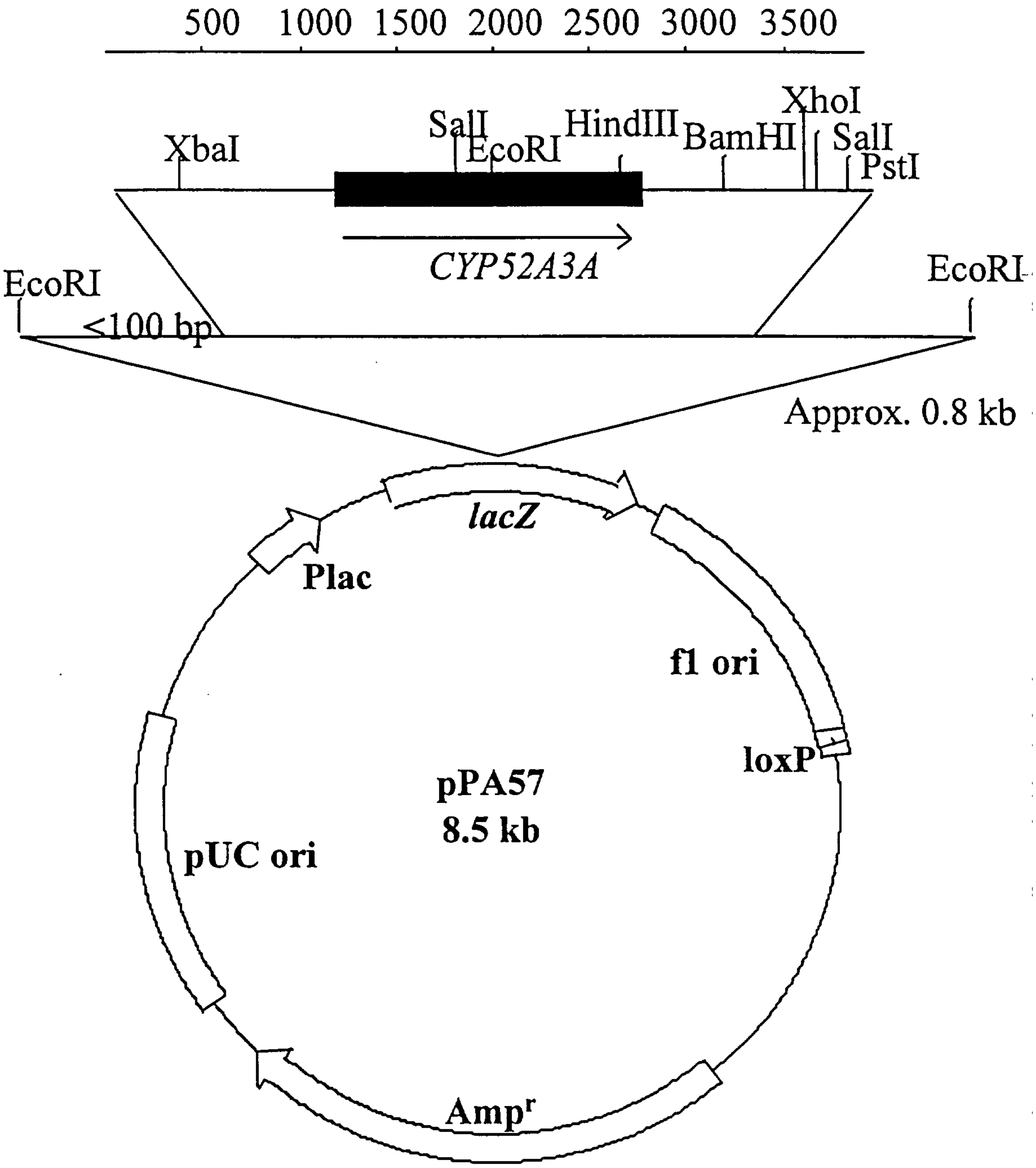
500
1000
1500
2000
2500
3000
3500
XbaI
SalI
EcoRI
HindIII
BamHI
XhoI
SalI
PstI
CYP52A3A
EcoRI
<100 bp
EcoRI
Approx. 0.8 kb
lacZ
Plac
f1 ori
loxP
pPA57
8.5 kb
pUC ori
Amp^r

Figure 29

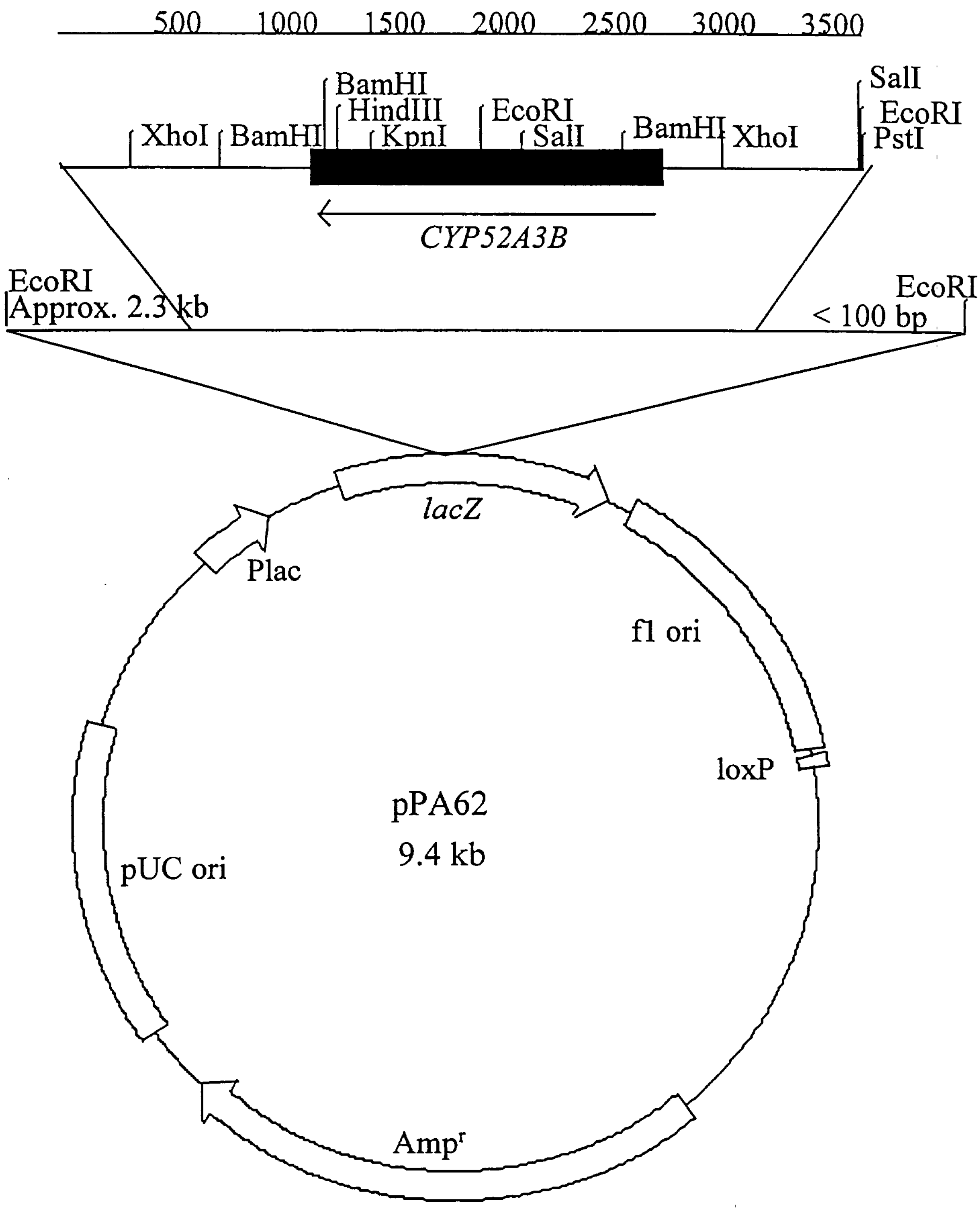
500
1000
1500
2000
2500
3000
3500
BamHI
HindIII
EcoRI
XhoI
BamHI
KpnI
SalI
BamHI
XhoI
SalI
EcoRI
PstI
CYP52A3B
EcoRI
Approx. 2.3 kb
EcoRI
< 100 bp
lacZ
Plac
f1 ori
loxP
pPA62
9.4 kb
pUC ori
Amp^r

Figure 30

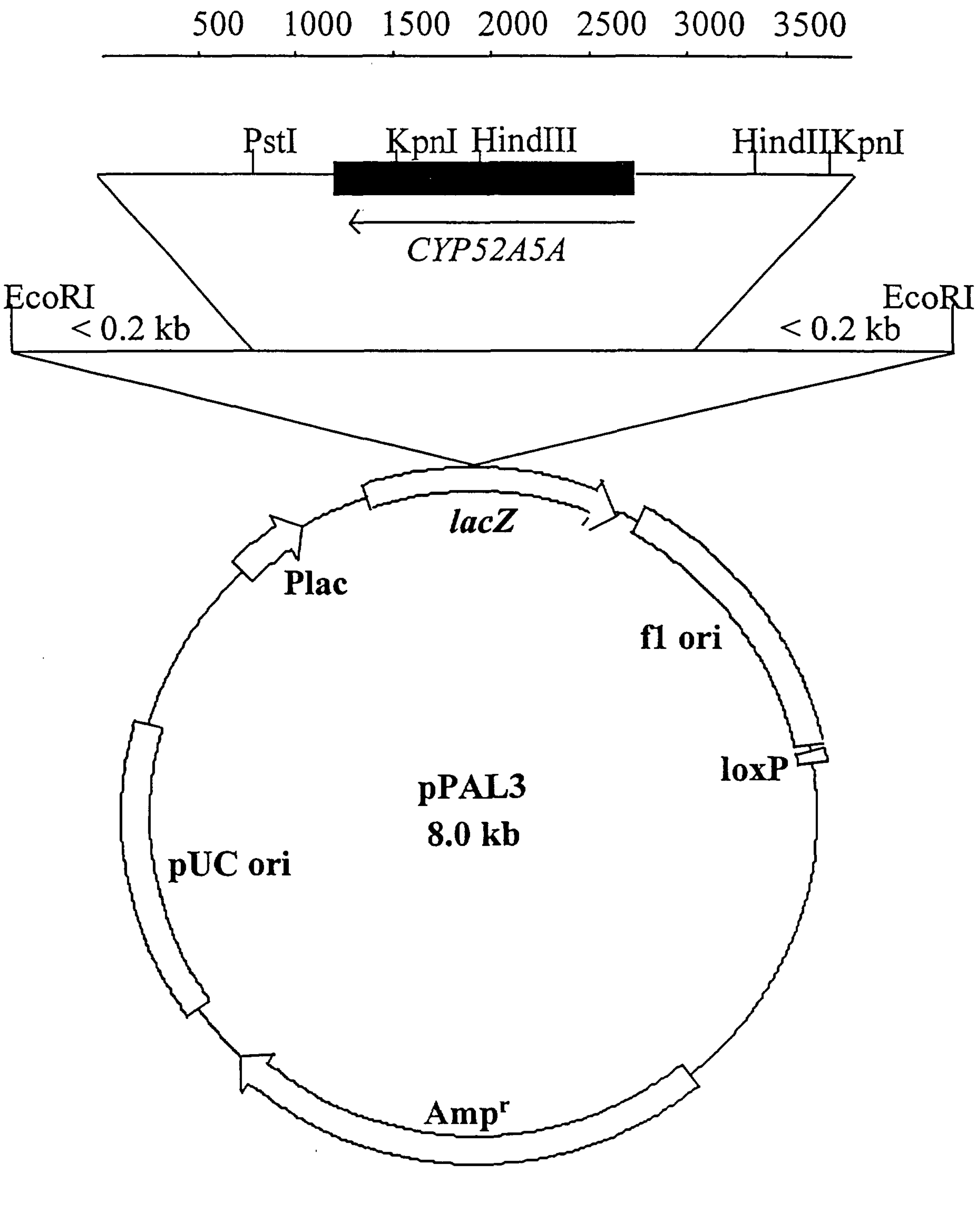
500
1000
1500
2000
2500
3000
3500
PstI
KpnI
HindIII
HindIII
KpnI
CYP52A5A
EcoRI
< 0.2 kb
EcoRI
< 0.2 kb
lacZ
Plac
f1 ori
loxP
pPAL3
8.0 kb
pUC ori
Amp^r

Figure 31

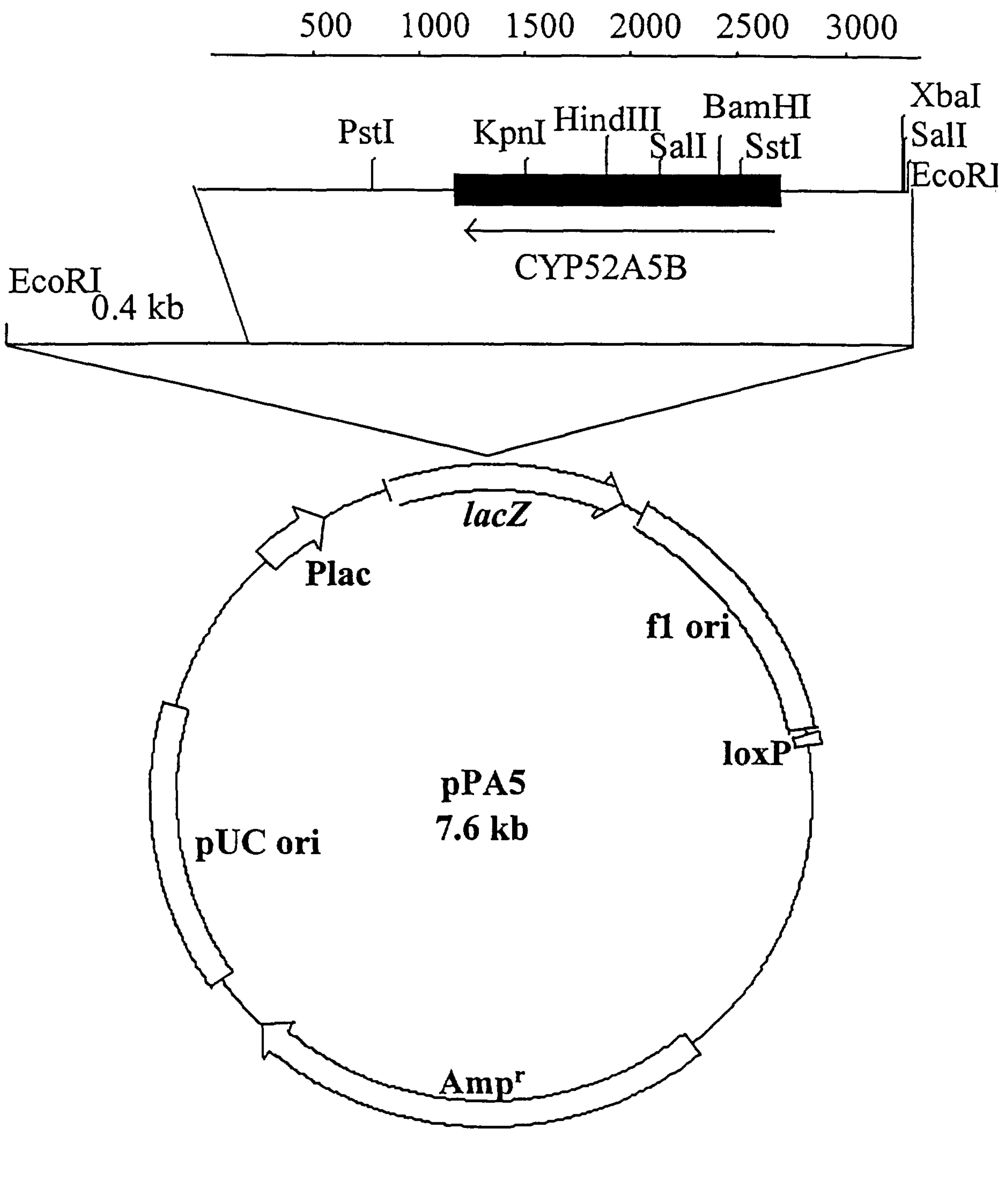
500
1000
1500
2000
2500
3000
PstI
KpnI
HindIII
SalI
BamHI
SstI
XbaI
SalI
EcoRI
CYP52A5B
EcoRI
0.4 kb
lacZ
Plac
f1 ori
loxP
pPA5
7.6 kb
pUC ori
Amp^r

Figure 32

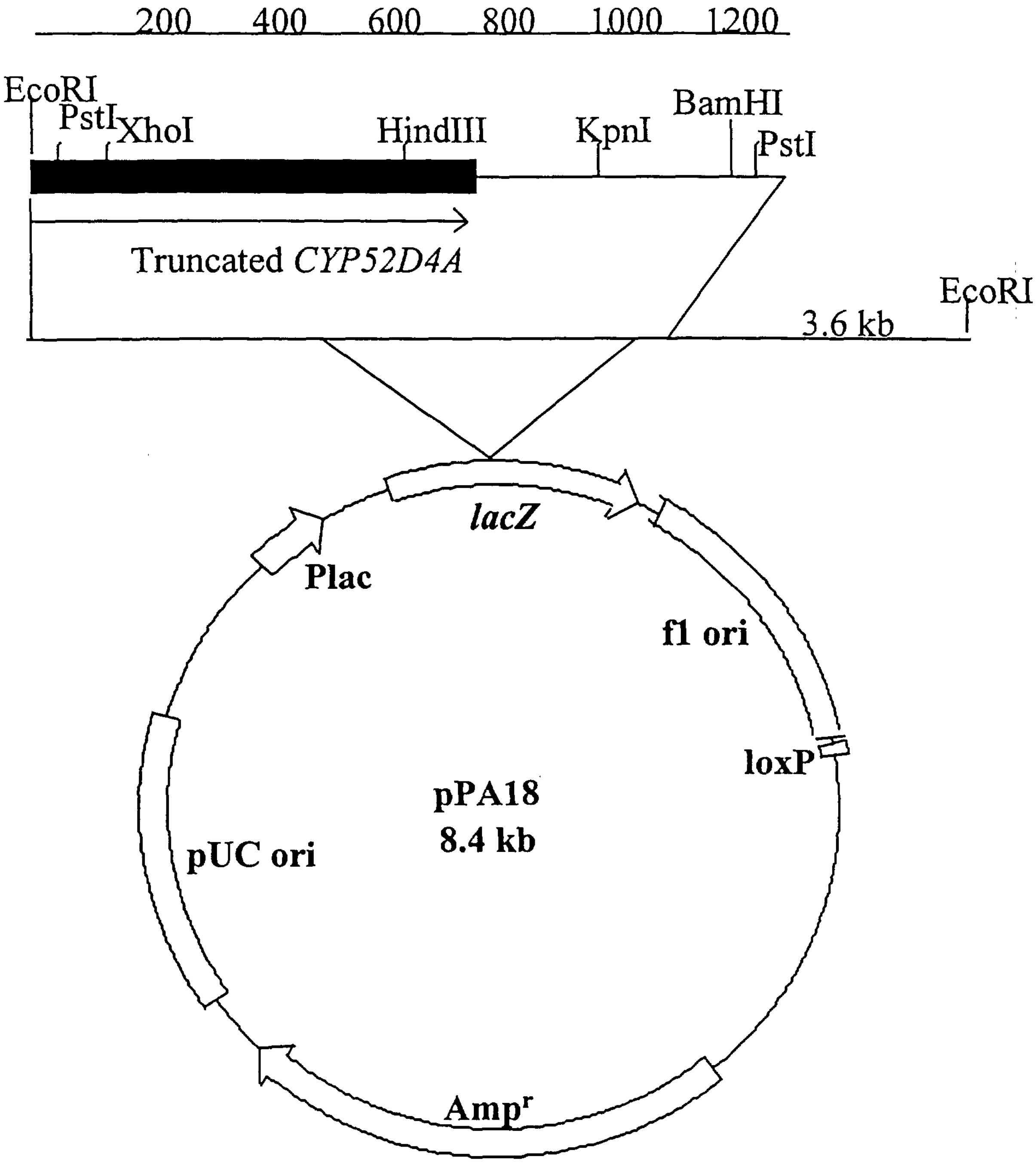
200
400
600
800
1000
1200
EcoRI
PstI
XhoI
HindIII
KpnI
BamHI
PstI
Truncated CYP52D4A
EcoRI
3.6 kb
lacZ
Plac
f1 ori
loxP
pPA18
8.4 kb
pUC ori
Amp^r

Figure 33

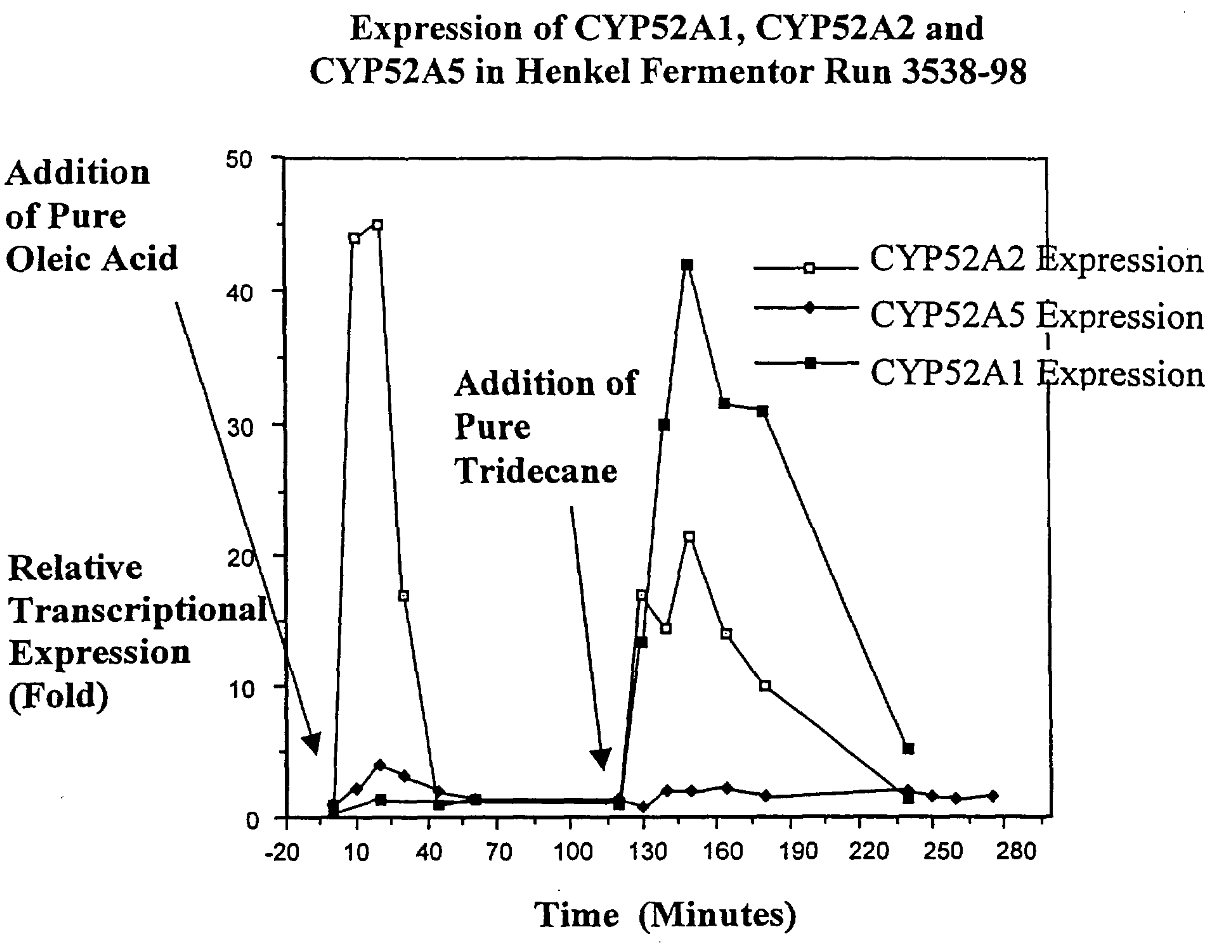
Expression of CYP52A1, CYP52A2 and
CYP52A5 in Henkel Fermentor Run 3538-98
Addition of Pure Oleic Acid
Addition of Pure Tridecane
Relative Transcriptional Expression (Fold)
50
40
30
20
10
0
-20
10
40
70
100
130
160
190
220
250
280
Time (Minutes)
CYP52A2 Expression
CYP52A5 Expression
CYP52A1 Expression

Figure 34

1 gram (g) Whole fermentation broth (70°C)
+
1g Internal standard C15:0 10g/l in 1N KOH (70°C)
+
0.8 ml 6N HCl
+
6 ml Methyl-t-Butyl-Ether (MtBE)
↓ Extract in 60 ml separatory funnel
1 ml MtBE phase pipeted in 12X75mm test tube
↓
Dry down to solids under $N_2$ stream
↓
Add 1 ml 12% BF3-Methanol (Kodak, 4°C) and stopper test tube
↓
Dissolve solids,esterify for 15 min.@ 60°C, quiescently
↓
Add 0.25 ml saturated NaCl solution (71.5g NaCl/200 ml H2O)
↓ Vortex to mix
Add 1 ml Mixed Ethers (50% diethyl ether 50% petroleum ether,v/v)
↓
Shake for 1 min. To extract methylesters
↓
Inject 5 ul of mixed ether phase into GC

<u>GC Parameters</u>

Column: HP-INNOWAX capillary column, 30m X 0.32 mm, 0.5um film thickness
Split ratio: 1:100
Column Head Pressure : 13.5 psig
Injector temperature: 240°C
FID Detector Temp. : 250°C
Temp. Prog.: 90°C for 0 min. to 190°C @ 7°C/min. for 0 min. to 235°C @ 12°C/min. for 30 min.

Figure 35

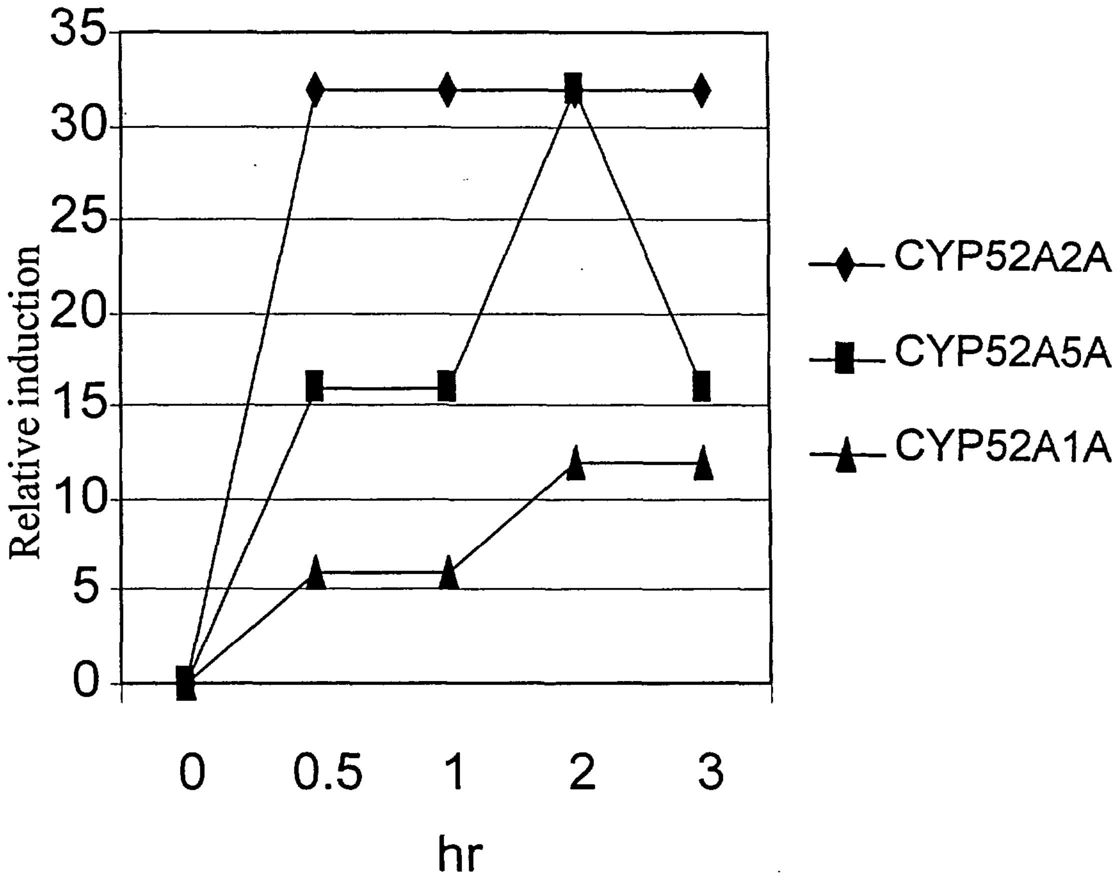
35
30
25
20
15
10
5
0
Relative induction
0
0.5
1
2
3
hr
CYP52A2A
CYP52A5A
CYP52A1A

Figure 36

SEQUENCE LISTING

<110> Henkel Corporation

<120> CYTOCHROME P450 MONOOXYGENASE AND NADPH CYTOCHROME P450 OXIDOREDUCTASE GENES AND PROTEINS RELATED TO THE OMEGA HYDROXYLASE COMPLEX OF CANDIDA TROPICALIS AND METHODS RELATING THERETO

<130> PCT

<140> PCT/US99/20797
<141> 1999-10-09

<160> 107

<170> PatentIn Ver. 2.1

<210> 1
<211> 32
<212> DNA
<213> Artificial Sequence

<220>
<223> Description of Artificial Sequence: Primer for CYP52A2A gene

<400> 1
ccttaattaa atgcacgaag cggagataaa ag 32

<210> 2
<211> 30
<212> DNA
<213> Artificial Sequence

<220>
<223> Description of Artificial Sequence: Primer for CYP52A2A gene

<400> 2
ccttaattaa gcataagctt gctcgagtct 30

<210> 3
<211> 31
<212> DNA
<213> Artificial Sequence

<220>
<223> Description of Artificial Sequence: Primer for CYP52A3A gene

<400> 3
ccttaattaa acgcaatggg aacatggagt g 31

<210> 4
<211> 34
<212> DNA
<213> Artificial Sequence

<220>
<223> Description of Artificial Sequence: Primer for CYP52A3A gene

<400> 4
ccttaattaa tcgcactacg gttattggta tcag 34

<210> 5
<211> 29
<212> DNA
<213> Artificial Sequence

<220>
<223> Description of Artificial Sequence: Primer for CYP52A5A gene

<400> 5
ccttaattaa tcaaagtacg ttcaggcgg 29

<210> 6
<211> 34
<212> DNA
<213> Artificial Sequence

<220>
<223> Description of Artificial Sequence: Primer for CYP52A5A gene

<400> 6
ccttaattaa ggcagacaac aacttggcaa agtc 34

<210> 7
<211> 31
<212> DNA
<213> Artificial Sequence

<220>
<223> Description of Artificial Sequence: Primer for CPRB gene

<400> 7
ccttaattaa gaggtcgttg gttgagtttt c 31

<210> 8
<211> 29
<212> DNA
<213> Artificial Sequence

<220>
<223> Description of Artificial Sequence: Primer for CPRB gene

<400> 8
ccttaattaa ttgataatga cgttgcggg 29

<210> 9
<211> 33
<212> DNA
<213> Artificial Sequence

<220>
<223> Description of Artificial Sequence: Primer for URA3A gene

<400> 9
aggcgcgccg gagtccaaaa agaccaacct ctg 33

<210> 10
<211> 34
<212> DNA
<213> Artificial Sequence

<220>
<223> Description of Artificial Sequence: Primer for URA3A gene

<400> 10
ccttaattaa tacgtggata ccttcaagca agtg 34

<210> 11
<211> 35
<212> DNA
<213> Artificial Sequence

<220>
<223> Description of Artificial Sequence: Primer for URA3A gene

<400> 11
ccttaattaa gctcacgagt tttgggattt tcgag 35

<210> 12
<211> 35
<212> DNA
<213> Artificial Sequence

<220>
<223> Description of Artificial Sequence: Primer for URA3A gene

<400> 12
gggtttaaac cgcagaggtt ggtctttttg gactc 35

<210> 13
<211> 10
<212> DNA
<213> Artificial Sequence

<220>
<223> Description of Artificial Sequence: PmeI restriction site

<400> 13
gggtttaaac 10

<210> 14
<211> 9
<212> DNA
<213> Artificial Sequence

<220>
<223> Description of Artificial Sequence: AscI restriction site

<400> 14
aggcgcgcc 9

<210> 15
<211> 10
<212> DNA
<213> Artificial Sequence

<220>
<223> Description of Artificial Sequence: PacI restriction site

<400> 15
ccttaattaa 10

<210> 16
<211> 21
<212> DNA
<213> Artificial Sequence

<220>
<223> Description of Artificial Sequence: Primer for CPR gene

<400> 16
tcycaaacwg gtacwgcwga a 21

<210> 17
<211> 21
<212> DNA
<213> Artificial Sequence

<220>
<223> Description of Artificial Sequence: Primer for CPR gene

<400> 17
ggtttgggta aytcwactta t 21

<210> 18

<211> 18
<212> DNA
<213> Artificial Sequence

<220>
<223> Description of Artificial Sequence: Primer for CPR gene

<400> 18
cgttattatc atttcttc 18

<210> 19
<211> 20
<212> DNA
<213> Artificial Sequence

<220>
<223> Description of Artificial Sequence: Primer for CPR gene

<400> 19
gcmacaccrg tacctggacc 20

<210> 20
<211> 18
<212> DNA
<213> Artificial Sequence

<220>
<223> Description of Artificial Sequence: Primer for CPR gene

<400> 20
atcccaatcg taatcagc 18

<210> 21
<211> 18
<212> DNA
<213> Artificial Sequence

<220>
<223> Description of Artificial Sequence: Primer for CPR gene

<400> 21

acttgtcttc gtttagca 18

<210> 22
<211> 18
<212> DNA
<213> Artificial Sequence

<220>
<223> Description of Artificial Sequence: Primer for CPR gene

<400> 22
ctacgtctgt ggtgatgc 18

<210> 23
<211> 17
<212> DNA
<213> Artificial Sequence

<220>
<223> Description of Artificial Sequence: Primer for CYP gene

<220>
<221> misc_feature
<222> (3)..(4)
<223> n=dATP or dCTP or dGTP or dTTP

<220>
<221> misc_feature
<222> (9)..(10)
<223> n=dATP or dCTP or dGTP or dTTP

<220>
<221> misc_feature
<222> (12)..(13)
<223> n=dATP or dCTP or dGTP or dTTP

<220>
<221> misc_feature
<222> (15)..(16)
<223> n=dATP or dCTP or dGTP or dTTP

<400> 23
cgngayacna cngcngg 17

<210> 24
<211> 17
<212> DNA
<213> Artificial Sequence

<220>
<223> Description of Artificial Sequence: Primer for CYP gene

<220>
<221> misc_feature
<222> (9)..(10)
<223> n=dATP or dCTP or dGTP or dTTP

<220>
<221> misc_feature
<222> (12)..(13)
<223> n=dATP or dCTP or dGTP or dTTP

<220>
<221> misc_feature
<222> (15)..(16)
<223> n=dATP or dCTP or dGTP or dTTP

<400> 24
agrgayacna cngcngg 17

<210> 25
<211> 17
<212> DNA
<213> Artificial Sequence

<220>
<223> Description of Artificial Sequence: Primer for CYP gene

<220>
<221> misc_feature
<222> (3)..(4)
<223> n=dATP or dCTP or dGTP or dTTP

<220>
<221> misc_feature
<222> (15)..(16)
<223> n=dATP or dCTP or dGTP or dTTP

<400> 25
agngcraayt gytgncc 17

<210> 26
<211> 18
<212> DNA
<213> Artificial Sequence

<220>
<223> Description of Artificial Sequence: Primer for CYP gene

<220>
<221> misc_feature
<222> (4)..(5)
<223> n=dATP or dCTP or dGTP or dTTP

<220>
<221> misc_feature
<222> (16)..(17)
<223> n=dATP or dCTP or dGTP or dTTP

<400> 26
yaangcraay tgytgncc 18

<210> 27
<211> 29
<212> DNA
<213> Artificial Sequence

<220>
<223> Description of Artificial Sequence: Primer for CYP gene

<400> 27
attcaacggt ggtccaagaa tctgtttgg 29

<210> 28
<211> 25
<212> DNA
<213> Artificial Sequence

<220>
<223> Description of Artificial Sequence: Primer for CYP gene

<400> 28
gagctatgtt gagaccacag tttgc 25

<210> 29
<211> 26
<212> DNA
<213> Artificial Sequence

<220>
<223> Description of Artificial Sequence: Primer for CYP gene

<400> 29
cttcagttaa agcaaattgt ttggcc 26

<210> 30
<211> 25
<212> DNA
<213> Artificial Sequence

<220>
<223> Description of Artificial Sequence: Primer for pTriplEX vector

<400> 30
ctcgggaagc gcgccattgt gttgg 25

<210> 31
<211> 29
<212> DNA
<213> Artificial Sequence

<220>
<223> Description of Artificial Sequence: Primer for pTriplEx vector

<400> 31
taatacgact cactataggg cgaattggc 29

<210> 32
<211> 21
<212> DNA
<213> Artificial Sequence

<220>
<223> Description of Artificial Sequence: Primer for CYP gene

<400> 32
tgrytcaaac catctytctg g 21

<210> 33
<211> 17
<212> DNA
<213> Artificial Sequence

<220>
<223> Description of Artificial Sequence: Primer for CYP gene

<400> 33
ggaccggcgt taaaggg 17

<210> 34
<211> 23
<212> DNA
<213> Artificial Sequence

<220>
<223> Description of Artificial Sequence: Primer for CYP gene

<400> 34
catagtcgwa tyatgcttag acc 23

<210> 35
<211> 17
<212> DNA
<213> Artificial Sequence

<220>
<223> Description of Artificial Sequence: Primer for CYP gene

<400> 35
ggaccaccat tgaatgg 17

<210> 36
<211> 540
<212> DNA
<213> Candida tropicalis

<400> 36
atgattgaac aactcctaga atattggtat gtcgttgtgc cagtgttgta catcatcaaa 60
caactccttg catacacaaa gactcgcgtc ttgatgaaaa agttgggtgc tgctccagtc 120
acaaacaagt tgtacgacaa cgctttcggt atcgtcaatg gatggaaggc tctccagttc 180
aagaaagagg gcagggctca agagtacaac gattacaagt ttgaccactc caagaaccca 240
agcgtgggca cctacgtcag tattcttttc ggcaccagga tcgtcgtgac caaagatcca 300
gagaatatca aagctatttt ggcaacccag tttggtgatt tttctttggg caagaggcac 360
actcttttta agcctttgtt aggtgatggg atcttcacat tggacggcga aggctggaag 420
cacagcagag ccatgttgag accacagttt gccagagaac aagttgctca tgtgacgtcg 480
ttggaaccac acttccagtt gttgaagaag catattctta agcacaaggg tgaatacttt 540

<210> 37
<211> 25
<212> DNA
<213> Artificial Sequence

<220>
<223> Description of Artificial Sequence: Primer for CYP52A1A gene

<400> 37
ccgatgaagt tttcgacgag taccc 25

<210> 38
<211> 26
<212> DNA
<213> Artificial Sequence

<220>
<223> Description of Artificial Sequence: Primer for CYP52A1A gene

<400> 38
aaggctttaa cgtgtccaat ctggtc 26

<210> 39
<211> 27
<212> DNA
<213> Artificial Sequence

<220>
<223> Description of Artificial Sequence: Primer for CYP52A2A gene

<400> 39
attatcgcca catacttcac caaatgg 27

<210> 40
<211> 24
<212> DNA
<213> Artificial Sequence

<220>
<223> Description of Artificial Sequence: Primer for CYP52A2A gene

<400> 40
cgagatcgtg gatacgctgg agtg 24

<210> 41
<211> 25
<212> DNA
<213> Artificial Sequence

<220>
<223> Description of Artificial Sequence: Primer for CYP52A3A gene

<400> 41
gccactcggt aactttgtca gggac 25

<210> 42
<211> 26
<212> DNA
<213> Artificial Sequence

<220>
<223> Description of Artificial Sequence: Primer for CYP52A3A gene

<400> 42
cattgaactg agtagccaaa acagcc 26

<210> 43

<211> 27
<212> DNA
<213> Artificial Sequence

<220>
<223> Description of Artificial Sequence: Primer for CYP52A3A and CYP52A3B genes

<400> 43
cctacgtttg gtatcgctac tccgttg 27

<210> 44
<211> 22
<212> DNA
<213> Artificial Sequence

<220>
<223> Description of Artificial Sequence: Primer for CYP52A3A and CYP52A3B genes

<400> 44
tttccagcca gcaccgtcca ag 22

<210> 45
<211> 24
<212> DNA
<213> Artificial Sequence

<220>
<223> Description of Artificial Sequence: Primer for CYP52D4A gene

<400> 45
gcagagccga tctatgttgc gtcc 24

<210> 46
<211> 25
<212> DNA
<213> Artificial Sequence

<220>
<223> Description of Artificial Sequence: Primer for CYP52D4A gene

<400> 46

tcattgaatg cttccaggaa cctcg 25

<210> 47
<211> 20
<212> DNA
<213> Artificial Sequence

<220>
<223> Description of Artificial Sequence: Primer for CYP52A5A and CYP52A5B genes

<400> 47
aagagggcag ggctcaagag 20

<210> 48
<211> 21
<212> DNA
<213> Artificial Sequence

<220>
<223> Description of Artificial Sequence: Primer for CYP52A5A and CYP52A5B genes

<400> 48
tccatgtgaa gatcccatca c 21

<210> 49
<211> 20
<212> DNA
<213> Artificial Sequence

<220>
<223> Description of Artificial Sequence: Primer for CYP52A8A gene

<400> 49
cttgaaggcc gtgttgaacg 20

<210> 50
<211> 21
<212> DNA
<213> Artificial Sequence

<220>

<223> Description of Artificial Sequence: Primer for CYP52A8A gene

<400> 50
caggatttgt ctgagttgcc g 21

<210> 51
<211> 28
<212> DNA
<213> Artificial Sequence

<220>
<223> Description of Artificial Sequence: Primer for POX4A and POX4B genes

<400> 51
ccattgcctt gagatacgcc attggtag 28

<210> 52
<211> 26
<212> DNA
<213> Artificial Sequence

<220>
<223> Description of Artificial Sequence: Primer for POX4A and POX4B genes

<400> 52
agccttggtg tcgttctttt caacgg 26

<210> 53
<211> 26
<212> DNA
<213> Artificial Sequence

<220>
<223> Description of Artificial Sequence: Primer for POX5A gene

<400> 53
ttgggtttgt ttgtttcctg tgtccg 26

<210> 54
<211> 27

<212> DNA
<213> Artificial Sequence

<220>
<223> Description of Artificial Sequence: Primer for POX5A gene

<400> 54
cctttgacct tcaatctggc gtagacg 27

<210> 55
<211> 26
<212> DNA
<213> Artificial Sequence

<220>
<223> Description of Artificial Sequence: Primer for CPRA gene

<400> 55
gtttgctgaa tacgctgaag gtgatg 26

<210> 56
<211> 27
<212> DNA
<213> Artificial Sequence

<220>
<223> Description of Artificial Sequence: Primer for CPRA gene

<400> 56
tggagctgaa caactctctc gtctcgg 27

<210> 57
<211> 20
<212> DNA
<213> Artificial Sequence

<220>
<223> Description of Artificial Sequence: Primer for CPRA and CPRB genes

<400> 57
ttcctcaaca cggacagcgg 20

<210> 58
<211> 24
<212> DNA
<213> Artificial Sequence

<220>
<223> Description of Artificial Sequence: Primer for CPRA and CPRB genes

<400> 58
agtcaaccag gtgtggaact cgtc 24

<210> 59
<211> 49
<212> DNA
<213> Artificial Sequence

<220>
<223> Description of Artificial Sequence: Primer for CYP52A5A gene

<400> 59
ggatcctaat acgactcact atagggagga agagggcagg gctcaagag 49

<210> 60
<211> 42
<212> DNA
<213> Artificial Sequence

<220>
<223> Description of Artificial Sequence: Primer for CYP52A5A gene

<400> 60
tccatgtgaa gatcccatca cgagtgtgcc tcttgcccaa ag 42

<210> 61
<211> 54
<212> DNA
<213> Artificial Sequence

<220>
<223> Description of Artificial Sequence: Primer for

CYP52A1A gene

<400> 61
ggatcctaat acgactcact atagggaggc cgatgaagtt ttcgacgagt accc 54

<210> 62
<211> 52
<212> DNA
<213> Artificial Sequence

<220>
<223> Description of Artificial Sequence: Primer for CYP52A1A gene

<400> 62
aaggctttaa cgtgtccaat ctggtcaaca tagctctgga gtgcttccaa cc 52

<210> 63
<211> 56
<212> DNA
<213> Artificial Sequence

<220>
<223> Description of Artificial Sequence: Primer for CYP52A2A gene

<400> 63
ggatcctaat acgactcact atagggagga ttatcgccac atacttcacc aaatgg 56

<210> 64
<211> 52
<212> DNA
<213> Artificial Sequence

<220>
<223> Description of Artificial Sequence: Primer for CYP52A2A gene

<400> 64
cgagatcgtg gatacgctgg agtgcgtcgc tcttcttctt caacaattca ag 52

<210> 65
<211> 49
<212> DNA

<213> Artificial Sequence

<220>
<223> Description of Artificial Sequence: Primer for CYP52A3A gene

<400> 65
cattgaactg agtagccaaa acagcccatg gtttcaatca atgggaggc 49

<210> 66
<211> 54
<212> DNA
<213> Artificial Sequence

<220>
<223> Description of Artificial Sequence: Primer for CYP52A3A gene

<400> 66
ggatcctaat acgactcact atagggaggg ccactcggta actttgtcag ggac 54

<210> 67
<211> 56
<212> DNA
<213> Artificial Sequence

<220>
<223> Description of Artificial Sequence: Primer for CYP52A3A and CYP52A3B genes

<400> 67
ggatcctaat acgactcact atagggaggc ctacgtttgg tatcgctact ccgttg 56

<210> 68
<211> 48
<212> DNA
<213> Artificial Sequence

<220>
<223> Description of Artificial Sequence: Primer for CYP52A3A and CYP52A3B genes

<400> 68
tttccagcca gcaccgtcca agcaacaagg agtacaagaa atcgtgtc 48

<210> 69
<211> 53
<212> DNA
<213> Artificial Sequence

<220>
<223> Description of Artificial Sequence: Primer for CYP52D4A gene

<400> 69
ggatcctaat acgactcact atagggaggg cagagccgat ctatgttgcg tcc 53

<210> 70
<211> 45
<212> DNA
<213> Artificial Sequence

<220>
<223> Description of Artificial Sequence: Primer for CYP52D4A gene

<400> 70
tcattgaatg cttccaggaa cctcgccaca tccatcgaga accgg 45

<210> 71
<211> 49
<212> DNA
<213> Artificial Sequence

<220>
<223> Description of Artificial Sequence: Primer for CYP52A8A gene

<400> 71
ggatcctaat acgactcact atagggaggc ttgaaggccg tgttgaacg 49

<210> 72
<211> 46
<212> DNA
<213> Artificial Sequence

<220>
<223> Description of Artificial Sequence: Primer for CYP52A8A gene

<400> 72
caggatttgt ctgagttgcc gcctgatcaa gataggatcc ttgccg 46

<210> 73
<211> 56
<212> DNA
<213> Artificial Sequence

<220>
<223> Description of Artificial Sequence: Primer for CPRA gene

<400> 73
ggatcctaat acgactcact atagggaggg gtttgctgaa tacgctgaag gtgatg 56

<210> 74
<211> 52
<212> DNA
<213> Artificial Sequence

<220>
<223> Description of Artificial Sequence: Primer for CPRA gene

<400> 74
tggagctgaa caactctctc gtctcgggtg gtcgaatgga cccttggtca ag 52

<210> 75
<211> 49
<212> DNA
<213> Artificial Sequence

<220>
<223> Description of Artificial Sequence: Primer for CPRA and CPRB genes

<400> 75
ggatcctaat acgactcact atagggaggt tcctcaacac ggacagcgg 49

<210> 76
<211> 49
<212> DNA
<213> Artificial Sequence

<220>
<223> Description of Artificial Sequence: Primer for CPRA and CPRB genes

<400> 76
agtcaaccag gtgtggaact cgtcggtggc aacaatgaaa aacaccaag 49

<210> 77
<211> 57
<212> DNA
<213> Artificial Sequence

<220>
<223> Description of Artificial Sequence: Primer for POX4A and POX4B genes

<400> 77
ggatcctaat acgactcact atagggaggc cattgccttg agatacgcca ttggtag 57

<210> 78
<211> 53
<212> DNA
<213> Artificial Sequence

<220>
<223> Description of Artificial Sequence: Primer for POX4A and POX4B genes

<400> 78
agccttggtg tcgttctttt caacggaagg tggtctcgat ggtgtgttca acc 53

<210> 79
<211> 55
<212> DNA
<213> Artificial Sequence

<220>
<223> Description of Artificial Sequence: Primer for POX5A gene

<400> 79
ggatcctaat acgactcact atagggaggt tgggtttgtt tgtttcctgt gtccg 55

<210> 80
<211> 50
<212> DNA
<213> Artificial Sequence

<220>
<223> Description of Artificial Sequence: Primer for POX5A gene

<400> 80
cctttgacct tcaatctggc gtagacgcag caccaccgat ccaccacttg 50

<210> 81
<211> 4206
<212> DNA
<213> Candida tropicalis

<400> 81
catcaagatc atctatgggg ataattacga cagcaacatt gcagaaagag cgttggtcac 60
aatcgaaaga gcctatggcg ttgccgtcgt tgaggcaaat gacagcacca acaataacga 120
tggtcccagt gaagagcctt cagaacagtc cattgttgac gcttaaggca cggataatta 180
cgtggggcaa aggaacgcgg aattagttat gggggatca aaagcggaag atttgtgttg 240
cttgtgggtt ttttccttta tttttcatat gatttctttg cgcaagtaac atgtgccaat 300
ttagtttgtg attagcgtgc cccacaattg gcatcgtgga cgggcgtgtt ttgtcatacc 360
ccaagtctta actagctcca cagtctcgac ggtgtctcga cgatgtcttc ttccacccct 420
cccatgaatc attcaaagtt gttgggggat ctccaccaag ggcaccggag ttaatgctta 480
tgtttctccc actttggttg tgattggggt agtctagtga gttggagatt ttcttttttt 540
cgcaggtgtc tccgatatcg aaatttgatg aatatagaga gaagccagat cagcacagta 600
gattgccttt gtagttagag atgttgaaca gcaactagtt gaattacacg ccaccacttg 660
acagcaagtg cagtgagctg taaacgatgc agccagagtg tcaccaccaa ctgacgttgg 720
gtggagttgt tgttgttgtt gttggcaggg ccatattgct aaacgaagac aagtagcaca 780
aaacccaagc ttaagaacaa aaataaaaaa aattcatacg acaattccaa agccattgat 840
ttacataatc aacagtaaga cagaaaaaac tttcaacatt tcaaagttcc ctttttccta 900
ttacttcttt tttttcttct ttccttcttt ccttctgttt ttcttacttt atcagtcttt 960
tacttgtttt tgcaattcct catcctcctc ctactcctcc tcaccatggc tttagacaag 1020
ttagatttgt atgtcatcat aacattggtg gtcgctgtag ccgcctattt tgctaagaac 1080
cagttccttg atcagcccca ggacaccggg ttcctcaaca cggacagcgg aagcaactcc 1140
agagacgtct tgctgacatt gaagaagaat aataaaaaca cgttgttgtt gtttgggtcc 1200
cagacgggta cggcagaaga ttacgccaac aaattgtcca gagaattgca ctccagattt 1260
ggcttgaaaa cgatggttgc agatttcgct gattacgatt gggataactt cggagatatc 1320
accgaagaca tcttggtgtt tttcattgtt gccacctatg gtgagggtga acctaccgat 1380
aatgccgacg agttccacac ctggttgact gaagaagctg acactttgag taccttgaaa 1440
tacaccgtgt tcgggttggg taactccacg tacgagttct tcaatgccat tggtagaaag 1500
tttgacagat tgttgagcga gaaaggtggt gacaggtttg ctgaatacgc tgaaggtgat 1560
gacggtactg gcaccttgga cgaagatttc atggcctgga aggacaatgt ctttgacgcc 1620
ttgaagaatg atttgaactt tgaagaaaag gaattgaagt acgaaccaaa cgtgaaattg 1680
actgagagag acgacttgtc tgctgctgac tcccaagttt ccttgggtga gccaaacaag 1740

```
aagtacatca actccgaggg catcgacttg accaagggtc cattcgacca cacccaccca 1800
tacttggcca gaatcaccga gacgagagag ttgttcagct ccaaggacag acactgtatc 1860
cacgttgaat ttgacatttc tgaatcgaac ttgaaataca ccaccggtga ccatctagct 1920
atctggccat ccaactccga cgaaaacatt aagcaatttg ccaagtgttt cggattggaa 1980
gataaactcg acactgttat tgaattgaag gcgttggact ccacttacac catcccattc 2040
ccaaccccaa ttacctacgg tgctgtcatt agacaccatt tagaaatctc cggtccagtc 2100
tcgagacaat tctttttgtc aattgctggg tttgctcctg atgaagaaac aaagaaggct 2160
tttaccagac ttggtggtga caagcaagaa ttcgccgcca aggtcacccg cagaaagttc 2220
aacattgccg atgccttgtt atattcctcc aacaacgctc catggtccga tgttcctttt 2280
gaattcctta ttgaaaacgt tccacacttg actccacgtt actactccat ttcgtcttcg 2340
tcattgagtg aaaagcaact catcaacgtt actgcagttg ttgaagccga agaagaagct 2400
gatggcagac cagtcactgg tgttgtcacc aacttgttga agaacgttga aattgtgcaa 2460
aacaagactg gcgaaaagcc acttgtccac tacgatttga gcggcccaag aggcaagttc 2520
aacaagttca agttgccagt gcatgtgaga agatccaact ttaagttgcc aaagaactcc 2580
accaccccag ttatcttgat tggtccaggt actggtgttg ccccattgag aggttttgtc 2640
agagaaagag ttcaacaagt caagaatggt gtcaatgttg gcaagacttt gttgttttat 2700
ggttgcagaa actccaacga ggactttttg tacaagcaag aatgggccga gtacgcttct 2760
gttttgggtg aaaactttga gatgttcaat gccttctcca gacaagaccc atccaagaag 2820
gtttacgtcc aggataagat tttagaaaac agccaacttg tgcacgagtt gttgactgaa 2880
ggtgccatta tctacgtctg tggtgatgcc agtagaatgg ctagagacgt gcagaccaca 2940
atttccaaga ttgttgctaa aagcagagaa attagtgaag acaaggctgc tgaattggtc 3000
aagtcctgga aggtccaaaa tagataccaa gaagatgttt ggtagactca aacgaatctc 3060
tctttctccc aacgcattta tgaatcttta ttctcattga agctttacat atgttctaca 3120
ctttattttt tttttttttt ttattattat attacgaaac ataggtcaac tatatatact 3180
tgattaaatg ttatagaaac aataactatt atctactcgt ctacttcttt ggcattgaca 3240
tcaacattac cgttcccatt accgttgccg ttggcaatgc cgggatattt agtacagtat 3300
ctccaatccg gatttgagct attgtagatc agctgcaagt cattctccac cttcaaccag 3360
tacttatact tcatctttga cttcaagtcc aagtcataaa tattacaagt tagcaagaac 3420
ttctggccat ccacgatata gacgttattc acgttattat gcgacgtatg gatgtggtta 3480
tccttattga acttctcaaa cttcaaaaac aaccccacgt cccgcaacgt cattatcaac 3540
gacaagttct ggctcacgtc gtcggagctc gtcaagttct caattagatc gttcttgtta 3600
ttgatcttct ggtactttct caattgctgg aacacattgt cctcgttgtt caaatagatc 3660
ttgaacaact ttttcaacgg gatcaacttc tcaatctggg ccaagatctc cgccgggatc 3720
ttcagaaaca agtcctgcaa cccctggtcg atggtctccg ggtacaacaa gtccaagggg 3780
cagaagtgtc taggcacgtg tttcaactgg ttcaacgaac atgttcgaca gtagttcgag 3840
ttatagttat cgtacaacca ttttggtttg atttcgaaaa tgacggagct gatgccatca 3900
ttctcctggt tcctctcata gtacaactgg cacttcttcg agaggctcaa ttcctcgtag 3960
ttcccgtcca agatattcgg caacaagagc ccgtaccgct cacggagcat caagtcgtgg 4020
ccctggttgt tcaacttgtt gatgaagtcc gaggtcaaga caatcaactg gatgtcgatg 4080
atctggtgcg ggaacaagtt cttgcatttt agctcgatga agtcgtacaa ctcacacgtc 4140
gagatatact cctgttcctc cttcaagagc cggatccgca agagcttgtg cttcaagtag 4200
tcgttg                                                            4206
```

<210> 82
<211> 4145
<212> DNA
<213> Candida tropicalis

<400> 82

```
tatatgatat atgatatatc ttcctgtgta attattattc gtattcgtta atacttacta 60
cattttttt tctttattta tgaagaaaag gagagttcgt aagttgagtt gagtagaata 120
ggctgttgtg catacgggga gcagaggaga gtatccgacg aggaggaact gggtgaaatt 180
tcatctatgc tgttgcgtcc tgtactgtac tgtaaatctt agatttccta gaggttgttc 240
tagcaaataa agtgtttcaa gatacaattt tacaggcaag ggtaaaggat caactgatta 300
gcggaagatt ggtgttgcct gtggggttct tttatttttc atatgatttc tttgcgcgag 360
taacatgtgc caatctagtt tatgattagc gtacctccac aattggcatc ttggacgggc 420
gtgttttgtc ttaccccaag ccttatttag ttccacagtc tcgacggtgt ctcgccgatg 480
tcttctccca cccctcgcag gaatcattcg aagttgttgg gggatctcct ccgcagttta 540
tgttcatgtc tttcccactt tggttgtgat tggggtagcg tagtgagttg gtgattttct 600
tttttcgcag gtgtctccga tatcgaagtt tgatgaatat aggagccaga tcagcatggt 660
atattgcctt tgtagataga gatgttgaac aacaactagc tgaattacac accaccgcta 720
aacgatgcgc acagggtgtc accgccaact gacgttgggt ggagttgttg ttggcagggc 780
catattgcta aacgaagaga agtagcacaa aacccaaggt taagaacaat taaaaaaatt 840
catacgacaa ttccacagcc atttacataa tcaacagcga caaatgagac agaaaaaact 900
ttcaacattt caaagttccc tttttcctat tacttctttt tttctttcct tcctttcatt 960
tcctttcctt ctgcttttat tactttacca gtcttttgct tgtttttgca attcctcatc 1020
ctcctcctca ccatggcttt agacaagtta gatttgtatg tcatcataac attggtggtc 1080
gctgtggccg cctattttgc taagaaccag ttccttgatc agccccagga caccgggttc 1140
ctcaacacgg acagcggaag caactccaga gacgtcttgc tgacattgaa gaagaataat 1200
aaaaacacgt tgttgttgtt tgggtcccag accggtacgg cagaagatta cgccaacaaa 1260
ttgtcaagag aattgcactc cagatttggc ttgaaaacca tggttgcaga tttcgctgat 1320
tacgattggg ataacttcgg agatatcacc gaagatatct tggtgttttt catcgttgcc 1380
acctacggtg agggtgaacc taccgacaat gccgacgagt tccacacctg gttgactgaa 1440
gaagctgaca ctttgagtac tttgagatat accgtgttcg ggttgggtaa ctccacctac 1500
gagttcttca atgctattgg tagaaagttt gacagattgt tgagtgagaa aggtggtgac 1560
agatttgctg aatatgctga aggtgacgac ggcactggca ccttggacga agatttcatg 1620
gcctggaagg ataatgtctt tgacgccttg aagaatgact tgaactttga agaaaaggaa 1680
ttgaagtacg aaccaaacgt gaaattgact gagagagatg acttgtctgc tgccgactcc 1740
caagtttcct tgggtgagcc aaacaagaag tacatcaact ccgagggcat cgacttgacc 1800
aagggtccat tcgaccacac ccacccatac ttggccagga tcaccgagac cagagagttg 1860
ttcagctcca aggaaagaca ctgtattcac gttgaatttg acatttctga atcgaacttg 1920
aaatacacca ccggtgacca tctagccatc tggccatcca actccgacga aaacatcaag 1980
caatttgcca agtgtttcgg attggaagat aaactcgaca ctgttattga attgaaggca 2040
ttggactcca cttacaccat tccattccca actccaatta cttacggtgc tgtcattaga 2100
caccatttag aaatctccgg tccagtctcg agacaattct tttgtcgat tgctgggttt 2160
gctcctgatg aagaaacaaa gaagactttc accagacttg gtggtgacaa acaagaattc 2220
gccaccaagg ttacccgcag aaagttcaac attgccgatg ccttgttata ttcctccaac 2280
aacactccat ggtccgatgt tccttttgag ttccttattg aaaacatcca acacttgact 2340
ccacgttact actccatttc ttcttcgtcg ttgagtgaaa aacaactcat caatgttact 2400
gcagtcgttg aggccgaaga agaagccgat ggcagaccag tcactggtgt tgttaccaac 2460
ttgttgaaga acattgaaat tgcgcaaaac aagactggcg aaaagccact tgttcactac 2520
gatttgagcg gcccaagagg caagttcaac aagttcaagt tgccagtgca cgtgagaaga 2580
tccaacttta agttgccaaa gaactccacc accccagtta tcttgattgg tccaggtact 2640
ggtgttgccc cattgagagg tttcgttaga gaaagagttc aacaagtcaa gaatggtgtc 2700
aatgttggca agactttgtt gttttatggt tgcagaaact ccaacgagga ctttttgtac 2760
```

```
aagcaagaat gggccgagta cgcttctgtt ttgggtgaaa actttgagat gttcaatgcc 2820
ttctctagac aagacccatc caagaaggtt tacgtccagg ataagatttt agaaaacagc 2880
caacttgtgc acgaattgtt gaccgaaggt gccattatct acgtctgtgg tgacgccagt 2940
agaatggcca gagacgtcca gaccacgatc tccaagattg ttgccaaaag cagagaaatc 3000
agtgaagaca aggccgctga attggtcaag tcctggaaag tccaaaatag ataccaagaa 3060
gatgtttggt agactcaaac gaatctctct ttctcccaac gcatttatga atattctcat 3120
tgaagtttta catatgttct atatttcatt tttttttat tatattacga aacataggtc 3180
aactatatat acttgattaa atgttataga aacaataatt attatctact cgtctacttc 3240
tttggcattg gcattggcat tggcattggc attgccgttg ccgttggtaa tgccgggata 3300
tttagtacag tatctccaat ccggatttga gctattgtaa atcagctgca agtcattctc 3360
caccttcaac cagtacttat acttcatctt tgacttcaag tccaagtcat aaatattaca 3420
agttagcaag aacttctggc catccacaat atagacgtta ttcacgttat tatgcgacgt 3480
atggatatgg ttatccttat tgaacttctc aaacttcaaa aacaacccca cgtcccgcaa 3540
cgtcattatc aacgacaagt tctgactcac gtcgtcggag ctcgtcaagt tctcaattag 3600
atcgttcttg ttattgatct tctggtactt tctcaactgc tggaacacat tgtcctcgtt 3660
gttcaaatag atcttgaaca acttcttcaa gggaatcaac ttttcgatct gggccaagat 3720
ttccgccggg atcttcagaa acaagtcctg caacccctgg tcgatggtct cggggtacaa 3780
caagtctaag gggcagaagt gtctaggcac gtgtttcaac tggttcaagg aacatgttcg 3840
acagtagttc gagttatagt tatcgtacaa ccactttggc ttgatttcga aaatgacgga 3900
gctgatccca tcattctcct ggttcctttc atagtacaac tggcatttct tcgagagact 3960
caactcctcg tagttcccgt ccaagatatt cggcaacaag agcccgtagc gctcacggag 4020
catcaagtcg tggccctggt tgttcaactt gttgatgaag tccgatgtca agacaatcaa 4080
ctggatgtcg atgatctggt gcggaaacaa gttcttgcac tttagctcga tgaagtcgta 4140
caact                                                             4145
```

<210> 83
<211> 679
<212> PRT
<213> Candida tropicalis

<400> 83

```
Met Ala Leu Asp Lys Leu Asp Leu Tyr Val Ile Ile Thr Leu Val Val
1               5                   10                  15

Ala Val Ala Ala Tyr Phe Ala Lys Asn Gln Phe Leu Asp Gln Pro Gln
            20                  25                  30

Asp Thr Gly Phe Leu Asn Thr Asp Ser Gly Ser Asn Ser Arg Asp Val
        35                  40                  45

Leu Leu Thr Leu Lys Lys Asn Asn Lys Asn Thr Leu Leu Leu Phe Gly
    50                  55                  60

Ser Gln Thr Gly Thr Ala Glu Asp Tyr Ala Asn Lys Leu Ser Arg Glu
65                  70                  75                  80

Leu His Ser Arg Phe Gly Leu Lys Thr Met Val Ala Asp Phe Ala Asp
```

```
                85                      90                      95

Tyr Asp Trp Asp Asn Phe Gly Asp Ile Thr Glu Asp Ile Leu Val Phe
            100                     105                     110

Phe Ile Val Ala Thr Tyr Gly Glu Gly Glu Pro Thr Asp Asn Ala Asp
        115                     120                     125

Glu Phe His Thr Trp Leu Thr Glu Glu Ala Asp Thr Leu Ser Thr Leu
    130                     135                     140

Lys Tyr Thr Val Phe Gly Leu Gly Asn Ser Thr Tyr Glu Phe Phe Asn
145                     150                     155                 160

Ala Ile Gly Arg Lys Phe Asp Arg Leu Leu Ser Glu Lys Gly Gly Asp
                165                     170                     175

Arg Phe Ala Glu Tyr Ala Glu Gly Asp Asp Gly Thr Gly Thr Leu Asp
            180                     185                     190

Glu Asp Phe Met Ala Trp Lys Asp Asn Val Phe Asp Ala Leu Lys Asn
        195                     200                     205

Asp Leu Asn Phe Glu Glu Lys Glu Leu Lys Tyr Glu Pro Asn Val Lys
    210                     215                     220

Leu Thr Glu Arg Asp Asp Leu Ser Ala Ala Asp Ser Gln Val Ser Leu
225                     230                     235                 240

Gly Glu Pro Asn Lys Lys Tyr Ile Asn Ser Glu Gly Ile Asp Leu Thr
                245                     250                     255

Lys Gly Pro Phe Asp His Thr His Pro Tyr Leu Ala Arg Ile Thr Glu
            260                     265                     270

Thr Arg Glu Leu Phe Ser Ser Lys Asp Arg His Cys Ile His Val Glu
        275                     280                     285

Phe Asp Ile Ser Glu Ser Asn Leu Lys Tyr Thr Thr Gly Asp His Leu
    290                     295                     300

Ala Ile Trp Pro Ser Asn Ser Asp Glu Asn Ile Lys Gln Phe Ala Lys
305                     310                     315                 320

Cys Phe Gly Leu Glu Asp Lys Leu Asp Thr Val Ile Glu Leu Lys Ala
                325                     330                     335

Leu Asp Ser Thr Tyr Thr Ile Pro Phe Pro Thr Pro Ile Thr Tyr Gly
```

340 345 350

Ala Val Ile Arg His His Leu Glu Ile Ser Gly Pro Val Ser Arg Gln
355 360 365

Phe Phe Leu Ser Ile Ala Gly Phe Ala Pro Asp Glu Glu Thr Lys Lys
370 375 380

Ala Phe Thr Arg Leu Gly Gly Asp Lys Gln Glu Phe Ala Ala Lys Val
385 390 395 400

Thr Arg Arg Lys Phe Asn Ile Ala Asp Ala Leu Leu Tyr Ser Ser Asn
405 410 415

Asn Ala Pro Trp Ser Asp Val Pro Phe Glu Phe Leu Ile Glu Asn Val
420 425 430

Pro His Leu Thr Pro Arg Tyr Tyr Ser Ile Ser Ser Ser Ser Leu Ser
435 440 445

Glu Lys Gln Leu Ile Asn Val Thr Ala Val Val Glu Ala Glu Glu Glu
450 455 460

Ala Asp Gly Arg Pro Val Thr Gly Val Val Thr Asn Leu Leu Lys Asn
465 470 475 480

Val Glu Ile Val Gln Asn Lys Thr Gly Glu Lys Pro Leu Val His Tyr
485 490 495

Asp Leu Ser Gly Pro Arg Gly Lys Phe Asn Lys Phe Lys Leu Pro Val
500 505 510

His Val Arg Arg Ser Asn Phe Lys Leu Pro Lys Asn Ser Thr Thr Pro
515 520 525

Val Ile Leu Ile Gly Pro Gly Thr Gly Val Ala Pro Leu Arg Gly Phe
530 535 540

Val Arg Glu Arg Val Gln Gln Val Lys Asn Gly Val Asn Val Gly Lys
545 550 555 560

Thr Leu Leu Phe Tyr Gly Cys Arg Asn Ser Asn Glu Asp Phe Leu Tyr
565 570 575

Lys Gln Glu Trp Ala Glu Tyr Ala Ser Val Leu Gly Glu Asn Phe Glu
580 585 590

Met Phe Asn Ala Phe Ser Arg Gln Asp Pro Ser Lys Lys Val Tyr Val

595 600 605

Gln Asp Lys Ile Leu Glu Asn Ser Gln Leu Val His Glu Leu Leu Thr
610 615 620

Glu Gly Ala Ile Ile Tyr Val Cys Gly Asp Ala Ser Arg Met Ala Arg
625 630 635 640

Asp Val Gln Thr Thr Ile Ser Lys Ile Val Ala Lys Ser Arg Glu Ile
645 650 655

Ser Glu Asp Lys Ala Ala Glu Leu Val Lys Ser Trp Lys Val Gln Asn
660 665 670

Arg Tyr Gln Glu Asp Val Trp
675

<210> 84
<211> 679
<212> PRT
<213> Candida tropicalis

<400> 84
Met Ala Leu Asp Lys Leu Asp Leu Tyr Val Ile Ile Thr Leu Val Val
1 5 10 15

Ala Val Ala Ala Tyr Phe Ala Lys Asn Gln Phe Leu Asp Gln Pro Gln
20 25 30

Asp Thr Gly Phe Leu Asn Thr Asp Ser Gly Ser Asn Ser Arg Asp Val
35 40 45

Leu Leu Thr Leu Lys Lys Asn Asn Lys Asn Thr Leu Leu Leu Phe Gly
50 55 60

Ser Gln Thr Gly Thr Ala Glu Asp Tyr Ala Asn Lys Leu Ser Arg Glu
65 70 75 80

Leu His Ser Arg Phe Gly Leu Lys Thr Met Val Ala Asp Phe Ala Asp
85 90 95

Tyr Asp Trp Asp Asn Phe Gly Asp Ile Thr Glu Asp Ile Leu Val Phe
100 105 110

Phe Ile Val Ala Thr Tyr Gly Glu Gly Glu Pro Thr Asp Asn Ala Asp
115 120 125

```
Glu Phe His Thr Trp Leu Thr Glu Glu Ala Asp Thr Leu Ser Thr Leu
    130                 135                 140

Arg Tyr Thr Val Phe Gly Leu Gly Asn Ser Thr Tyr Glu Phe Phe Asn
145                 150                 155                 160

Ala Ile Gly Arg Lys Phe Asp Arg Leu Leu Ser Glu Lys Gly Gly Asp
                165                 170                 175

Arg Phe Ala Glu Tyr Ala Glu Gly Asp Asp Gly Thr Gly Thr Leu Asp
            180                 185                 190

Glu Asp Phe Met Ala Trp Lys Asp Asn Val Phe Asp Ala Leu Lys Asn
        195                 200                 205

Asp Leu Asn Phe Glu Glu Lys Glu Leu Lys Tyr Glu Pro Asn Val Lys
    210                 215                 220

Leu Thr Glu Arg Asp Asp Leu Ser Ala Ala Asp Ser Gln Val Ser Leu
225                 230                 235                 240

Gly Glu Pro Asn Lys Lys Tyr Ile Asn Ser Glu Gly Ile Asp Leu Thr
                245                 250                 255

Lys Gly Pro Phe Asp His Thr His Pro Tyr Leu Ala Arg Ile Thr Glu
            260                 265                 270

Thr Arg Glu Leu Phe Ser Ser Lys Glu Arg His Cys Ile His Val Glu
        275                 280                 285

Phe Asp Ile Ser Glu Ser Asn Leu Lys Tyr Thr Thr Gly Asp His Leu
    290                 295                 300

Ala Ile Trp Pro Ser Asn Ser Asp Glu Asn Ile Lys Gln Phe Ala Lys
305                 310                 315                 320

Cys Phe Gly Leu Glu Asp Lys Leu Asp Thr Val Ile Glu Leu Lys Ala
                325                 330                 335

Leu Asp Ser Thr Tyr Thr Ile Pro Phe Pro Thr Pro Ile Thr Tyr Gly
            340                 345                 350

Ala Val Ile Arg His His Leu Glu Ile Ser Gly Pro Val Ser Arg Gln
        355                 360                 365

Phe Phe Leu Ser Ile Ala Gly Phe Ala Pro Asp Glu Glu Thr Lys Lys
    370                 375                 380
```

```
Thr Phe Thr Arg Leu Gly Gly Asp Lys Gln Glu Phe Ala Thr Lys Val
385                 390                 395                 400

Thr Arg Arg Lys Phe Asn Ile Ala Asp Ala Leu Leu Tyr Ser Ser Asn
                405                 410                 415

Asn Thr Pro Trp Ser Asp Val Pro Phe Glu Phe Leu Ile Glu Asn Ile
            420                 425                 430

Gln His Leu Thr Pro Arg Tyr Tyr Ser Ile Ser Ser Ser Ser Leu Ser
        435                 440                 445

Glu Lys Gln Leu Ile Asn Val Thr Ala Val Val Glu Ala Glu Glu Glu
    450                 455                 460

Ala Asp Gly Arg Pro Val Thr Gly Val Val Thr Asn Leu Leu Lys Asn
465                 470                 475                 480

Ile Glu Ile Ala Gln Asn Lys Thr Gly Glu Lys Pro Leu Val His Tyr
                485                 490                 495

Asp Leu Ser Gly Pro Arg Gly Lys Phe Asn Lys Phe Lys Leu Pro Val
            500                 505                 510

His Val Arg Arg Ser Asn Phe Lys Leu Pro Lys Asn Ser Thr Thr Pro
        515                 520                 525

Val Ile Leu Ile Gly Pro Gly Thr Gly Val Ala Pro Leu Arg Gly Phe
    530                 535                 540

Val Arg Glu Arg Val Gln Gln Val Lys Asn Gly Val Asn Val Gly Lys
545                 550                 555                 560

Thr Leu Leu Phe Tyr Gly Cys Arg Asn Ser Asn Glu Asp Phe Leu Tyr
                565                 570                 575

Lys Gln Glu Trp Ala Glu Tyr Ala Ser Val Leu Gly Glu Asn Phe Glu
            580                 585                 590

Met Phe Asn Ala Phe Ser Arg Gln Asp Pro Ser Lys Lys Val Tyr Val
        595                 600                 605

Gln Asp Lys Ile Leu Glu Asn Ser Gln Leu Val His Glu Leu Leu Thr
    610                 615                 620

Glu Gly Ala Ile Ile Tyr Val Cys Gly Asp Ala Ser Arg Met Ala Arg
625                 630                 635                 640
```

Asp Val Gln Thr Thr Ile Ser Lys Ile Val Ala Lys Ser Arg Glu Ile
645 650 655

Ser Glu Asp Lys Ala Ala Glu Leu Val Lys Ser Trp Lys Val Gln Asn
660 665 670

Arg Tyr Gln Glu Asp Val Trp
675

<210> 85
<211> 4115
<212> DNA
<213> Candida tropicalis

<400> 85
catatgcgct aatcttcttt ttctttttat cacaggagaa actatcccac ccccacttcg 60
aaacacaatg acaactcctg cgtaacttgc aaattcttgt ctgactaatt gaaaactccg 120
gacgagtcag acctccagtc aaacggacag acagacaaac acttggtgcg atgttcatac 180
ctacagacat gtcaacgggt gttagacgac ggtttcttgc aaagacaggt gttggcatct 240
cgtacgatgg caactgcagg aggtgtcgac ttctccttta ggcaatagaa aaagactaag 300
agaacagcgt ttttacaggt tgcattggtt aatgtagtat ttttttagtc ccagcattct 360
gtgggttgct ctgggtttct agaataggaa atcacaggag aatgcaaatt cagatggaag 420
aacaaagaga taaaaaacaa aaaaaaactg agttttgcac caatagaatg tttgatgata 480
tcatccactc gctaaacgaa tcatgtgggt gatcttctct ttagttttgg tctatcataa 540
aacacatgaa agtgaaatcc aaatacacta cactccgggt attgtccttc gttttacaga 600
tgtctcattg tcttactttt gaggtcatag gagttgcctg tgagagatca cagagattat 660
cacactcaca tttatcgtag tttcctatct catgctgtgt gtctctggtt ggttcatgag 720
tttggattgt tgtacattaa aggaatcgct ggaaagcaaa gctaactaaa ttttctttgt 780
cacaggtaca ctaacctgta aaacttcact gccacgccag tctttcctga ttgggcaagt 840
gcacaaacta caacctgcaa aacagcactc cgcttgtcac aggttgtctc ctctcaacca 900
acaaaaaaat aagattaaac tttctttgct catgcatcaa tcggagttat ctctgaaaga 960
gttgcctttg tgtaatgtgt gccaaactca aactgcaaaa ctaaccacag aatgatttcc 1020
ctcacaatta tataaactca cccacatttc cacagaccgt aatttcatgt ctcactttct 1080
cttttgctct tcttttactt agtcaggttt gataacttcc tttttatta ccctatctta 1140
tttatttatt tattcattta taccaaccaa ccaaccatgg ccacacaaga aatcatcgat 1200
tctgtacttc cgtacttgac caaatggtac actgtgatta ctgcagcagt attagtcttc 1260
cttatctcca caaacatcaa gaactacgtc aaggcaaaga aattgaaatg tgtcgatcca 1320
ccatacttga aggatgccgg tctcactggt attctgtctt tgatcgccgc catcaaggcc 1380
aagaacgacg gtagattggc taactttgcc gatgaagttt tcgacgagta cccaaaccac 1440
accttctact tgtctgttgc cggtgctttg aagattgtca tgactgttga cccagaaaac 1500
atcaaggctg tcttggccac ccaattcact gacttctcct tgggtaccag acacgcccac 1560
tttgctcctt tgttgggtga cggtatcttc accttggacg gagaaggttg gaagcactcc 1620
agagctatgt tgagaccaca gtttgctaga gaccagattg gacacgttaa agccttggaa 1680
ccacacatcc aaatcatggc taagcagatc aagttgaacc agggaaagac tttcgatatc 1740
caagaattgt tctttagatt taccgtcgac accgctactg agttcttgtt tggtgaatcc 1800
gttcactcct tgtacgatga aaaattgggc atcccaactc caaacgaaat cccaggaaga 1860
gaaaactttg ccgctgcttt caacgtttcc caacactact tggccaccag aagttactcc 1920

cagactttttt actttttgac caaccctaag gaattcagag actgtaacgc caaggtccac 1980
cacttggcca agtactttgt caacaaggcc ttgaacttta ctcctgaaga actcgaagag 2040
aaatccaagt ccggttacgt tttcttgtac gaattggtta agcaaaccag agatccaaag 2100
gtcttgcaag atcaattgtt gaacattatg gttgccggaa gagacaccac tgccggtttg 2160
ttgtcctttg ctttgtttga attggctaga cacccagaga tgtggtccaa gttgagagaa 2220
gaaatcgaag ttaactttgg tgttggtgaa gactcccgcg ttgaagaaat taccttcgaa 2280
gccttgaaga gatgtgaata cttgaaggct atccttaacg aaaccttgcg tatgtaccca 2340
tctgttcctg tcaactttag aaccgccacc agagacacca ctttgccaag aggtggtggt 2400
gctaacggta ccgacccaat ctacattcct aaaggctcca ctgttgctta cgttgtctac 2460
aagacccacc gtttggaaga atactacggt aaggacgcta acgacttcag accagaaaga 2520
tggtttgaac catctactaa gaagttgggc tgggcttatg ttccattcaa cggtggtcca 2580
agagtctgct tgggtcaaca attcgccttg actgaagctt cttatgtgat cactagattg 2640
gcccagatgt ttgaaactgt ctcatctgat ccaggtctcg aataccctcc accaaagtgt 2700
attcacttga ccatgagtca caacgatggt gtctttgtca agatgtaaag tagtcgatgc 2760
tgggtattcg attacatgtg tataggaaga ttttggtttt ttattcgttc tttttttttaa 2820
tttttgttaa attagtttag agatttcatt aatacataga tgggtgctat ttccgaaact 2880
ttacttctat cccctgtatc ccttattatc cctctcagtc acatgattgc tgtaattgtc 2940
gtgcaggaca caaactccct aacggactta aaccataaac aagctcagaa ccataagccg 3000
acatcactcc ttcttctctc ttctccaacc aatagcatgg acagacccac cctcctatcc 3060
gaatcgaaga cccttattga ctccataccc acctggaagc ccctcaagcc acacacgtca 3120
tccagcccac ccatcaccac atccctctac tcgacaacgt ccaaagacgg cgagttctgg 3180
tgtgcccgga aatcagccat cccggccaca tacaagcagc cgttgattgc gtgcatactc 3240
ggcgagccca caatgggagc cacgcattcg gaccatgaag caaagtacat tcacgagatc 3300
acgggtgttt cagtgtcgca gattgagaag ttcgacgatg gatggaagta cgatctcgtt 3360
gcggattacg acttcggtgg gttgttatct aaacgaagat tctatgagac gcagcatgtg 3420
tttcggttcg aggattgtgc gtacgtcatg agtgtgcctt ttgatggacc caaggaggaa 3480
ggttacgtgg ttgggacgta cagatccatt gaaaggttga gctggggtaa agacggggac 3540
gtggagtgga ccatggcgac gacgtcggat cctggtgggt ttatcccgca atggataact 3600
cgattgagca tccctggagc aatcgcaaaa gatgtgccta gtgtattaaa ctacatacag 3660
aaataaaaac gtgtcttgat tcattggttt ggttcttgtt gggttccgag ccaatatttc 3720
acatcatctc ctaaattctc caagaatccc aacgtagcgt agtccagcac gccctctgag 3780
atcttattta atatcgactt ctcaaccacc ggtggaatcc cgttcagacc attgttacct 3840
gtagtgtgtt tgctcttgtt cttgatgaca atgatgtatt tgtcacgata cctgaaataa 3900
taaaacatcc agtcattgag cttattactc gtgaacttat gaaagaactc attcaagccg 3960
ttcccaaaaa acccagaatt gaagatcttg ctcaactggt catgcaagta gtagatcgcc 4020
atgatctgat actttaccaa gctatcctct ccaagttctc ccacgtacgg caagtacggc 4080
aacgagctct ggaagctttg ttgtttgggg tcata 4115

<210> 86
<211> 3948
<212> DNA
<213> Candida tropicalis

<400> 86
gacctgtgac gcttccggtg tcttgccacc agtctccaag ttgaccgacg cccaagtcat 60
gtaccacttt atttccggtt acacttccaa gatggctggt actgaagaag gtgtcacgga 120
accacaagct actttctccg cttgtttcgg tcaaccattc ttggtgttgc acccaatgaa 180

gtacgctcaa caattgtctg acaagatctc gcaacacaag gctaacgcct ggttgttgaa 240
caccggttgg gttggttctt ctgctgctag aggtggtaag agatgctcat tgaagtacac 300
cagagccatt ttggacgcta tccactctgg tgaattgtcc aaggttgaat acgaaacttt 360
cccagtcttc aacttgaatg tcccaacctc ctgtccaggt gtcccaagtg aaatcttgaa 420
cccaaccaag gcctggaccg gaaggtgttg actccttcaa caaggaaatc aagtctttgg 480
ctggtaagtt tgctgaaaac ttcaagacct atgctgacca agctaccgct gaagtgagag 540
ctgcaggtcc agaagcttaa agatatttat tcattattta gtttgcctat ttatttctca 600
ttacccatca tcattcaaca ctatatataa agttacttcg gatatcattg taatcgtgcg 660
tgtcgcaatt ggatgatttg gaactgcgct tgaaacggat tcatgcacga agcggagata 720
aaagattacg taatttatct cctgagacaa ttttagccgt gttcacacgc ccttctttgt 780
tctgagcgaa ggataaataa ttagacttcc acagctcatt ctaatttccg tcacgcgaat 840
attgaagggg ggtacatgtg gccgctgaat gtgggggcag taaacgcagt ctctcctctc 900
ccaggaatag tgcaacggag gaaggataac ggatagaaag cggaatgcga ggaaaatttt 960
gaacgcgcaa gaaaagcaat atccgggcta ccaggttttg agccagggaa cacactccta 1020
tttctgctca atgactgaac atagaaaaaa caccaagacg caatgaaacg cacatggaca 1080
tttagacctc cccacatgtg atagtttgtc ttaacagaaa agtataataa gaacccatgc 1140
cgtccctttt ctttcgccgc ttcaactttt ttttttttat cttacacaca tcacgaccat 1200
gactgtacac gatattatcg ccacatactt caccaaatgg tacgtgatag taccactcgc 1260
tttgattgct tatagagtcc tcgactactt ctatggcaga tacttgatgt acaagcttgg 1320
tgctaaacca tttttccaga aacagacaga cggctgtttc ggattcaaag ctccgcttga 1380
attgttgaag aagaagagcg acggtaccct catagacttc acactccagc gtatccacga 1440
tctcgatcgt cccgatatcc caactttcac attcccggtc ttttccatca accttgtcaa 1500
tacccttgag ccggagaaca tcaaggccat cttggccact cagttcaacg atttctcctt 1560
gggtaccaga cactcgcact ttgctccttt gttgggtgat ggtatcttta cgttggatgg 1620
cgccggctgg aagcacagca gatctatgtt gagaccacag tttgccagag aacagatttc 1680
ccacgtcaag ttgttggagc cacacgttca ggtgttcttc aaacacgtca gaaaggcaca 1740
gggcaagact tttgacatcc aggaattgtt tttcagattg accgtcgact ccgccaccga 1800
gtttttgttt ggtgaatccg ttgagtcctt gagagatgaa tctatcggca tgtccatcaa 1860
tgcgcttgac tttgacggca aggctggctt tgctgatgct tttaactatt cgcagaatta 1920
tttggcttcg agagcggtta tgcaacaatt gtactgggtg ttgaacggga aaaagtttaa 1980
ggagtgcaac gctaaagtgc acaagtttgc tgactactac gtcaacaagg ctttggactt 2040
gacgcctgaa caattggaaa agcaggatgg ttatgtgttt ttgtacgaat tggtcaagca 2100
aaccagagac aagcaagtgt tgagagacca attgttgaac atcatggttg ctggtagaga 2160
caccaccgcc ggtttgttgt cgtttgtttt ctttgaattg gccagaaacc cagaagttac 2220
caacaagttg agagaagaaa ttgaggacaa gtttggactc ggtgagaatg ctagtgttga 2280
agacatttcc tttgagtcgt tgaagtcctg tgaatacttg aaggctgttc tcaacgaaac 2340
cttgagattg tacccatccg tgccacagaa tttcagagtt gccaccaaga acactaccct 2400
cccaagaggt ggtggtaagg acgggttgtc tcctgttttg gtgagaaagg gtcagaccgt 2460
tatttacggt gtctacgcag cccacagaaa cccagctgtt tacggtaagg acgctcttga 2520
gtttagacca gagagatggt ttgagccaga gacaaagaag cttggctggg ccttcctccc 2580
attcaacggt ggtccaagaa tctgtttggg acagcagttt gccttgacag aagcttcgta 2640
tgtcactgtc aggttgctcc aggagtttgc acacttgtct atggacccag acaccgaata 2700
tccacctaag aaaatgtcgc atttgaccat gtcgcttttc gacggtgcca atattgagat 2760
gtattagagg gtcatgtgtt attttgattg tttagtttgt aattactgat taggttaatt 2820
catggattgt tatttattga taggggtttg cgcgtgttgc attcacttgg gatcgttcca 2880
ggttgatgtt tccttccatc ctgtcgagtc aaaaggagtt ttgttttgta actccggacg 2940
atgttttaaa tagaaggtcg atctccatgt gattgttttg actgttactg tgattatgta 3000
atctgcggac gttatacaag catgtgattg tggttttgca gccttttgca cgacaaatga 3060

tcgtcagacg attacgtaat ctttgttaga ggggtaaaaa aaaacaaaat ggcagccaga 3120
atttcaaaca ttctgcaaac aatgcaaaaa atgggaaact ccaacagaca aaaaaaaaaa 3180
ctccgcagca ctccgaaccc acagaacaat ggggcgccag aattattgac tattgtgact 3240
tttttacgct aacgctcatt gcagtgtagt gcgtcttaca cggggtattg ctttctacaa 3300
tgcaagggca cagttgaagg tttgcaccta acgttgcccc gtgtcaactc aatttgacga 3360
gtaacttcct aagctcgaat tatgcagctc gtgcgtcaac ctatgtgcag gaaagaaaaa 3420
atccaaaaaa atcgaaaatg cgactttcga ttttgaataa accaaaaaga aaaatgtcgc 3480
actttttttct cgctctcgct ctctcgaccc aaatcacaac aaatcctcgc gcgcagtatt 3540
tcgacgaaac cacaacaaat aaaaaaaaca aattctacac cacttctttt tcttcaccag 3600
tcaacaaaaa acaacaaatt atacaccatt tcaacgattt ttgctcttat aaatgctata 3660
taatggttta attcaactca ggtatgttta ttttactgtt ttcagctcaa gtatgttcaa 3720
atactaacta cttttgatgt ttgtcgcttt tctagaatca aaacaacgcc cacaacacgc 3780
cgagcttgtc gaatagacgg tttgtttact cattagatgg tcccagatta cttttcaagc 3840
caaagtctct cgagttttgt ttgctgtttc cccaattcct aactatgaag ggtttttata 3900
aggtccaaag accccaaggc atagtttttt tggttccttc ttgtcgtg 3948

<210> 87
<211> 3755
<212> DNA
<213> Candida tropicalis

<400> 87
gctcaacaat tgtctgacaa gatctcgcaa cacaaggcta acgcctggtt gttgaacact 60
ggttgggttg gttcttctgc tgctagaggt ggtaagagat gttcattgaa gtacaccaga 120
gccattttgg acgctatcca ctctggtgaa ttgtccaagg ttgaatacga gactttccca 180
gtcttcaact tgaatgtccc aacctcctgc ccaggtgtcc caagtgaaat cttgaaccca 240
accaaggcct ggaccgaagg tgttgactcc ttcaacaagg aaatcaagtc tttggctggt 300
aagtttgctg aaaacttcaa gacctatgct gaccaagcta ccgctgaagt tagagctgca 360
ggtccagaag cttaaagata tttattcact atttagtttg cctatttatt tctcatcacc 420
catcatcatt caacaatata tataaagtta tttcggaact catatatcat tgtaatcgtg 480
cgtgttgcaa ttgggtaatt tgaaactgta gttggaacgg attcatgcac gatgcggaga 540
taacacgaga ttatctccta agacaatttt ggcctcattc acacgccctt cttctgagct 600
aaggataaat aattagactt cacaagttca ttaaaatatc cgtcacgcga aaactgcaac 660
aataaggaag ggggggtag acgtagccga tgaatgtggg gtgccagtaa acgcagtctc 720
tctctccccc cccccccccc cccccctcagg aatagtacaa cggggggaagg ataacggata 780
gcaagtggaa tgcgaggaaa attttgaatg cgcaaggaaa gcaatatccg ggctatcagg 840
ttttgagcca ggggacacac tcctcttctg cacaaaaact taacgtagac aaaaaaaaaa 900
aactccacca agacacaatg aatcgcacat ggacatttag acctccccac atgtgaaagc 960
ttctctggcg aaagcaaaaa aagtataata aggacccatg ccttccctct tcctgggccg 1020
tttcaacttt ttcttttct ttgtctatca acacacacac acctcacgac catgactgca 1080
caggatatta tcgccacata catcaccaaa tggtacgtga tagtaccact cgctttgatt 1140
gcttataggg tcctcgacta cttttacggc agatacttga tgtacaagct tggtgctaaa 1200
ccgtttttcc agaaacaaac agacggttat ttcggattca aagctccact tgaattgtta 1260
aaaaagaaga gtgacggtac cctcatagac ttcactctcg agcgtatcca agcgctcaat 1320
cgtccagata tcccaacttt tacattccca atcttttcca tcaaccttat cagcaccctt 1380
gagccggaga acatcaaggc tatcttggcc acccagttca acgatttctc cttgggcacc 1440
agacactcgc actttgctcc tttgttgggc gatggtatct ttaccttgga cggtgccggc 1500

tggaagcaca gcagatctat gttgagacca cagtttgcca gagaacagat ttcccacgtc 1560
aagttgttgg agccacacat gcaggtgttc ttcaagcacg tcagaaaggc acagggcaag 1620
acttttgaca tccaagaatt gtttttcaga ttgaccgtcg actccgccac tgagtttttg 1680
tttggtgaat ccgttgagtc cttgagagat gaatctattg ggatgtccat caatgcactt 1740
gactttgacg gcaaggctgg ctttgctgat gcttttaact actcgcagaa ctatttggct 1800
tcgagagcgg ttatgcaaca attgtactgg gtgttgaacg ggaaaaagtt taaggagtgc 1860
aacgctaaag tgcacaagtt tgctgactat tacgtcagca aggctttgga cttgacacct 1920
gaacaattgg aaaagcagga tggttatgtg ttcttgtacg agttggtcaa gcaaaccaga 1980
gacaggcaag tgttgagaga ccagttgttg aacatcatgg ttgccggtag agacaccacc 2040
gccggtttgt tgtcgtttgt tttctttgaa ttggccagaa acccagaggt gaccaacaag 2100
ttgagagaag aaatcgagga caagtttggt cttggtgaga atgctcgtgt tgaagacatt 2160
tcctttgagt cgttgaagtc atgtgaatac ttgaaggctg ttctcaacga aactttgaga 2220
ttgtacccat ccgtgccaca gaatttcaga gttgccacca aaaacactac ccttccaagg 2280
ggaggtggta aggacgggtt atctcctgtt ttggtcagaa agggtcaaac cgttatgtac 2340
ggtgtctacg ctgcccacag aaacccagct gtctacggta aggacgccct tgagtttaga 2400
ccagagaggt ggtttgagcc agagacaaag aagcttggct gggccttcct tccattcaac 2460
ggtggtccaa gaatttgctt gggacagcag tttgccttga cagaagcttc gtatgtcact 2520
gtcagattgc tccaagagtt tggacacttg tctatggacc ccaacaccga atatccacct 2580
aggaaaatgt cgcatttgac catgtccctt ttcgacggtg ccaacattga gatgtattag 2640
aggatcatgt gttatttttg attggtttag tctgtttgta gctattgatt aggttaattc 2700
acggattgtt atttattgat aggggtgcg tgtgtgtgtg tgtgttgcat tcacatggga 2760
tcgttccagg ttgttgtttc cttccatcct gttgagtcaa aaggagtttt gttttgtaac 2820
tccggacgat gtcttagata gaaggtcgat ctccatgtga ttgtttgact gctactctga 2880
ttatgtaatc tgtaaagcct agacgttatg caagcatgtg attgtggttt ttgcaacctg 2940
tttgcacgac aaatgatcga cagtcgatta cgtaatccat attatttaga ggggtaataa 3000
aaaataaatg gcagccagaa tttcaaacat tttgcaaaca atgcaaaaga tgagaaactc 3060
caacagaaaa aataaaaaaa ctccgcagca ctccgaacca acaaaacaat gggggcgcc 3120
agaattattg actattgtga cttttttta tttttccgt taactttcat tgcagtgaag 3180
tgtgttacac ggggtggtga tggtgttggt ttctacaatg caagggcaca gttgaaggtt 3240
tccacataac gttgcaccat atcaactcaa tttatcctca ttcatgtgat aaaagaagag 3300
ccaaaaggta attggcagac cccccaaggg gaacacggag tagaaagcaa tggaaacacg 3360
cccatgacag tgccatttag cccacaacac atctagtatt cttttttttt tttgtgcgca 3420
ggtgcacacc tggactttag ttattgcccc ataaagttaa caatctcacc tttggctctc 3480
ccagtgtctc cgcctccaga tgctcgtttt acaccctcga gctaacgaca acacaacacc 3540
catgagggga atgggcaaag ttaaacactt ttggtttcaa tgattcctat ttgctactct 3600
cttgttttgt gttttgattt gcaccatgtg aaataaacga caattatata tacctttcg 3660
tctgtcctcc aatgtctctt tttgctgcca ttttgctttt tgctttttgc ttttgcactc 3720
tctcccactc ccacaatcag tgcagcaaca cacaa 3755

<210> 88
<211> 3900
<212> DNA
<213> Candida tropicalis

<400> 88
gacatcataa tgacccggtt atttcgccct caggttgctt atttgagccg taaagtgcag 60
tagaaacttt gccttgggtt caaactctag tataatggtg ataactggtt gcactcttgc 120

cataggcatg aaaataggcc gttatagtac tatatttaat aagcgtagga gtataggatg 180
catatgaccg gtttttctat atttttaaga taatctctag taaattttgt attctcagta 240
ggatttcatc aaatttcgca accaattctg gcgaaaaaat gattctttta cgtcaaaagc 300
tgaatagtgc agtttaaagc acctaaaatc acatatacag cctctagata cgacagagaa 360
gctctttatg atctgaagaa gcattagaat agctactatg agccactatt ggtgtatata 420
ttagggattg gtgcaattaa gtacgtacta ataaacagaa gaaaatactt aaccaatttc 480
tggtgtatac ttagtggtga gggacctttt ctgaacattc gggtcaaact tttttttgga 540
gtgcgacatc gatttttcgt ttgtgtaata atagtgaacc tttgtgtaat aaatcttcat 600
gcaagacttg cataattcga gcttgggagt tcacgccaat ttgacctcgt tcatgtgata 660
aaagaaaagc caaaaggtaa ttagcagacg caatgggaac atggagtgga aagcaatgga 720
agcacgccca ggacggagta atttagtcca cactacatct gggggttttt tttttgtgcg 780
caagtacaca cctggacttt agtttttgcc ccataaagtt aacaatctaa cctttggctc 840
tccaactctc tccgccccca aatattcgtt tttacaccct caagctagcg acagcacaac 900
acccattaga ggaatggggc aaagttaaac acttttggct tcaatgattc ctattcgcta 960
ctacattctt ctcttgtttt gtgctttgaa ttgcaccatg tgaaataaac gacaattata 1020
tatacctttt catccctcct cctatatctc tttttgctac attttgtttt ttacgtttct 1080
tgcttttgca ctctcccact cccacaaaga aaaaaaaact acactatgtc gtcttctcca 1140
tcgtttgccc aagaggttct cgctaccact agtccttaca tcgagtactt tcttgacaac 1200
tacaccagat ggtactactt catacctttg gtgcttcttt cgttgaactt tataagtttg 1260
ctccacacaa ggtacttgga acgcaggttc cacgccaagc cactcggtaa ctttgtcagg 1320
gaccctacgt ttggtatcgc tactccgttg cttttgatct acttgaagtc gaaaggtacg 1380
gtcatgaagt ttgcttgggg cctctggaac aacaagtaca tcgtcagaga cccaaagtac 1440
aagacaactg ggctcaggat tgttggcctc ccattgattg aaaccatgga cccagagaac 1500
atcaaggctg ttttggctac tcagttcaat gatttctctt tgggaaccag acacgatttc 1560
ttgtactcct tgttgggtga cggtattttc accttggacg gtgctggctg gaaacatagt 1620
agaactatgt tgagaccaca gtttgctaga gaacaggttt ctcacgtcaa gttgttggag 1680
ccacacgttc aggtgttctt caagcacgtt agaaagcacc gcggtcaaac gttcgacatc 1740
caagaattgt tcttcaggtt gaccgtcgac tccgccaccg agttcttgtt tggtgagtct 1800
gctgaatcct tgagggacga atctattgga ttgaccccaa ccaccaagga tttcgatggc 1860
agaagagatt tcgctgacgc tttcaactat tcgcagactt accaggccta cagatttttg 1920
ttgcaacaaa tgtactggat cttgaatggc tcggaattca gaaagtcgat tgctgtcgtg 1980
cacaagtttg ctgaccacta tgtgcaaaag gctttggagt tgaccgacga tgacttgcag 2040
aaacaagacg gctatgtgtt cttgtacgag ttggctaagc aaaccagaga cccaaaggtc 2100
ttgagagacc agttattgaa cattttggtt gccggtagag acacgaccgc cggtttgttg 2160
tcatttgttt tctacgagtt gtcaagaaac cctgaggtgt ttgctaagtt gagagaggag 2220
gtggaaaaca gatttggact cggtgaagaa gctcgtgttg aagagatctc gtttgagtcc 2280
ttgaagtctt gtgagtactt gaaggctgtc atcaatgaaa ccttgagatt gtacccatcg 2340
gttccacaca actttagagt tgctaccaga aacactaccc tcccaagagg tggtggtgaa 2400
gatggatact cgccaattgt cgtcaagaag ggtcaagttg tcatgtacac tgttattgct 2460
acccacagag acccaagtat ctacggtgcc gacgctgacg tcttcagacc agaaagatgg 2520
tttgaaccag aaactagaaa gttgggctgg gcatacgttc cattcaatgg tggtccaaga 2580
atctgtttgg gtcaacagtt tgccttgacc gaagcttcat acgtcactgt cagattgctc 2640
caggagtttg cacacttgtc tatggaccca gacaccgaat atccaccaaa attgcagaac 2700
accttgacct tgtcgctctt tgatggtgct gatgttagaa tgtactaagg ttgcttttcc 2760
ttgctaattt tcttctgtat agcttgtgta tttaaattga atcggcaatt gatttttctg 2820
ataccaataa ccgtagtgcg atttgaccaa aaccgttcaa actttttgtt ctctcgttga 2880
cgtgctcgct catcagcact gtttgaagac gaaagagaaa atttttttgta aacaacactg 2940
tccaaattta cccaacgtga accattatgc aaatgagcgg ccctttcaac tggtcgctgg 3000

aagcattcgg ggatatctac aacgccctta agtttgaaac agacattgat ttagacacca 3060
tagatttcag cggcatcaag aatgaccttg cccacatttt gacgacccca acaccactgg 3120
aagaatcacg ccagaaacta ggcgatggat ccaagcctgt gaccttgccc aatggagacg 3180
aagtggagtt gaaccaagcg ttcctagaag ttaccacatt attgtcgaat gagtttgact 3240
tggaccaatt gaacgcggca gagttgttat actacgctgg cgacatatcc tacaagaagg 3300
gcacatcaat cgcagacagt gccagattgt cttattattt gagagcaaac tacatcttga 3360
acatacttgg gtatttgatt tcgaagcagc gattggattt gatagtcacg gacaacgacg 3420
cgttgtttga tagtattttg aaaagttttg aaaagatcta caagttgata agcgtgttga 3480
acgatatgat tgacaagcaa aaggtgacaa gcgacatcaa cagtctagca ttcatcaatt 3540
gcatcaacta ctcgagaggt caactattct ccgcacacga acttttggga ctggttttgt 3600
ttggattggt cgacatctat ttcaaccagt ttggcacatt agacaactac aagaaggtat 3660
tggcattgat actgaagaac atcagcgatg aagacatctt gatcatacac ttcctcccat 3720
cgacactaca attgtttaag ctggtgttgg acaagaaaga cgacgctgca gttgaacagt 3780
tctacaagta catcacttca acagtgtcac gagactacaa ctccaacatc ggctccacag 3840
ccaaagatga tatcgatttg tccaaaacca aactcagtgg ctttgaggtg ttgacgagtt 3900

<210> 89
<211> 3668
<212> DNA
<213> Candida tropicalis

<400> 89
cctgcagaat tcgcggccgc gtcgacagag tagcagttat gcaagcatgt gattgtggtt 60
tttgcaacct gtttgcacga caaatgatcg acagtcgatt acgtaatcca tattatttag 120
aggggtaata aaaaataaat ggcagccaga atttcaaaca ttttgcaaac aatgcaaaag 180
atgagaaact ccaacagaaa aaataaaaaa actccgcagc actccgaacc aacaaaacaa 240
tggggggcgc cagaattatt gactattgtg actttttttt attttttccg ttaactttca 300
ttgcagtgaa gtgtgttaca cggggtggtg atggtgttgg tttctacaat gcaagggcac 360
agttgaaggt ttccacataa cgttgcacca tatcaactca atttatcctc attcatgtga 420
taaaagaaga gccaaaaggt aattggcaga ccccccaagg ggaacacgga gtagaaagca 480
atggaaacac gcccatgaca gtgccattta gcccacaaca catctagtat tctttttttt 540
ttttgtgcgc aggtgcacac ctggacttta gttattgccc cataaagtta acaatctcac 600
ctttggctct cccagtgtct ccgcctccag atgctcgttt tacaccctcg agctaacgac 660
aacacaacac ccatgagggg aatgggcaaa gttaaacact tttggtttca atgattccta 720
tttgctactc tcttgttttg tgttttgatt tgcaccatgt gaaataaacg acaattatat 780
ataccttttc gtctgtcctc caatgtctct ttttgctgcc attttgcttt ttgctttttg 840
cttttgcact ctctcccact cccacaatca gtgcagcaac acacaaagaa gaaaaataaa 900
aaaacctaca ctatgtcgtc ttctccatcg tttgctcagg aggttctcgc taccactagt 960
ccttacatcg agtactttct tgacaactac accagatggt actacttcat ccctttggtg 1020
cttctttcgt tgaacttcat cagcttgctc cacacaaagt acttggaacg caggttccac 1080
gccaagccgc tcggtaacgt cgtgttggat cctacgtttg gtatcgctac tccgttgatc 1140
ttgatctact taaagtcgaa aggtacagtc atgaagtttg cctggagctt ctggaacaac 1200
aagtacattg tcaaagaccc aaagtacaag accactggcc ttagaattgt cggcctccca 1260
ttgattgaaa ccatagaccc agagaacatc aaagctgtgt tggctactca gttcaacgat 1320
ttctccttgg gaactagaca cgatttcttg tactccttgt tgggcgatgg tatttttacc 1380
ttggacggtg ctggctggaa acacagtaga actatgttga gaccacagtt tgctagagaa 1440
caggtttccc acgtcaagtt gttggaacca cacgttcagg tgttcttcaa gcacgttaga 1500

aaacaccgcg gtcagacttt tgacatccaa gaattgttct tcagattgac cgtcgactcc 1560
gccaccgagt tcttgtttgg tgagtctgct gaatccttga gagacgactc tgttggtttg 1620
accccaacca ccaaggattt cgaaggcaga ggagatttcg ctgacgcttt caactactcg 1680
cagacttacc aggcctacag atttttgttg caacaaatgt actggatttt gaatggcgcg 1740
gaattcagaa agtcgattgc catcgtgcac aagtttgctg accactatgt gcaaaaggct 1800
ttggagttga ccgacgatga cttgcagaaa caagacggct atgtgttctt gtacgagttg 1860
gctaagcaaa ctagagaccc aaaggtcttg agagaccagt tgttgaacat tttggttgcc 1920
ggtagagaca cgaccgccgg tttgttgtcg tttgtgttct acgagttgtc gagaaaccct 1980
gaagtgtttg ccaagttgag agaggaggtg gaaaacagat ttggactcgg cgaagaggct 2040
cgtgttgaag agatctcttt tgagtccttg aagtcctgtg agtacttgaa ggctgtcatc 2100
aatgaagcct tgagattgta cccatctgtt ccacacaact tcagagttgc caccagaaac 2160
actacccttc caagaggcgg tggtaaagac ggatgctcgc caattgttgt caagaagggt 2220
caagttgtca tgtacactgt cattggtacc cacagagacc caagtatcta cggtgccgac 2280
gccgacgtct tcagaccaga aagatggttc gagccagaaa ctagaaagtt gggctgggca 2340
tatgttccat tcaatggtgg tccaagaatc tgtttgggtc agcagtttgc cttgactgaa 2400
gcttcatacg tcactgtcag attgctccaa gagtttggaa acttgtccct ggatccaaac 2460
gctgagtacc caccaaaatt gcagaacacc ttgaccttgt cactctttga tggtgctgac 2520
gttagaatgt tctaaggttg cttatccttg ctagtgttat ttatagtttg tgtatttaaa 2580
ttgaatcggc gattgatttt tctggtacta ataactgtag tgggttttga ccaaaaccgt 2640
tcaaactttt tttttttttt tcttcccct accttcgttg ctcgctcatc agcactgttt 2700
gaaaacgaaa aaagaaaatt ttttgtaaac aacattgccc aaacttaccc aacgtgaacc 2760
attataacca aatgagcggc gctttcaact ggtcactgga ggcattcggg gatatctaca 2820
acacccttaa gtttgaggaa gacattgatt tagacaccat agatttcagc ggcatcaaga 2880
atgaccttgt ccacattttg acaaccccaa caccactgga agaatcgcgc cagaaactag 2940
gcgatggatc caagcctgtg gccttgccca atggagacga agtggagttg aaccaagcgt 3000
tcctagaagt taccacatta ttgtcgaacg agtttgactt ggaccaattg aacgcggccg 3060
agttgttata ctacgccggc gacatatcct acaagaaggg cacatcaatt gccgacagtg 3120
ccagattgtc ttactatttg agagcaaact acatcttgaa catacttggg tactttattt 3180
cgaagcagcg attggatgtg atagtcaccg acaacaacgc gttgtttgat aatattttga 3240
aaagttttga aaagatctac aagttgataa gcgcgttgaa cgatatgatt gacaagcaaa 3300
aggtgacaag cgacatcaac agtctagcat ttatcaactg catcaactac tcgaggggtc 3360
aactattctc cgcacacgaa cttttgggac tggttttgtt tggattggtt gacaactatt 3420
tcaaccagtt tggctcatta gacaactaca agaaagtatt ggcattgata ctgaagaaca 3480
tcagtgatga agatatcttg atcgtacgct tcctcccatc gacactacaa ttgtttaagc 3540
tggtgttgga taagaaagac gacgccactg ttgaccagtt ctacaagtac atcacctcaa 3600
cagtgtcgca agactacaac tccaacatcg gagccacagc caaagatgat atcgatttgt 3660
ccaaagcc 3668

<210> 90
<211> 3826
<212> DNA
<213> Candida tropicalis

<400> 90
tggagtcgcc agacttgctc acttttgact cccttcgaaa ctcaaagtac gttcaggcgg 60
tgctcaacga aacgctccgt atctacccgg gggtaccacg aaacatgaag acagctacgt 120
gcaacacgac gttgccacgc ggaggaggca aagacggcaa ggaacctatc ttggtgcaga 180

agggacagtc cgttgggttg attactattg ccacgcagac ggacccagag tattttgggg 240
ccgacgctgg tgagtttaag ccggagagat ggtttgattc aagcatgaag aacttggggt 300
gtaaatactt gccgttcaat gctgggccac ggacttgctt ggggcagcag tacactttga 360
ttgaagcgag ctacttgcta gtccggttgg cccagaccta ccgggcaata gatttgcagc 420
caggatcggc gtacccacca agaaagaagt cgttgatcaa catgagtgct gccgacgggg 480
tgtttgtaaa gctttataag gatgtaacgg tagatggata gttgtgtagg aggagcggag 540
ataaattaga tttgattttg tgtaaggttt tggatgtcaa cctactccgc acttcatgca 600
gtgtgtgtga cacaagggtg tactacgtgt gcgtgtgcgc caagagacag cccaaggggg 660
tggtagtgtg tgttggcgga agtgcatgtg acacaacgcg tgggttctgg ccaatggtgg 720
actaagtgca ggtaagcagc gacctgaaac attcctcaac gcttaagaca ctggtggtag 780
agatgcggac caggctattc ttgtcgtgct acccggcgca tggaaaatca actgcgggaa 840
gaataaattt atccgtagaa tccacagagc ggataaattt gcccacctcc atcatcaacc 900
acgccgccac taactacatc actcccctat tttctctctc tctctttgtc ttactccgct 960
cccgtttcct tagccacaga tacacaccca ctgcaaacag cagcaacaat tataaagata 1020
cgccaggccc accttctttc tttttcttca cttttttgac tgcaactttc tacaatccac 1080
cacagccacc accacagccg ctatgattga acaactccta gaatattggt atgtcgttgt 1140
gccagtgttg tacatcatca aacaactcct tgcatacaca aagactcgcg tcttgatgaa 1200
aaagttgggt gctgctccag tcacaaacaa gttgtacgac aacgctttcg gtatcgtcaa 1260
tggatggaag gctctccagt tcaagaaaga gggcagggct caagagtaca acgattacaa 1320
gtttgaccac tccaagaacc caagcgtggg cacctacgtc agtattcttt tcggcaccag 1380
gatcgtcgtg accaaagatc cagagaatat caaagctatt ttggcaaccc agtttggtga 1440
tttttctttg ggcaagaggc acactctttt taagcctttg ttaggtgatg ggatcttcac 1500
attggacggc gaaggctgga agcacagcag agccatgttg agaccacagt ttgccagaga 1560
acaagttgct catgtgacgt cgttggaacc acacttccag ttgttgaaga agcatattct 1620
taagcacaag ggtgaatact ttgatatcca ggaattgttc tttagattta ccgttgattc 1680
ggccacggag ttcttatttg gtgagtccgt gcactcctta aaggacgaat ctattggtat 1740
caaccaagac gatatagatt ttgctggtag aaaggacttt gctgagtcgt tcaacaaagc 1800
ccaggaatac ttggctatta gaaccttggt gcagacgttc tactggttgg tcaacaacaa 1860
ggagtttaga gactgtacca agctggtgca caagttcacc aactactatg ttcagaaagc 1920
tttggatgct agcccagaag agcttgaaaa gcaaagtggg tatgtgttct tgtacgagct 1980
tgtcaagcag acaagagacc ccaatgtgtt gcgtgaccag tctttgaaca tcttgttggc 2040
cggaagagac accactgctg ggttgttgtc gtttgctgtc tttgagttgg ccagacaccc 2100
agagatctgg gccaagttga gagaggaaat tgaacaacag tttggtcttg gagaagactc 2160
tcgtgttgaa gagattacct ttgagagctt gaagagatgt gagtacttga aagcgttcct 2220
taatgaaacc ttgcgtattt acccaagtgt cccaagaaac ttcagaatcg ccaccaagaa 2280
cacgacattg ccaaggggcg gtggttcaga cggtacctcg ccaatcttga tccaaaaggg 2340
agaagctgtg tcgtatggta tcaactctac tcatttggac cctgtctatt acggccctga 2400
tgctgctgag ttcagaccag agagatggtt tgagccatca accaaaaagc tcggctgggc 2460
ttacttgcca ttcaacggtg gtccaagaat ctgtttgggt cagcagtttg ccttgacgga 2520
agctggctat gtgttggtta gattggtgca agagttctcc cacgttaggc tggacccaga 2580
cgaggtgtac ccgccaaaga ggttgaccaa cttgaccatg tgtttgcagg atggtgctat 2640
tgtcaagttt gactagcggc gtggtgaatg cgtttgattt tgtagtttct gtttgcagta 2700
atgagataac tattcagata aggcgagtgg atgtacgttt tgtaagagtt tccttacaac 2760
cttggtgggg tgtgtgaggt tgaggttgca tcttggggag attacacctt ttgcagctct 2820
ccgtatacac ttgtactctt tgtaacctct atcaatcatg tggggggggg ggttcattgt 2880
ttggccatgg tggtgcatgt taaatccgcc aactacccaa tctcacatga aactcaagca 2940
cactaaaaaa aaaaaagatg ttggggggaaa actttggttt cccttcttag taattaaaca 3000
ctctcactct cactctcact ctctccactc agacaaacca accacctggg ctgcagacaa 3060

ccagaaaaaa aaagaacaaa atccagatag aaaaacaaag ggctggacaa ccataaataa 3120
acaatctagg gtctactcca tcttccactg tttcttcttc ttcagactta gctaacaaac 3180
aactcacttc accatggatt acgcaggcat cacgcgtggc tccatcagag gcgaggcctt 3240
gaagaaactc gcagaattga ccatccagaa ccagccatcc agcttgaaag aaatcaacac 3300
cggcatccag aaggacgact ttgccaagtt gttgtctgcc accccgaaaa tccccaccaa 3360
gcacaagttg aacggcaacc acgaattgtc tgaggtcgcc attgccaaaa aggagtacga 3420
ggtgttgatt gccttgagcg acgccacaaa agacccaatc aaagtgacct cccagatcaa 3480
gatcttgatt gacaagttca aggtgtactt gtttgagttg cctgaccaga agttctccta 3540
ctccatcgtg tccaactccg tcaacatcgc cccctggacc ttgctcgggg agaagttgac 3600
cacgggcttg atcaacttgg ccttccagaa caacaagcag cacttggacg aggtcattga 3660
catcttcaac gagttcatcg acaagttctt ggcaacacg gagccgcaat tgaccaactt 3720
cttgaccttg tgcggtgtgt tggacgggtt gattgaccat gccaacttct tgagcgtgtc 3780
ctcgcggacc ttcaagatct tcttgaactt ggactcgtat gtggac 3826

<210> 91
<211> 3910
<212> DNA
<213> Candida tropicalis

<400> 91
ttacaatcat ggagctcgct aggaacccag atgtctggga gaagctccgc gaagaggtca 60
acacgaactt tggcatggag tcgccagact tgctcacttt tgactctctt agaagctcaa 120
agtacgttca ggcggtgctc aacgaaacgc ttcgtatcta cccgggggtg ccacgaaaca 180
tgaagacagc tacgtgcaac acgacgttgc cgcgtggagg aggcaaagac ggtaaggaac 240
ctattttggt gcagaagggc cagtccgttg ggttgattac tattgccacg cagacggacc 300
cagagtattt tggggcagat gctggtgagt tcaaaccgga gagatggttt gattcaagca 360
tgaagaactt ggggtgtaag tacttgccgt tcaatgctgg gccccggact tgtttggggc 420
agcagtacac tttgattgaa gcgagctatt tgctagtcag gttggcgcag acctaccggg 480
taatcgattt gctgccaggg tcggcgtacc caccaagaaa gaagtcgttg atcaatatga 540
gtgctgccga tggggtggtt gtaaagtttc acaaggatct agatggatat gtaaggtgtg 600
taggaggagc ggagataaat tagatttgat tttgtgtaag gtttagcacg tcaagctact 660
ccgcactttg tgtgtaggga gcacatactc cgtctgcgcc tgtgccaaga gacggcccag 720
gggtagtgtg tggtggtgga agtgcatgtg acacaatacc ctggttctgg ccaattgggg 780
atttagtgta ggtaagctgc gacctgaaac actcctcaac gcttgagaca ctggtgggta 840
gagatgcggg ccaggaggct attcttgtcg tgctacccgt gcacggaaaa tcgattgagg 900
gaagaacaaa tttatccgtg aaatccacag agcggataaa tttgtcacat tgctgcgttg 960
cccacccaca gcattctctt ttctctctct ttgtcttact ccgctcctgt ttccttatcc 1020
agaaatacac accaactcat ataaagatac gctagcccag ctgtctttct ttttcttcac 1080
ttttttttggt gtgttgcttt tttggctgct actttctaca accaccacca ccaccaccac 1140
catgattgaa caaatcctag aatattggta tattgttgtg cctgtgttgt acatcatcaa 1200
acaactcatt gcctacagca agactcgcgt cttgatgaaa cagttgggtg ctgctccaat 1260
cacaaaccag ttgtacgaca acgttttcgg tatcgtcaac ggatggaagg ctctccagtt 1320
caagaaagag ggcagagctc aagagtacaa cgatcacaag tttgacagct ccaagaaccc 1380
aagcgtcggc acctatgtca gtattctttt tggcaccaag attgtcgtga ccaaggatcc 1440
agagaatatc aaagctattt tggcaaccca gtttggcgat ttttctttgg gcaagagaca 1500
cgctcttttt aaacctttgt taggtgatgg gatcttcacc ttggacggcg aaggctggaa 1560
gcatagcaga tccatgttaa gaccacagtt tgccagagaa caagttgctc atgtgacgtc 1620

gttggaacca cacttccagt tgttgaagaa gcatatcctt aaacacaagg gtgagtactt 1680
tgatatccag gaattgttct ttagatttac tgtcgactcg gccacggagt tcttatttgg 1740
tgagtccgtg cactccttaa aggacgaaac tatcggtatc aaccaagacg atatagattt 1800
tgctggtaga aaggactttg ctgagtcgtt caacaaagcc caggagtatt tgtctattag 1860
aattttggtg cagaccttct actggttgat caacaacaag gagtttagag actgtaccaa 1920
gctggtgcac aagtttacca actactatgt tcagaaagct ttggatgcta ccccagagga 1980
acttgaaaag caaggcgggt atgtgttctt gtatgagctt gtcaagcaga cgagagaccc 2040
caaggtgttg cgtgaccagt ctttgaacat cttgttggca ggaagagaca ccactgctgg 2100
gttgttgtcc tttgctgtgt ttgagttggc cagaaaccca cacatctggg ccaagttgag 2160
agaggaaatt gaacagcagt ttggtcttgg agaagactct cgtgttgaag agattacctt 2220
tgagagcttg aagagatgtg agtacttgaa agcgttcctt aacgaaacct tgcgtgttta 2280
cccaagtgtc ccaagaaact tcagaatcgc caccaagaat acaacattgc caaggggtgg 2340
tggtccagac ggtacccagc caatcttgat ccaaaaggga gaaggtgtgt cgtatggtat 2400
caactctacc cacttagatc ctgtctatta tggccctgat gctgctgagt tcagaccaga 2460
gagatggttt gagccatcaa ccagaaagct cggctgggct tacttgccat tcaacggtgg 2520
gccacgaatc tgtttgggtc agcagtttgc cttgaccgaa gctggttacg ttttggtcag 2580
attggtgcaa gagttctccc acattaggct ggacccagat gaagtgtatc caccaaagag 2640
gttgaccaac ttgaccatgt gtttgcagga tggtgctatt gtcaagtttg actagtacgt 2700
atgagtgcgt ttgattttgt agtttctgtt tgcagtaatg agataactat tcagataagg 2760
cgggtggatg tacgttttgt aagagtttcc ttacaaccct ggtgggtgtg tgaggttgca 2820
tcttagggag agatagcacc ttttgcagct ctccgtatac agttttactc tttgtaacct 2880
atgccaatca tgtggggatt cattgtttgc ccatggtggt gcatgcaaaa tccccccaac 2940
tacccaatct cacatgaaac tcaagcacac tagaaaaaaa agatgttgcg tgggttcttt 3000
tgatgttggg gaaaactttc gtttcctttc tcagtaatta aacgttctca ctcagacaaa 3060
ccacctgggc tgcagacaac cagaaaaaac aaaatccaga tagaagaaga aagggctgga 3120
caaccataaa taaacaacct aggtccact ccatctttca cttcttcttc ttcagactta 3180
tctaacaaac gactcacttc accatggatt acgcaggtat cacgcgtggg tccatcagag 3240
gcgaagcctt gaagaaactc gccgagttga ccatccagaa ccagccatcc agcttgaaag 3300
aaatcaacac cggcatccag aaggacgact ttgccaagtt gttgtcttcc accccgaaaa 3360
tccacaccaa gcacaagttg aatggcaacc acgaattgtc cgaagtcgcc attgccaaaa 3420
aggagtacga ggtgttgatt gccttgagcg acgccacgaa agaaccaatc aaagtcacct 3480
cccagatcaa gatcttgatt gacaagttca aggtgtactt gtttgagttg cccgaccaga 3540
agttctccta ctccatcgtg tccaactccg ttaacattgc cccctggacc ttgctcggtg 3600
agaagttgac cacgggcttg atcaacttgg cgttccagaa caacaagcag cacttggacg 3660
aagtcatcga catcttcaac gagttcatcg acaagttctt tggcaacaca gagccgcaat 3720
tgaccaactt cttgaccttg tccggtgtgt tggacgggtt gattgaccat gccaacttct 3780
tgagcgtgtc ctccaggacc ttcaagatct tcttgaactt ggactcgttt gtggacaact 3840
cggacttctt gaacgacgtg gagaactact ccgacttttt gtacgacgag ccgaacgagt 3900
accagaactt 3910

<210> 92
<211> 3150
<212> DNA
<213> Candida tropicalis

<400> 92
gaattctttg gatctaattc cagctgatct tgctaatcct tatcaacgta gttgtgatca 60

ttgtttgtct gaattataca caccagtgga agaatatggt ctaatttgca cgtcccactg 120
gcattgtgtg tttgtggggg ggggggggtg cacacatttt tagtgccatt ctttgttgat 180
taccccctccc ccctatcatt cattcccaca ggattagttt tttcctcact ggaattcgct 240
gtccacctgt caaccccccc cccccccccc cccactgccc taccctgccc tgccctgcac 300
gtcctgtgtt ttgtgctgtg tctttcccac gctataaaag ccctggcgtc cggccaaggt 360
ttttccaccc agccaaaaaa acagtctaaa aaatttggtt gatccttttt ggttgcaagg 420
ttttccacca ccacttccac cacctcaact attcgaacaa aagatgctcg atcagatctt 480
acattactgg tacattgtct tgccattgtt ggccattatc aaccagatcg tggctcatgt 540
caggaccaat tatttgatga agaaattggg tgctaagcca ttcacacacg tccaacgtga 600
cgggtggttg ggcttcaaat tcggccgtga attcctcaaa gcaaaaagtg ctgggagact 660
ggttgattta atcatctccc gtttccacga taatgaggac actttctcca gctatgcttt 720
tggcaaccat gtggtgttca ccagggaccc cgagaatatc aaggcgcttt tggcaaccca 780
gtttggtgat ttttcattgg gcagcagggt caagttcttc aaaccattat tggggtacgg 840
tatcttcaca ttggacgccg aaggctggaa gcacagcaga gccatgttga gaccacagtt 900
tgccagagaa caagttgctc atgtgacgtc gttggaacca cacttccagt tgttgaagaa 960
gcatatcctt aaacacaagg gtgagtactt tgatatccag gaattgttct ttagatttac 1020
tgtcgactcg gccacggagt tcttatttgg tgagtccgtg cactccttaa aggacgagga 1080
aattggctac gacacgaaag acatgtctga agaaagacgc agatttgccg acgcgttcaa 1140
caagtcgcaa gtctacgtgg ccaccagagt tgctttacag aacttgtact ggttggtcaa 1200
caacaaagag ttcaaggagt gcaatgacat tgtccacaag tttaccaact actatgttca 1260
gaaagccttg gatgctaccc cagaggaact tgaaaagcaa ggcgggtatg tgttcttgta 1320
tgagcttgtc aagcagacga gagaccccaa ggtgttgcgt gaccagtctt tgaacatctt 1380
gttggcagga agagacacca ctgctgggtt gttgtccttt gctgtgtttg agttggccag 1440
aaacccacac atctgggcca agttgagaga ggaaattgaa cagcagtttg gtcttggaga 1500
agactctcgt gttgaagaga ttacctttga gagcttgaag agatgtgagt acttgaaggc 1560
cgtgttgaac gaaactttga gattacaccc aagtgtccca agaaacgcaa gatttgcgat 1620
taaagacacg actttaccaa gaggcggtgg ccccaacggc aaggatccta tcttgatcag 1680
gaaggatgag gtggtgcagt actccatctc ggcaactcag acaaatcctg cttattatgg 1740
cgccgatgct gctgatttta gaccggaaag atggtttgaa ccatcaacta gaaacttggg 1800
atgggctttc ttgccattca acggtggtcc aagaatctgt ttgggacaac agtttgcttt 1860
gactgaagcc ggttacgttt tggttagact tgttcaggag tttccaaact tgtcacaaga 1920
ccccgaaacc aagtacccac cacctagatt ggcacacttg acgatgtgct tgtttgacgg 1980
tgcacacgtc aagatgtcat aggtttcccc atacaagtag ttcagtaatt atacactgtt 2040
tttactttct cttcatacca aatggacaaa agttttaagc atgcctaaca acgtgaccgg 2100
acaattgtgt cgcactagta tgtaacaatt gtaaaaatag tgtacactaa tttgtggtgg 2160
ccggagataa attacagttt ggttttgtgt aaactcgcgg atatctctgg cagtttctct 2220
tctccgcagc agctttgcca cgggtttgct ctggggccaa caaattcaaa agggggagaa 2280
acttaacacc ccttatctct ccactctagg ttgtagctct tgtggggatg caattgtcgt 2340
acgtttttta tgttttgtct agactttgat gattacgttg gatttcttat gtctgaggcg 2400
tgcttgaaag aagtgtcaaa atgtgacagg cgacgctatt cgacatgaac gcgaaagggt 2460
tatttgcatc aatacgaggg gctgactcta gtctaggatg gcagtcctag gttgcaaaca 2520
tgttgcacca tatccctcct ggagttggtc gacctcgcct acgccacccct cagcgatcgg 2580
cactttccgt tgttcaatat ttctccttcc cattgttcca ggggttatca acaacgttgc 2640
cggcctcctc cccaaattac aagaaaaata aattgtcgca cggcaccgat ctgtcaaaga 2700
tacagataaa ccttaaatct gcaaaaacaa gacccctccc catagcctag aagcaccagc 2760
aagatgatgg agcaactcct ccagtactgg tacatcgcac tctctgtatg gttcatcctt 2820
cgctacttgg cttcccacgc acgagccgtc tacttgcgcc acaagctcgg cgcggcgcca 2880
ttcacgcaca cccagtacga cggctggtat gggttcaagt ttgggcggga gtttctcaag 2940

gcgaagaaga tcgggcggca gacggacttg gtgcatgcgc ggttccgtgg cggcatggac 3000
accttctcga gctacacttt cggcatccat atcatcctta cccgggaccc ggagaacatc 3060
aaggcggtct tggcgacgca gttcgatgac ttctcgctcg gtggcaggat caggttcttg 3120
aagccgttgt tggggtatgg gatattcacg 3150

<210> 93
<211> 3579
<212> DNA
<213> Candida tropicalis

<400> 93
aaaaccgata caagaagaag acagtcaaca agaacgttaa tgtcaaccag gcgccaagaa 60
gacggtttgg cggacttgga agaatgtggc atttgcccat gatgtttatg ttctggagag 120
gtttttcaag gaatcgtcat cctccgccac cacaagaacc accagttaac gagatccata 180
ttcacaaccc accgcaaggt gacaatgctc aacaacaaca gcaacaacaa caaccccccac 240
aagaacagtg gaataatgcc agtcaacaaa gagtggtgac agacgaggga gaaaacgcaa 300
gcaacagtgg ttctgatgca agatcagcta caccgcttca tcaggaaaag caggagctcc 360
caccaccata tgcccatcac gagcaacacc agcaggttag tgtatagtag tctgtagtta 420
agtcaatgca atgtaccaat aagactatcc cttcttacaa ccaagttttc tgccgcgcct 480
gtctggcaac agatgctggc cgacacactt tcaactgagt ttggtctaga attcttgcac 540
atgcacgaca aggaaactct tacaaagaca acacttgtgc tctgatgcca cttgatcttg 600
ctaagcctta tcaacgtaat tgagatcatt gtttgtctga attatacaca ccagtggaag 660
aatctggtct aatctgcacg cctcatgggc attgtgtgtt ttgggggggg gggggggggt 720
gcacacattt ttagtgcgaa tgtttgtttg ctggttcccc ctcccccctc ccccctatca 780
tgcccacagg attagttttt tcctcactgg aattcgctgt ccacctgtca accccctcac 840
tgccctgccc tgccctgcac gccctgtgtt ttgtgctgtg gcactcccac gctataaaag 900
ccctggcgta cggccaaggt ttttcctcac agccaaaaaa aaatttggct gatccttttg 960
ggctgcaagg tttttcacca ccaccaccac caccacctca actattcaaa caaaggatgc 1020
tcgaccagat cttccattac tggtacattg tcttgccatt gttggtcatt atcaagcaga 1080
tcgtggctca tgccaggacc aattatttga tgaagaagtt gggcgctaag ccattcacac 1140
atgtccaact agacgggtgg tttggcttca aatttggccg tgaattcctc aaagctaaaa 1200
gtgctgggag gcaggttgat ttaatcatct cccgtttcca cgataatgag gacactttct 1260
ccagctatgc ttttggcaac catgtggtgt tcaccaggga ccccgagaat atcaaggcgc 1320
ttttggcaac ccagtttggt gatttttcat tgggaagcag ggtcaaattc ttcaaaccat 1380
tgttggggta cggtatcttc accttggacg gcgaaggctg gaagcacagc agagccatgt 1440
tgagaccaca gtttgccaga gagcaagttg ctcatgtgac gtcgttggaa ccacatttcc 1500
agttgttgaa gaagcatatt cttaagcaca agggtgaata ctttgatatc caggaattgt 1560
tctttagatt taccgttgat tcagcgacgg agttcttatt tggtgagtcc gtgcactcct 1620
taagggacga ggaaattggc tacgatacga aggacatggc tgaagaaaga cgcaaatttg 1680
ccgacgcgtt caacaagtcg caagtctatt tgtccaccag agttgcttta cagacattgt 1740
actggttggt caacaacaaa gagttcaagg agtgcaacga cattgtccac aagttcacca 1800
actactatgt tcagaaagcc ttggatgcta ccccagagga acttgaaaaa caaggcgggt 1860
atgtgttctt gtacgagctt gccaagcaga cgaaagaccc caatgtgttg cgtgaccagt 1920
ctttgaacat cttgttggct ggaagggaca ccactgctgg gttgttgtcc tttgctgtgt 1980
ttgagttggc caggaaccca cacatctggg ccaagttgag agaggaaatt gaatcacact 2040
ttgggctggg tgaggactct cgtgttgaag agattacctt tgagagcttg aagagatgtg 2100
agtacttgaa agccgtgttg aacgaaacgt tgagattaca cccaagtgtc ccaagaaacg 2160

caagatttgc gattaaagac acgactttac caagaggcgg tggccccaac ggcaaggatc 2220
ctatcttgat cagaaagaat gaggtggtgc aatactccat ctcggcaact cagacaaatc 2280
ctgcttatta tggcgccgat gctgctgatt ttagaccgga aagatggttt gagccatcaa 2340
ctagaaactt gggatgggct tacttgccat tcaacggtgg tccaagaatc tgcttgggac 2400
aacagtttgc tttgaccgaa gccggttacg ttttggttag acttgttcag gaattcccta 2460
gcttgtcaca ggaccccgaa actgagtacc caccacctag attggcacac ttgacgatgt 2520
gcttgtttga cggggcatac gtcaagatgc aataggtttt ggtttgactt tgtttccata 2580
tgcaagtagt tcagtaatta cacactaatt tgtggtggcc ggcgataaat taccgtttgg 2640
ttttgtgtaa aaattcggac atctctggtg gtttcccttc tccgcagcag ctttgccacg 2700
ggtttgctct gcggccaaca aattcgaaag gggggggggg gggggagaaa gttaacaccc 2760
cctgttccca ccgtaggctg tagctcttgt gggggatgt aattgtcgta cgttttcatg 2820
tttggcccag actttgatga ttacgtaggc tttcttatgt ctaaggcgtg cttgacacaa 2880
gtgtcaaaag gtgacaggcg acgttattcg acatgaacgc aaaagggtaa tttgcatcga 2940
tacgaggggt tgcctctggt ctaagaagga ccccccaggt tgcaaacatg ttgcactgca 3000
tcccactcag agttggtcga ccacgcctac gcttaccctc agcgatcggc actttccgtt 3060
gctcaatatt tctctccccc ctgcttcccc ccattgttcc aggattatc aacaacgttg 3120
ccggtctcct ctcccccccc tccccccagt tatgtacaag aaaattaaat tgtcgcacgg 3180
caccgatacg tcaaagatac agagaaacct taatccctcc catagcctag aagcatcaaa 3240
aagatgattg agcaactcct ccagtactgg tacattgcac tccctgtatg gttcattctc 3300
cgctacgtgg cttcccacgc acgaaccatc tacttgcgcc acaagctcgg cgcggcgccg 3360
ttcacgcaca cccagtacga cggatggtat gggttcaagt ttgggcggga gtttctcaag 3420
gcgaagaaga ttggaaggca gacggacttg gtgcatgcgc ggttccgtgg aggggcatg 3480
gatactttct cgagctatac tttcggcatc catatcattc ttactcggga cccggagaac 3540
atcaaggcgg tcttggcgac gcagttcgat gacttttcg 3579

<210> 94
<211> 3348
<212> DNA
<213> Candida tropicalis

<400> 94
gatgtggtgc ttgatttctc gagacacatc cttgtgaggt gccatgaatc tgtacctgtc 60
tgtaagcaca gggaactgct tcaacacctt attgcatatt ctgtctattg caagcgtgtg 120
ctgcaacgat atctgccaag gtatatagca gaacgtgctg atggttcctc cggtcatatt 180
ctgttggtag ttctgcaggt aaatttggat gtcaggtagt ggagggaggt ttgtatcggt 240
tgtgttttct tcttcctctc tctctgattc aacctccacg tctccttcgg gttctgtgtc 300
tgtgtctgag tcgtactgtt ggattaagtc catcgcatgt gtgaaaaaaa gtagcgctta 360
tttagacaac cagttcgttg ggcgggtatc agaaatagtc tgttgtgcac gaccatgagt 420
atgcaacttg acgagacgtc gttaggaatc cacagaatga tagcaggaag cttactacgt 480
gagagattct gcttagagga tgttctcttc ttgttgattc cattaggtgg gtatcatctc 540
cggtggtgac aacttgacac aagcagttcc gagaaccacc cacaacaatc accattccag 600
ctatcacttc tacatgtcaa cctacgatgt atctcatcac catctagttt cttggcaatc 660
gtttatttgt tatgggtcaa catccaatac aactccacca atgaagaaga aaaacggaaa 720
gcagaatacc agaatgacag tgtgagttcc tgaccattgc taatctatgg ctatatctag 780
tttgctatcg tgggatgtga tctgtgtcgt cttcatttgc gtttgtgttt atttcgggta 840
tgaatattgt tatactaaat acttgatgca caaacatggc gctcgagaaa tcgagaatgt 900
gatcaacgat gggttctttg ggttccgctt acctttgcta ctcatgcgag ccagcaatga 960

```
gggccgactt atcgagttca gtgtcaagag attcgagtcg gcgccacatc cacagaacaa 1020
gacattggtc aaccgggcat tgagcgttcc tgtgatactc accaaggacc cagtgaatat 1080
caaagcgatg ctatcgaccc agtttgatga cttttccctt gggttgagac tacaccagtt 1140
tgcgccgttg ttggggaaag gcatctttac tttggacggc ccagagtgga agcagagccg 1200
atctatgttg cgtccgcaat ttgccaaaga tcgggtttct catatcctgg atctagaacc 1260
gcattttgtg ttgcttcgga agcacattga tggccacaat ggagactact tcgacatcca 1320
ggagctctac ttccggttct cgatggatgt ggcgacgggg tttttgtttg gcgagtctgt 1380
ggggtcgttg aaagacgaag atgcgaggtt cctggaagca ttcaatgagt cgcagaagta 1440
tttggcaact agggcaacgt tgcacgagtt gtactttctt tgtgacgggt ttaggtttcg 1500
ccagtacaac aaggttgtgc gaaagttctg cagccagtgt gtccacaagg cgttagatgt 1560
tgcaccggaa gacaccagcg agtacgtgtt tctccgcgag ttggtcaaac acactcgaga 1620
tcccgttgtt ttacaagacc aagcgttgaa cgtcttgctt gctggacgcg acaccaccgc 1680
gtcgttatta tcgtttgcaa catttgagct agcccggaat gaccacatgt ggaggaagct 1740
acgagaggag gttatcctga cgatgggacc gtccagtgat gaaataaccg tggccgggtt 1800
gaagagttgc cgttacctca aagcaatcct aaacgaaact cttcgactat acccaagtgt 1860
gcctaggaac gcgagatttg ctacgaggaa tacgacgctt cctcgtggcg gaggtccaga 1920
tggatcgttt ccgattttga taagaaaggg ccagccagtg gggtatttca tttgtgctac 1980
acacttgaat gagaaggtat atgggaatga tagccatgtg tttcgaccgg agagatgggc 2040
tgcgttagag ggcaagagtt tgggctggtc gtatcttcca ttcaacggcg gcccgagaag 2100
ctgccttggt cagcagtttg caatccttga agcttcgtat gttttggctc gattgacaca 2160
gtgctacacg acgatacagc ttagaactac cgagtaccca ccaaagaaac tcgttcatct 2220
cacgatgagt cttctcaacg ggtgtacat ccgaactaga acttgattat gtgtttatgg 2280
ttaatcgggg caaagcactg caagtcattg atgtttgtgg aagcccagca ttggtgttcc 2340
ggagcatcaa taaccaatgt cttgaagggt ttgattttct tgaccttctt cttcctgagc 2400
ttctttccgt caaacttgta cagaatggcc atcatttcag gaacaaccac gtacgacggc 2460
cggtaccgca tctggagtat ctcgccgtcg ttcaagtagc acgaaaacag caacgacgtc 2520
accatctgct tcccaatctt gacacccaca gatacccctg cggcttcatg gatcaaaaac 2580
gtcggcaacc ccgcgtatat gtccatgtaa ttctccatgg ccacctccat caacacactg 2640
atggagcgac tgacggtgcc accactgccc tcggttgagt caaggcagta tgatgccggg 2700
atccagtact ccaatgggaa cctctgcacg gtgtcgctgc agtttttgag gcgtatttcg 2760
atccatgatc gttctttggt gctgtagtat aacgagctct tggtgtcctt gaaatggaac 2820
aggttggatg tgttgttgag tttgtctgcg tgcttggttt gcaagtcttc gatcgagcgt 2880
agtgagtaga cagttggcgg gggtggtggc tcgggcttta ttctgtgttt gtgtttcctt 2940
cttagtcttg gaatgacgct gttatcgacg gttcgtagta taagtagcgc caatatgaga 3000
atgtatatcc gcatcaccca agactcttca gcctgttaca acgactgagg ctgttggccg 3060
tgtgaccaat tggtttcttt ggtgacctag attggtcccg cagggaaagc aagggctgct 3120
agggggggcat accaaacaag gtcgtgtaat cagtatctat ggtgctacca tgtgtgtggt 3180
tggggggaaa ttcccgcatt tttgtgtaac gaaagttcta gaaagttctc gtgggttctg 3240
agaatctgct ggaaccatcc acccgcattt ccgttgccaa agtgggaaga gcaatcaacc 3300
caccctgctt tgcccaatca gccattcccc tgggaatata aattcaac           3348
```

<210> 95
<211> 523
<212> PRT
<213> Candida tropicalis

<400> 95

```
Met Ala Thr Gln Glu Ile Ile Asp Ser Val Leu Pro Tyr Leu Thr Lys
 1               5                  10                  15

Trp Tyr Thr Val Ile Thr Ala Ala Val Leu Val Phe Leu Ile Ser Thr
            20                  25                  30

Asn Ile Lys Asn Tyr Val Lys Ala Lys Lys Leu Lys Cys Val Asp Pro
        35                  40                  45

Pro Tyr Leu Lys Asp Ala Gly Leu Thr Gly Ile Leu Ser Leu Ile Ala
    50                  55                  60

Ala Ile Lys Ala Lys Asn Asp Gly Arg Leu Ala Asn Phe Ala Asp Glu
65                  70                  75                  80

Val Phe Asp Glu Tyr Pro Asn His Thr Phe Tyr Leu Ser Val Ala Gly
                85                  90                  95

Ala Leu Lys Ile Val Met Thr Val Asp Pro Glu Asn Ile Lys Ala Val
            100                 105                 110

Leu Ala Thr Gln Phe Thr Asp Phe Ser Leu Gly Thr Arg His Ala His
        115                 120                 125

Phe Ala Pro Leu Leu Gly Asp Gly Ile Phe Thr Leu Asp Gly Glu Gly
    130                 135                 140

Trp Lys His Ser Arg Ala Met Leu Arg Pro Gln Phe Ala Arg Asp Gln
145                 150                 155                 160

Ile Gly His Val Lys Ala Leu Glu Pro His Ile Gln Ile Met Ala Lys
                165                 170                 175

Gln Ile Lys Leu Asn Gln Gly Lys Thr Phe Asp Ile Gln Glu Leu Phe
            180                 185                 190

Phe Arg Phe Thr Val Asp Thr Ala Thr Glu Phe Leu Phe Gly Glu Ser
        195                 200                 205

Val His Ser Leu Tyr Asp Glu Lys Leu Gly Ile Pro Thr Pro Asn Glu
    210                 215                 220

Ile Pro Gly Arg Glu Asn Phe Ala Ala Ala Phe Asn Val Ser Gln His
225                 230                 235                 240

Tyr Leu Ala Thr Arg Ser Tyr Ser Gln Thr Phe Tyr Phe Leu Thr Asn
                245                 250                 255
```

```
Pro Lys Glu Phe Arg Asp Cys Asn Ala Lys Val His His Leu Ala Lys
                260                 265                 270

Tyr Phe Val Asn Lys Ala Leu Asn Phe Thr Pro Glu Glu Leu Glu Glu
            275                 280                 285

Lys Ser Lys Ser Gly Tyr Val Phe Leu Tyr Glu Leu Val Lys Gln Thr
    290                 295                 300

Arg Asp Pro Lys Val Leu Gln Asp Gln Leu Leu Asn Ile Met Val Ala
305                 310                 315                 320

Gly Arg Asp Thr Thr Ala Gly Leu Leu Ser Phe Ala Leu Phe Glu Leu
                325                 330                 335

Ala Arg His Pro Glu Met Trp Ser Lys Leu Arg Glu Glu Ile Glu Val
            340                 345                 350

Asn Phe Gly Val Gly Glu Asp Ser Arg Val Glu Glu Ile Thr Phe Glu
        355                 360                 365

Ala Leu Lys Arg Cys Glu Tyr Leu Lys Ala Ile Leu Asn Glu Thr Leu
    370                 375                 380

Arg Met Tyr Pro Ser Val Pro Val Asn Phe Arg Thr Ala Thr Arg Asp
385                 390                 395                 400

Thr Thr Leu Pro Arg Gly Gly Gly Ala Asn Gly Thr Asp Pro Ile Tyr
                405                 410                 415

Ile Pro Lys Gly Ser Thr Val Ala Tyr Val Val Tyr Lys Thr His Arg
            420                 425                 430

Leu Glu Glu Tyr Tyr Gly Lys Asp Ala Asn Asp Phe Arg Pro Glu Arg
        435                 440                 445

Trp Phe Glu Pro Ser Thr Lys Lys Leu Gly Trp Ala Tyr Val Pro Phe
    450                 455                 460

Asn Gly Gly Pro Arg Val Cys Leu Gly Gln Gln Phe Ala Leu Thr Glu
465                 470                 475                 480

Ala Ser Tyr Val Ile Thr Arg Leu Ala Gln Met Phe Glu Thr Val Ser
                485                 490                 495

Ser Asp Pro Gly Leu Glu Tyr Pro Pro Pro Lys Cys Ile His Leu Thr
            500                 505                 510
```

Met Ser His Asn Asp Gly Val Phe Val Lys Met
515 520

<210> 96
<211> 522
<212> PRT
<213> Candida tropicalis

<400> 96
Met Thr Val His Asp Ile Ile Ala Thr Tyr Phe Thr Lys Trp Tyr Val
1 5 10 15

Ile Val Pro Leu Ala Leu Ile Ala Tyr Arg Val Leu Asp Tyr Phe Tyr
20 25 30

Gly Arg Tyr Leu Met Tyr Lys Leu Gly Ala Lys Pro Phe Phe Gln Lys
35 40 45

Gln Thr Asp Gly Cys Phe Gly Phe Lys Ala Pro Leu Glu Leu Leu Lys
50 55 60

Lys Lys Ser Asp Gly Thr Leu Ile Asp Phe Thr Leu Gln Arg Ile His
65 70 75 80

Asp Leu Asp Arg Pro Asp Ile Pro Thr Phe Thr Phe Pro Val Phe Ser
85 90 95

Ile Asn Leu Val Asn Thr Leu Glu Pro Glu Asn Ile Lys Ala Ile Leu
100 105 110

Ala Thr Gln Phe Asn Asp Phe Ser Leu Gly Thr Arg His Ser His Phe
115 120 125

Ala Pro Leu Leu Gly Asp Gly Ile Phe Thr Leu Asp Gly Ala Gly Trp
130 135 140

Lys His Ser Arg Ser Met Leu Arg Pro Gln Phe Ala Arg Glu Gln Ile
145 150 155 160

Ser His Val Lys Leu Leu Glu Pro His Val Gln Val Phe Phe Lys His
165 170 175

Val Arg Lys Ala Gln Gly Lys Thr Phe Asp Ile Gln Glu Leu Phe Phe
180 185 190

Arg Leu Thr Val Asp Ser Ala Thr Glu Phe Leu Phe Gly Glu Ser Val
195 200 205

```
Glu Ser Leu Arg Asp Glu Ser Ile Gly Met Ser Ile Asn Ala Leu Asp
    210                 215                 220

Phe Asp Gly Lys Ala Gly Phe Ala Asp Ala Phe Asn Tyr Ser Gln Asn
225                 230                 235                 240

Tyr Leu Ala Ser Arg Ala Val Met Gln Gln Leu Tyr Trp Val Leu Asn
                245                 250                 255

Gly Lys Lys Phe Lys Glu Cys Asn Ala Lys Val His Lys Phe Ala Asp
            260                 265                 270

Tyr Tyr Val Asn Lys Ala Leu Asp Leu Thr Pro Glu Gln Leu Glu Lys
        275                 280                 285

Gln Asp Gly Tyr Val Phe Leu Tyr Glu Leu Val Lys Gln Thr Arg Asp
    290                 295                 300

Lys Gln Val Leu Arg Asp Gln Leu Leu Asn Ile Met Val Ala Gly Arg
305                 310                 315                 320

Asp Thr Thr Ala Gly Leu Leu Ser Phe Val Phe Phe Glu Leu Ala Arg
                325                 330                 335

Asn Pro Glu Val Thr Asn Lys Leu Arg Glu Glu Ile Glu Asp Lys Phe
            340                 345                 350

Gly Leu Gly Glu Asn Ala Ser Val Glu Asp Ile Ser Phe Glu Ser Leu
        355                 360                 365

Lys Ser Cys Glu Tyr Leu Lys Ala Val Leu Asn Glu Thr Leu Arg Leu
    370                 375                 380

Tyr Pro Ser Val Pro Gln Asn Phe Arg Val Ala Thr Lys Asn Thr Thr
385                 390                 395                 400

Leu Pro Arg Gly Gly Gly Lys Asp Gly Leu Ser Pro Val Leu Val Arg
                405                 410                 415

Lys Gly Gln Thr Val Ile Tyr Gly Val Tyr Ala Ala His Arg Asn Pro
            420                 425                 430

Ala Val Tyr Gly Lys Asp Ala Leu Glu Phe Arg Pro Glu Arg Trp Phe
        435                 440                 445

Glu Pro Glu Thr Lys Lys Leu Gly Trp Ala Phe Leu Pro Phe Asn Gly
    450                 455                 460
```

```
Gly Pro Arg Ile Cys Leu Gly Gln Gln Phe Ala Leu Thr Glu Ala Ser
465                 470                 475                 480

Tyr Val Thr Val Arg Leu Leu Gln Glu Phe Ala His Leu Ser Met Asp
                485                 490                 495

Pro Asp Thr Glu Tyr Pro Pro Lys Lys Met Ser His Leu Thr Met Ser
            500                 505                 510

Leu Phe Asp Gly Ala Asn Ile Glu Met Tyr
        515                 520


<210> 97
<211> 522
<212> PRT
<213> Candida tropicalis

<400> 97
Met Thr Ala Gln Asp Ile Ile Ala Thr Tyr Ile Thr Lys Trp Tyr Val
  1               5                  10                  15

Ile Val Pro Leu Ala Leu Ile Ala Tyr Arg Val Leu Asp Tyr Phe Tyr
             20                  25                  30

Gly Arg Tyr Leu Met Tyr Lys Leu Gly Ala Lys Pro Phe Phe Gln Lys
         35                  40                  45

Gln Thr Asp Gly Tyr Phe Gly Phe Lys Ala Pro Leu Glu Leu Leu Lys
     50                  55                  60

Lys Lys Ser Asp Gly Thr Leu Ile Asp Phe Thr Leu Glu Arg Ile Gln
 65                  70                  75                  80

Ala Leu Asn Arg Pro Asp Ile Pro Thr Phe Thr Phe Pro Ile Phe Ser
                 85                  90                  95

Ile Asn Leu Ile Ser Thr Leu Glu Pro Glu Asn Ile Lys Ala Ile Leu
            100                 105                 110

Ala Thr Gln Phe Asn Asp Phe Ser Leu Gly Thr Arg His Ser His Phe
        115                 120                 125

Ala Pro Leu Leu Gly Asp Gly Ile Phe Thr Leu Asp Gly Ala Gly Trp
    130                 135                 140

Lys His Ser Arg Ser Met Leu Arg Pro Gln Phe Ala Arg Glu Gln Ile
```

145 150 155 160

Ser His Val Lys Leu Leu Glu Pro His Met Gln Val Phe Phe Lys His
165 170 175

Val Arg Lys Ala Gln Gly Lys Thr Phe Asp Ile Gln Glu Leu Phe Phe
180 185 190

Arg Leu Thr Val Asp Ser Ala Thr Glu Phe Leu Phe Gly Glu Ser Val
195 200 205

Glu Ser Leu Arg Asp Glu Ser Ile Gly Met Ser Ile Asn Ala Leu Asp
210 215 220

Phe Asp Gly Lys Ala Gly Phe Ala Asp Ala Phe Asn Tyr Ser Gln Asn
225 230 235 240

Tyr Leu Ala Ser Arg Ala Val Met Gln Gln Leu Tyr Trp Val Leu Asn
245 250 255

Gly Lys Lys Phe Lys Glu Cys Asn Ala Lys Val His Lys Phe Ala Asp
260 265 270

Tyr Tyr Val Ser Lys Ala Leu Asp Leu Thr Pro Glu Gln Leu Glu Lys
275 280 285

Gln Asp Gly Tyr Val Phe Leu Tyr Glu Leu Val Lys Gln Thr Arg Asp
290 295 300

Arg Gln Val Leu Arg Asp Gln Leu Leu Asn Ile Met Val Ala Gly Arg
305 310 315 320

Asp Thr Thr Ala Gly Leu Leu Ser Phe Val Phe Phe Glu Leu Ala Arg
325 330 335

Asn Pro Glu Val Thr Asn Lys Leu Arg Glu Glu Ile Glu Asp Lys Phe
340 345 350

Gly Leu Gly Glu Asn Ala Arg Val Glu Asp Ile Ser Phe Glu Ser Leu
355 360 365

Lys Ser Cys Glu Tyr Leu Lys Ala Val Leu Asn Glu Thr Leu Arg Leu
370 375 380

Tyr Pro Ser Val Pro Gln Asn Phe Arg Val Ala Thr Lys Asn Thr Thr
385 390 395 400

Leu Pro Arg Gly Gly Gly Lys Asp Gly Leu Ser Pro Val Leu Val Arg

405 410 415

Lys Gly Gln Thr Val Met Tyr Gly Val Tyr Ala Ala His Arg Asn Pro
420 425 430

Ala Val Tyr Gly Lys Asp Ala Leu Glu Phe Arg Pro Glu Arg Trp Phe
435 440 445

Glu Pro Glu Thr Lys Lys Leu Gly Trp Ala Phe Leu Pro Phe Asn Gly
450 455 460

Gly Pro Arg Ile Cys Leu Gly Gln Gln Phe Ala Leu Thr Glu Ala Ser
465 470 475 480

Tyr Val Thr Val Arg Leu Leu Gln Glu Phe Gly His Leu Ser Met Asp
485 490 495

Pro Asn Thr Glu Tyr Pro Pro Arg Lys Met Ser His Leu Thr Met Ser
500 505 510

Leu Phe Asp Gly Ala Asn Ile Glu Met Tyr
515 520

<210> 98
<211> 540
<212> PRT
<213> Candida tropicalis

<400> 98
Met Ser Ser Ser Pro Ser Phe Ala Gln Glu Val Leu Ala Thr Thr Ser
1 5 10 15

Pro Tyr Ile Glu Tyr Phe Leu Asp Asn Tyr Thr Arg Trp Tyr Tyr Phe
20 25 30

Ile Pro Leu Val Leu Leu Ser Leu Asn Phe Ile Ser Leu Leu His Thr
35 40 45

Arg Tyr Leu Glu Arg Arg Phe His Ala Lys Pro Leu Gly Asn Phe Val
50 55 60

Arg Asp Pro Thr Phe Gly Ile Ala Thr Pro Leu Leu Leu Ile Tyr Leu
65 70 75 80

Lys Ser Lys Gly Thr Val Met Lys Phe Ala Trp Gly Leu Trp Asn Asn
85 90 95

```
Lys Tyr Ile Val Arg Asp Pro Lys Tyr Lys Thr Thr Gly Leu Arg Ile
            100                 105                 110

Val Gly Leu Pro Leu Ile Glu Thr Met Asp Pro Glu Asn Ile Lys Ala
        115                 120                 125

Val Leu Ala Thr Gln Phe Asn Asp Phe Ser Leu Gly Thr Arg His Asp
    130                 135                 140

Phe Leu Tyr Ser Leu Leu Gly Asp Gly Ile Phe Thr Leu Asp Gly Ala
145                 150                 155                 160

Gly Trp Lys His Ser Arg Thr Met Leu Arg Pro Gln Phe Ala Arg Glu
                165                 170                 175

Gln Val Ser His Val Lys Leu Leu Glu Pro His Val Gln Val Phe Phe
            180                 185                 190

Lys His Val Arg Lys His Arg Gly Gln Thr Phe Asp Ile Gln Glu Leu
        195                 200                 205

Phe Phe Arg Leu Thr Val Asp Ser Ala Thr Glu Phe Leu Phe Gly Glu
    210                 215                 220

Ser Ala Glu Ser Leu Arg Asp Glu Ser Ile Gly Leu Thr Pro Thr Thr
225                 230                 235                 240

Lys Asp Phe Asp Gly Arg Arg Asp Phe Ala Asp Ala Phe Asn Tyr Ser
                245                 250                 255

Gln Thr Tyr Gln Ala Tyr Arg Phe Leu Leu Gln Gln Met Tyr Trp Ile
            260                 265                 270

Leu Asn Gly Ser Glu Phe Arg Lys Ser Ile Ala Val Val His Lys Phe
        275                 280                 285

Ala Asp His Tyr Val Gln Lys Ala Leu Glu Leu Thr Asp Asp Asp Leu
    290                 295                 300

Gln Lys Gln Asp Gly Tyr Val Phe Leu Tyr Glu Leu Ala Lys Gln Thr
305                 310                 315                 320

Arg Asp Pro Lys Val Leu Arg Asp Gln Leu Leu Asn Ile Leu Val Ala
                325                 330                 335

Gly Arg Asp Thr Thr Ala Gly Leu Leu Ser Phe Val Phe Tyr Glu Leu
            340                 345                 350
```

Ser Arg Asn Pro Glu Val Phe Ala Lys Leu Arg Glu Glu Val Glu Asn
355 360 365

Arg Phe Gly Leu Gly Glu Glu Ala Arg Val Glu Glu Ile Ser Phe Glu
370 375 380

Ser Leu Lys Ser Cys Glu Tyr Leu Lys Ala Val Ile Asn Glu Thr Leu
385 390 395 400

Arg Leu Tyr Pro Ser Val Pro His Asn Phe Arg Val Ala Thr Arg Asn
405 410 415

Thr Thr Leu Pro Arg Gly Gly Gly Glu Asp Gly Tyr Ser Pro Ile Val
420 425 430

Val Lys Lys Gly Gln Val Val Met Tyr Thr Val Ile Ala Thr His Arg
435 440 445

Asp Pro Ser Ile Tyr Gly Ala Asp Ala Asp Val Phe Arg Pro Glu Arg
450 455 460

Trp Phe Glu Pro Glu Thr Arg Lys Leu Gly Trp Ala Tyr Val Pro Phe
465 470 475 480

Asn Gly Gly Pro Arg Ile Cys Leu Gly Gln Gln Phe Ala Leu Thr Glu
485 490 495

Ala Ser Tyr Val Thr Val Arg Leu Leu Gln Glu Phe Ala His Leu Ser
500 505 510

Met Asp Pro Asp Thr Glu Tyr Pro Pro Lys Leu Gln Asn Thr Leu Thr
515 520 525

Leu Ser Leu Phe Asp Gly Ala Asp Val Arg Met Tyr
530 535 540

<210> 99
<211> 540
<212> PRT
<213> Candida tropicalis

<400> 99
Met Ser Ser Ser Pro Ser Phe Ala Gln Glu Val Leu Ala Thr Thr Ser
1 5 10 15

Pro Tyr Ile Glu Tyr Phe Leu Asp Asn Tyr Thr Arg Trp Tyr Tyr Phe
20 25 30

```
Ile Pro Leu Val Leu Leu Ser Leu Asn Phe Ile Ser Leu Leu His Thr
        35                  40                  45

Lys Tyr Leu Glu Arg Arg Phe His Ala Lys Pro Leu Gly Asn Val Val
    50                  55                  60

Leu Asp Pro Thr Phe Gly Ile Ala Thr Pro Leu Ile Leu Ile Tyr Leu
65                  70                  75                  80

Lys Ser Lys Gly Thr Val Met Lys Phe Ala Trp Ser Phe Trp Asn Asn
                85                  90                  95

Lys Tyr Ile Val Lys Asp Pro Lys Tyr Lys Thr Thr Gly Leu Arg Ile
            100                 105                 110

Val Gly Leu Pro Leu Ile Glu Thr Ile Asp Pro Glu Asn Ile Lys Ala
        115                 120                 125

Val Leu Ala Thr Gln Phe Asn Asp Phe Ser Leu Gly Thr Arg His Asp
    130                 135                 140

Phe Leu Tyr Ser Leu Leu Gly Asp Gly Ile Phe Thr Leu Asp Gly Ala
145                 150                 155                 160

Gly Trp Lys His Ser Arg Thr Met Leu Arg Pro Gln Phe Ala Arg Glu
                165                 170                 175

Gln Val Ser His Val Lys Leu Leu Glu Pro His Val Gln Val Phe Phe
            180                 185                 190

Lys His Val Arg Lys His Arg Gly Gln Thr Phe Asp Ile Gln Glu Leu
        195                 200                 205

Phe Phe Arg Leu Thr Val Asp Ser Ala Thr Glu Phe Leu Phe Gly Glu
    210                 215                 220

Ser Ala Glu Ser Leu Arg Asp Asp Ser Val Gly Leu Thr Pro Thr Thr
225                 230                 235                 240

Lys Asp Phe Glu Gly Arg Gly Asp Phe Ala Asp Ala Phe Asn Tyr Ser
                245                 250                 255

Gln Thr Tyr Gln Ala Tyr Arg Phe Leu Leu Gln Gln Met Tyr Trp Ile
            260                 265                 270

Leu Asn Gly Ala Glu Phe Arg Lys Ser Ile Ala Ile Val His Lys Phe
        275                 280                 285
```

Ala Asp His Tyr Val Gln Lys Ala Leu Glu Leu Thr Asp Asp Asp Leu
290 295 300

Gln Lys Gln Asp Gly Tyr Val Phe Leu Tyr Glu Leu Ala Lys Gln Thr
305 310 315 320

Arg Asp Pro Lys Val Leu Arg Asp Gln Leu Leu Asn Ile Leu Val Ala
325 330 335

Gly Arg Asp Thr Thr Ala Gly Leu Leu Ser Phe Val Phe Tyr Glu Leu
340 345 350

Ser Arg Asn Pro Glu Val Phe Ala Lys Leu Arg Glu Glu Val Glu Asn
355 360 365

Arg Phe Gly Leu Gly Glu Glu Ala Arg Val Glu Glu Ile Ser Phe Glu
370 375 380

Ser Leu Lys Ser Cys Glu Tyr Leu Lys Ala Val Ile Asn Glu Ala Leu
385 390 395 400

Arg Leu Tyr Pro Ser Val Pro His Asn Phe Arg Val Ala Thr Arg Asn
405 410 415

Thr Thr Leu Pro Arg Gly Gly Gly Lys Asp Gly Cys Ser Pro Ile Val
420 425 430

Val Lys Lys Gly Gln Val Val Met Tyr Thr Val Ile Gly Thr His Arg
435 440 445

Asp Pro Ser Ile Tyr Gly Ala Asp Ala Asp Val Phe Arg Pro Glu Arg
450 455 460

Trp Phe Glu Pro Glu Thr Arg Lys Leu Gly Trp Ala Tyr Val Pro Phe
465 470 475 480

Asn Gly Gly Pro Arg Ile Cys Leu Gly Gln Gln Phe Ala Leu Thr Glu
485 490 495

Ala Ser Tyr Val Thr Val Arg Leu Leu Gln Glu Phe Gly Asn Leu Ser
500 505 510

Leu Asp Pro Asn Ala Glu Tyr Pro Pro Lys Leu Gln Asn Thr Leu Thr
515 520 525

Leu Ser Leu Phe Asp Gly Ala Asp Val Arg Met Phe
530 535 540

<210> 100
<211> 517
<212> PRT
<213> Candida tropicalis

<400> 100
```
Met Ile Glu Gln Leu Leu Glu Tyr Trp Tyr Val Val Val Pro Val Leu
 1               5                  10                  15

Tyr Ile Ile Lys Gln Leu Leu Ala Tyr Thr Lys Thr Arg Val Leu Met
            20                  25                  30

Lys Lys Leu Gly Ala Ala Pro Val Thr Asn Lys Leu Tyr Asp Asn Ala
        35                  40                  45

Phe Gly Ile Val Asn Gly Trp Lys Ala Leu Gln Phe Lys Lys Glu Gly
    50                  55                  60

Arg Ala Gln Glu Tyr Asn Asp Tyr Lys Phe Asp His Ser Lys Asn Pro
65                  70                  75                  80

Ser Val Gly Thr Tyr Val Ser Ile Leu Phe Gly Thr Arg Ile Val Val
                85                  90                  95

Thr Lys Asp Pro Glu Asn Ile Lys Ala Ile Leu Ala Thr Gln Phe Gly
            100                 105                 110

Asp Phe Ser Leu Gly Lys Arg His Thr Leu Phe Lys Pro Leu Leu Gly
        115                 120                 125

Asp Gly Ile Phe Thr Leu Asp Gly Glu Gly Trp Lys His Ser Arg Ala
    130                 135                 140

Met Leu Arg Pro Gln Phe Ala Arg Glu Gln Val Ala His Val Thr Ser
145                 150                 155                 160

Leu Glu Pro His Phe Gln Leu Leu Lys Lys His Ile Leu Lys His Lys
                165                 170                 175

Gly Glu Tyr Phe Asp Ile Gln Glu Leu Phe Phe Arg Phe Thr Val Asp
            180                 185                 190

Ser Ala Thr Glu Phe Leu Phe Gly Glu Ser Val His Ser Leu Lys Asp
        195                 200                 205

Glu Ser Ile Gly Ile Asn Gln Asp Asp Ile Asp Phe Ala Gly Arg Lys
```

210 215 220

Asp Phe Ala Glu Ser Phe Asn Lys Ala Gln Glu Tyr Leu Ala Ile Arg
225 230 235 240

Thr Leu Val Gln Thr Phe Tyr Trp Leu Val Asn Asn Lys Glu Phe Arg
245 250 255

Asp Cys Thr Lys Leu Val His Lys Phe Thr Asn Tyr Tyr Val Gln Lys
260 265 270

Ala Leu Asp Ala Ser Pro Glu Glu Leu Glu Lys Gln Ser Gly Tyr Val
275 280 285

Phe Leu Tyr Glu Leu Val Lys Gln Thr Arg Asp Pro Asn Val Leu Arg
290 295 300

Asp Gln Ser Leu Asn Ile Leu Leu Ala Gly Arg Asp Thr Thr Ala Gly
305 310 315 320

Leu Leu Ser Phe Ala Val Phe Glu Leu Ala Arg His Pro Glu Ile Trp
325 330 335

Ala Lys Leu Arg Glu Glu Ile Glu Gln Gln Phe Gly Leu Gly Glu Asp
340 345 350

Ser Arg Val Glu Glu Ile Thr Phe Glu Ser Leu Lys Arg Cys Glu Tyr
355 360 365

Leu Lys Ala Phe Leu Asn Glu Thr Leu Arg Ile Tyr Pro Ser Val Pro
370 375 380

Arg Asn Phe Arg Ile Ala Thr Lys Asn Thr Thr Leu Pro Arg Gly Gly
385 390 395 400

Gly Ser Asp Gly Thr Ser Pro Ile Leu Ile Gln Lys Gly Glu Ala Val
405 410 415

Ser Tyr Gly Ile Asn Ser Thr His Leu Asp Pro Val Tyr Tyr Gly Pro
420 425 430

Asp Ala Ala Glu Phe Arg Pro Glu Arg Trp Phe Glu Pro Ser Thr Lys
435 440 445

Lys Leu Gly Trp Ala Tyr Leu Pro Phe Asn Gly Gly Pro Arg Ile Cys
450 455 460

Leu Gly Gln Gln Phe Ala Leu Thr Glu Ala Gly Tyr Val Leu Val Arg

465 470 475 480

Leu Val Gln Glu Phe Ser His Val Arg Leu Asp Pro Asp Glu Val Tyr
485 490 495

Pro Pro Lys Arg Leu Thr Asn Leu Thr Met Cys Leu Gln Asp Gly Ala
500 505 510

Ile Val Lys Phe Asp
515

<210> 101
<211> 517
<212> PRT
<213> Candida tropicalis

<400> 101
Met Ile Glu Gln Ile Leu Glu Tyr Trp Tyr Ile Val Val Pro Val Leu
1 5 10 15

Tyr Ile Ile Lys Gln Leu Ile Ala Tyr Ser Lys Thr Arg Val Leu Met
20 25 30

Lys Gln Leu Gly Ala Ala Pro Ile Thr Asn Gln Leu Tyr Asp Asn Val
35 40 45

Phe Gly Ile Val Asn Gly Trp Lys Ala Leu Gln Phe Lys Lys Glu Gly
50 55 60

Arg Ala Gln Glu Tyr Asn Asp His Lys Phe Asp Ser Ser Lys Asn Pro
65 70 75 80

Ser Val Gly Thr Tyr Val Ser Ile Leu Phe Gly Thr Lys Ile Val Val
85 90 95

Thr Lys Asp Pro Glu Asn Ile Lys Ala Ile Leu Ala Thr Gln Phe Gly
100 105 110

Asp Phe Ser Leu Gly Lys Arg His Ala Leu Phe Lys Pro Leu Leu Gly
115 120 125

Asp Gly Ile Phe Thr Leu Asp Gly Glu Gly Trp Lys His Ser Arg Ser
130 135 140

Met Leu Arg Pro Gln Phe Ala Arg Glu Gln Val Ala His Val Thr Ser
145 150 155 160

Leu Glu Pro His Phe Gln Leu Leu Lys Lys His Ile Leu Lys His Lys
165 170 175

Gly Glu Tyr Phe Asp Ile Gln Glu Leu Phe Phe Arg Phe Thr Val Asp
180 185 190

Ser Ala Thr Glu Phe Leu Phe Gly Glu Ser Val His Ser Leu Lys Asp
195 200 205

Glu Thr Ile Gly Ile Asn Gln Asp Asp Ile Asp Phe Ala Gly Arg Lys
210 215 220

Asp Phe Ala Glu Ser Phe Asn Lys Ala Gln Glu Tyr Leu Ser Ile Arg
225 230 235 240

Ile Leu Val Gln Thr Phe Tyr Trp Leu Ile Asn Asn Lys Glu Phe Arg
245 250 255

Asp Cys Thr Lys Leu Val His Lys Phe Thr Asn Tyr Tyr Val Gln Lys
260 265 270

Ala Leu Asp Ala Thr Pro Glu Glu Leu Glu Lys Gln Gly Gly Tyr Val
275 280 285

Phe Leu Tyr Glu Leu Val Lys Gln Thr Arg Asp Pro Lys Val Leu Arg
290 295 300

Asp Gln Ser Leu Asn Ile Leu Leu Ala Gly Arg Asp Thr Thr Ala Gly
305 310 315 320

Leu Leu Ser Phe Ala Val Phe Glu Leu Ala Arg Asn Pro His Ile Trp
325 330 335

Ala Lys Leu Arg Glu Glu Ile Glu Gln Gln Phe Gly Leu Gly Glu Asp
340 345 350

Ser Arg Val Glu Glu Ile Thr Phe Glu Ser Leu Lys Arg Cys Glu Tyr
355 360 365

Leu Lys Ala Phe Leu Asn Glu Thr Leu Arg Val Tyr Pro Ser Val Pro
370 375 380

Arg Asn Phe Arg Ile Ala Thr Lys Asn Thr Thr Leu Pro Arg Gly Gly
385 390 395 400

Gly Pro Asp Gly Thr Gln Pro Ile Leu Ile Gln Lys Gly Glu Gly Val
405 410 415

Ser Tyr Gly Ile Asn Ser Thr His Leu Asp Pro Val Tyr Tyr Gly Pro
420 425 430

Asp Ala Ala Glu Phe Arg Pro Glu Arg Trp Phe Glu Pro Ser Thr Arg
435 440 445

Lys Leu Gly Trp Ala Tyr Leu Pro Phe Asn Gly Gly Pro Arg Ile Cys
450 455 460

Leu Gly Gln Gln Phe Ala Leu Thr Glu Ala Gly Tyr Val Leu Val Arg
465 470 475 480

Leu Val Gln Glu Phe Ser His Ile Arg Leu Asp Pro Asp Glu Val Tyr
485 490 495

Pro Pro Lys Arg Leu Thr Asn Leu Thr Met Cys Leu Gln Asp Gly Ala
500 505 510

Ile Val Lys Phe Asp
515

<210> 102
<211> 512
<212> PRT
<213> Candida tropicalis

<400> 102
Met Leu Asp Gln Ile Leu His Tyr Trp Tyr Ile Val Leu Pro Leu Leu
1 5 10 15

Ala Ile Ile Asn Gln Ile Val Ala His Val Arg Thr Asn Tyr Leu Met
20 25 30

Lys Lys Leu Gly Ala Lys Pro Phe Thr His Val Gln Arg Asp Gly Trp
35 40 45

Leu Gly Phe Lys Phe Gly Arg Glu Phe Leu Lys Ala Lys Ser Ala Gly
50 55 60

Arg Leu Val Asp Leu Ile Ile Ser Arg Phe His Asp Asn Glu Asp Thr
65 70 75 80

Phe Ser Ser Tyr Ala Phe Gly Asn His Val Val Phe Thr Arg Asp Pro
85 90 95

Glu Asn Ile Lys Ala Leu Leu Ala Thr Gln Phe Gly Asp Phe Ser Leu
100 105 110

```
Gly Ser Arg Val Lys Phe Phe Lys Pro Leu Leu Gly Tyr Gly Ile Phe
        115                 120                 125

Thr Leu Asp Ala Glu Gly Trp Lys His Ser Arg Ala Met Leu Arg Pro
    130                 135                 140

Gln Phe Ala Arg Glu Gln Val Ala His Val Thr Ser Leu Glu Pro His
145                 150                 155                 160

Phe Gln Leu Leu Lys Lys His Ile Leu Lys His Lys Gly Glu Tyr Phe
                165                 170                 175

Asp Ile Gln Glu Leu Phe Phe Arg Phe Thr Val Asp Ser Ala Thr Glu
            180                 185                 190

Phe Leu Phe Gly Glu Ser Val His Ser Leu Lys Asp Glu Glu Ile Gly
        195                 200                 205

Tyr Asp Thr Lys Asp Met Ser Glu Glu Arg Arg Arg Phe Ala Asp Ala
    210                 215                 220

Phe Asn Lys Ser Gln Val Tyr Val Ala Thr Arg Val Ala Leu Gln Asn
225                 230                 235                 240

Leu Tyr Trp Leu Val Asn Asn Lys Glu Phe Lys Glu Cys Asn Asp Ile
                245                 250                 255

Val His Lys Phe Thr Asn Tyr Tyr Val Gln Lys Ala Leu Asp Ala Thr
            260                 265                 270

Pro Glu Glu Leu Glu Lys Gln Gly Gly Tyr Val Phe Leu Tyr Glu Leu
        275                 280                 285

Val Lys Gln Thr Arg Asp Pro Lys Val Leu Arg Asp Gln Ser Leu Asn
    290                 295                 300

Ile Leu Leu Ala Gly Arg Asp Thr Thr Ala Gly Leu Leu Ser Phe Ala
305                 310                 315                 320

Val Phe Glu Leu Ala Arg Asn Pro His Ile Trp Ala Lys Leu Arg Glu
                325                 330                 335

Glu Ile Glu Gln Gln Phe Gly Leu Gly Glu Asp Ser Arg Val Glu Glu
            340                 345                 350

Ile Thr Phe Glu Ser Leu Lys Arg Cys Glu Tyr Leu Lys Ala Val Leu
        355                 360                 365
```

Asn Glu Thr Leu Arg Leu His Pro Ser Val Pro Arg Asn Ala Arg Phe
370 375 380

Ala Ile Lys Asp Thr Thr Leu Pro Arg Gly Gly Gly Pro Asn Gly Lys
385 390 395 400

Asp Pro Ile Leu Ile Arg Lys Asp Glu Val Val Gln Tyr Ser Ile Ser
405 410 415

Ala Thr Gln Thr Asn Pro Ala Tyr Tyr Gly Ala Asp Ala Ala Asp Phe
420 425 430

Arg Pro Glu Arg Trp Phe Glu Pro Ser Thr Arg Asn Leu Gly Trp Ala
435 440 445

Phe Leu Pro Phe Asn Gly Gly Pro Arg Ile Cys Leu Gly Gln Gln Phe
450 455 460

Ala Leu Thr Glu Ala Gly Tyr Val Leu Val Arg Leu Val Gln Glu Phe
465 470 475 480

Pro Asn Leu Ser Gln Asp Pro Glu Thr Lys Tyr Pro Pro Pro Arg Leu
485 490 495

Ala His Leu Thr Met Cys Leu Phe Asp Gly Ala His Val Lys Met Ser
500 505 510

<210> 103
<211> 512
<212> PRT
<213> Candida tropicalis

<400> 103
Met Leu Asp Gln Ile Phe His Tyr Trp Tyr Ile Val Leu Pro Leu Leu
1 5 10 15

Val Ile Ile Lys Gln Ile Val Ala His Ala Arg Thr Asn Tyr Leu Met
20 25 30

Lys Lys Leu Gly Ala Lys Pro Phe Thr His Val Gln Leu Asp Gly Trp
35 40 45

Phe Gly Phe Lys Phe Gly Arg Glu Phe Leu Lys Ala Lys Ser Ala Gly

50 55 60

Arg Gln Val Asp Leu Ile Ile Ser Arg Phe His Asp Asn Glu Asp Thr
65 70 75 80

Phe Ser Ser Tyr Ala Phe Gly Asn His Val Val Phe Thr Arg Asp Pro
85 90 95

Glu Asn Ile Lys Ala Leu Leu Ala Thr Gln Phe Gly Asp Phe Ser Leu
100 105 110

Gly Ser Arg Val Lys Phe Phe Lys Pro Leu Leu Gly Tyr Gly Ile Phe
115 120 125

Thr Leu Asp Gly Glu Gly Trp Lys His Ser Arg Ala Met Leu Arg Pro
130 135 140

Gln Phe Ala Arg Glu Gln Val Ala His Val Thr Ser Leu Glu Pro His
145 150 155 160

Phe Gln Leu Leu Lys Lys His Ile Leu Lys His Lys Gly Glu Tyr Phe
165 170 175

Asp Ile Gln Glu Leu Phe Phe Arg Phe Thr Val Asp Ser Ala Thr Glu
180 185 190

Phe Leu Phe Gly Glu Ser Val His Ser Leu Arg Asp Glu Glu Ile Gly
195 200 205

Tyr Asp Thr Lys Asp Met Ala Glu Glu Arg Arg Lys Phe Ala Asp Ala
210 215 220

Phe Asn Lys Ser Gln Val Tyr Leu Ser Thr Arg Val Ala Leu Gln Thr
225 230 235 240

Leu Tyr Trp Leu Val Asn Asn Lys Glu Phe Lys Glu Cys Asn Asp Ile
245 250 255

Val His Lys Phe Thr Asn Tyr Tyr Val Gln Lys Ala Leu Asp Ala Thr
260 265 270

Pro Glu Glu Leu Glu Lys Gln Gly Gly Tyr Val Phe Leu Tyr Glu Leu
275 280 285

Ala Lys Gln Thr Lys Asp Pro Asn Val Leu Arg Asp Gln Ser Leu Asn
290 295 300

Ile Leu Leu Ala Gly Arg Asp Thr Thr Ala Gly Leu Leu Ser Phe Ala

305 310 315 320

Val Phe Glu Leu Ala Arg Asn Pro His Ile Trp Ala Lys Leu Arg Glu
325 330 335

Glu Ile Glu Ser His Phe Gly Leu Gly Glu Asp Ser Arg Val Glu Glu
340 345 350

Ile Thr Phe Glu Ser Leu Lys Arg Cys Glu Tyr Leu Lys Ala Val Leu
355 360 365

Asn Glu Thr Leu Arg Leu His Pro Ser Val Pro Arg Asn Ala Arg Phe
370 375 380

Ala Ile Lys Asp Thr Thr Leu Pro Arg Gly Gly Gly Pro Asn Gly Lys
385 390 395 400

Asp Pro Ile Leu Ile Arg Lys Asn Glu Val Val Gln Tyr Ser Ile Ser
405 410 415

Ala Thr Gln Thr Asn Pro Ala Tyr Tyr Gly Ala Asp Ala Ala Asp Phe
420 425 430

Arg Pro Glu Arg Trp Phe Glu Pro Ser Thr Arg Asn Leu Gly Trp Ala
435 440 445

Tyr Leu Pro Phe Asn Gly Gly Pro Arg Ile Cys Leu Gly Gln Gln Phe
450 455 460

Ala Leu Thr Glu Ala Gly Tyr Val Leu Val Arg Leu Val Gln Glu Phe
465 470 475 480

Pro Ser Leu Ser Gln Asp Pro Glu Thr Glu Tyr Pro Pro Pro Arg Leu
485 490 495

Ala His Leu Thr Met Cys Leu Phe Asp Gly Ala Tyr Val Lys Met Gln
500 505 510

<210> 104
<211> 499
<212> PRT
<213> Candida tropicalis

<400> 104

Met Ala Ile Ser Ser Leu Leu Ser Trp Asp Val Ile Cys Val Val Phe
1               5                   10                  15

Ile Cys Val Cys Val Tyr Phe Gly Tyr Glu Tyr Cys Tyr Thr Lys Tyr
            20                  25                  30

Leu Met His Lys His Gly Ala Arg Glu Ile Glu Asn Val Ile Asn Asp
        35                  40                  45

Gly Phe Phe Gly Phe Arg Leu Pro Leu Leu Leu Met Arg Ala Ser Asn
    50                  55                  60

Glu Gly Arg Leu Ile Glu Phe Ser Val Lys Arg Phe Glu Ser Ala Pro
65                  70                  75                  80

His Pro Gln Asn Lys Thr Leu Val Asn Arg Ala Leu Ser Val Pro Val
                85                  90                  95

Ile Leu Thr Lys Asp Pro Val Asn Ile Lys Ala Met Leu Ser Thr Gln
            100                 105                 110

Phe Asp Asp Phe Ser Leu Gly Leu Arg Leu His Gln Phe Ala Pro Leu
        115                 120                 125

Leu Gly Lys Gly Ile Phe Thr Leu Asp Gly Pro Glu Trp Lys Gln Ser
    130                 135                 140

Arg Ser Met Leu Arg Pro Gln Phe Ala Lys Asp Arg Val Ser His Ile
145                 150                 155                 160

Leu Asp Leu Glu Pro His Phe Val Leu Leu Arg Lys His Ile Asp Gly
                165                 170                 175

His Asn Gly Asp Tyr Phe Asp Ile Gln Glu Leu Tyr Phe Arg Phe Ser
            180                 185                 190

Met Asp Val Ala Thr Gly Phe Leu Phe Gly Glu Ser Val Gly Ser Leu
        195                 200                 205

Lys Asp Glu Asp Ala Arg Phe Leu Glu Ala Phe Asn Glu Ser Gln Lys
    210                 215                 220

Tyr Leu Ala Thr Arg Ala Thr Leu His Glu Leu Tyr Phe Leu Cys Asp
225                 230                 235                 240

Gly Phe Arg Phe Arg Gln Tyr Asn Lys Val Val Arg Lys Phe Cys Ser
                245                 250                 255

```
Gln Cys Val His Lys Ala Leu Asp Val Ala Pro Glu Asp Thr Ser Glu
            260                 265                 270

Tyr Val Phe Leu Arg Glu Leu Val Lys His Thr Arg Asp Pro Val Val
        275                 280                 285

Leu Gln Asp Gln Ala Leu Asn Val Leu Leu Ala Gly Arg Asp Thr Thr
    290                 295                 300

Ala Ser Leu Leu Ser Phe Ala Thr Phe Glu Leu Ala Arg Asn Asp His
305                 310                 315                 320

Met Trp Arg Lys Leu Arg Glu Glu Val Ile Leu Thr Met Gly Pro Ser
                325                 330                 335

Ser Asp Glu Ile Thr Val Ala Gly Leu Lys Ser Cys Arg Tyr Leu Lys
            340                 345                 350

Ala Ile Leu Asn Glu Thr Leu Arg Leu Tyr Pro Ser Val Pro Arg Asn
        355                 360                 365

Ala Arg Phe Ala Thr Arg Asn Thr Thr Leu Pro Arg Gly Gly Gly Pro
    370                 375                 380

Asp Gly Ser Phe Pro Ile Leu Ile Arg Lys Gly Gln Pro Val Gly Tyr
385                 390                 395                 400

Phe Ile Cys Ala Thr His Leu Asn Glu Lys Val Tyr Gly Asn Asp Ser
                405                 410                 415

His Val Phe Arg Pro Glu Arg Trp Ala Ala Leu Glu Gly Lys Ser Leu
            420                 425                 430

Gly Trp Ser Tyr Leu Pro Phe Asn Gly Gly Pro Arg Ser Cys Leu Gly
        435                 440                 445

Gln Gln Phe Ala Ile Leu Glu Ala Ser Tyr Val Leu Ala Arg Leu Thr
    450                 455                 460

Gln Cys Tyr Thr Thr Ile Gln Leu Arg Thr Thr Glu Tyr Pro Pro Lys
465                 470                 475                 480

Lys Leu Val His Leu Thr Met Ser Leu Leu Asn Gly Val Tyr Ile Arg
                485                 490                 495

Thr Arg Thr
```

<210> 105
<211> 1712
<212> DNA
<213> Candida tropicalis

<400> 105
ggtaccgagc tcacgagttt tgggattttc gagtttggat tgtttccttt gttgattgaa 60
ttgacgaaac cagaggtttt caagacagat aagattgggt ttatcaaaac gcagtttgaa 120
atattccagt tggtttccaa gatatcttga agaagattga cgatttgaaa tttgaagaag 180
tggagaagat ctggtttgga ttgttggaga atttcaagaa tctcaagatt tactctaacg 240
acgggtacaa cgagaattgt attgaattga tcaagaacat gatcttggtg ttacagaaca 300
tcaagttctt ggaccagact gagaatgcca cagatataca aggcgtcatg tgataaaatg 360
gatgagattt atcccacaat tgaagaaaga gtttatggaa agtggtcaac cagaagctaa 420
acaggaagaa gcaaacgaag aggtgaaaca agaagaagaa ggtaaataag tattttgtat 480
tatataacaa acaaagtaag gaatacagat ttatacaata aattgccata ctagtcacgt 540
gagatatctc atccattccc caactcccaa gaaaaaaaaa aagtgaaaaa aaaaatcaaa 600
cccaaagatc aacctcccca tcatcatcgt catcaaaccc ccagctcaat tcgcaatggt 660
tagcacaaaa acatacacag aaagggcatc agcacacccc tccaaggttg cccaacgttt 720
attccgctta atggagtcca aaaagaccaa cctctgcgcc tcgatcgacg tgaccacaac 780
cgccgagttc ctttcgctca tcgacaagct cggtccccac atctgtctcg tgaagacgca 840
catcgatatc atctcagact tcagctacga gggcacgatt gagccgttgc ttgtgcttgc 900
agagcgccac gggttcttga tattcgagga caggaagttt gctgatatcg gaaacaccgt 960
gatgttgcag tacacctcgg gggtataccg gatcgcggcg tggagtgaca tcacgaacgc 1020
gcacggagtg actgggaagg gcgtcgttga agggttgaaa cgcggtgcgg aggggggtaga 1080
aaaggaaagg ggcgtgttga tgttggcgga gttgtcgagt aaaggctcgt tggcgcatgg 1140
tgaatatacc cgtgagacga tcgagattgc gaagagtgat cgggagttcg tgattgggtt 1200
catcgcgcag cgggacatgg ggggtagaga agaagggttt gattggatca tcatgacgcc 1260
tggtgtgggg ttggatgata aaggcgatgc gttgggccag cagtatagga ctgttgatga 1320
ggtggttctg actggtaccg atgtgattat tgtcgggaga gggttgtttg gaaaaggaag 1380
agaccctgag gtggagggaa agagatacag ggatgctgga tggaaggcat acttgaagag 1440
aactggtcag ttagaataaa tattgtaata aataggtcta tatacataca ctaagcttct 1500
aggacgtcat tgtagtcttc gaagttgtct gctagtttag ttctcatgat ttcgaaaacc 1560
aataacgcaa tggatgtagc agggatggtg gttagtgcgt tcctgacaaa cccagagtac 1620
gccgcctcaa accacgtcac attcgccctt tgcttcatcc gcatcacttg cttgaaggta 1680
tccacgtacg agttgtaata caccttgaag aa 1712

<210> 106
<211> 267
<212> PRT
<213> Candida tropicalis

<400> 106
Met Val Ser Thr Lys Thr Tyr Thr Glu Arg Ala Ser Ala His Pro Ser
1 5 10 15

Lys Val Ala Gln Arg Leu Phe Arg Leu Met Glu Ser Lys Lys Thr Asn

20 25 30

Leu Cys Ala Ser Ile Asp Val Thr Thr Thr Ala Glu Phe Leu Ser Leu
35 40 45

Ile Asp Lys Leu Gly Pro His Ile Cys Leu Val Lys Thr His Ile Asp
50 55 60

Ile Ile Ser Asp Phe Ser Tyr Glu Gly Thr Ile Glu Pro Leu Leu Val
65 70 75 80

Leu Ala Glu Arg His Gly Phe Leu Ile Phe Glu Asp Arg Lys Phe Ala
85 90 95

Asp Ile Gly Asn Thr Val Met Leu Gln Tyr Thr Ser Gly Val Tyr Arg
100 105 110

Ile Ala Ala Trp Ser Asp Ile Thr Asn Ala His Gly Val Thr Gly Lys
115 120 125

Gly Val Val Glu Gly Leu Lys Arg Gly Ala Glu Gly Val Glu Lys Glu
130 135 140

Arg Gly Val Leu Met Leu Ala Glu Leu Ser Ser Lys Gly Ser Leu Ala
145 150 155 160

His Gly Glu Tyr Thr Arg Glu Thr Ile Glu Ile Ala Lys Ser Asp Arg
165 170 175

Glu Phe Val Ile Gly Phe Ile Ala Gln Arg Asp Met Gly Gly Arg Glu
180 185 190

Glu Gly Phe Asp Trp Ile Ile Met Thr Pro Gly Val Gly Leu Asp Asp
195 200 205

Lys Gly Asp Ala Leu Gly Gln Gln Tyr Arg Thr Val Asp Glu Val Val
210 215 220

Leu Thr Gly Thr Asp Val Ile Ile Val Gly Arg Gly Leu Phe Gly Lys
225 230 235 240

Gly Arg Asp Pro Glu Val Glu Gly Lys Arg Tyr Arg Asp Ala Gly Trp
245 250 255

Lys Ala Tyr Leu Lys Arg Thr Gly Gln Leu Glu
260 265

```
<210> 107
<211> 473
<212> DNA
<213> Artificial Sequence

<220>
<223> Description of Artificial Sequence: Probe for
      CYP52A1 gene

<400> 107
gtcaaagcaa attgttggcc caagcagact cttggaccac cgttgaatgg aacataagcc 60
cagcccaact tcttagtaga tggttcaaac catctttctg gtctgaagtc gttagcgtcc 120
ttaccgtagt attcttccaa acggtgggtc ttgtagacaa cgtaagcaac agtggagcct 180
ttaggaatgt agattgggtc ggtaccgtta gcaccaccac ctcttggcaa agtggtgtct 240
ctggtggcgg ttctaaagtt gacaggaaca gatgggtaca tacgcaaggt ttcgttaagg 300
atagccttca agtattcaca tctcttcaag gcttcgaaag taatttcttc aacgcgggag 360
tcttcaccaa caccaaagtt aacttcgatt tcttctctca acttggacca catctctggg 420
tgtctagcca attcaaacaa agcaaaggac aacaaacccg cggtggtgtc tct       473
```